(19) **Europäisches Patentamt / European Patent Office / Office européen des brevets**

(11) **EP 3 883 584 B1**

(12) # EUROPEAN PATENT SPECIFICATION

(45) Date of publication and mention
of the grant of the patent:
**18.02.2026   Bulletin 2026/08**

(21) Application number: **19886343.3**

(22) Date of filing: **17.11.2019**

(51) International Patent Classification (IPC):
*A61K 35/17* $^{(2025.01)}$      *A61K 9/50* $^{(2006.01)}$
*A61P 31/00* $^{(2006.01)}$      *A61K 40/42* $^{(2025.01)}$
*A61K 40/31* $^{(2025.01)}$      *A61K 40/11* $^{(2025.01)}$
*A61K 47/00* $^{(2006.01)}$

(52) Cooperative Patent Classification (CPC):
**A61K 9/5068; A61K 9/0019; A61K 35/17;
A61K 40/11; A61K 40/31; A61K 40/4205;
A61K 40/4211; A61K 40/4217; A61P 31/00;**
A61K 2239/31; A61K 2239/38; A61K 2239/48

(86) International application number:
**PCT/IL2019/051250**

(87) International publication number:
**WO 2020/105034 (28.05.2020 Gazette 2020/22)**

(54) **EARLY APOPTOTIC CELLS FOR USE TREATING ORGAN FAILURE IN SEPSIS**

FRÜHE APOPTOTISCHE ZELLEN ZUR BEHANDLUNG VON ORGANVERSAGEN IN SEPSIS

CELLULES APOPTOTIQUES PRÉCOCES DESTINÉES À ÊTRE UTILISÉES DANS LE TRAITEMENT DE DÉFAILLANCE D'ORGANES EN CAS DE SEPTICÉMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2018   US 201816194417
07.10.2019   US 201916594463
04.11.2019   US 201916672547**

(43) Date of publication of application:
**29.09.2021   Bulletin 2021/39**

(73) Proprietor: **Enlivex Therapeutics R&D Ltd
7403618 Nes-Ziona (IL)**

(72) Inventors:
• **NOVIK, Shai
4721249 Ramat Hasharon (IL)**
• **MEVORACH, Dror
9574630 Jerusalem (IL)**

(74) Representative: **Pearl Cohen Zedek Latzer Baratz UK LLP
The Gridiron Building
One Pancras Square
London N1C 4AG (GB)**

(56) References cited:
**EP-A1- 3 285 877        WO-A1-2014/087408
WO-A1-2016/170541      US-A1- 2017 360 836
US-A1- 2018 094 244     US-A1- 2019 083 535**

• **MEVORACH ET AL: "Apoptotic Cells for the Prevention of Cytokine Release Syndrome (CRS) in CAR T-Cell Therapy", BLOOD JOURNAL, vol. 128, no. 22, 1 December 2016 (2016-12-01), pages 1626, XP055409892**
• **BURKOVSKIY IAN ET AL: "Cytokine release in sepsis", ADVANCES IN BIOSCIENCE AND BIOTECHNOLOGY, vol. 04, no. 09, 1 January 2013 (2013-01-01), pages 860 - 865, XP055960933, ISSN: 2156-8456, DOI: 10.4236/ abb.2013.49114**

- BRINK A J ET AL: "Pharmacokinetics of once-daily dosing of ertapenem in critically ill patients with severe sepsis", INTERNATIONAL JOURNAL OF ANTIMICROBIAL AGENTS, ELSEVIER, AMSTERDAM, NL, vol. 33, no. 5, 1 May 2009 (2009-05-01), pages 432 - 436, XP026073919, ISSN: 0924-8579, [retrieved on 20081216], DOI: 10.1016/J.IJANTIMICAG.2008.10.005

- MEVORACH D.: "Effect of Allocetra-OTS (off-the-shelf apoptotic cells) Therapy in Sepsis", CYTOTHERAPY, vol. 22, no. 5, 1 May 2020 (2020-05-01), GB, pages S19, XP055960925, ISSN: 1465-3249, DOI: 10.1016/j.jcyt.2020.03.488

**Description**

**FIELD OF INTEREST**

Disclosed herein are compositions for use in preventing, inhibiting or reducing the incidence of organ failure in a subject having sepsis.

**BACKGROUND**

While standard treatments for cancer are surgery, chemotherapy, and radiation therapy, improved methods, such as targeted immunological therapies, are currently being developed and tested. One promising technique uses adoptive cell transfer (ACT), in which immune cells are modified to recognize and attack their tumors. One example of ACT is when a patient's own cytotoxic T-cells, or a donor's, are engineered to express a chimeric antigen receptor (CAR T-cells) targeted to a tumor specific antigen expressed on the surface of the tumor cells. These CAR T-cells are then cytotoxic only to cells expressing the tumor specific antigen. Clinical trials have shown that CAR T-cell therapy has great potential in controlling advanced acute lymphoblastic leukemia (ALL) and lymphoma, among others.

However, some patients given CAR T-cell therapy and other immune therapies experience a dangerous and sometimes life-threatening side effect called cytokine release syndrome (CRS), in which the infused, activated T-cells produce a systemic inflammatory response in which there is a rapid and massive release of cytokines into the bloodstream, leading to dangerously low blood pressure, high fever and shivering.

In severe cases of CRS, patients experience a cytokine storm (a.k.a. cytokine cascade or hypercytokinemia), in which there is a positive feedback loop between cytokines and white blood cells with highly elevated levels of cytokines. This can lead to potentially life-threatening complications including cardiac dysfunction, adult respiratory distress syndrome, neurologic toxicity, renal and/or hepatic failure, pulmonary edema and disseminated intravascular coagulation.

For example, six patients in a recent phase I trial who were administered the monoclonal antibody TGN1412, which binds to the CD28 receptor on T-cells, exhibited severe cases of cytokine storm and multi-organ failure. This happened despite the fact that the TGN1412 dose was 500-times lower than that found to be safe in animals (St. Clair EW: The calm after the cytokine storm: Lessons from the TGN1412 trial. J Clin Invest 118: 1344-1347, 2008).

To date, corticosteroids, biological therapies such as anti-IL6 therapies and anti-inflammatory drugs are being evaluated to control cytokine release syndrome in patients administered CAR T-cell therapy. However, steroids may affect CAR T-cells' activity and/or proliferation and put the patients in danger of sepsis and opportunistic infections. Anti-inflammatory drugs may not be effective in controlling cytokine release syndromes or cytokine storms, because the cytokine storm includes a very large number of cytokines while there is limited ability to infuse patients with anti-inflammatory drugs. Novel strategies are needed to control cytokine release syndromes, and especially cytokine storms, in order to realize the potential of CAR T-cell therapy.

Cytokine storms are also a problem after other infectious and non-infectious stimuli. In a cytokine storm, numerous proinflammatory cytokines, such as interleukin-1 (IL-1), IL-6, g-interferon (g-IFN), and tumor necrosis factor-a. (TNFα), are released, resulting in hypotension, hemorrhage, and, ultimately, multiorgan failure. The relatively high death rate in young people, with presumably healthy immune systems, in the 1918 H1N1 influenza pandemic and the more recent bird flu H5N1 infection are attributed to cytokine storms. This syndrome has been also known to occur in advanced or terminal cases of severe acute respiratory syndrome (SARS), Epstein-Barr virus-associated hemophagocytic lymphohistiocytosis, sepsis, gram-negative sepsis, malaria and numerous other infectious diseases, including Ebola infection.

Cytokine storm may also stem from non-infectious causes, such as acute pancreatitis, severe burns or trauma, or acute respiratory distress syndrome. Novel strategies are therefore needed to control cytokine release syndrome, and especially cytokine storms.

Cancer is an abnormal state in which uncontrolled proliferation of one or more cell populations interferes with normal biological functioning. The proliferative changes are usually accompanied by other changes in cellular properties, including reversion to a less differentiated, more developmentally primitive state. The *in vitro* correlate of cancer is called cellular transformation. Transformed cells generally display several or all of the following properties: spherical morphology, expression of fetal antigens, growth-factor independence, lack of contact inhibition, anchorage-independence, and growth to high density.

The primary cause of lethality of malignant diseases such as lung and skin cancer arise from metastatic spread. In many cases, it is not possible to prevent the onset of metastatic disease since cancers are often metastatic by the time of diagnosis, and even in cases where cancers are diagnosed prior to this stage, complete surgical removal or destruction of primary lesion tissues which are capable of eventually generating metastases may not be feasible. Metastatic disease may be impossible to diagnose at early stages due to the small size of metastatic lesions, and/or the absence of reliable markers in primary lesions upon which to reliably predict their existence. Such lesions may be difficult or impossible to treat via ablative methods due to their being inaccessible, disseminated, and/or poorly localized. Chemotherapy/radiotherapy, the current methods of choice for treatment of certain metastatic malignancies are often ineffective or suboptimal, and have the significant disadvantage of being associated with particularly harmful and/or potentially lethal side-effects.

Immunotherapeutic cancer treatment methods, such as those involving antigen presenting cell (APC) vaccinations, have the potential to be optimally effective for treatment of inaccessible, disseminated, microscopic, recurrent and/or poorly localized cancer lesions. One promising immunotherapy avenue involves the use of professional APCs, such as dendritic cells (DCs), to elicit systemic anti-cancer immunity.

Dendritic cells are antigen-producing and presenting cells of the mammalian immune system that process antigen material and present it on the cell surface to the T-cells of the immune system and are thereby capable of sensitizing T-cells to both new and recall antigens. DCs are the most potent antigen-producing cells, acting as messengers between the innate and the adaptive immune systems. DC cells may be used, to prime specific antitumor immunity through the generation of effector cells that attack and lyse tumors.

Sepsis is the body's overwhelming and life-threatening response to infection that can lead to tissue damage, organ failure and death. In other words, it's a body's overactive and toxic response to an infection.

The immune system usually works to fight any germs (bacteria, viruses, fungi or parasites) to prevent infection. If an infection does occur, the immune system will try to fight it, although it may need help from medication such as antibiotics, antivirals, antifungals and antiparasitics. However, for reasons researchers do not understand, the immune system sometimes stops fighting the "invaders," and begins to turn on itself. This is the start of sepsis.

People who are at higher risk of developing sepsis are generally people who are at higher risk of contracting an infection. These could include the very young, the very old, those with chronic illnesses and those with a weakened or impaired immune system. Patients are diagnosed with sepsis when they develop a set of signs and symptoms related to sepsis. Sepsis is not diagnosed based on an infection itself. If a person has more than one of the symptoms of sepsis, especially if there are signs of an infection or if someone falls into one of the higher risk groups, the physician will likely suspect sepsis.

Sepsis, which has been identified by the World Health Organization (WHO) as a global health priority, has no proven pharmacologic treatment other than appropriate antibiotic agents, fluids, vasopressors as needed, and possibly corticosteroids (Venkatesh, B., Finfer, S., Cohen, J., Rajbhandari, D., Arabi, Y., Bellomo, R., Billot, L., Correa, M., Glass, P., Harward, M., et al. (2018). Adjunctive Glucocorticoid Therapy in Patients with Septic Shock. N. Engl. J. Med. 378, 797-808). Reported death rates among hospitalized patients range between 30% and 45% ( Finfer, S., Bellomo, R., Lipman, J., French, C., Dobb, G., and Myburgh, J. (2004). Adult-population incidence of severe sepsis in Australian and New Zealand intensive care units. Intensive Care Med. 30, 589-596; Fleischmann, C., Scherag, A., Adhikari, N.K.J., Hartog, C.S., Tsaganos, T., Schlattmann, P., Angus, D.C., Reinhart, K., and International Forum of Acute Care Trialists (2016). Assessment of Global Incidence and Mortality of Hospital-treated Sepsis. Current Estimates and Limitations. Am. J. Respir. Crit. Care Med. 193, 259-272; Liu, V., Escobar, G.J., Greene, J.D., Soule, J., Whippy, A., Angus, D.C., and Iwashyna, T.J. (2014). Hospital Deaths in Patients With Sepsis From 2 Independent Cohorts. JAMA 312, 90; Machado, F.R., Cavalcanti, A.B., Bozza, F.A., Ferreira, E.M., Angotti Carrara, F.S., Sousa, J.L., Caixeta, N., Salomao, R., Angus, D.C., Pontes Azevedo, L.C., et al. (2017). The epidemiology of sepsis in Brazilian intensive care units (the Sepsis PREvalence Assessment Database, SPREAD): an observational study. Lancet Infect. Dis. 17, 1180-1189; Reinhart, K., Daniels, R., Kissoon, N., Machado, F.R., Schachter, R.D., and Finfer, S. (2017). Recognizing Sepsis as a Global Health Priority - A WHO Resolution. N. Engl. J. Med. 377, 414-417; Rhee, C., Dantes, R., Epstein, L., Murphy, D.J., Seymour, C.W., Iwashyna, T.J., Kadri, S.S., Angus, D.C., Danner, R.L., Fiore, A.E., et al. (2017). Incidence and Trends of Sepsis in US Hospitals Using Clinical vs Claims Data, 2009-2014. JAMA 318, 1241).

Sepsis is generally initiated by simultaneous recognition of either pathogen-associated molecular patterns (PAMPs) or damage-associated molecular patterns (DAMPs) by components of the innate immune system, including complement proteins, Toll-like receptors, NOD-like receptors, RIG-like receptors, mannose-binding lectin, and scavenger receptors ( HOTShkiss, R.S., Moldawer, L.L., Opal, S.M., Reinhart, K., Turnbull, I.R., and Vincent, J.-L. (2016). Sepsis and septic shock. Nat. Rev. Dis. Prim. 2, 16045). Recognition induces a complex intracellular signaling system with redundant and complementary activities, and activation of these multiple signaling pathways ultimately leads to the expression of several common classes of genes that are involved in inflammation, adaptive immunity, and cellular metabolism ( Tang, D., Kang, R., Coyne, C.B., Zeh, H.J., and Lotze, M.T. (2012). PAMPs and DAMPs: signal 0s that spur autophagy and immunity. Immunol. Rev. 249, 158-175).

Sepsis elicits dysregulated immune responses manifested by a cytokine/chemokine elevation (also known as 'cytokine storm') that correlates well with disease severity and poor prognosis (Chaudhry, H., Zhou, J., Zhong, Y., Ali, M.M., McGuire, F., Nagarkatti, P.S., and Nagarkatti, M. (2015). Role of cytokines as a double-edged sword in sepsis. In Vivo 27, 669-684; Matsumoto, H., Ogura, H., Shimizu, K., Ikeda, M., Hirose, T., Matsuura, H., Kang, S., Takahashi, K., Tanaka, T., and Shimazu, T. (2018). The clinical importance of a cytokine network in the acute phase of sepsis. Sci. Rep. 8, 1-4). This exaggerated immune response deleteriously affects physiological homeostasis of vital organs, including the kidney, liver, lungs, and heart, and often evolves into multi-organ failure, also termed Multiple Organ Dysfunction Syndrome (MODS) ( Marshall, J.C., Cook, D.J., Christou, N. V, Bernard, G.R., Sprung, C.L., and Sibbald, W.J. (1995). Multiple organ dysfunction score: a reliable descriptor of a complex clinical outcome. Crit. Care Med. 23, 1638-16525; Vincent, J.-L. (2006). Organ Dysfunction in Patients with Severe Sepsis. Surg. Infect. (Larchmt). 7, s-69-s-72).

Sepsis progresses to severe sepsis when, in addition to signs of sepsis, the patient experiences indications of organ dysfunction, such as difficulty breathing (lungs), low or no urine output (kidneys), abnormal liver tests (liver), and changes in mental status (brain). Nearly all patients with severe sepsis require treatment in an intensive care unit (ICU). Septic shock is the most severe level and is diagnosed when a patient's blood pressure drops to dangerous levels.

The current treatment for sepsis includes: the administration of antibiotics and, when indicated, surgical or interventional radiological approaches for eliminating or at least controlling the source of infection; the administration of intravenous fluids (crystalloid solutions such as 0.9% sodium chloride solution, or colloid solutions such as 5% albumin solution) to restore and maintain adequate intravascular volume; the infusion of titratable vasoconstricting and/or inotropic drugs, such as vasopressin or noradrenaline, as needed, to change the strength of a heart's contractions; and, when indicated, mechanical ventilation, various forms of renal replacement therapy and, in rare cases, venovenous or venoarterial extracorporeal membrane oxygenation.

Due to the high rates of mortality and morbidity from sepsis, and the associated economic burden, the need for a novel pharmacological therapy is obvious. Unfortunately, a recent study examining the role of glucocorticoids in patients with septic shock who were undergoing mechanical ventilation, found that administration of a continuous infusion of hydrocortisone did not result in lower 90-day mortality compared to placebo.

It appears that previous attempts to find a therapy for sepsis failed due to the parallel course of biological activities that occur within a sepsis patient. While the medical team is administering the best standard of care, mainly antibiotics, a Cytokine Release Syndrome ramps up at the same time. A Cytokine Release Syndrome is difficult to treat with traditional small molecules or biotech drugs as the condition involves dozens of cytokines that induce multiple biological paths of hyper immune activity. Such hyper immune activity may result in an attack of immune killer cells (e.g., T-Cells, B-Cells, Natural Killer Cells) on healthy organs of the patient, such as heart, brain, lungs, liver, kidney and others. This outcome of this attack may lead to organ damage, multiple organ failure and mortality. If the Cytokine Release Syndrome could be prevented, the medical team would have ample time to eradicate the core source of the sepsis (i.e., an antibiotic-resistant bacteria), and most likely significantly increase the patient's chance of survival and survival statistics.

Apoptotic cells present one pathway of physiological cell death, most commonly occurring via apoptosis, which elicits a series of molecular homeostatic mechanisms comprising recognition, an immune response and a removal process. Moreover, apoptotic cells are immunomodulatory cells capable of directly and indirectly inducing immune tolerance to dendritic cells and macrophages. Apoptotic cells have been shown to modulate dendritic cells and macrophages and to render them tolerogenic and inhibit proinflammatory activies such as secretion of proinflammatory cytokiens and expression of costimulatory molecules.

As many as $3 \times 10^8$ cells undergo apoptosis every hour in the human body. One of the primary "eat me" signals expressed by apoptotic cells is phosphatidylserine (PtdSer) membrane exposure. Apoptotic cells themselves are major contributors to the "non-inflammatory" nature of the engulfment process, some by secreting thrombospondin-1 (TSP-1) or adenosine monophosphate and possibly other immune modulating "calm-down" signals that interact with macrophages and DCs. Apoptotic cells also produce "find me" and "tolerate me" signals to attract and immunomodulate macrophages and DCs that express specific receptors for some of these signals.

The pro-homeostatic nature of apoptotic cell interaction with the immune system is illustrated in known apoptotic cell signaling events in macrophages and DCs that are related to Toll-like receptors (TLRs), NF-κB, inflammasome, lipid-activated nuclear receptors, Tyro3, Axl, and Mertk receptors. In addition, induction of signal transducers, activation of transcription 1, and suppression of cytokine signaling lead to immune system silencing and DC tolerance Trahtemberg, U., and Mevorach, D. (2017). Apoptotic cells induced signaling for immune homeostasis in macrophages and dendritic cells. Front. Immunol. 8).

As summarized recently (Trahtemberg and Mevorach, 2017, *ibid),* apoptotic cells may have a beneficial effect on aberrant immune response, with downregulation of both anti- and pro-inflammatory cytokines derived from PAMPs and DAMPs, in both animal and in vitro models.

There remains an unmet need for compositions which are useful in the treatment of sepsis, including for the prevention of organ failure and mortality in patients with sepsis.

The compositions for use described herein address the need to increasing survival of a subject suffering from sepsis, and provide an unexpected solution for treating sepsis, including preventing organ failure.

## SUMMARY

The present invention provides a composition comprising an early apoptotic cell population for use in treating, preventing, inhibiting, or reducing the incidence of organ failure in a subject having sepsis, wherein said population comprises a mononuclear enriched early apoptotic cell population, optionally wherein said population comprises a mononuclear apoptotic cell population comprising a decreased percentage of non-quiescent non-apoptotic cells, a suppressed cellular activation of any living non-apoptotic cells, or a reduced proliferation of any living non-apoptotic cells, or any combination thereof.

In an embodiment, the sepsis comprises mild, severe, acute, or highly aggressive sepsis. In some embodiments, the source of sepsis comprises pneumonia, an endovascular methicillin-resistant Staphylococcus aureus (MRSA) infection, or a urinary tract infection (UTI).

In an embodiment, the composition increases the survival of said subject. In an embodiment, the incidence of organ failure or organ dysfunction, or organ damage, or a combination thereof, in a subject is reduced. In an embodiment, the organ failure comprises acute multiple organ failure.

In an embodiment, the early apoptotic cell population comprises a pooled population of early apoptotic cells or comprises an irradiated population of early apoptotic cells, wherein said irradiation was post induction of apoptosis; or comprises a pooled population of irradiated early apoptotic cells, wherein said irradiation was post induction of apoptosis.

In an embodiment, the subject in need is a human subject.

In an embodiment, the administering comprises a single infusion of said early apoptotic cell population. In an embodiment, the administering comprises multiple infusions of said apoptotic cell population. In an embodiment, the administering comprises intravenous administration.

In an embodiment, an additional therapy for treating, preventing, inhibiting, reducing the incidence of, ameliorating, or alleviating sepsis, or any combination thereof, is administered to the subject. In a further embodiment, the additional therapy is administered prior to, concurrent with, or following administration of said early apoptotic cells.

In an embodiment, the composition comprises a first-line therapy. In an embodiment, the composition comprises an adjuvant therapy.

In an embodiment, the composition reduces the secretion of one or more proinflammatory cytokine/chemokine.

In an embodiment, the composition reduces the secretion of one or more anti-inflammatory cytokine/chemokine.

In an embodiment, the composition reduces secretion of one or more pro-inflammatory cytokines/chemokines and one or more anti-inflammatory cytokines/chemokines.

In an embodiment, the composition prevents, inhibits, reduces the incidence of, or reduces the severity of a cytokine and chemokine storm in the subject.

## BRIEF DESCRIPTION OF THE DRAWINGS

The subject matter disclosed herein is particularly pointed out and distinctly claimed in the concluding portion of the specification. The compositions disclosed herein, together with objects, features, and advantages thereof, may best be understood by reference to the following detailed description when read with the accompanying drawings.

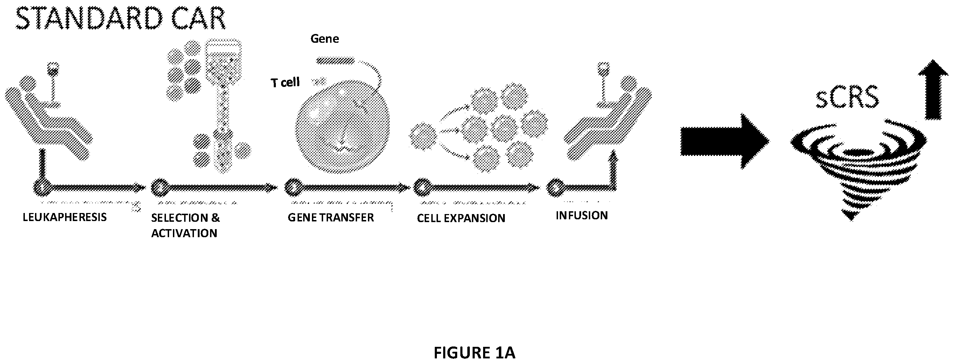

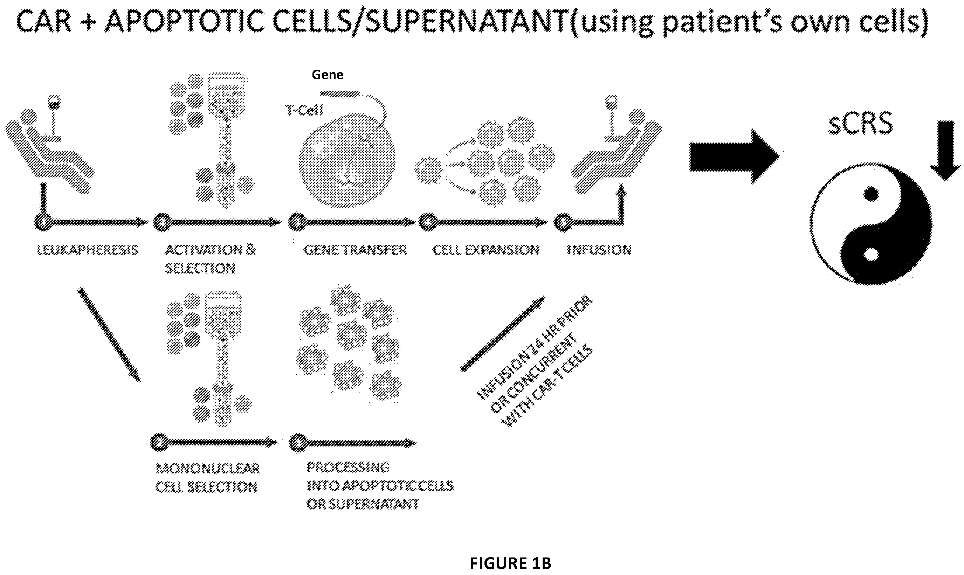

**Figures 1A-1B. Schematic showing standard CAR T-cell therapy (Figure 1A) and safe and efficacious CAR T-cell cancer therapy in a patient** using patients' own cells (autologous) **(Figure 1B)** to produce apoptotic cells or an apoptotic cell supernatant.

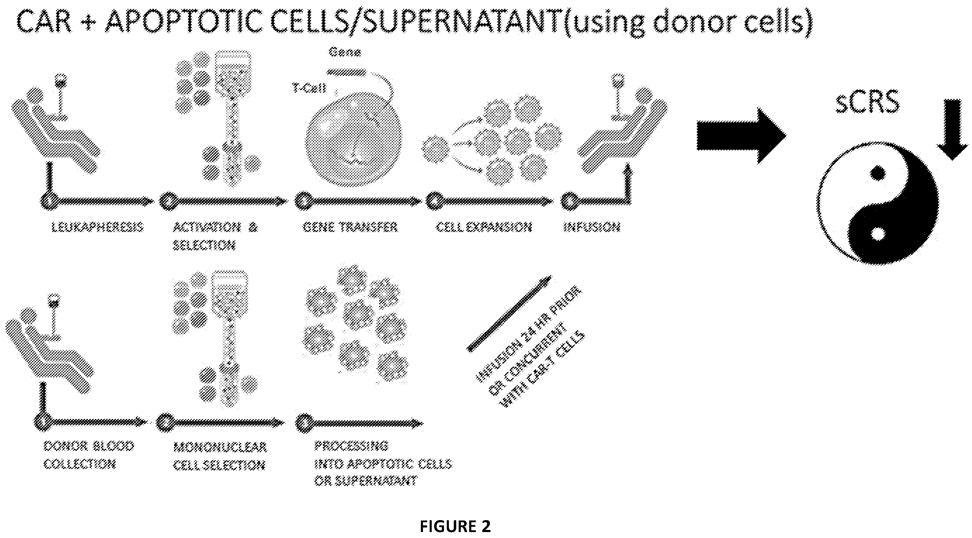

**Figure 2. Schematic showing safe and efficacious CAR T-cell cancer therapy in a patient,** using donor cells to produce apoptotic cells or an apoptotic supernatant.

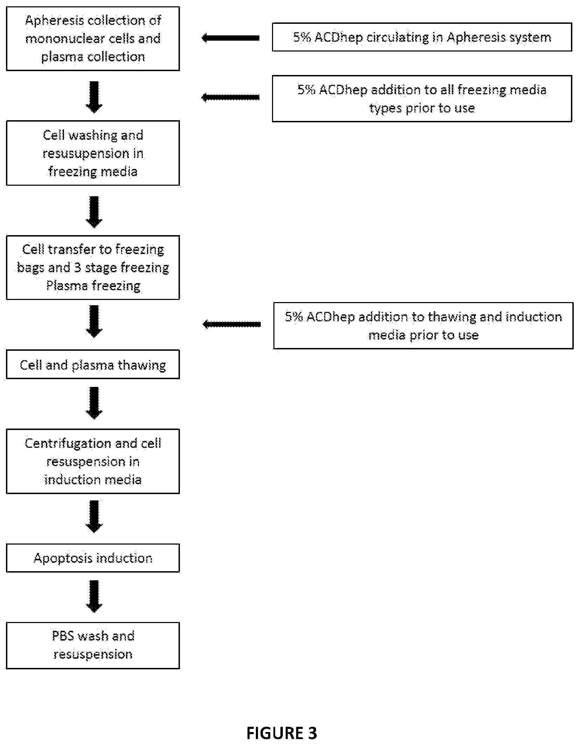

**Figure 3. Flow chart** presenting the steps during the manufacturing process of an early apoptotic cell population, wherein anti-coagulants were included in the process.

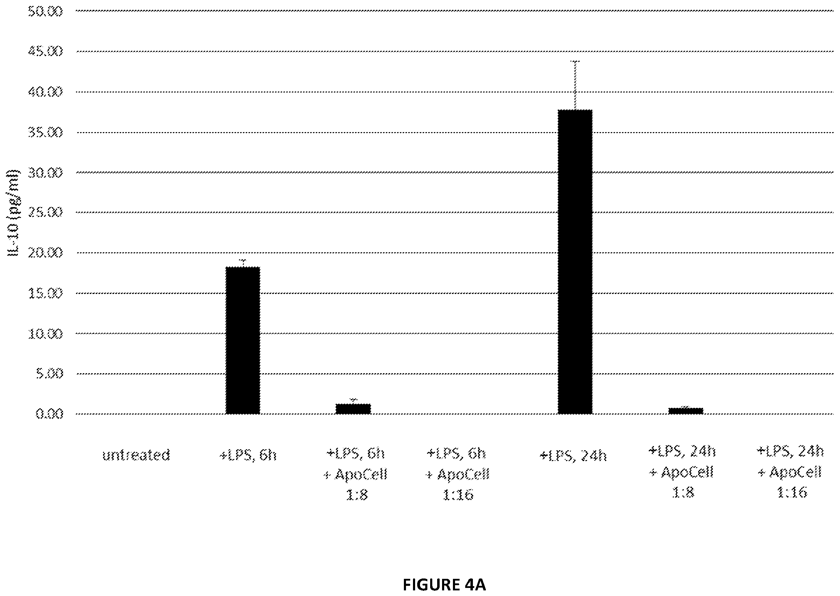

**Figures 4A - 4J. Apoptotic cells prevent cytokine storm in *in vitro* model of cytokine storm induced in LPS-Sterile model of macrophage activation syndrome in a cancer environment. Figure 4A** shows the reduction of LPS induced IL-10 levels in the macrophage activation syndrome model in the presence of cancer following administration of Apocells at a macrophage/monocyte:Apocell ratio of 1:8 and 1:16, at two time periods (6 hours and 24 hours).

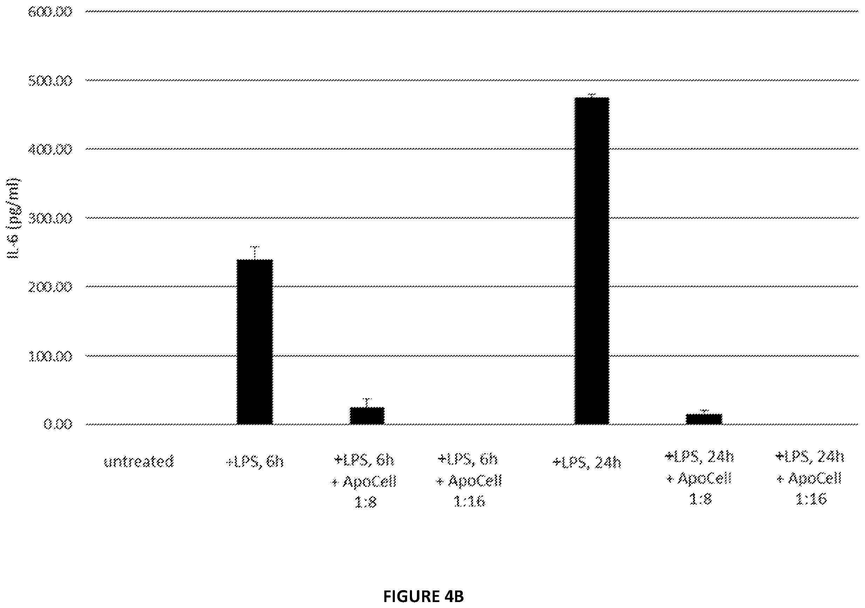

**Figure 4B** shows the reduction of LPS induced IL-6 levels in the macrophage activation syndrome model following administration of Apocells in the presence of cancer and CAR-19, at a macrophage/monocyte:Apocell ratio of 1:8 and 1:16, at two time periods (6 hours and 24 hours).

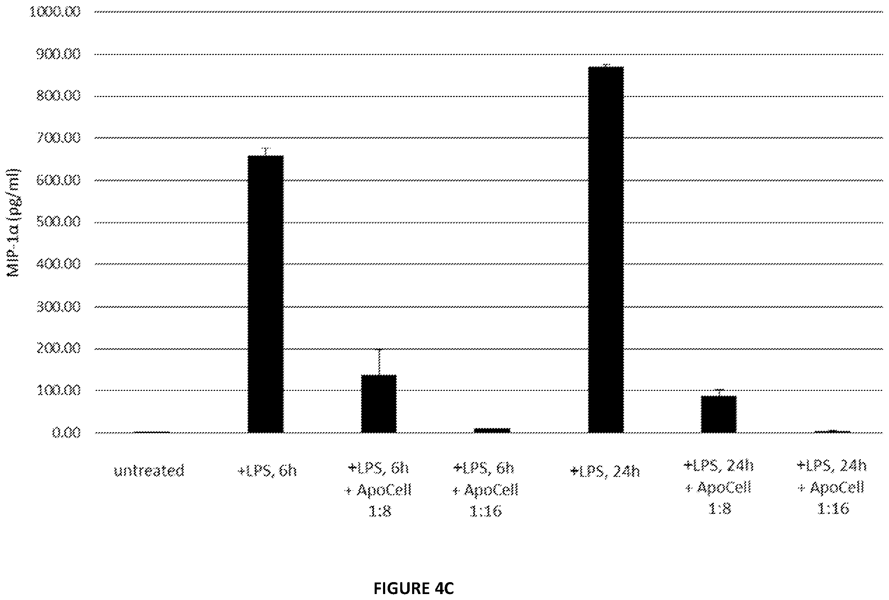

**Figure 4C** shows the reduction of LPS induced MIP-1$\alpha$ levels in the macrophage activation syndrome model in the presence of cancer and CAR-19, following administration of Apocells at a macrophage/monocyte:Apocell ratio of 1:8 and 1:16, at two time periods (6 hours and 24 hours).

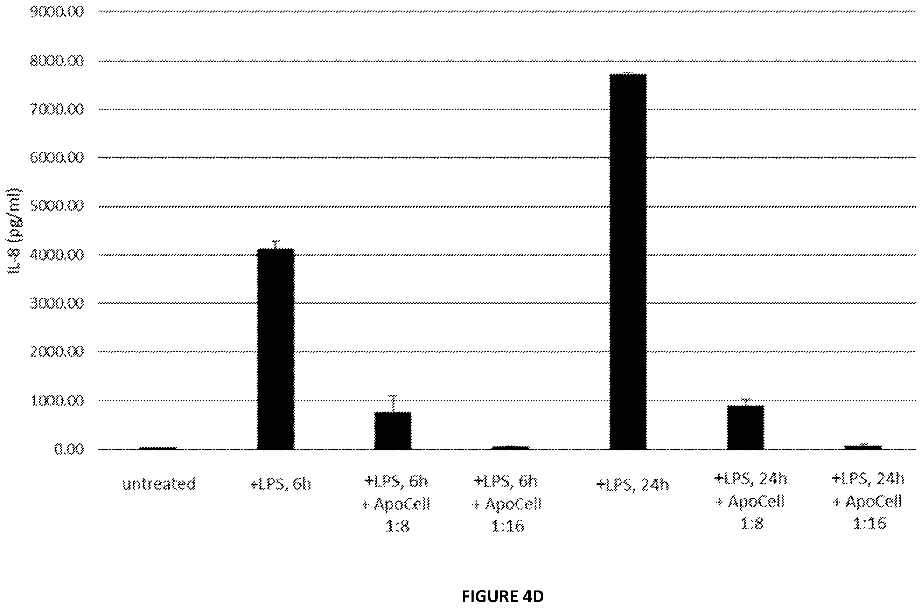

**Figure 4D** shows the reduction of LPS induced IL-8 levels in the macrophage activation syndrome model in the presence of cancer and CAR-19, following administration of Apocells at a macrophage/monocyte:Apocell ratio of 1:8 and 1:16, at two time periods (6 hours and 24 hours).

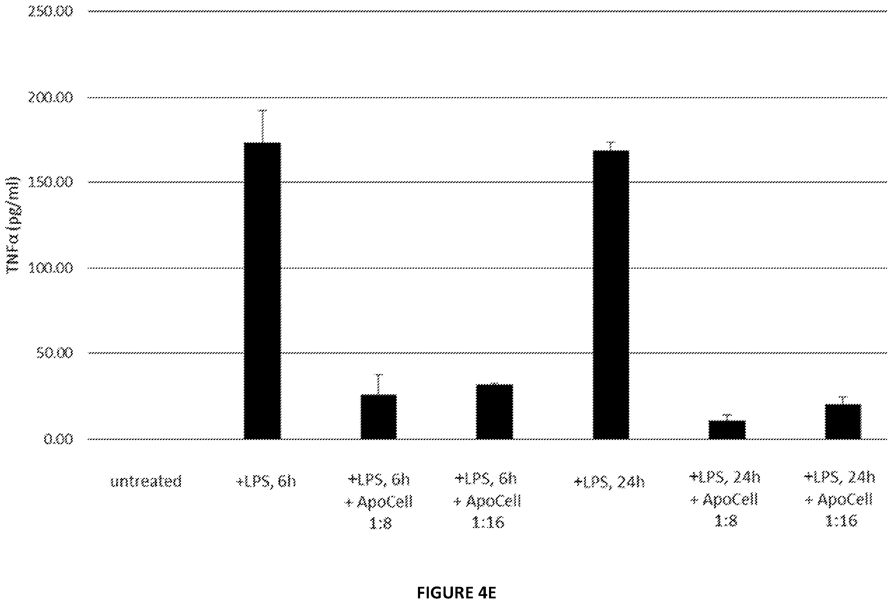

**Figure 4E** shows the reduction of LPS induced TNF-$\alpha$ levels in the macrophage activation syndrome model in the presence of cancer and CAR-19, following administration of Apocells at a macrophage/monocyte:Apocell ratio of 1:8 and 1:16, at TWO time periods (6 hours and 24 hours).

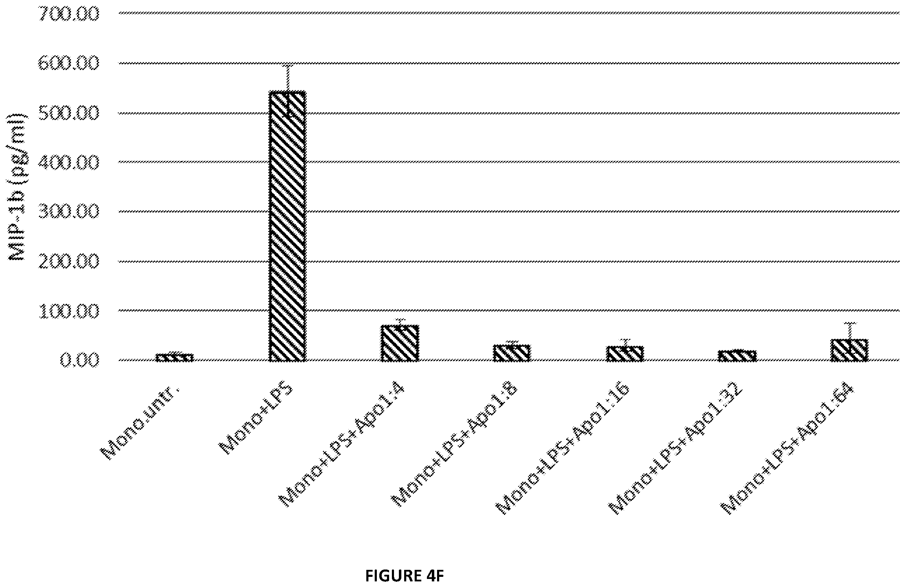

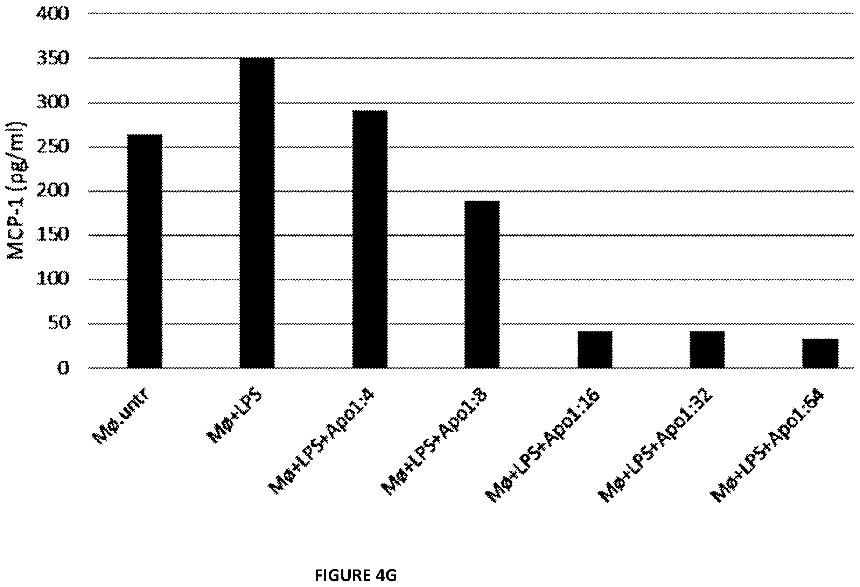

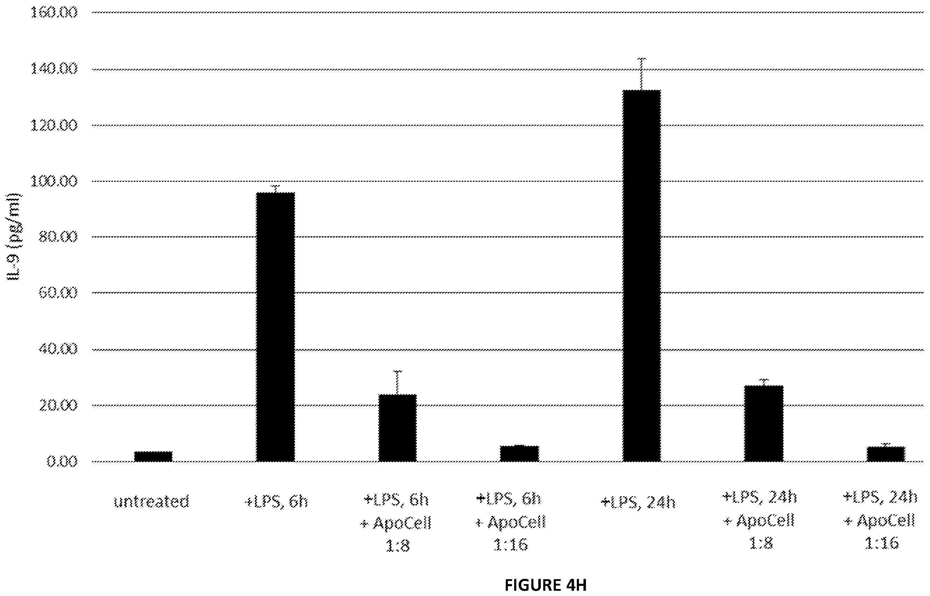

**Figure 4F** shows the reduction of LPS induced MIP-1β levels in the macrophage activation syndrome model in the presence of cancer and CAR-19, following administration of Apocells at a macrophage/monocyte:Apocell ratio of 1:4, 1:8, 1:16, 1:32, and 1:64 at 24 hours. **Figure 4G** shows the reduction of LPS induced MCP-1 levels in the macrophage activation syndrome model in the presence of cancer and CAR-19, following administration of Apocells at a macrophage/monocyte:Apocell ratio of 1:4, 1:8, 1:16, 1:32, and 1:64 at 24 hours. **Figure 4H** shows the reduction of LPS induced IL-9 levels in the macrophage activation syndrome model in the presence of cancer and CAR-19, following administration of Apocells at a macrophage/monocyte:Apocell ratio of 1:8 and1:16, at two time periods (6 hours and 24 hours).

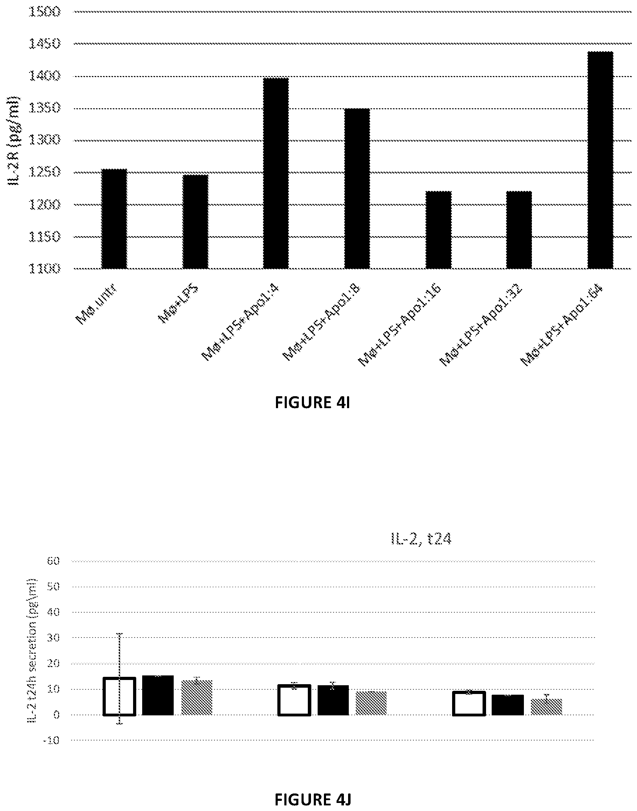

**Figure 4I** shows the increase of LPS induced IL-2R levels in the macrophage activation syndrome model in the presence of cancer and CAR-19, following administration of Apocells at a macrophage/monocyte:Apocell ratio of 1:4, 1:8, 1:16, 1:32, and 1:64 at 24 hours. **Figure 4J** shows that apoptotic cells do not down regulate IL-2 release from cells. Apoptotic cells were incubated with macrophages/monocytes in the presence of cancer and CAR-19, over a 24 hour time period with increasing doses of apoptotic cells (n=3). Empty bar (outline only) - 2.5 x $10^6$ apoptotic cells per well; Black - 5 x $10^6$ apoptotic cells per well; Grey - 10 x$10^6$ apoptotic cells per well.

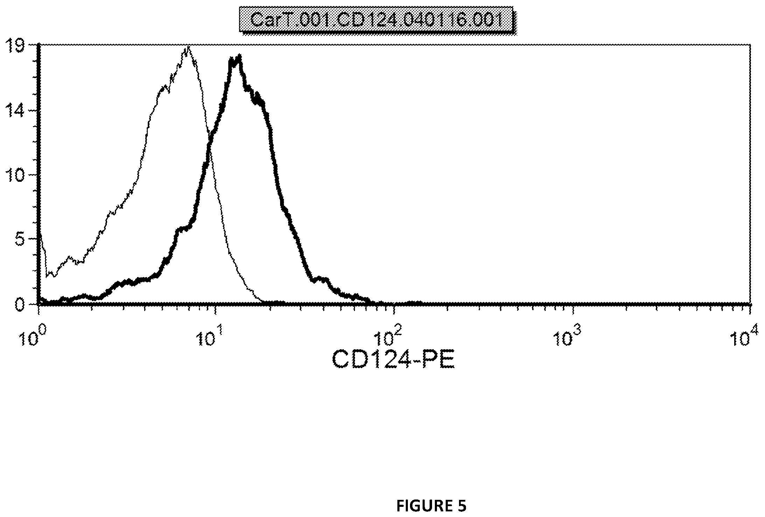

**Figure 5. Verification of Transduction of T-cells** showing the flow cytometry results of anti-CD124 analysis of transduced T4$^+$CAR-T cells.

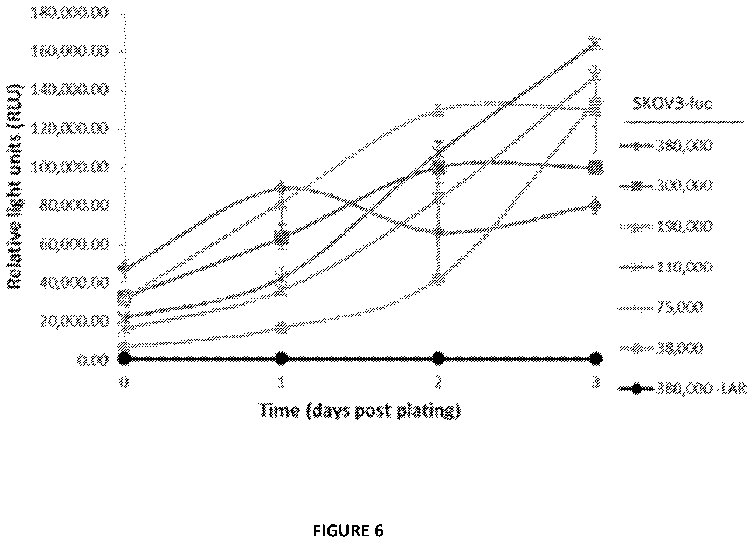

**Figure 6. T4$^+$CAR T-Cells reduced proliferation of SKOV3-luc ovarian adenocarcinoma cells.** The results of the cytotoxicity assay, wherein a monolayer of SKOV3-luc cells were cultured either by non-transduced T cells or by T4+ CAR-T cells, are presented in a bar graph.

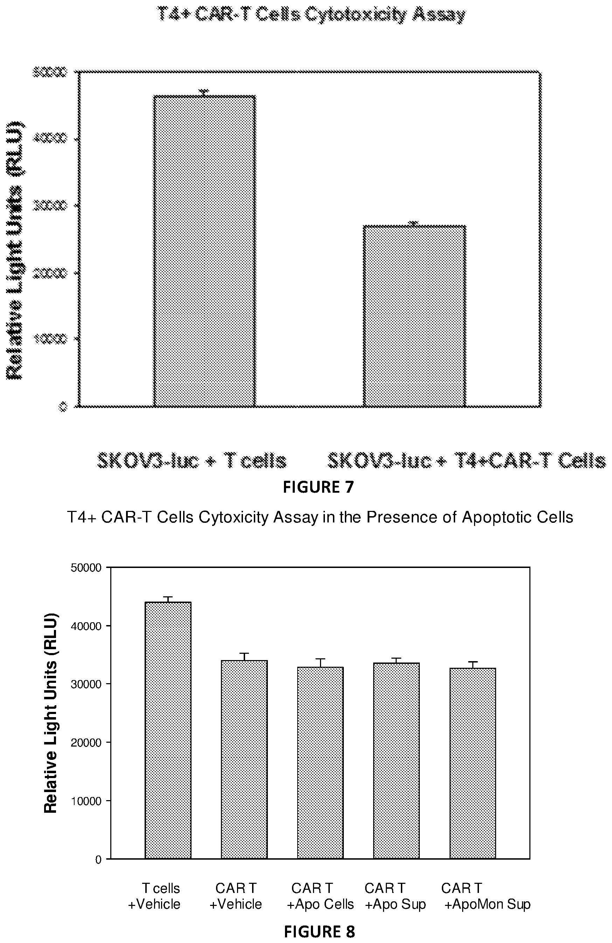

**Figure 7. Apoptotic Cells do not abrogate T4$^+$CAR-T cells anti-tumor activity.** Results are based on a cytotoxicity assay, wherein a monolayer of SKOV3-luc cells were cultured either with non-transduced T cells or with T4$^+$CAR-T cells in the presence of a vehicle (Hartmann solution), or apoptotic cells (Apocell), or a supernatant of apoptotic cells (ApoSup), or supernatant of co-culture of apoptotic cells and monocytes/macrophages (ApoMon Sup).

**Figure 8. 11-6, secreted at high levels during cytotoxicity, is down-regulated by apoptotic cells.** The results shown here demonstrate the effect of co-culture of SKOV3-luc and human monocytes/macrophages were exposed to apoptotic cells (ApoCell), or ApoCell supernatant (ApoSup), or apoptotic cells and monocyte/macrophage co-culture (ApoMon Sup).

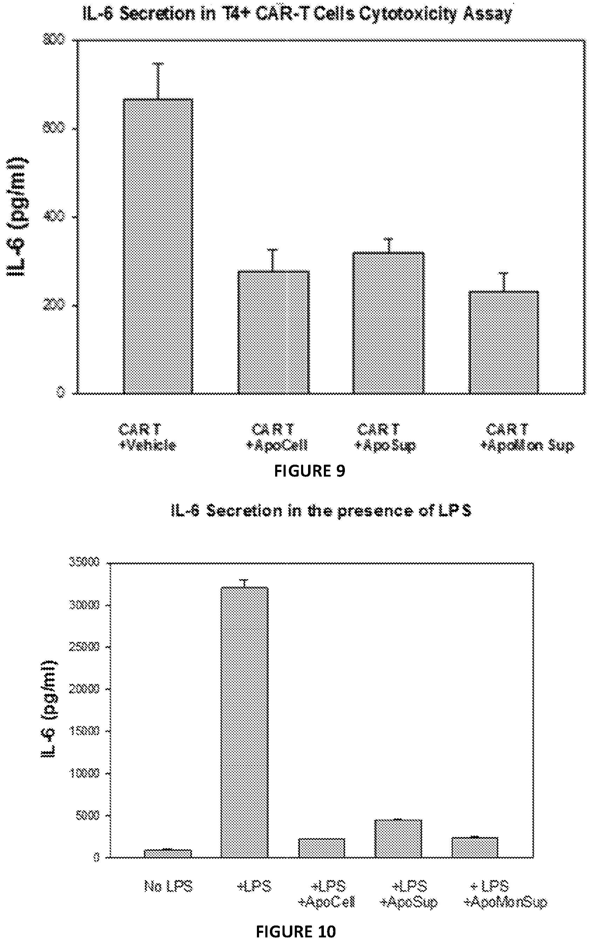

**Figure 9. Effect of Apoptotic Cells or Apoptotic Cell Supernatant or a co-culture of Apoptotic cells and Monocytes following LPS exposure during CAR-T cell therapy.** Extremely high secretion of IL-6 was documented when lipopolysaccharides (LPS) were added to the cytotoxic assay. Results show that exposure to Apoptotic cells (Apocell), or supernatant of apoptotic cells (ApoSup) or supernatant of co-culture of apoptotic cells and monocytes/macrophages (ApoMon Sup), down regulated IL-6, wherein IL-6 was reduced to acceptable levels.

**Figure 10. Effect of Apoptotic Cells or Apoptotic Cell Supernatant or a co-culture of Apoptotic cells and Monocytes following LPS exposure during CAR T-cell treatment mimicking CAR T-cell clinical therapy.** Extremely high secretion of IL-6 was documented when lipopolysaccharides (LPS) were added to the cytotoxic assay. Results show that exposure to Apoptotic cells (Apocell), or supernatant of apoptotic cells (ApoSup) or supernatant of co-culture of apoptotic cells and monocytes/macrophages (ApoMon Sup), down regulated IL-6, wherein IL-6 was reduced to acceptable levels.

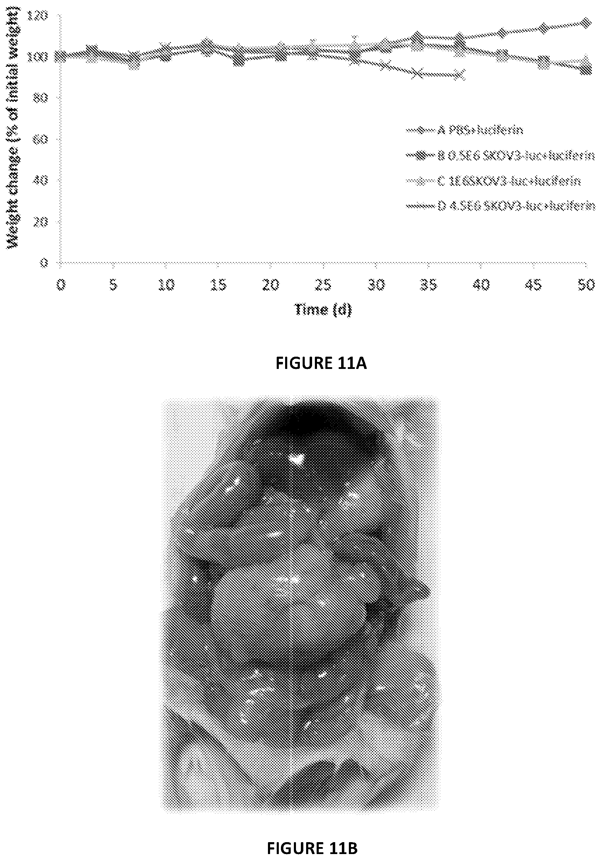

**Figures 11A-11B. Weight and Tumor Size in Mice at time of Culling. Figure 11A** shows Weight change over the experimental time period. Blue-control no 4.5x$10^6$ SKOV3-luc cells administrated. Red- 0.5x$10^6$ SKOV3-luc cells. Green-1.0x$10^6$ SKOV3-luc cells. Purple-4.5x$10^6$ SKOV3-luc cells **Figure 11B** presents a representative SKOV3-luc tumor for a mouse receiving 4.5x$10^6$ SKOV3-luc cells, 39 days after injection.

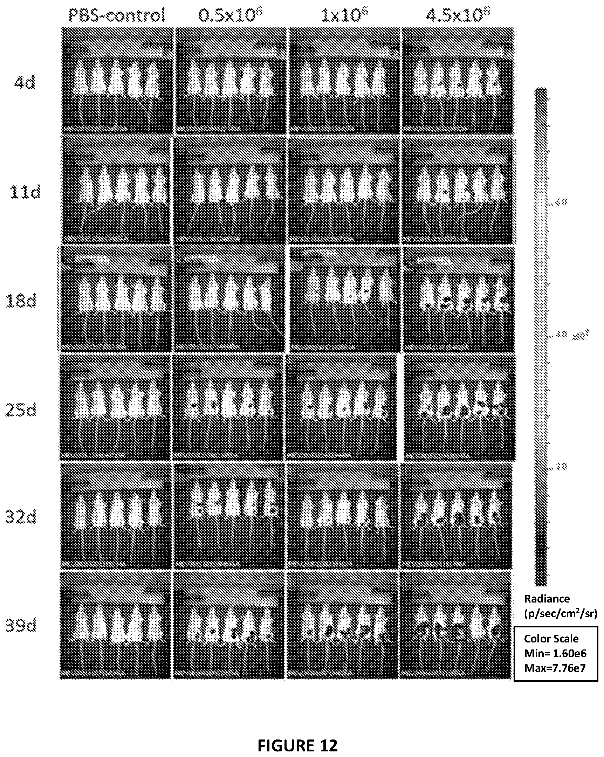

**Figure 12. SKOV3-luc Tumor Growth.** Mice bearing SKOV3-luc tumors imaged by Bioluminescent imaging (BLI) are presented showing the differences between control (PBS) and inoculation with 0.5x$10^6$, 1x$10^6$, and 4.5x$10^6$ SKOV3-luc cells.

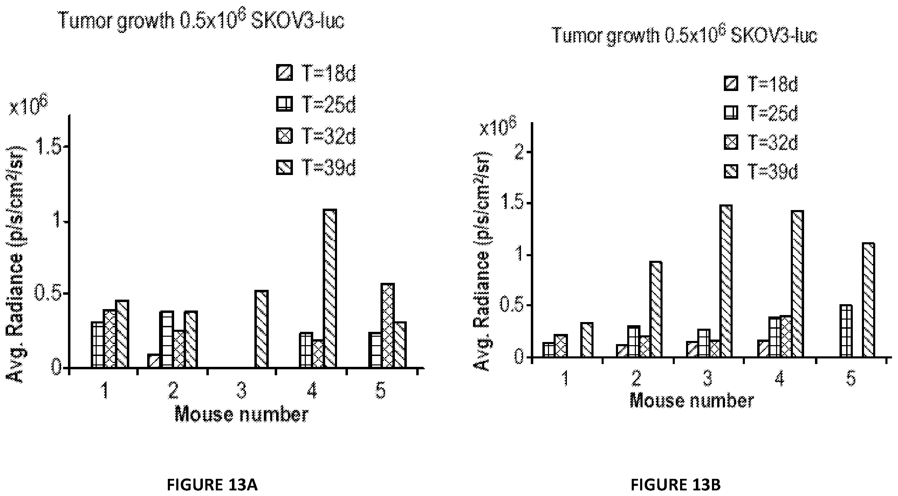

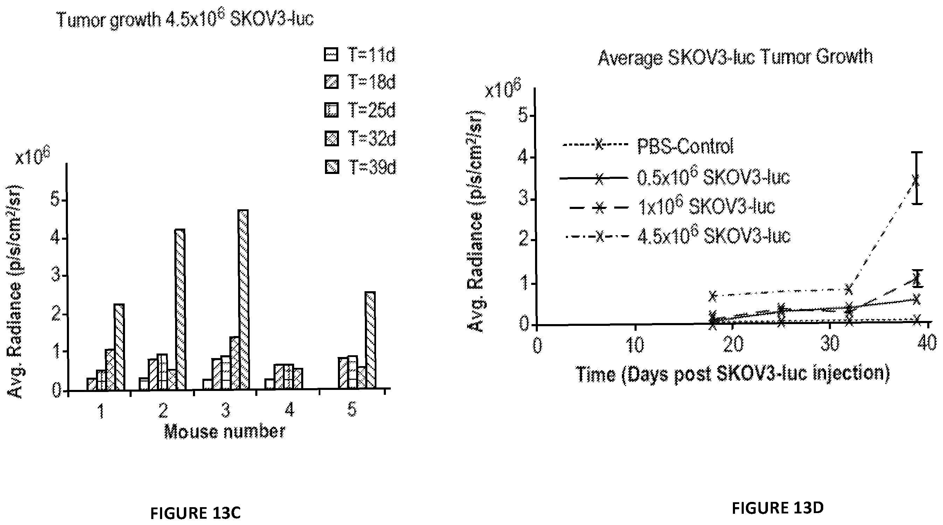

**Figures 13A-13D. SKOV3-luc Tumor Burden.** Quantification of bioluminescence (BLI) of SKOV3-luc tumors *in vivo* (See Figure 12). A 600 photon count cut-off was implemented as instructed by the manufacturer. **Figure 13A,** mice inoculated with 0.5x106 SKOV3-luc. **Figure 13B,** mice inoculated with 1x106 SKOV3-luc. **Figure 13C,** mice inoculated with 4.5x106 SKOV3-luc. **Figure 13D,** Average SKOV3-luc tumor growth.

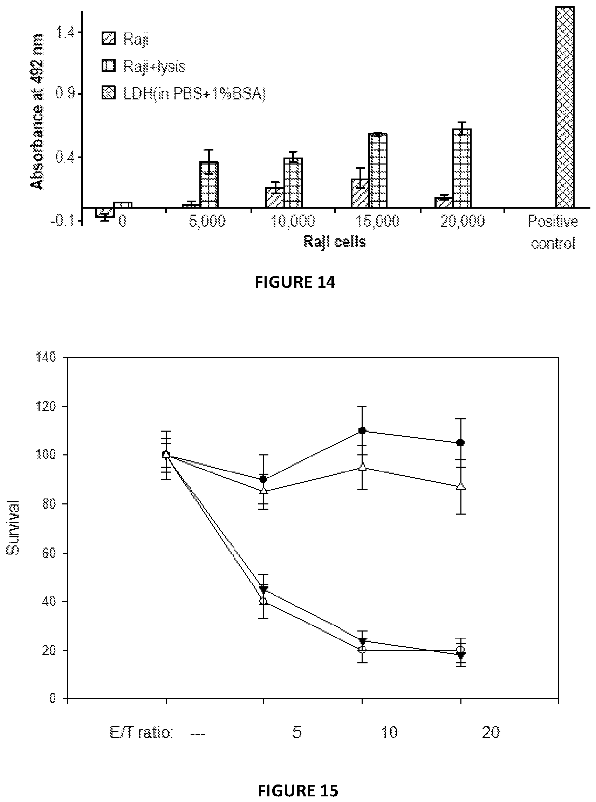

**Figure 14. Cytotoxic Calibration for Raji Burkett Lymphoma Cells.** Raji cells were plated at various cell densities, with cell lysis occurring immediately prior to centrifugation. The results show Raji cell number (x-axis) vs. at absorbance at 492 nm (y-axis). All cell numbers exhibited significant readings relative to the unlysed counterpart.

**Figure 15. Addition of early apoptotic cells does not affect CAR T-cell anti-tumor activity.** E/T ratio shows the CD19+CAR T-cell to HeLa cell ratio. Survival is of CD19+ Tumor cells. Filled circle CD19+ Hela; Empty triangle CD19+ Hela + Naive T cells; Filled triangle CD19+ Hela + CAR T-CD19; Empty circle CD19+ Hela + CAR T-CD19 +ApoCells.

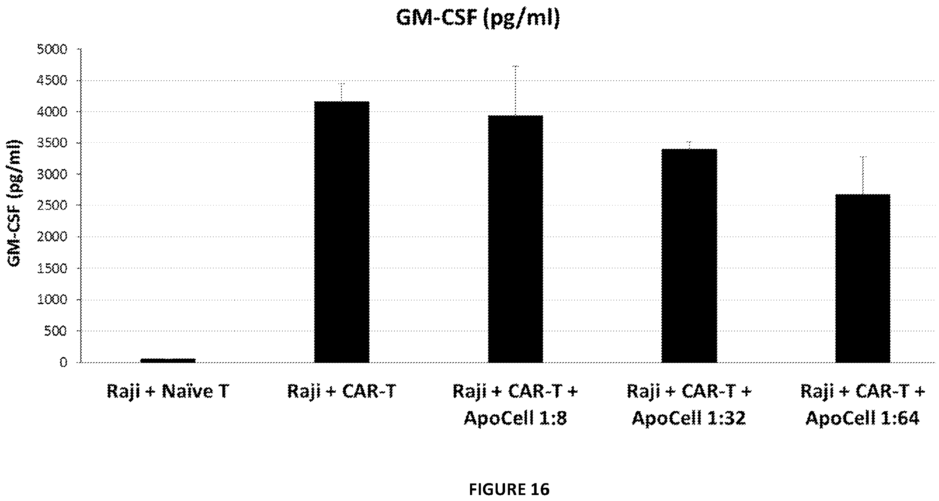

**Figure 16. Cytokine Analysis (GM-CSF) in Raji Burkett Lymphoma Cells in the Presence and Absence of Apoptotic cells.** The bar graph presents the concentration measurements of cytokine GM-CSF (pg/ml) found in culture supernatants of Raji cells incubated in the presence of monocytes and LPS, followed by addition of Naïve T-cells (Raji + Naive T), CD19+ CAR T-cells (Raji + CAR T), CD19+ CAR T-cells and apoptotic cells (ApoCell) at a ratio of 1:8 CAR T-cells:ApoCells (Raji + CAR T+ApoCell 1:8), CD19+ CAR T-cells and apoptotic cells (ApoCell) at a ratio of 1:32 CAR T-cells:ApoCells (Raji + CAR T+ApoCell 1:32), and CD19+ CAR T-cells and apoptotic cells (ApoCell) at a ratio of 1:64 CAR T-cells:ApoCells (Raji + CAR T+ApoCell 1:64).

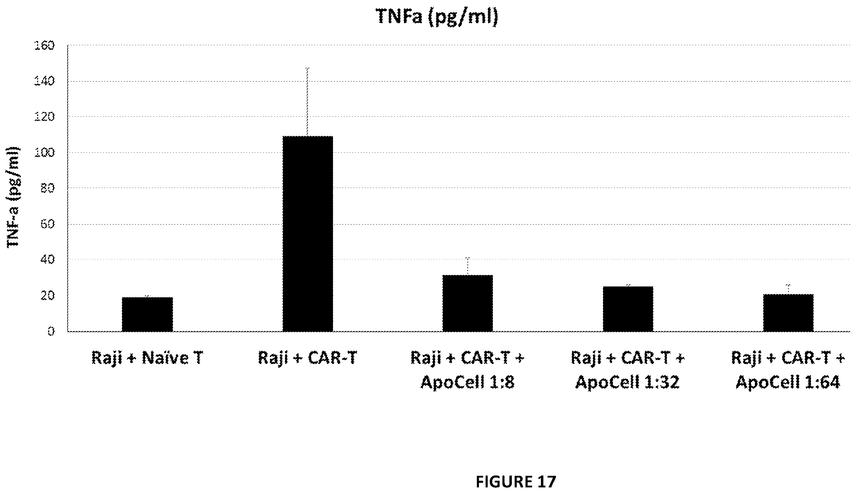

**Figure 17. Cytokine Analysis (TNF-alpha) in Raji Burkett Lymphoma Cells in the Presence and Absence of Apoptotic cells.** The bar graph presents the concentration measurements of cytokine TNF-alpha (TNF-a) (pg/ml) found in culture supernatants of Raji cells incubated in the presence of monocytes and LPS, followed by addition of Naive T-cells (Raji + Naive T), CD19+ CAR T-cells (Raji + CAR T), CD19+ CAR T-cells and apoptotic cells (ApoCell) at a ratio of 1:8 CAR T-cells:ApoCells (Raji + CAR T+ApoCell 1:8), CD19+ CAR T-cells and apoptotic cells (ApoCell) at a ratio of 1:32 CAR T-cells:ApoCells (Raji + CAR T+ApoCell 1:32), and CD19+ CAR T-cells and apoptotic cells (ApoCell) at a ratio of 1:64 CAR T-cells:ApoCells (Raji + CAR T+ApoCell 1:64).

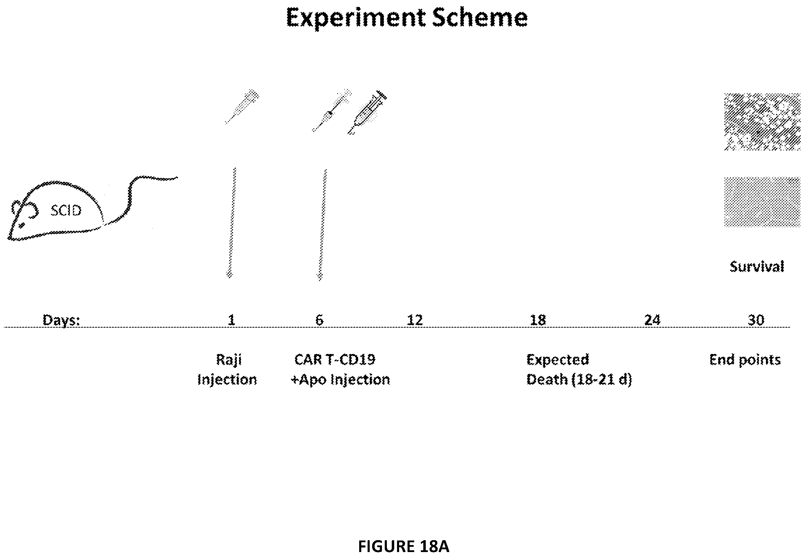

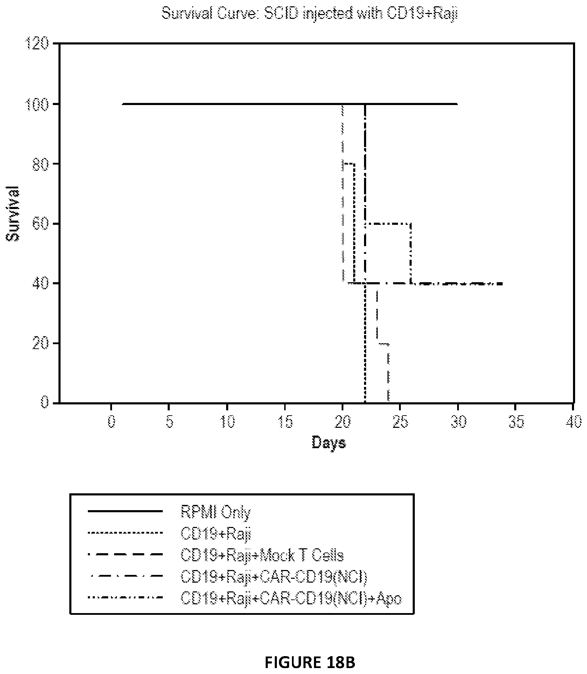

**Figures 18A and 18B. Experimental Scheme. Figure 18A** presents the experimental scheme to analyze the influence of apoptotic cells on CAR T-cell therapy. SCID mice were injected on day 1 with Raji cancer cells, followed on day 6 by administration of CAR T-CD19 cells (CAR T-cell therapy) and Apoptotic cells. **Figure 18B** shows that CAR T-cell therapy was not negatively influenced by co-administration of ApoCells. Survival Curve: SCID mice were injected with CD19+ Raji cells with or without addition of early apoptotic cells.

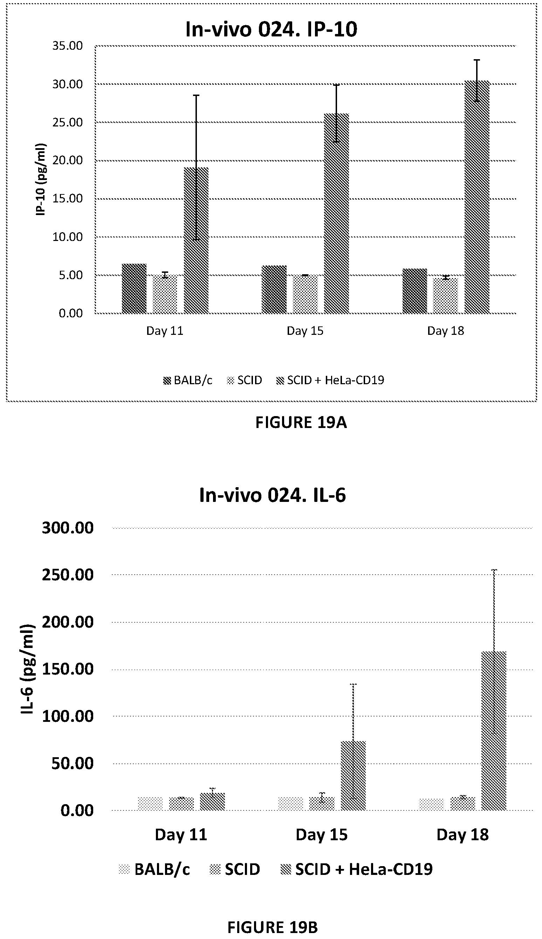

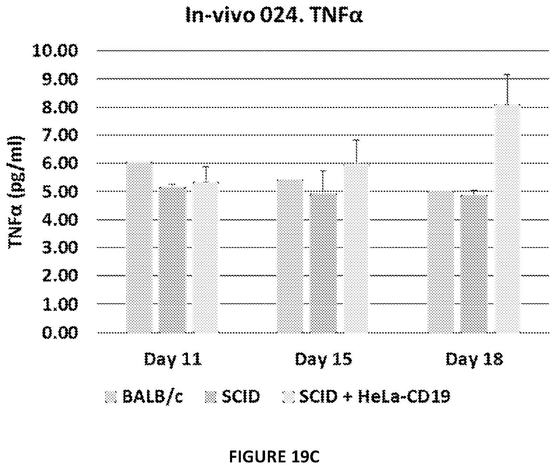

**Figures 19A, 19B, and 19C. Increased release of pro-inflammatory cytokines from a tumor, in a solid tumor *in vivo* model. Figure 19A** shows slight increase of IL-6 released from a solid tumor present in the peritoneum of BALB/c and SCID mice, wherein the IL-6 release is significantly increased in the presence of HeLa CAR-CD-19 CAR T-cells. Similarly, **Figure 19B** shows a slight increase of IP-10 released from a solid tumor present in the peritoneum of BALB/c and SCID mice, wherein the IP-10 release is significantly increased in the presence of HeLa CAR-CD-19 CAR T-cells, and **Figure 19C** shows that surprisingly even TNF-$\alpha$ release is increased by in the presence of HeLa CAR-CD-19 CAR T-cells.

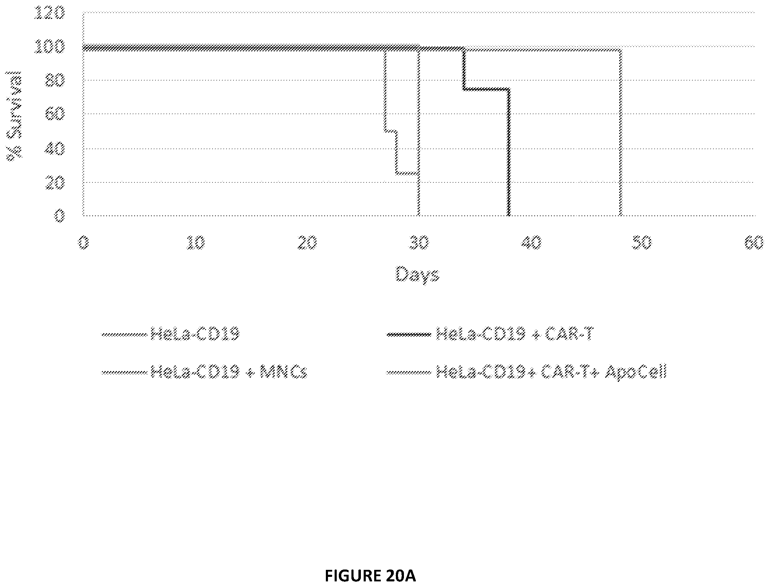

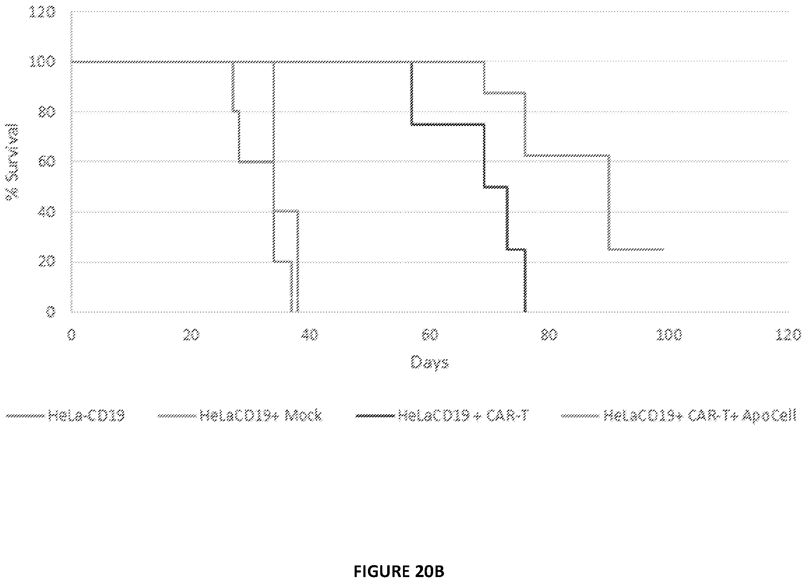

**Figures 20A and 20B. Testing the efficacy of CD19-CAR-T cells in an IP model of HeLaCD19 (Leukemia), in the presence or absence of ApoCell.** HeLa-CD19 - Blue; HeLaCD19+Mock - Green; HeLaCD19 + CAR-T - Purple; and HeLaCD19 + CAR-T + ApoCell - orange. **Figure 20A** was with $0.5 \times 10^6$ CAR-T positive cells. **Figure 20B** was with $2.2 \times 10^6$ CAR-T positive cells.

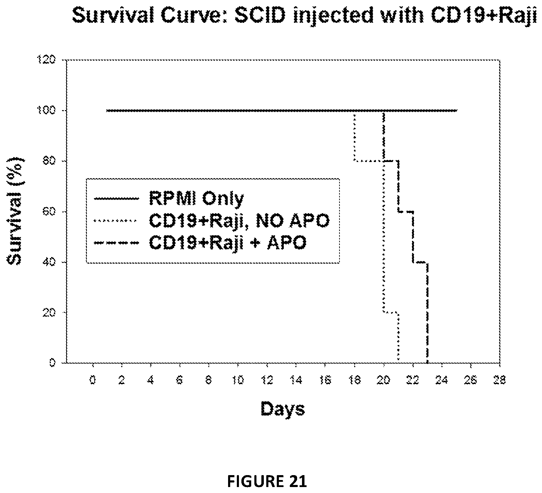

**Figure 21. Survival curves for *in vivo* diffuse tumor SCID mouse model.** The curves show that administration of early apoptotic cells (APO; broad dashed lines ----) extended survival compared with mice not administered apoptotic cells (NO APO; dotted line ·····), wherein control SCID mice showed 100% survival (solid line -_).

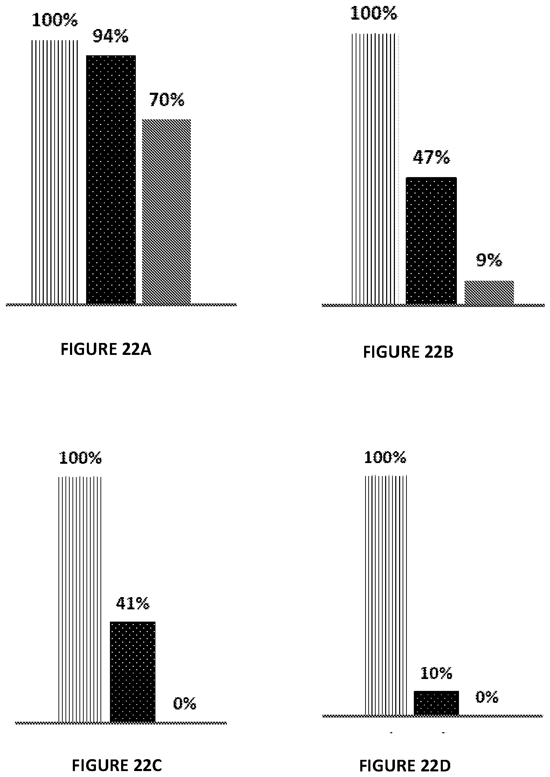

**Figures 22A-22D. Apoptotic cell infusions increased the lifespan of leukemic mice and increased the number of mice attaining complete remission.** Cohorts: No leukemia (Control-striped pattern); Leukemia + early apoptotic cells (spotted); Leukemia only (solid grey). n=51 in total (p<0.001) **Figure 22A.** Apoptotic cell infusions increased the percentage of mice surviving through the expected life-span post leukemia induction. **Figure 22B.** Apoptotic cell infusions increased the percentage of mice surviving up to 12% of the expected life-span post leukemia induction. **Figure 22C.** Apoptotic cell infusions increased the percentage of mice surviving up to 30% of the expected life-span post leukemia induction. **Figure 22D.** Apoptotic cell infusions increased the percentage of mice surviving up to 100% of the expected life-span post leukemia induction and attaining complete remission.

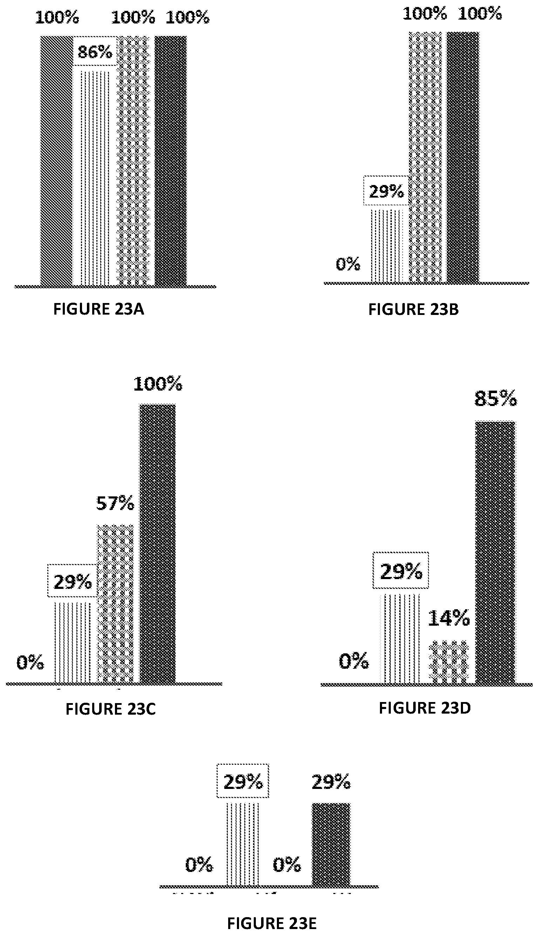

**Figures 23A-23E. Apoptotic cell infusions increased the life-span of leukemic mice, increased the number of mice attaining complete remission, and enhanced the anti-CD20 monoclonal antibody (mAb) therapeutic effect.** Cohorts: Leukemia only (solid grey); Leukemia + early apoptotic cells (striped pattern); Leukemia + anti-CD20 mAb (checkered); Leukemia + anti-CD20 + early apoptotic cells (spotted). n=28 in total (p<0.002) **Figure 23A.** Shows the percent (%) survival through the expected lifespan of mice following induction of leukemia with Raji cells. **Figure 23B.** Apoptotic cell infusions increased the percentage of mice surviving up to 24% longer than the expected life-span post leukemia induction. **Figure 23C.** Apoptotic cell infusions increased the percentage of mice surviving up to 59% longer than the expected life-span post leukemia induction and enhanced the anti-CD20 mAb effect on the life-span of leukemic mice. **Figure 23D.** Apoptotic cell infusions increased the percentage of mice surviving up to 76% longer than the expected life-span post leukemia induction and enhanced the anti-CD20 mAb effect on the life-span of leukemic mice. **Figure 23E.** Apoptotic cell infusions increased the percentage of mice attaining complete remission.

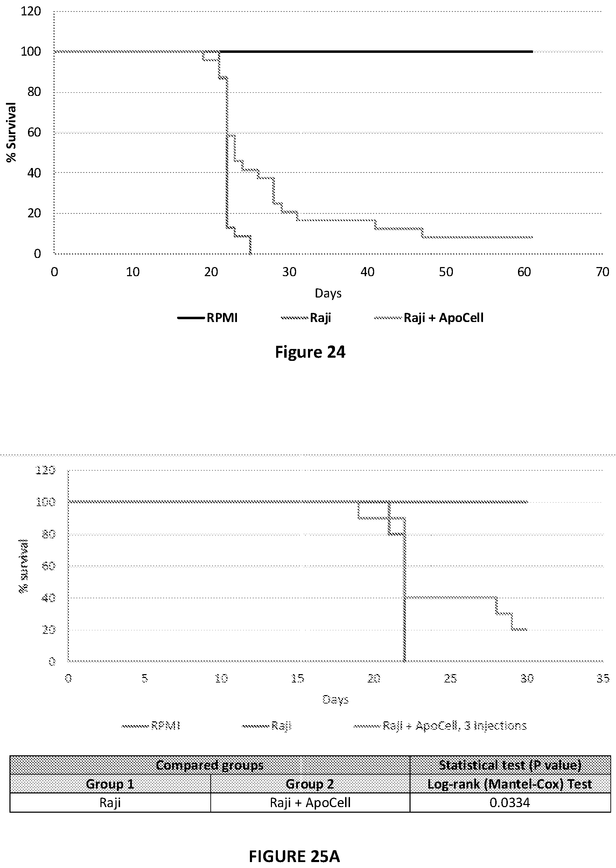

**Figure 24. Kaplan-Meier survival plot of SCID-Bg mice with Raji leukemia/lymphoma, receiving ApoCell.** (RPMI group, n = 15; Raji group, n = 23; Raji + ApoCell group, n = 24) RPMI (control) - Black; Raji only - Orange; Raji + ApoCell - Blue.

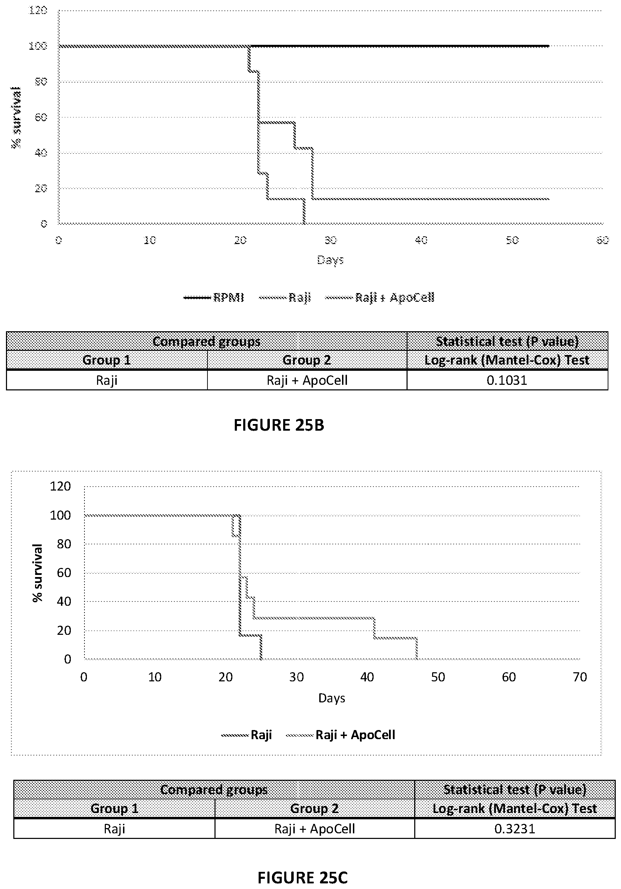

**Figures 25A-25C. Kaplan-Meier survival plots. Figure 25A** presents data from a study wherein female SCID-Bg mice, 7-weeks-old (ENVIGO, Jerusalem, Israel), were injected IV with $0.1 \times 10^6$ Raji cells per mouse (n = 10 per group, three groups). Mice received three IV doses (days 5, 8, 11) of $30 \times 10^6$ ApoCell. (RPMI-light blue; Raji-orange; and Raji + ApoCell - dark blue) **Figure 25B** presents data from a study wherein female SCID-Bg mice, 7-weeks-old (ENVIGO, Jerusalem, Israel), were injected IV with $0.1 \times 10^6$ Raji cells per mouse (n = 10 per group, three groups). Mice received three IV doses (days 5, 8, 11) of $30 \times 10^6$ ApoCell. (RPMI-black; Raji-orange; and Raji + ApoCell - dark blue) **Figure**

**25C** presents data from a study wherein female SCID-Bg mice, 8-9-weeks-old (ENVIGO, Jerusalem, Israel), were injected IV with $0.1 \times 10^6$ Raji cells per mouse (n=10 per group, 2 groups). Mice received three IV doses (days 5, 8, 12) of $30 \times 10^6$ ApoCell. (Raji-orange; and Raji + ApoCell - dark blue)

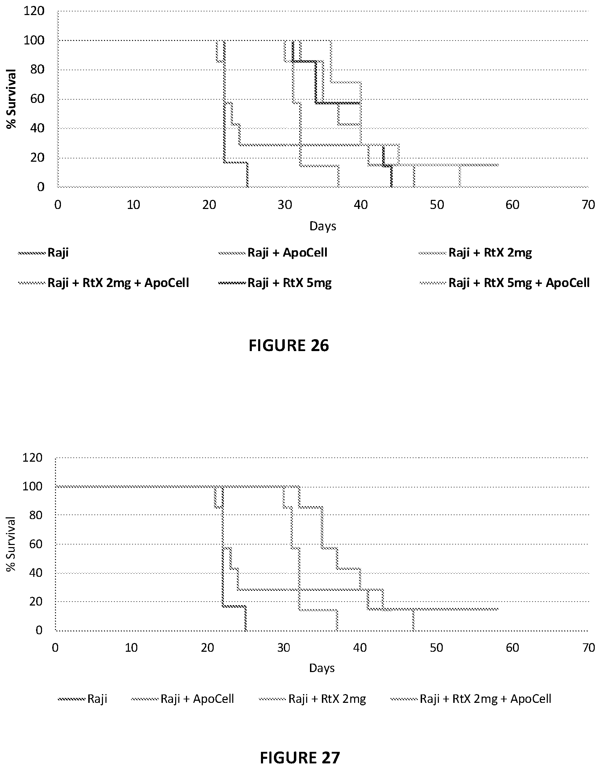

**Figure 26. Kaplan-Meier survival plot of SCID-Bg mice with Raji leukemia/lymphoma, receiving RtX and ApoCell.** (Raji alone - orange; Raji + ApoCell - blue; Raji + RtX 2mg - green; Raji + RtX 2mg + ApoCell - yellow; Raji + RtX 5mg - purple; Raji + RtX 5mg + ApoCell - grey.)

**Figure 27. Kaplan-Meier survival plot of SCID-Bg mice with Raji leukemia/lymphoma, receiving rtx and ApoCell.** (Raji alone - orange; Raji + ApoCell - blue; Raji + RtX 2mg - green; Raji + RtX 2mg + ApoCell - yellow.)

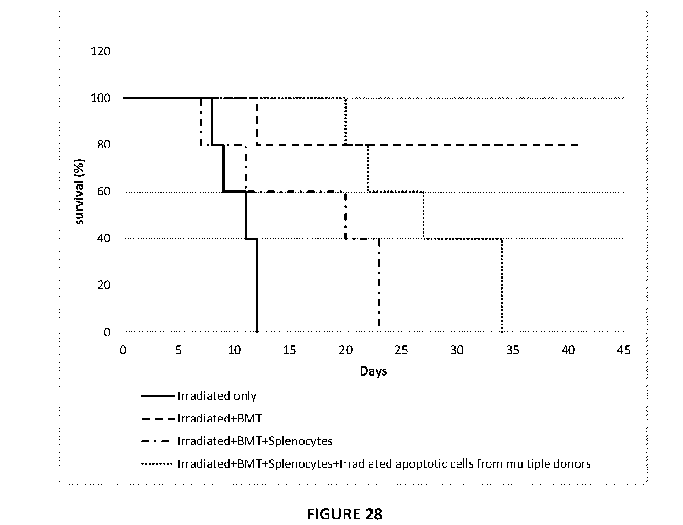

**Figure 28. Effect of Pooled ApoCell Preparation. Figure 28** presents a graph showing the clear effect (p<0.01) of a single apoptotic cell preparation injection from multiple individual donors (blue) on survival. The graph presented is a Kaplan-Meier survival curve in a GvHD mouse model that was treated with a single dose irradiated pooled apoptotic cell preparation from multiple individual donors.

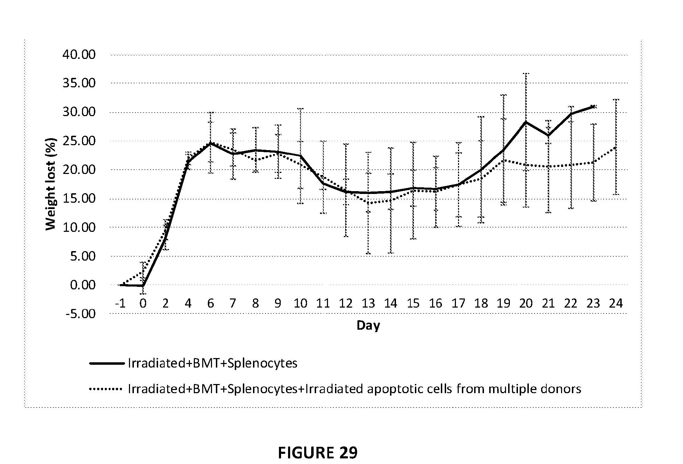

**Figure 29. Effect of Pooled ApoCell Preparation. Figure 29** presents a graph showing the clear effect (p<0.01) of a single apoptotic cell preparation injection from multiple individual donors (blue) on percentage of weight loss of the 2 compared groups.

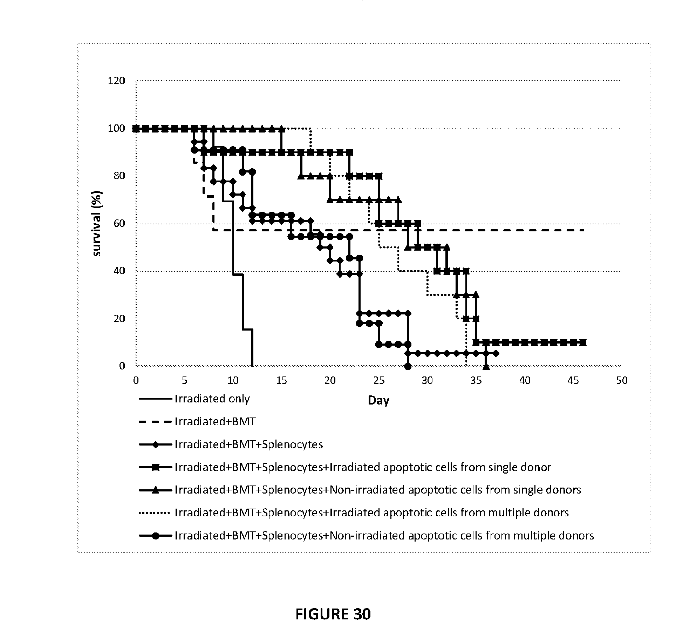

**Figure 30. Comparison of Single Donor versus Pooled ApoCell Preparation. Figure 30** presents a graph showing comparison between the administration of a single dose of single-donor and multiple-donor apoptotic cell preparations +/- irradiation on % survival using a mouse model of induced GvHD.

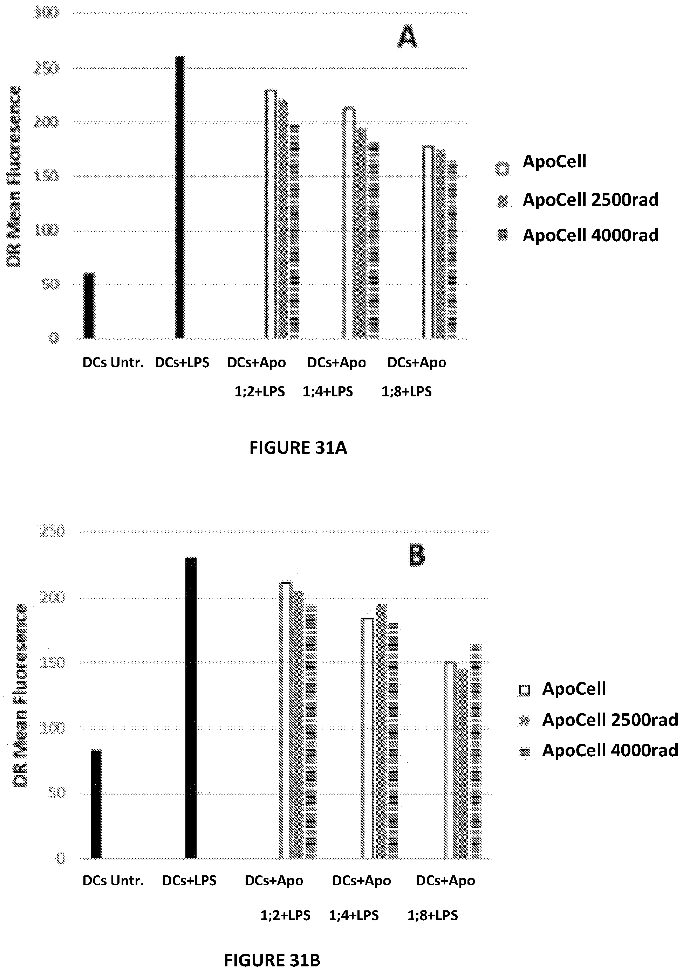

**Figures 31A-31B. Potency Test. Figures 31A-31B** present the results of a potency test that shows the inhibition of maturation of dendritic cells (DCs) following interaction with apoptotic cells, measured by expression of HLA-DR. **Figure 31A.** HLA DR mean fluorescence of fresh final product A (t0). **Figure 31B.** HLA DR mean fluorescence of final product A, following 24h at 2-8°C.

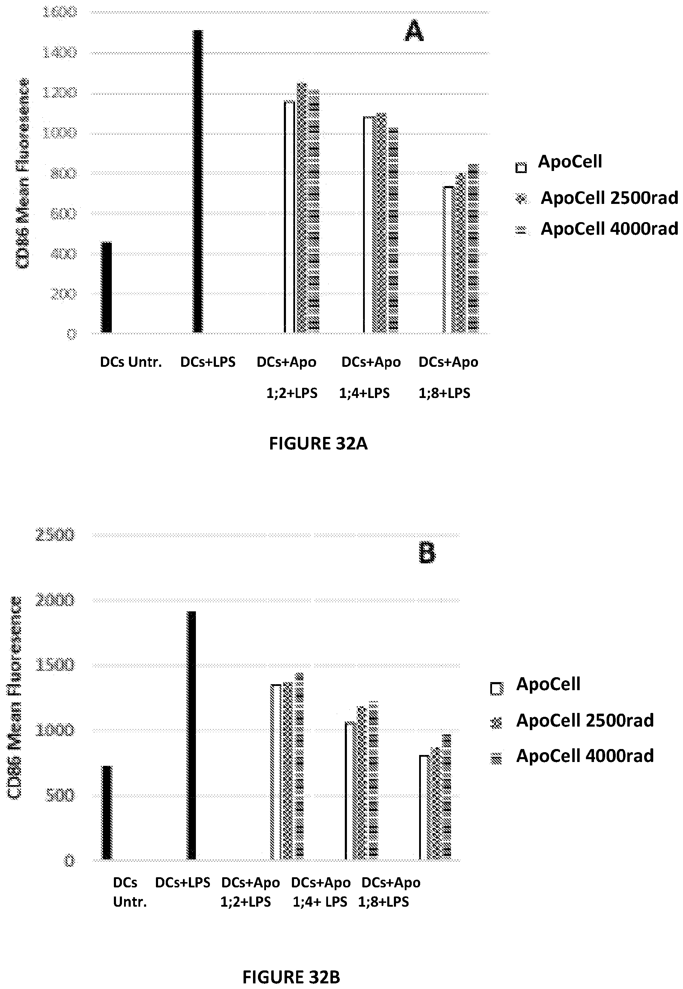

**Figures 32A-32B. Potency Test. Figures 32A-32B** present the results of a potency test that shows the inhibition of maturation of dendritic cells (DCs) following interaction with apoptotic cells, measured by expression of CD86. **Figure 32A.** CD86 Mean fluorescence of fresh final product A (t0). **Figure 32B.** CD86 Mean fluorescence of final product A, following 24h at 2-8°C.

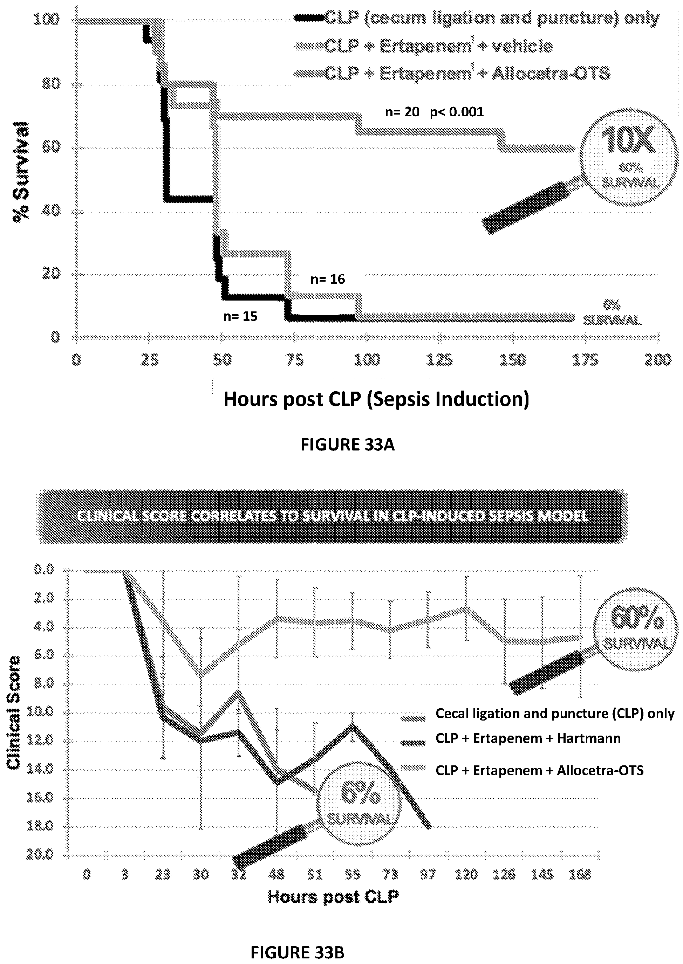

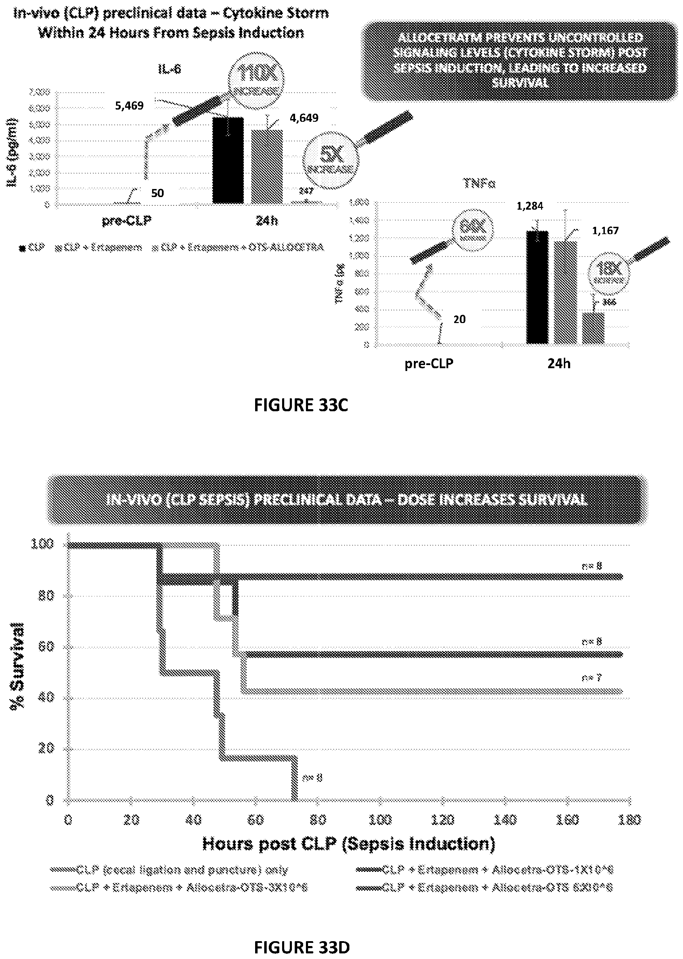

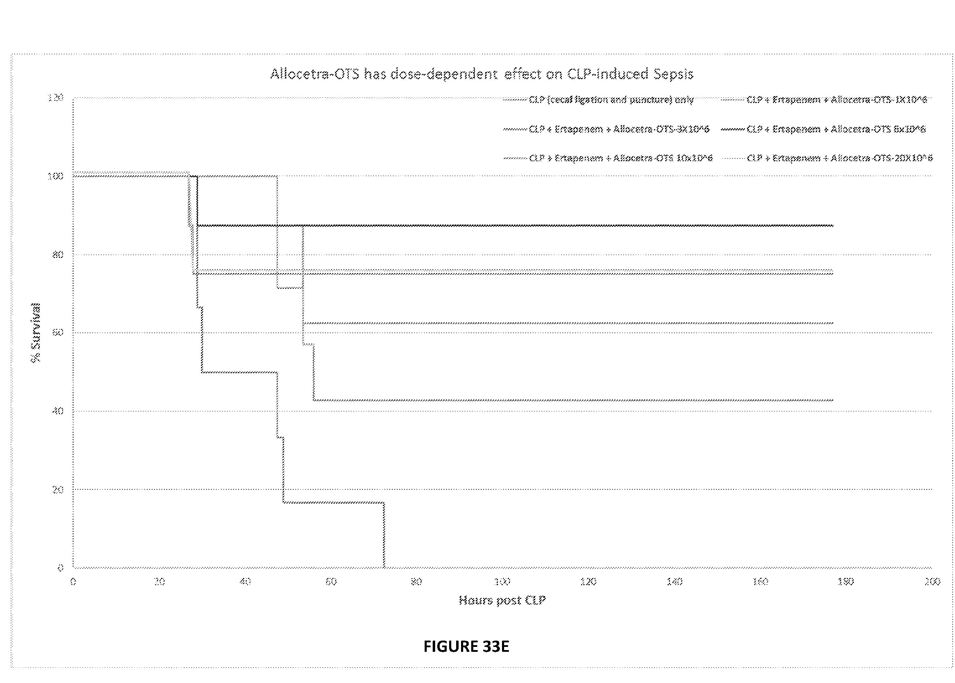

**Figures 33A-33E.** Results of preclinical analysis of use of early apoptotic cells in the treatment of sepsis. **Figure 33A** shows increased survival in mice receiving antibiotic and Allocetra-OTS (early apoptotic cells as described herein). **Figure 33B** shows the clinical scores of the different cohorts, wherein the clinical score correlates with the survival of mice in the CLP-induced sepsis model subjects. **Figure 33C** shows Allocetra prevents uncontrolled cytokine signaling events, i.e., a cytokine storm, following sepsis induction, which lead to increased survival in the CLP-induced sepsis model subjects. **Figures 33D** shows dose dependent increased survival of the CLP-induced sepsis model subjects treated with Allocetra. **Figure 33E** also shows dose dependent increased survival of the CLP-induced sepsis model subjects treated with Allocetra.

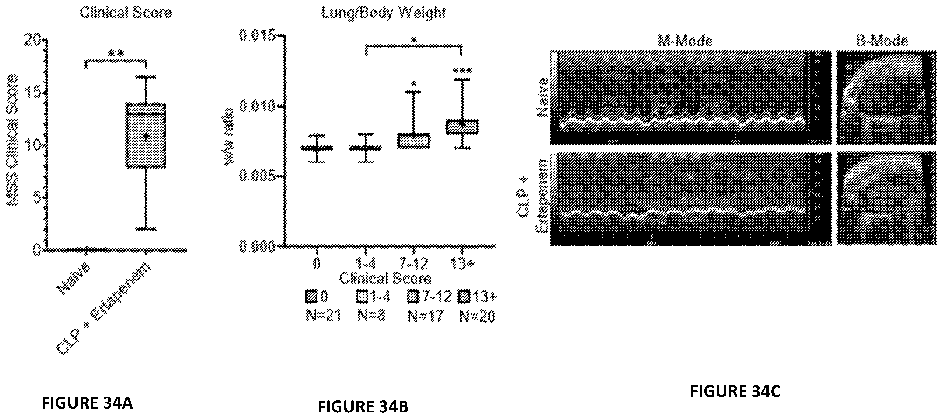

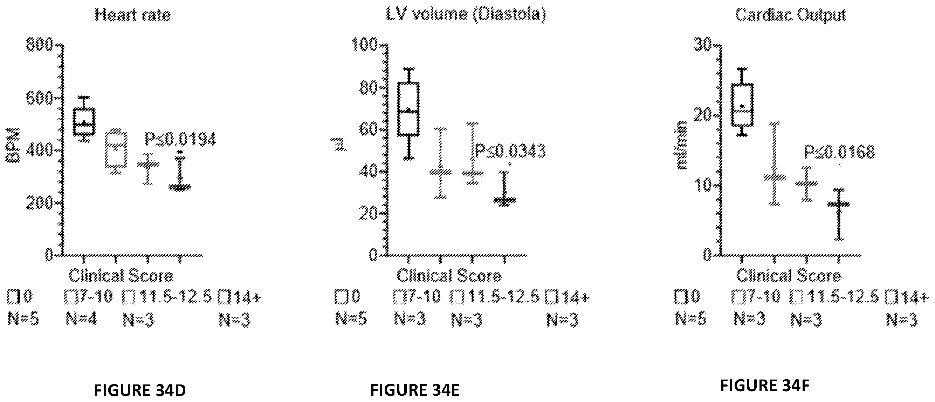

**Figures 34A-34F.** CLP mice display signs of respiratory and cardiovascular dysfunction that correlate with sepsis severity. **(Figure 34A)** 24h post-CLP, naïve mice (n=21) showed no signs of illness, while the majority of CLP mice (n=40) had severe clinical signs (median MSS Clinical Score of 13; 95% CI of median 9-14); \*\*P<0.0001 by a two-tailed Mann-Whitney test. **(Figure 34B)** The lung-to-body weight ratio significantly increases with sepsis severity. **(Figure 34C)** Representative 2D echocardiograms of naïve (top panels) and CLP-mice (bottom panels), showing the time-lapse view (M-Mode) and top view (B-Mode). LV internal distances, heart rate, and posterior wall thickness were measured for the calculation of various parameters of LV structure and function, including **(Figure 34D)** heart rate, **(Figure 34E)** LV volume, and **(Figure 34F)** cardiac output. Data are presented as the median within the inter-quartile range (IQR); mean values are marked with a '+' sign; error bars represent the 5-95 percentile range; group sizes (N) are indicated below their respective legends; \*P≤0.01, \*\*P≤0.001, \*\*\* P≤0.0001 by the Kruskal-Wallis nonparametric ANOVA, with multiple comparisons adjusted using Dunn's test. P values above the bars indicate the significant differences from the control group, and those above the brackets indicate the significant differences between the two other groups.

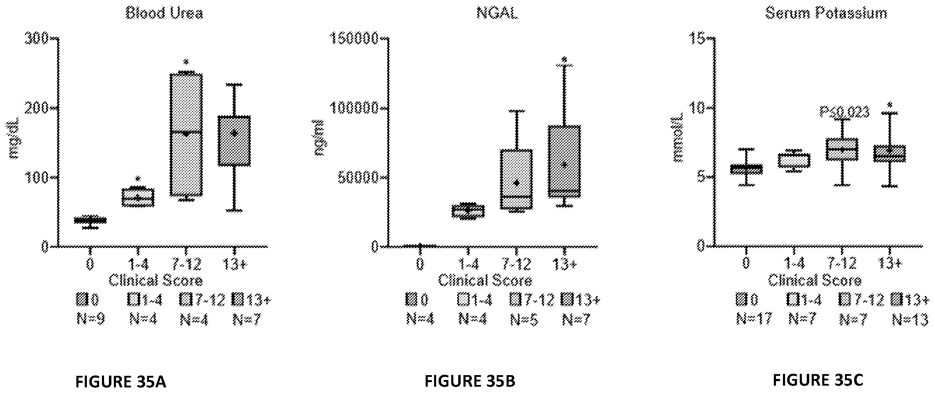

**Figures 35A-35C.** CLP mice display signs of renal dysfunction that correlate with sepsis severity. Renal dysfunction is indicated by increasing concentrations of **(Figure 35A)** blood urea, **(Figure 35B)** neutrophil gelatinase-associated lipocalin (NGAL), and **(Figure 35C)** serum potassium. Data are presented as the median within the inter-quartile range (IQR); mean values are marked with a '+' sign; error bars represent the 5-95 percentile range; group sizes (N) are indicated below their respective legends; \*P≤0.01, \*\*P≤0.001, \*\*\* P≤0.0001 by the Kruskal-Wallis nonparametric ANOVA, with multiple comparisons adjusted using Dunn's test. P values above the bars indicate differences from the control group, and those above the brackets indicate differences between the two other groups.

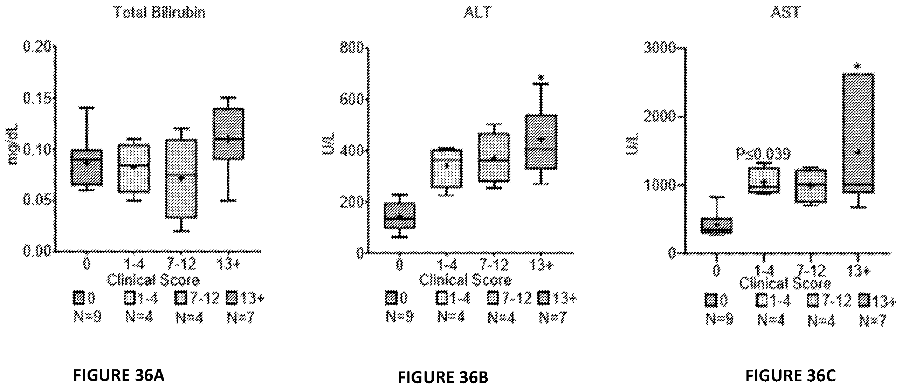

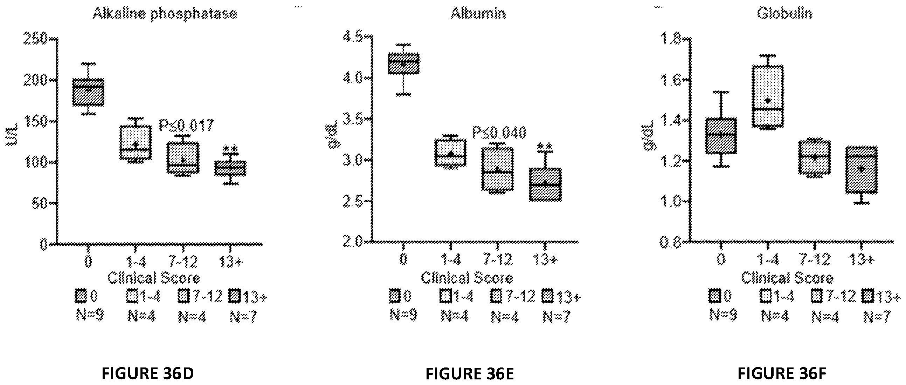

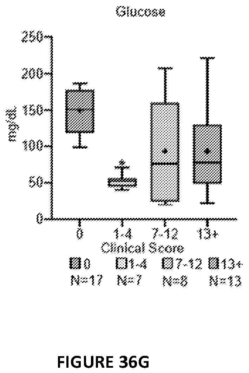

**Figures 36A-36G.** Markers for hepatic dysfunction strongly correlate with MSS clinical score in CLP mice. **(Figure 36A)** 24h post-CLP, mice with severe sepsis (MSS Clinical Score >13) had a slight and insignificant (p>0.93) increase in total bilirubin serum concentration, while **(Figure 36B)** alanine aminotransferase (ALT) and **(Figure 36C)** aspartate aminotransferase (AST) levels were significantly decreased with sepsis severity. **(Figure 36D)** Alkaline phosphatase and **(Figure 36E)** albumin levels were significantly decreased with sepsis severity, while **(Figure 36F)** globulin serum concentrations were not significantly altered. **(Figure 36G)** Glucose levels of septic mice, notably in mildly septic mice (MSS Clinical score of 1-4), were lower than those in naive mice. Data are presented as the median within the inter-quartile range (IQR); mean values are marked with a '+' sign; error bars represent the 5-95 percentile range; group sizes (N) are indicated below their respective legends; *$P \leq 0.01$, **$P \leq 0.001$, *** $P \leq 0.0001$ by the Kruskal-Wallis non-parametric ANOVA, with multiple comparisons adjusted using Dunn's test. P values above the bars indicate the differences from the control group, and those above the brackets indicate differences between the two other groups.

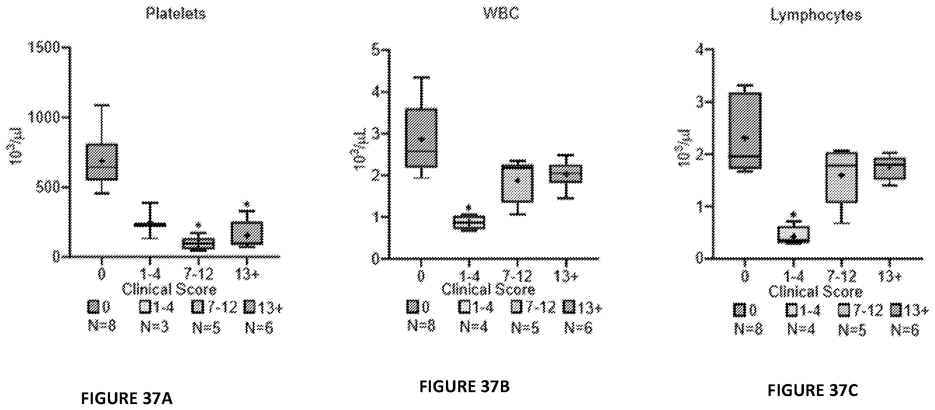

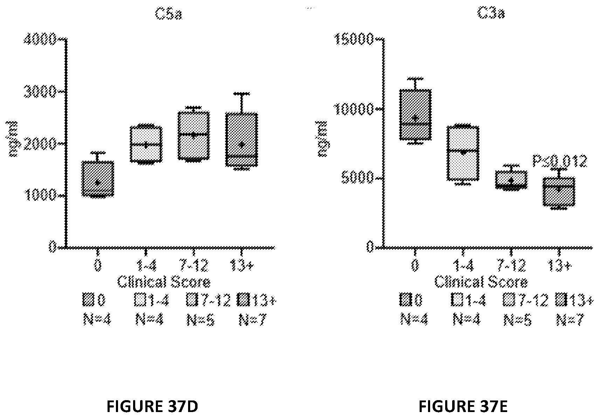

**Figures 37A-37E.** Marked thrombocytopenia and lymphopenia and aberrant complement activation in septic mice. **(Figure 37A)** 24h post-CLP, septic mice had significantly lower platelet counts than naive mice. Decreased **(Figure 37B)** WBC and **(Figure 37C)** lymphocyte counts in CLP-mice, predominantly in mice with mild sepsis (MSS clinical score of 1-4). **(Figure 37D)** C5a serum concentration is higher in septic mice, regardless of their clinical score. **(Figure 37E)** C3a serum concentration is lower in septic mice with correlation to clinical score. Data are presented as the median within the inter-quartile range (IQR); mean values are marked with a '+' sign; error bars represent the 5-95 percentile range; group sizes (N) are indicated below the irrespective legends; *$P \leq 0.01$, **$P \leq 0.001$, ***$P \leq 0.0001$ by the Kruskal-Wallis non-parametric ANOVA, with multiple-comparisons adjusted by using the Dunn's test. P values above the bars indicate the significant differences from the control group, and those above the brackets indicate the significant differences between two other groups.

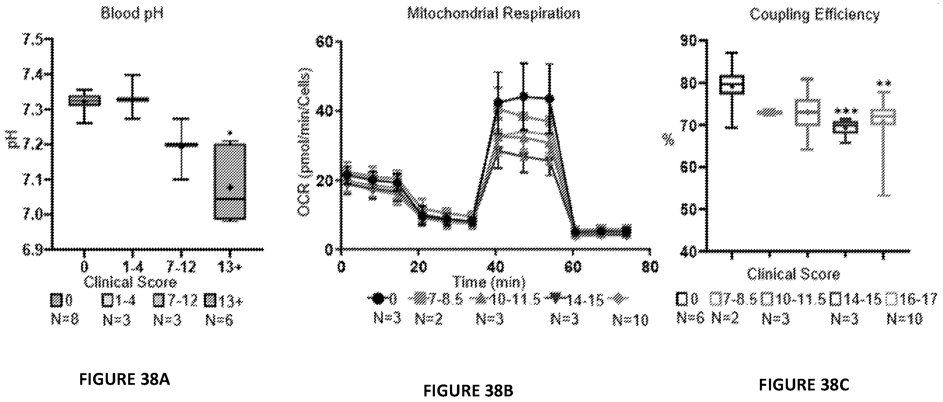

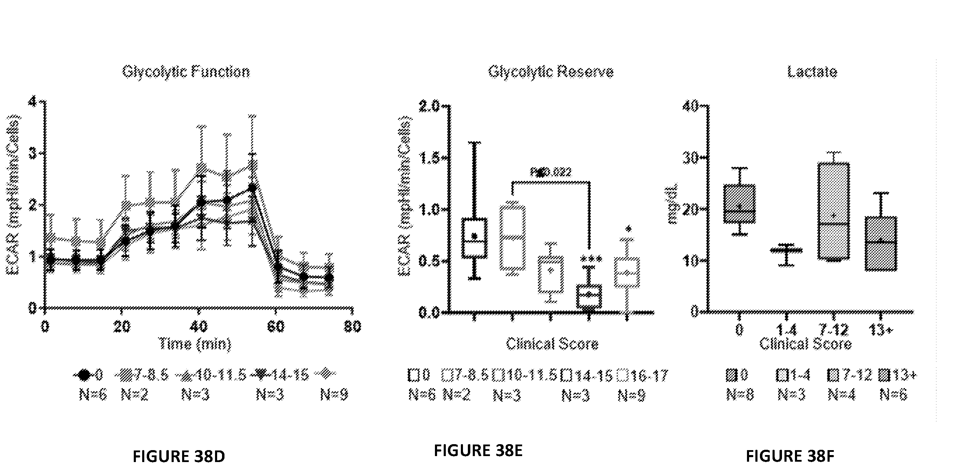

**Figure 38A-38F.** CLP mice are presented with adverse metabolic changes. **(Figure 38A)** 24h after CLP, blood pH significantly decreased with sepsis severity. **(Figure 38B)** OCR measurements of PBMCs from naïve and CLP-mice showed aberrant mitochondrial respiration, predominantly in severely septic mice (MSS Clinical score >10), which was manifested primarily by **(Figure 38C)** a decreased coupling efficiency. **(Figure 38D)** extracellular acidification rate (ECAR) measurements of PBMCs from naive and CLP-mice showed only mild changes in the general glycolytic function, which was slightly increased in moderately septic mice (MSS Clinical Score 7-8.5); **(Figure 38E)** The glycolytic reserve of PBMCs in this assay was significantly decreased in severely septic mice (MSS Clinical score >14). **(Figure 38F)** Blood lactate concentration was slightly lower in CLP-mice. Data in **Figures 38A, 38C, 38E,** and **38F** are presented as the median within the inter-quartile range (IQR); mean values are marked with a '+' sign; error bars represent the 5-95 percentile range; data in **Figures 38B** and **38D** are presented as the mean $\pm$ standard deviation; group sizes (N) are indicated below their respective legends; *$P \leq 0.01$, **$P \leq 0.001$, ***$P \leq 0.0001$ by the Kruskal-Wallis nonparametric ANOVA, with multiple comparisons adjusted using Dunn's test. P values above the bars indicate differences from the control group, and those above the brackets indicate differences between two other groups.

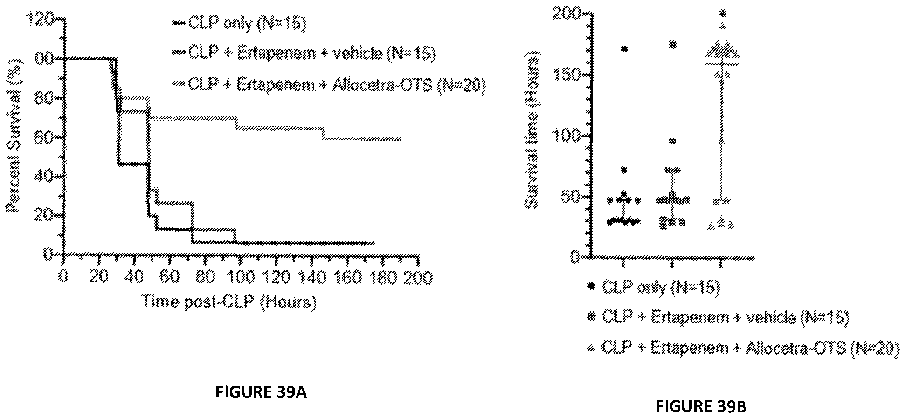

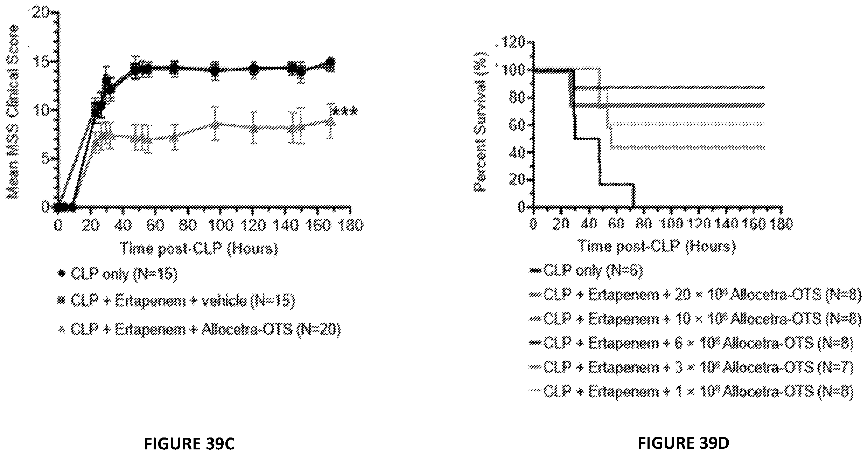

**Figures 39A-39D.** Beneficial effects of Allocetra-OTS on CLP mice. 4 hours after CLP, mice were injected IV with ertapenem and either Hartmann's solution (vehicle) or $20 \times 10^6$ Allocetra-OTS, unless indicated otherwise. Mice were monitored for well-being and euthanized when the MSS Clinical Score was > 15. **(Figure 39A)** Kaplan-Meier survival curves of CLP mice treated with either ertapenem + vehicle or ertapenem + Allocetra-OTS. **(Figure 39B)** Increased median survival time of Allocetra-OTS-treated mice; error bars represent the 95% CI; *$P \leq 0.01$ by the Kruskal-Wallis nonparametric ANOVA, with multiple-comparisons adjusted by using the Dunn's test. **(Figure 39C)** Decreased mean MSS Clinical Score of Allocetra-OTS-treated mice; error bars represent the standard error; ***$P \leq 0.0001$ by ordinary one-way ANOVA of the non-linear curve fits. **(Figure 39D)** Kaplan-Meier survival curves of CLP mice treated with ertapenem + varying doses of Allocetra-OTS. The numbers of mice in each group (N) are indicated beside their respective legends.

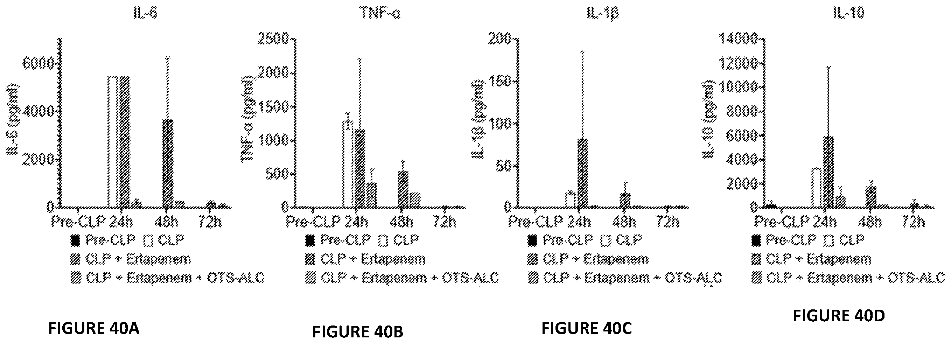

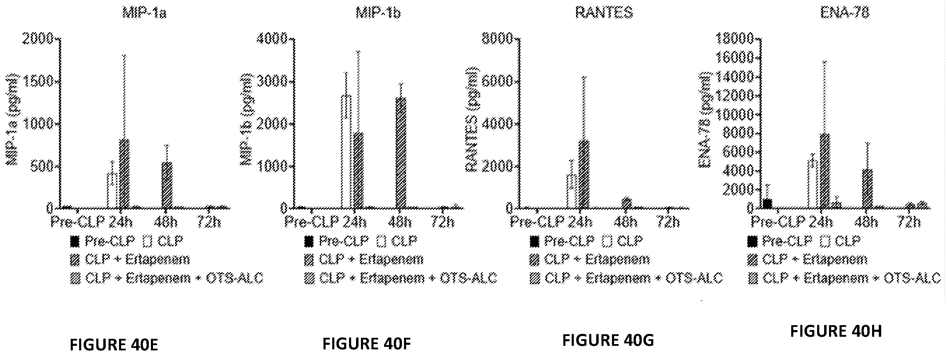

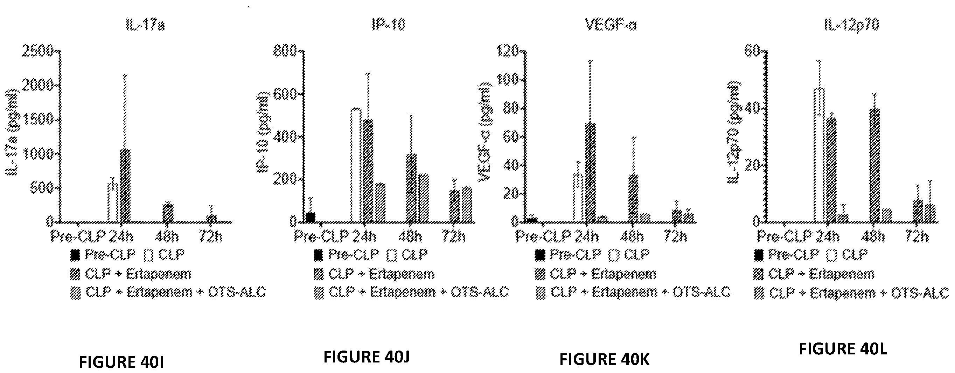

**Figures 40A-40L.** Allocetra-OTS treatment attenuates cytokine/chemokine release following CLP. Blood samples were taken from C57BL/6 mice before CLP and 24h, 48h, and 72h post-CLP, after treatment with either ertapenem or a combination of ertapenem + Allocetra-OTS (OTS-ALC). Non-treated CLP mice did not survive past 24h and therefore, data are not shown. **(Figures 40A-40L)** Cytokine/chemokine levels were measured by Luminex, including: IL-6 **(Figure 40A),** TNF-$\alpha$ **(Figure 40B),** IL-1$\beta$ **(Figure 40C),** IL-10 **(Figure 40D),** MIP-1a **(Figure 40E),** MIP-1b **(Figure 40F),** RANTES **(Figure 40G),** ENA-78 **(Figure 40H),** IL-17a **(Figure 40I),** IP-10 **(Figure 40J),** VEGF-$\alpha$ **(Figure 40K),** and IL-12p70 **(Figure 40L).** Figures 40A-40L demonstrate that both pro-inflammatory and anti-inflammatory cytokine are reduced following treatment of Allocetra-OTS. Data are presented as the mean $\pm$ standard deviation.

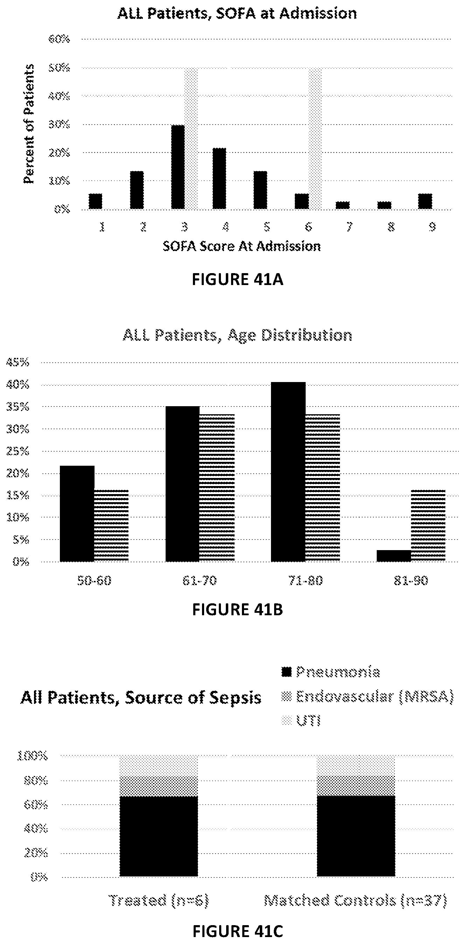

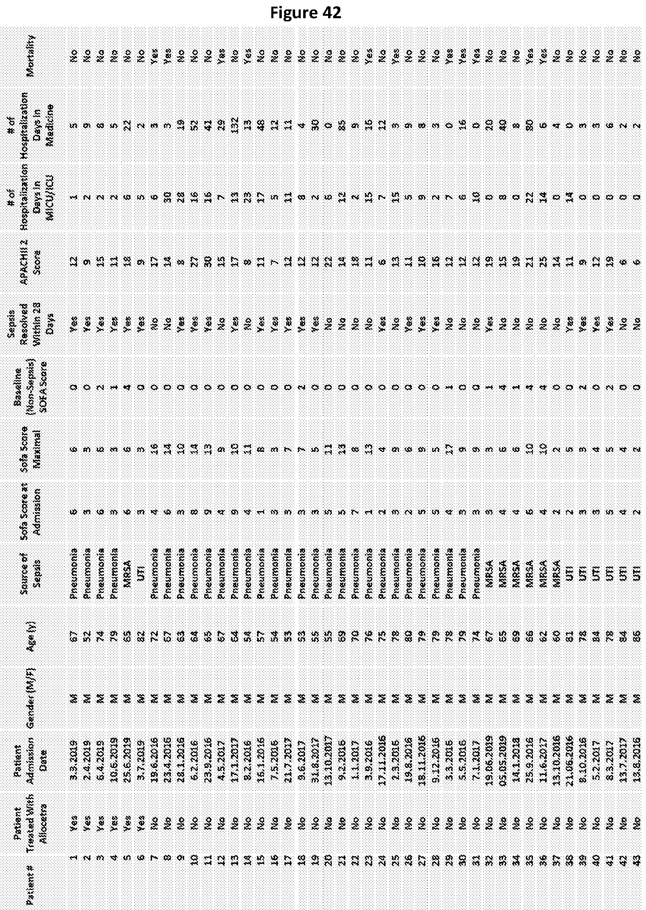

**Figures 41A-41C.** Patients Characteristics - All historical-matched controls between the years 2016-2018, hospitalized in the Medical Intensive Unit at Hadassah Ein Kerem Hospital, Jerusalem, Israel, were reviewed. Historical controls were matched with patients, based on Age ($\pm$3 years), Gender matching, the Sequential Organ Failure Assessment (SOFA) score at admission ($\pm$2), and Source of Sepsis. The probability of survival of the treatment arm of 6 patients based on APACHE II score taken in the first 24 hours of admission (a score that predicts mortality according to general status and chronic diseases), was 52.95%. However, no patient died in the treated group. **Figure 41A** shows the SOFA Score of patients (Treated and Controls) at the time of admission. (Black - Matched controls; Light grey - Treated). **Figure 41B** shows the age distribution of patients at the time of admission. (Black - Matched controls; Striped - Treated). **Figure 41C** shows the percent of patients and their source of sepsis (Treated and Controls) from pneumonia, endovascular Methicillin-resistant Staphylococcus aureus (MRSA), or urinary tract infection (UTI). The Y axis for **Figures 41B and 41C** is Percent of patients. The X-axis for **Figure 41B** is Age. **Figure 42.** Comparative Interim Data: Treated and Untreated Matched Controls Patient Population. **Figure 42** shows in tabular form the comparative interim data of the six (6) treated patients and thirty-seven (37) matched control patients.

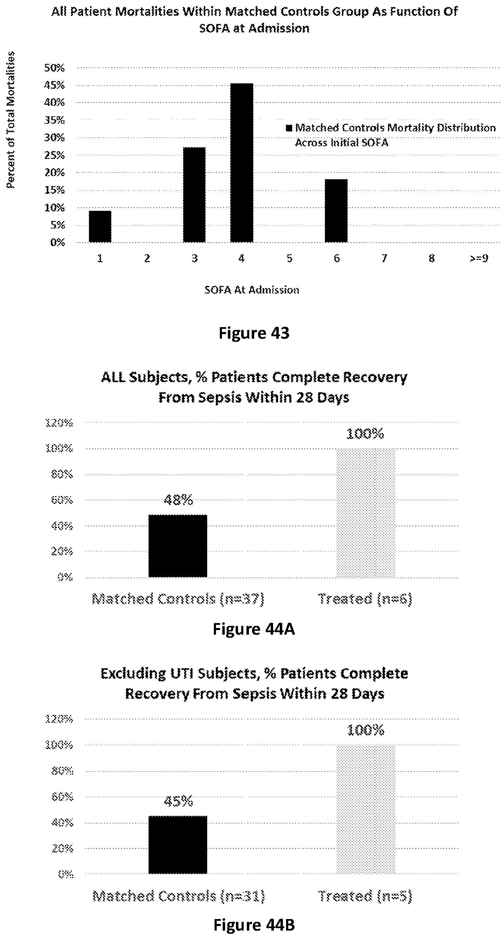

**Figure 43.** Comparative Interim Data: SOFA at Admission & Sepsis-Associated Mortality. **Figure 43** shows that for the Matched-Controls Group, mortalities were associated mostly with low SOFA scores at admission.

**Figures 44A and 44B.** Comparative Interim Data: Recovery from Sepsis. **Figures 44A and 44B** show that Allocetra-OTS is highly effective in treatment of Sepsis. **Figure 44A** shows that 100% of all patients treated with Allocetra-OTS recovered from sepsis within 28 days, independent of the sources of sepsis, which included pneumonia, endovascular Methicillin-resistant Staphylococcus aureus (MRSA), and urinary tract infection (UTI), compared with only 48% of matched controls. **Figure 44B** shows that 100% of sepsis patients (sources of sepsis: pneumonia and endovascular Methicillin-resistant Staphylococcus aureus (MRSA)) treated with Allocetra-OTS recovered from sepsis within 28 days, compared with only 45% of matched controls. (Black - Matched Controls; Light Grey - Treated with Allocetra-OTS)

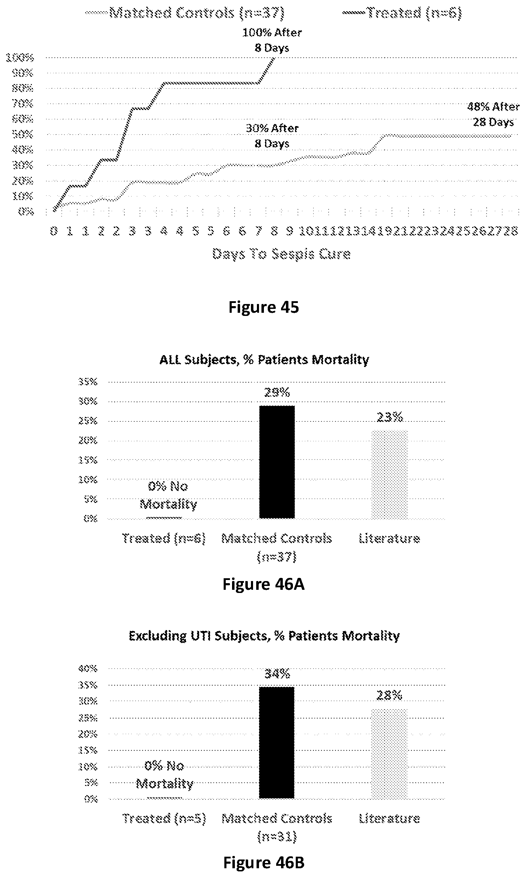

**Figure 45.** Comparative Interim Data: Recovery from Sepsis: Allocetra-OTS is highly effective in treatment of Sepsis Swiftly After Admission. **Figure 45** shows % of patients having complete recovery from sepsis based on the days from admission, wherein 100% of treated patients (dark grey line) recovered by day 8, compared with less than 40% of matched controls (light grey line).

**Figures 46A and 46B.** Comparative Top-Line Data: Recovery from Sepsis: Mortality Data showing that Allocetra-OTS is highly effective in prevention of Sepsis-associated mortality. **Figure 46A** shows no deaths of Allocetra treated patients, independent of the source of sepsis, which included pneumonia, endovascular Methicillin-resistant Staphylococcus aureus (MRSA), and urinary tract infection (UTI), compared with mortality rates of 29% (matched controls) and 23% (literature). **Figure 46B** shows no deaths of Allocetra treated patients, when the source of sepsis included pneumonia and endovascular Methicillin-resistant Staphylococcus aureus (MRSA), compared with mortality rates of 34% (matched controls) and 28% (literature). (Black - Matched Controls; Light Grey - Literature)

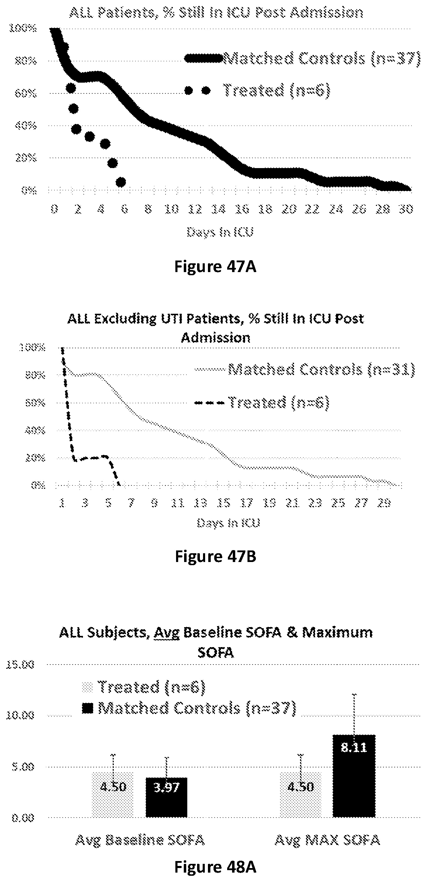

**Figures 47A and 47B.** Comparative Interim Data: Duration of ICU Hospitalization showing that Allocetra-OTS improves patients' clinical state and speeds up release from ICU. **Figure 47A** shows that after 6 days, only 43% of all Matched Controls were released from the ICU, compared with 100% of patients treated with Allocetra-OTS. (Treated - dashed black lines; Matched Control - dotted lines) **Figure 47B** shows that after 6 days, only 35% of Matched Controls (Excluding UTI controls) were released from the ICU, compared with 100% of patients treated with Allocetra-OTS. (Treated - dashed black lines; Matched Control - solid grey lines; Data collection started at 100%)

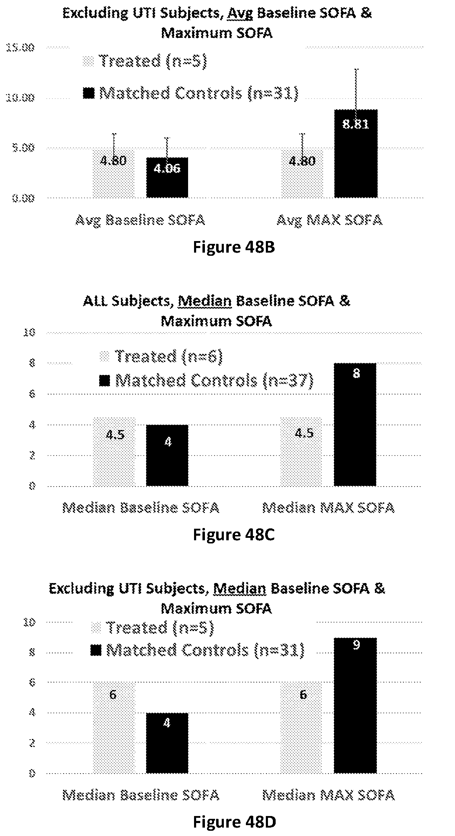

**Figures 48A-48D.** Comparative Interim Data: Organ Dysfunction & Failure showing that Allocetra-OTS prevents organ dysfunction and failure. **Figure 48A** shows the average baseline SOFA and the maximum SOFA for all subjects, independent of the source of sepsis, which included pneumonia, endovascular Methicillin-resistant Staphylococcus aureus (MRSA), and urinary tract infection (UTI) (Treated and Matched Controls). **Figure 48B** shows the average baseline SOFA and the maximum SOFA for subjects (Treated and Matched Controls) excluding the UTI patients. **Figure 48C** shows the median baseline SOFA and the maximum SOFA for all subjects, independent of the source of sepsis, which included pneumonia, endovascular Methicillin-resistant Staphylococcus aureus (MRSA), and urinary tract infection (UTI) (Treated and Matched Controls). There was no difference between treated patients and historical controls at the baseline SOFA but there was a dramatic difference between maximal SOFA, indicating that treated patients did not progress in their sepsis course. **Figure 48D** shows the median baseline SOFA and the maximum SOFA for subjects (Treated and Matched Controls) excluding the UTI patients. (Black - Matched Controls; Light Grey - Treated with Allocetra-OTS)

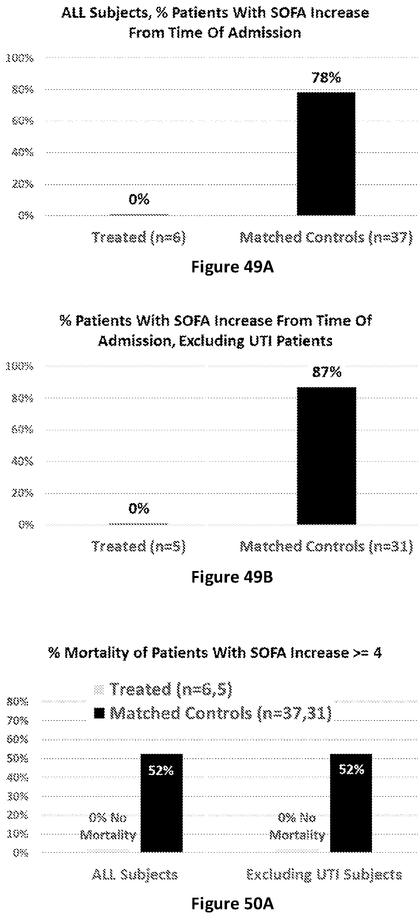

**Figures 49A and 49B.** Comparative Interim Data: Organ Dysfunction & Failure showing that treatment with Allocetra-OTS prevented an increase in SOFA scores. **Figure 49A** shows the percent of all patients with increased SOFA scores from time of admission, independent of the source of sepsis, which included pneumonia, endovascular Methicillin-resistant Staphylococcus aureus (MRSA), and urinary tract infection (UTI). **Figure 49B** shows the percent of patients with increased SOFA scores from time of admission, wherein the source of sepsis included pneumonia and endovascular Methicillin-resistant Staphylococcus aureus (MRSA). (Black - Matched Controls; Light Grey - Allocetra-OTS Treated)

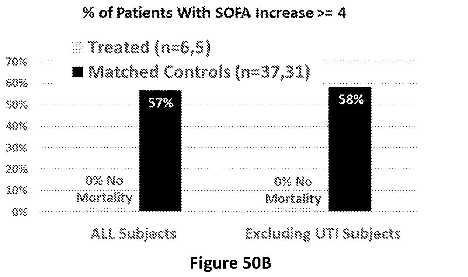

**Figures 50A and 50B.** Comparative Interim Data: Organ Dysfunction & Failure showing that prevention of SOFA increase by 4 or more points is critical to prevent mortality and that treatment with Allocetra-OTS prevents SOFA increase by 4 or more points. **Figure 50A** shows the percent of mortality of patients with a SOFA increase of greater than or equal to 4. **Figure 50B** shows the percent of patients with a SOFA increase of greater than or equal to 4. (Black - Matched Controls; Light Grey - Allocetra-OTS Treated)

## DETAILED DESCRIPTION

In the following detailed description, numerous specific details are set forth in order to provide a thorough understanding of the disclosure herein. However, it will be understood by those skilled in the art that the invention may be practiced without these specific details. In other instances, well-known methods, procedures, and components have not been described in detail so as not to obscure the invention disclosed herein.

The present invention provides a composition comprising an early apoptotic cell population for use in treating, preventing, inhibiting, or reducing the incidence of organ failure in a subject having sepsis, wherein said population comprises a mononuclear enriched early apoptotic cell population, optionally wherein said population comprises a mononuclear apoptotic cell population comprising a decreased percentage of non-quiescent non-apoptotic cells, a suppressed cellular activation of any living non-apoptotic cells, or a reduced proliferation of any living non-apoptotic cells, or any combination thereof.

In some embodiments, the compositions comprise early apoptotic cells in combination with an additional agent. In some embodiments, the additional agent may be an antibody. In some embodiments, the antibody comprises rituximab or a functional fragment thereof.

In some embodiments, compositions of early apoptotic cells comprise a population of mononuclear apoptotic cell comprising mononuclear cells in an early-apoptotic state, wherein said mononuclear apoptotic cell population comprises: a decreased percent of non-quiescent non-apoptotic viable cells; a suppressed cellular activation of any living non-apoptotic cells; or a reduced proliferation of any living non-apoptotic cells; or any combination thereof.

In some embodiments, a pooled mononuclear apoptotic cell preparation comprises mononuclear cells in an early apoptotic state, wherein said pooled mononuclear apoptotic cells preparation comprises pooled individual mononuclear cell populations, and wherein said pooled mononuclear apoptotic cell preparation comprises a decreased percent of living non-apoptotic cells, a suppressed cellular activation of any living non-apoptotic cells, or a reduced proliferation of any living non-apoptotic cells, or any combination thereof. In another embodiment, the pooled mononuclear apoptotic cells have been irradiated. In another embodiment, the pooled mononuclear apoptotic cell preparation uses the white blood cell fraction (WBC) obtained from donated blood. Often this WBC fraction is discarded at blood banks or is targeted for use in research.

In some embodiments, a cell population disclosed herein is inactivated. In another embodiment, inactivation comprises irradiation. In another embodiment, inactivation comprises T-cell receptor inactivation. In another embodiment, inactivation comprises T-cell receptor editing. In another embodiment, inactivation comprises suppressing or eliminating an immune response in said preparation. In another embodiment, inactivation comprises suppressing or eliminating cross-reactivity between multiple individual populations comprised in the preparation. In other embodiment, inactivation comprises reducing or eliminating T-cell receptor activity between multiple individual populations comprised in the preparation. In another embodiment, an inactivated cell preparation comprises a decreased percent of living non-apoptotic cells, suppressed cellular activation of any living non-apoptotic cells, or a reduce proliferation of any living non-apoptotic cells, or any combination thereof.

In another embodiment, an inactivated cell population comprises a reduced number of non-quiescent non-apoptotic cells compared with a non-radiated cell preparation. In some embodiments, an inactivated cell population comprises 50 percent (%) of living non-apoptotic cells. In some embodiments, an inactivated cell population comprises 40% of living non-apoptotic cells. In some embodiments, an inactivated cell population comprises 30% of living non-apoptotic cells. In some embodiments, an inactivated cell population comprises 20% of living non-apoptotic cells. In some embodiments, an inactivated cell population comprises 100% of living non-apoptotic cells. In some embodiments, an inactivated cell population comprises 0% of living non-apoptotic cells.

Disclosed herein but not forming part of the present invention is a method for producing a population of mononuclear apoptotic cell comprising a decreased percent of non-quiescent non-apoptotic viable cells; a suppressed cellular activation of any living non-apoptotic cells; or a reduced proliferation of any living non-apoptotic cells; or any combination thereof, said method comprising the following steps,

obtaining a mononuclear-enriched cell population of peripheral blood;
freezing said mononuclear-enriched cell population in a freezing medium comprising an anticoagulant;
thawing said mononuclear-enriched cell population;
incubating said mononuclear-enriched cell population in an apoptosis inducing incubation medium comprising methylprednisolone at a final concentration of about 10-100 $\mu$g/mL and an anticoagulant;
resuspending said apoptotic cell population in an administration medium; and
inactivating said mononuclear-enriched population, wherein said inactivation occurs following induction,

wherein said method produces a population of mononuclear apoptotic cell comprising a decreased percent of non-quiescent non-apoptotic cells; a suppressed cellular activation of any living non-apoptotic cells; or a reduced proliferation of any living non-apoptotic cells; or any combination thereof.

In another embodiment, the irradiation comprises gamma irradiation or UV irradiation. In yet another embodiment, the irradiated preparation has a reduced number of non-quiescent non-apoptotic cells compared with a non-irradiated cell preparation.

In another embodiment, the pooled mononuclear apoptotic cells have undergone T-cell receptor inactivation. In another embodiment, the pooled mononuclear apoptotic cells have undergone T-cell receptor editing.

Pooled blood may comprise 3rd party blood from HLA matched or HLA unmatched sources, with respect to a recipient.

Disclosed herein but not part of the present invention are methods of production of a pharmaceutical composition comprising a pooled mononuclear apoptotic cell preparation comprising pooled individual mononuclear cell populations in an early apoptotic state, wherein said composition comprises a decreased percent of living non-apoptotic cells, a preparation having a suppressed cellular activation of any living non-apoptotic cells, or a preparation having reduced proliferation of any living non-apoptotic cells, or any combination thereof. The methods can provide a pharmaceutical composition which comprises a pooled mononuclear apoptotic cell preparation comprising pooled individual mononuclear cell populations in an early apoptotic state, wherein said composition comprises a decreased percent of non-quiescent non-apoptotic cells.

*Cytokine Storm and Cytokine Release Syndrome*

A skilled artisan would appreciate that decreasing toxic cytokine release or toxic cytokine levels comprises decreasing or inhibiting production of toxic cytokine levels in a subject, or inhibiting or reducing the incidence of cytokine release syndrome or a cytokine storm in a subject. In another embodiment, toxic cytokine levels are reduced during CRS or a cytokine storm. In another embodiment, decreasing or inhibiting the production of toxic cytokine levels comprises treating CRS or a cytokine storm. In another embodiment, decreasing or inhibiting the production of toxic cytokine levels comprises preventing CRS or a cytokine storm. In another embodiment, decreasing or inhibiting the production of toxic cytokine levels comprises alleviating CRS or a cytokine storm. In another embodiment, decreasing or inhibiting the production of toxic cytokine levels comprises ameliorating CRS or a cytokine storm. In another embodiment, the toxic cytokines comprise pro-inflammatory cytokines. In another embodiment, pro-inflammatory cytokines comprise IL-6. In another embodiment, pro-inflammatory cytokines comprise IL-1β. In another embodiment, pro-inflammatory cytokines comprise TNF-α, In another embodiment, pro-inflammatory cytokines comprise IL-6, IL-1β, or TNF-α, or any combination thereof.

In one embodiment, cytokine release syndrome is characterized by elevated levels of several inflammatory cytokines and adverse physical reactions in a subject such as low blood pressure, high fever and shivering. In another embodiment, inflammatory cytokines comprise IL-6, IL-1β, and TNF-α. In another embodiment, CRS is characterized by elevated levels of IL-6, IL-1β, or TNF-α, or any combination thereof. In another embodiment, CRS is characterized by elevated levels of IL-8, or IL-13, or any combination thereof. In another embodiment, a cytokine storm is characterized by increases in TNF-alpha, IFN-gamma, IL-1beta, IL-2, IL-6, IL-8, IL-10, IL-13, GM-CSF, IL-5, fracktalkine, or a combination thereof or a subset thereof. In yet another embodiment, IL-6 comprises a marker of CRS or cytokine storm.

In another embodiment, cytokines increased in CRS or a cytokine storm in humans and mice may comprise any combination of cytokines listed in Tables 1 and 2 below.

**Table 1: Panel of Cytokines Increased in CRS or Cytokine Storm in Humans and/or Mice**

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| Flt-3L | * | | | | DC (?) | |
| Fractalkine | * | | | | APC, Endothelial cells (?) | = CX3CL1, Neurotactin (Mouse) |
| M-CSF | | | | | | = CSF1 |
| GM-CSF | * | | | * *(in vitro)* | T cell, MØ | |
| IFN-alpha | * | | | | T cell, MØ, Monocyte | |
| IFN-beta | ? | | | ? | T cell, MØ, Monocyte | |

(continued)

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| IFN-gamma | * | * | | * (in vitro) | cytotoxic T cells, helper T cells, NK cells, MØ, Monocyte, DC | |
| IL- 1 alpha | * | | | | Monocyte, MØ, Epithel | |
| IL- 1 beta | * | | | * | Macrophages, DCs, fibroblasts, endothelial cells, hepatocytes | |
| IL-1R alpha | * | | | | | |
| IL- 2 | * | * | | * (in vitro) | T cells | |
| IL- 2R alpha | * | | | | lymphocytes | |
| IL- 4 | * | * | | * (in vitro) | Th2 cells | |
| IL- 5 | * | * | | * | T cells | |
| IL- 6 | * | | * | * | monocytes/ macrophages, dendritic cells, T cells, fibroblasts, keratinocytes, endothelial cells, adipocytes, myocytes, mesangial cells, and osteoblasts | |
| IL- 7 | * | | | * | *In vitro* by BM stromal cells | |
| IL- 8 | * | | | | Macrophages, monocytes | |
| IL- 9 | * | * | | | T cells, T helper | |
| IL- 10 | * | * | * | * (in vitro) | monocytes/macrophag es, mast cells, B cells, regulatory T cells, and helper T cells | |
| IL- 12 | * | | | * | MØ, Monocyte, DC, activated lymphocytes, neutrophils | = p70 (p40+p35) |
| IL- 13 | * | * | | | T cells | |

In some embodiments, cytokines Flt-3L, Fractalkine, GM-CSF, IFN-γ, IL-1β, IL-2, IL-2Rα, IL-4, IL-5, IL-6, IL-7, IL-8, IL-9, IL-10, IL-12, and IL-13 of Table 1 are considered to be significant in CRS or cytokine storm. In another embodiment, IFN-α, IFN-β, IL-1, and IL-1Rα of Table 1 appear to be important in CRS or cytokine storm. In another embodiment, M-CSF has unknown importance. In another embodiment, any cytokine listed in Table 1, or combination thereof, may be used as a marker of CRS or cytokine storm.

**Table 2: Panel of Cytokines Increased in CRS or Cytokine Storm in Humans and/or Mice**

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| IL- 15 | * | | | * | Fibroblasts, monocytes (?) | 22 |
| IL- 17 | * | | | * | T cells | |
| IL- 18 | | | | | Macrophages | |
| IL- 21 | * | | | | T helper cells, NK cells | |

(continued)

| Cytokine (Analyte) | Human model (clinical trials) | Mouse model (pre-clinical) | | | Cells secreting this cytokine | Notes / other |
|---|---|---|---|---|---|---|
| | | CAR-T (H) origin | Mouse origin | Not specified | | |
| IL- 22 | * | | | | activated DC and T cells | |
| IL- 23 | | | | | | |
| IL- 25 | | | | | | Protective? |
| IL- 27 | * | | | | APC | |
| IP-10 | * | | | | Monocytes (?) | |
| MCP-1 | * | | | | Endothel, fibroblast, epithel, monocytes | = CXCL10 |
| MCP-3 | * | | | | **PBMCs, MØ** (?) | = CCL2 |
| MIP-1α | * | | | * (in vitro) | T cells | = CXCL9 |
| MIP-1β | * | | | | T cells | = CCL3 |
| PAF | ? | | | | platelets, endothelial cells, neutrophils, monocytes, and macrophages, mesangial cells | = CCL4 |
| PGE2 | * | | | * | Gastrointestinal mucosa and other | |
| RANTES | * | | | | Monocytes | |
| TGF-β | * | | | * | MØ, lymphocytes, endothel, platelets ... | = CCL5 |
| TNF-α | * | * | * | * (in vitro) | Macrophages, NK cells, T cells | |
| TNF-αR1 | * | | | | | |
| HGF | | | | | | |
| MIG | * | | | | T cell chemoattractant, induced by IFN-γ | |

In one embodiment, IL-15, IL-17, IL-18, IL-21, IL-22, IP-10, MCP-1, MIP-1α, MIP-1β, and TNF-α of Table 2 are considered to be significant in CRS or cytokine storm. In another embodiment, IL-27, MCP-3, PGE2, RANTES, TGF-β, TNF-αR1, and MIG of Table 2 appear to be important in CRS or cytokine storm. In another embodiment, IL-23 and IL-25 have unknown importance. In another embodiment, any cytokine listed in Table 2, or combination thereof, may be used as a marker of CRS or cytokine storm. In another embodiment, mouse cytokines IL-10, IL-1β, IL-2, IP-10, IL-4, IL-5, IL-6, IFNα, IL-9, IL-13, IFN-γ, IL-12p70, GM-CSF, TNF-α, MIP-1α, MIP-1β, IL-17A, IL-15/IL-15R and IL-7 appear to be important in CRS or cytokine storm.

A skilled artisan would appreciate that the term "cytokine" may encompass cytokines (e.g., interferon gamma (IFN-γ), granulocyte macrophage colony stimulating factor, tumor necrosis factor alpha), chemokines (e.g., MIP 1 alpha, MIP 1 beta, RANTES), and other soluble mediators of inflammation, such as reactive oxygen species and nitric oxide.

In one embodiment, increased release of a particular cytokine, whether significant, important or having unknown importance, does not a priori mean that the particular cytokine is part of a cytokine storm. In one embodiment, an increase of at least one cytokine is not the result of a cytokine storm or CRS. In another embodiment, CAR T-cells may be the source of increased levels of a particular cytokine or group of cytokines.

In another embodiment, cytokine release syndrome is characterized by any or all of the following symptoms: Fever with or without rigors, malaise, fatigue, anorexia, myalgias, arthalgias, nausea, vomiting, headache Skin Rash, Nausea, vomiting, diarrhea, Tachypnea, hypoxemia Cardiovascular Tachycardia, widened pulse pressure, hypotension, increased cardiac output (early), potentially diminished cardiac output (late), Elevated D-dimer, hypofibrinogenemia with or without bleeding, Azotemia Hepatic Transaminitis, hyperbilirubinemia, Headache, mental status changes, confusion, delirium, word finding difficulty or frank aphasia, hallucinations, tremor, dymetria, altered gait, seizures. In another embodiment, a cytokine storm is characterized by IL-2 release and lymphoproliferation. In another embodiment, a cytokine storm is characterized by increases in cytokines released by CAR T-cells. In another embodiment, a cytokine storm is characterized by increases in cytokines released by cells other than CAR T-cells.

In another embodiment, cytokine storm leads to potentially life-threatening complications including cardiac dysfunction, adult respiratory distress syndrome, neurologic toxicity, renal and/or hepatic failure, and disseminated intravascular coagulation.

A skilled artisan would appreciate that the characteristics of a cytokine release syndrome (CRS) or cytokine storm are estimated to occur a few days to several weeks following the trigger for the CRS or cytokine storm. In one embodiment, CAR T-cells are a trigger for CRS or a cytokine storm. In another embodiment, a trigger for CRS or a cytokine storm is not CAR T-cells.

In one embodiment, measurement of cytokine levels or concentration, as an indicator of cytokine storm, may be expressed as -fold increase, per cent (%) increase, net increase or rate of change in cytokine levels or concentration. In another embodiment, absolute cytokine levels or concentrations above a certain level or concentration may be an indication of a subject undergoing or about to experience a cytokine storm. In another embodiment, absolute cytokine levels or concentration at a certain level or concentration, for example a level or concentration normally found in a control subject not undergoing CAR-T cell therapy, may be an indication for inhibiting or reducing the incidence of a cytokine storm in a subject undergoing CAR T-cell.

A skilled artisan would appreciate that the term "cytokine level" may encompass a measure of concentration, a measure of fold change, a measure of percent (%) change, or a measure of rate change. Further, the methods for measuring cytokines in blood, saliva, serum, urine, and plasma are well known in the art.

In one embodiment, despite the recognition that cytokine storm is associated with elevation of several inflammatory cytokines, IL-6 levels may be used as a common measure of cytokine storm and/or as a common measure of the effectiveness of a treatment for cytokine storms. A skilled artisan would appreciate that other cytokines may be used as markers of a cytokine storm, for example TNF-$\alpha$, IB-1$\alpha$, IL-8, IL-13, or INF-$\gamma$. Further, assay methods for measuring cytokines are well known in the art. A skilled artisan would appreciate that methods affecting a cytokine storm may similarly affect cytokine release syndrome.

In one embodiment, the composition decreases or inhibits cytokine production in a subject experiencing cytokine release syndrome or a cytokine storm. In another embodiment, the composition decreases or inhibits cytokine production in a subject vulnerable to experiencing cytokine release syndrome or a cytokine storm. In another embodiment, the composition decreases or inhibits cytokine production in a subject experiencing cytokine release syndrome or a cytokine storm, wherein production of any cytokine or group of cytokines listed in Tables 1 and/or 2 is decreased or inhibited. In another embodiment, cytokine IL-6 production is decreased or inhibited. In another embodiment, cytokine IL-beta1 production is decreased or inhibited. In another embodiment, cytokine IL-8 production is decreased or inhibited. In another embodiment, cytokine IL-13 production is decreased or inhibited. In another embodiment, cytokine TNF-alpha production is decreased or inhibited. In another embodiment, cytokines IL-6 production, IL-1beta production, or TNF-alpha production, or any combination thereof is decreased or inhibited.

In one embodiment, cytokine release syndrome is graded. In another embodiment, Grade 1 describes cytokine release syndrome in which symptoms are not life threatening and require symptomatic treatment only, e.g., fever, nausea, fatigue, headache, myalgias, malaise. In another embodiment, Grade 2 symptoms require and respond to moderate intervention, such as oxygen, fluids or vasopressor for hypotension. In another embodiment, Grade 3 symptoms require and respond to aggressive intervention. In another embodiment, Grade 4 symptoms are life-threatening symptoms and require ventilator and patients display organ toxicity.

In another embodiment, a cytokine storm is characterized by IL-6 and interferon gamma release. In another embodiment, a cytokine storm is characterized by release of any cytokine or combination thereof, listed in Tables 1 and 2. In another embodiment, a cytokine storm is characterized by release of any cytokine or combination thereof, known in the art.

In one embodiment, symptoms onset begins minutes to hours after the infusion begins. In another embodiment, symptoms coincide with peak cytokine levels.

Any appropriate method of quantifying cytotoxicity can be used to determine whether activity in an immune cell modified to express a CAR remains substantially unchanged. For example, cytotoxicity can be quantified using a cell culture-based assay such as the cytotoxic assays described in the Examples. Cytotoxicity assays can employ dyes that preferentially stain the DNA of dead cells. In other cases, fluorescent and luminescent assays that measure the relative number of live and dead cells in a cell population can be used. For such assays, protease activities serve as markers for cell viability and cell toxicity, and a labeled cell permeable peptide generates fluorescent signals that are proportional to the number of viable cells in the sample. For example a cytotoxicity assay may use 7-AAD in a flow cytometry analysis. Kits for various cytotoxicity assays are commercially available from manufacturers such as Promega, Abcam, and Life Technologies.

In another embodiment, a measure of cytotoxicity may be qualitative. In another embodiment, a measure of cytotoxicity may be quantitative. In a further embodiment, a measure of cytotoxicity may be related to the change in expression of a cytotoxic cytokine. In another embodiment, a measure of cytotoxicity may be determined by survival curve and tumor load in bone marrow and liver.

In one embodiment, apoptotic cells are heterologous to the subject. In one embodiment, apoptotic cells are derived from one or more donors. In one embodiment, apoptotic cells are derived from one or more bone marrow donors. In another embodiment, apoptotic cells are derived from one or more blood bank donations. In one embodiment, the donors are matched donors. In another embodiment, apoptotic cells are from unmatched third party donors. In one embodiment, apoptotic cells are universal allogeneic apoptotic cells. In another embodiment, apoptotic cells are from a syngeneic donor. In another embodiment, apoptotic cells are from pooled third party donor cells. In one embodiment, the donor is a bone marrow donor. In another embodiment, the donor is a blood bank donor. In another embodiment, apoptotic cells are autologous to the subject. In this embodiment, the patient's own cells are used.

According to some embodiments, the therapeutic mononuclear-enriched cell preparation disclosed herein is administered to the subject systemically. In another embodiment, administration is via the intravenous route. Alternately, the therapeutic mononuclear enriched cell may be administered to the subject according to various other routes, including the parenteral, intraperitoneal, intra-articular, intramuscular and subcutaneous routes.

According to some embodiments, the therapeutic mononuclear-enriched cell preparation disclosed herein or the additional agent is administered to the subject systemically. In another embodiment, administration is via the intravenous route. Alternately, the therapeutic mononuclear enriched cell or the additional agent may be administered to the subject according to various other routes, including the parenteral, intraperitoneal, intra-articular, intramuscular and subcutaneous routes.

In one embodiment, the preparation is administered in a local rather than systemic manner, for example, via injection of the preparation directly into a specific region of a patient's body.

In another embodiment, the therapeutic mononuclear enriched cells are administered to the subject suspended in a suitable physiological buffer, such as saline solution, PBS, HBSS, and the like. In addition the suspension medium may further comprise supplements conducive to maintaining the viability of the cells. In another embodiment, the additional agent is administered to the subject suspended in a suitable physiological buffer, such as saline solution, PBS, HBSS, and the like.

According to some embodiments, the pharmaceutical composition is administered intravenously. According to another embodiment, the pharmaceutical composition is administered in a single dose. According to alternative embodiments, the pharmaceutical composition is administered in multiple doses. According to another embodiment, the pharmaceutical composition is administered in two doses. According to another embodiment, the pharmaceutical composition is administered in three doses. According to another embodiment, the pharmaceutical composition is administered in four doses. According to another embodiment, the pharmaceutical composition is administered in five or more doses. According to some embodiments, the pharmaceutical composition is formulated for intravenous injection.

*Cytokine Release Associated with Non CAR T-cell Applications*

In one embodiment, the composition decreases or inhibits cytokine production in a subject experiencing cytokine release syndrome or cytokine storm or vulnerable to cytokine release syndrome or cytokine storm. In another embodiment, decrease or inhibition of cytokine production is compared with a subject experiencing cytokine release syndrome or cytokine storm or vulnerable to cytokine release syndrome or cytokine storm and not administered apoptotic cells. In another embodiment, decreasing or inhibiting cytokine production decreases or inhibits pro-inflammatory cytokine production. In another embodiment, decreasing or inhibiting cytokine production decreases or inhibits production of at least one pro-inflammatory cytokine. In another embodiment, decreasing or inhibiting cytokine production decreases or inhibits production of at least cytokine IL-6. In another embodiment, decreasing or inhibiting cytokine production decreases or inhibits production of at least cytokine IL-1beta. In another embodiment, decreasing or inhibiting cytokine production decreases or inhibits production of at least cytokine TNF-alpha. In another embodiment, decreasing or inhibiting cytokine production results in reduction or inhibition of production of cytokines IL-6, IL-1$\beta$, or TNF-$\alpha$, or any combination in said subject compared with a subject experiencing cytokine release syndrome or cytokine storm or vulnerable to cytokine release syndrome or cytokine storm and not administered apoptotic cells.

Cancers or tumors may also affect the absolute level of cytokines including pro-inflammatory cytokines. The level of tumor burden in a subject may affect cytokine levels, particularly pro-inflammatory cytokines. A skilled artisan would appreciate that the phrase "decrease or inhibit" or grammatical variants thereof may encompass fold decrease or inhibition of cytokine production, or a net decrease or inhibition of cytokine production, or percent (%) decrease or inhibition, or may encompass a rate of change of decrease or inhibition of a cytokine production.

In another embodiment, the composition decreases or inhibits cytokine production in a subject experiencing cytokine release syndrome or cytokine storm or vulnerable to cytokine release syndrome or cytokine storm.

In one embodiment, an infection causes the cytokine release syndrome or cytokine storm in the subject. In one embodiment, the infection is an influenza infection. In one embodiment, the influenza infection is H1N1. In another embodiment, the influenza infection is an H5N1 bird flu. In another embodiment, the infection is severe acute respiratory syndrome (SARS). In another embodiment, the subject has Epstein-Barr virus-associated hemophagocytic lymphohistiocytosis (HLH). In one embodiment, the sepsis is gram-negative. In another embodiment, the infection is malaria. In another embodiment, the infection is an Ebola virus infection. In another embodiment, the infection is variola virus. In another embodiment, the infection is a systemic Gram-negative bacterial infection. In another embodiment, the infection is Jarisch-Herxheimer syndrome.

In one embodiment, the cause of the cytokine release syndrome or cytokine storm in a subject is hemophagocytic lymphohistiocytosis (HLH). In another embodiment, HLH is sporadic HLH. In another embodiment, HLH is macrophage activation syndrome (MAS). In another embodiment, the cause of the cytokine release syndrome or cytokine storm in a subject is MAS.

In one embodiment, the cause of the cytokine release syndrome or cytokine storm in a subject is chronic arthritis. In another embodiment, the cause of the cytokine release syndrome or cytokine storm in a subject is systemic Juvenile Idiopathic Arthritis (sJIA), also known as Still's Disease.

In one embodiment, the cause of the cytokine release syndrome or cytokine storm in a subject is Cryopyrin-associated Periodic Syndrome (CAPS). In another embodiment, CAPS comprises Familial Cold Auto-inflammatory Syndrome (FCAS), also known as Familial Cold Urticaria (FCU). In another embodiment, CAPS comprises Muckle-Well Syndrome (MWS). In another embodiment, CAPS comprises Chronic Infantile Neurological Cutaneous and Articular (CINCA) Syndrome. In yet another embodiment, CAPS comprises FCAS, FCU, MWS, or CINCA Syndrome, or any combination thereof. In another embodiment, the cause of the cytokine release syndrome or cytokine storm in a subject is FCAS. In another embodiment, the cause of the cytokine release syndrome or cytokine storm in a subject is FCU. In another embodiment, the cause of the cytokine release syndrome or cytokine storm in a subject is MWS. In another embodiment, the cause of the cytokine release syndrome or cytokine storm in a subject is CINCA Syndrome. In still another embodiment, the cause of the cytokine release syndrome or cytokine storm in a subject is FCAS, FCU, MWS, or CINCA Syndrome, or any combination thereof.

In another embodiment, the cause of the cytokine release syndrome or cytokine storm in a subject is a cryopyrinopathy comprising inherited or de novo gain of function mutations in the NLRP3 gene, also known as the CIAS1 gene.

In one embodiment, the cause of the cytokine release syndrome or cytokine storm in a subject is a hereditary auto-inflammatory disorder.

In one embodiment, the trigger for the release of inflammatory cytokines is a lipopolysaccharide (LPS), Gram-positive toxins, fungal toxins, glycosylphosphatidylinositol (GPI) or modulation of RIG-1 gene expression.

In another embodiment, the subject experiencing cytokine release syndrome or cytokine storm does not have an infectious disease. In one embodiment, the subject has acute pancreatitis. In another embodiment, the subject has tissue injury, which in on embodiment, is severe burns or trauma. In another embodiment, the subject has acute respiratory distress syndrome. In another embodiment, the subject has cytokine release syndrome or cytokine storm secondary to drug use. In another embodiment, the subject has cytokine release syndrome or cytokine storm secondary to toxin inhalation.

In another embodiment, the subject has cytokine release syndrome or cytokine storm secondary to receipt of immunotherapy, which in one embodiment is immunotherapy with superagonistic CD28-specific monoclonal antibodies (CD28SA). In one embodiment, the CD28SA is TGN1412. In another embodiment, the immunotherapy is CAR T-cell therapy.

Examples of diseases for which control of inflammatory cytokine production can be beneficial include cancers, allergies, any type of infection, toxic shock syndrome, sepsis, any type of autoimmune disease, arthritis, Crohn's disease, lupus, psoriasis, or any other disease for which the hallmark feature is toxic cytokine release that causes deleterious effects in a subject.

*Sepsis*

The present invention provides a composition comprising an early apoptotic cell population for use in treating, preventing, inhibiting, or reducing the incidence of organ failure in a subject having sepsis, wherein said population comprises a mononuclear enriched early apoptotic cell population, optionally wherein said population comprises a mononuclear apoptotic cell population comprising a decreased percentage of non-quiescent non-apoptotic cells, a suppressed cellular activation of any living non-apoptotic cells, or a reduced proliferation of any living non-apoptotic cells, or any combination thereof.

In some embodiments, sepsis comprises severe sepsis. In some embodiments, sepsis comprises mild sepsis. In some embodiments, sepsis comprises acute sepsis. In some embodiments, sepsis comprises highly aggressive sepsis.

In some embodiments, the source of sepsis comprises pneumonia. In some embodiments, the source of sepsis comprises endovascular Methicillin-resistant Staphylococcus aureus (MRSA). In some embodiments, the source of sepsis comprises a urinary tract infection (UTI). In some embodiments, the source of sepsis comprises a biliary tract infection.

In some embodiments, the composition is useful in the prevention, inhibiting, reducing the incidence of organ dysfunction. In some embodiments, the composition is useful in the prevention, inhibiting, reducing the incidence of organ damage. In some embodiments, the composition is useful in the prevention, inhibiting, reducing the incidence of acute multiple organ failure.

In some embodiments, administering early apoptotic cells to a subject suffering from sepsis results in preventing, inhibiting, reducing the incidence of organ failure. In some embodiments, administering early apoptotic cells to a subject suffering from sepsis results in preventing, inhibiting, reducing the incidence of organ dysfunction. In some embodiments, administering early apoptotic cells to a subject suffering from sepsis results in preventing inhibiting, reducing the incidence of organ failure. In some embodiments, administering early apoptotic cells to a subject suffering from sepsis results in preventing, inhibiting, reducing the incidence of organ damage. In some embodiments, administering early apoptotic cells to a subject suffering from sepsis results in preventing inhibiting, reducing the incidence of acute multiple organ failure.

In some embodiments, organ failure during sepsis comprises failure of a vital organ, for example lung, heart, kidney, liver, and blood organs. In some embodiments, multiple organ failure as a component of sepsis comprises failure of a combination of lung, the heart, a kidney, liver, and blood. In some embodiments, hematological aberrations during sepsis comprise thrombocytopenia, lymphopenia, neutropenia, or neutrophilia, or any combination thereof. In some embodiments, organ failure may be measured using standards known in the art including the Sequential Organ Failure Assessment (SOFA) scores. In some embodiments, measurements of sepsis use standards known in the art including the Glasgow coma scale (GCS).

In some embodiments, treating, preventing, inhibiting, reducing the incidence of, ameliorating, or alleviating sepsis in a subject in need comprises prevention, inhibiting, reducing the incidence of organ dysfunction. In some embodiments, treating, preventing, inhibiting, reducing the incidence of, ameliorating, or alleviating sepsis in a subject in need comprises prevention, inhibiting, reducing the incidence of multiple organ dysfunction. In some embodiments, treating, preventing, inhibiting, reducing the incidence of, ameliorating, or alleviating sepsis in a subject in need comprises prevention, inhibiting, reducing the incidence of vital organ dysfunction.

In some embodiments, administering early apoptotic cells to a subject suffering from sepsis results in preventing, inhibiting, reducing the incidence of organ dysfunction. In some embodiments, administering early apoptotic cells to a subject suffering from sepsis results in preventing, inhibiting, reducing the incidence of multiple organ dysfunction. In some embodiments, administering early apoptotic cells to a subject suffering from sepsis results in preventing, inhibiting, reducing the incidence of vital organ dysfunction. In some embodiments, administering early apoptotic cells to a subject suffering from sepsis results in preventing an increase in vital organ dysfunction, compared with subjects not administered early apoptotic cells.

In some embodiments, administering early apoptotic cells to a subject suffering from sepsis is highly effective in the treatment of sepsis. In some embodiments, measure of an effective treatment of sepsis includes the percent of patients that recover from sepsis within a given timeframe. In some embodiments, measure of an effective treatment of sepsis includes the percent of patients that are released from intensive care compared with the percent of patients not administered early apoptotic cells. In some embodiments, a subject suffering from sepsis administered early apoptotic cells recovers more quickly than a subject suffering from sepsis and not administered early apoptotic cells. In some embodiments, a subject suffering from sepsis administered early apoptotic cells recovers more completely than a subject suffering from sepsis and not administered early apoptotic cells. In some embodiments, the mortality rate of patients suffering from sepsis and treated with early apoptotic cells is decreased, compared with patients not administered early apoptotic cells.

In some embodiments, treating, preventing, inhibiting, reducing the incidence of, ameliorating, or alleviating sepsis in a subject in need comprises prevention, inhibiting, reducing the incidence of cardiovascular dysfunction. In some embodiments, treating, preventing, inhibiting, reducing the incidence of, ameliorating, or alleviating sepsis in a subject in need comprises prevention, inhibiting, reducing the incidence of acute kidney injury. In some embodiments, treating, preventing, inhibiting, reducing the incidence of, ameliorating, or alleviating sepsis in a subject in need comprises prevention, inhibiting, reducing the incidence of lung dysfunction. In some embodiments, treating, preventing, inhibiting, reducing the incidence of, ameliorating, or alleviating sepsis in a subject in need comprises prevention, inhibiting, reducing the incidence of liver dysfunction. In some embodiments, treating, preventing, inhibiting, reducing the incidence of, ameliorating, or alleviating sepsis in a subject in need comprises prevention, inhibiting, reducing the incidence of hematological aberrations. In some embodiments, treating, preventing, inhibiting, reducing the incidence of, ameliorating, or alleviating sepsis in a subject in need comprises prevention, inhibiting, reducing the incidence of a combination of any of cardiovascular dysfunction, acute kidney injury, lung dysfunction, and hematological aberrations.

In some embodiments, administering early apoptotic cells to a subject suffering from sepsis results in preventing, inhibiting, reducing the incidence of cardiovascular dysfunction. In some embodiments, administering early apoptotic cells to a subject suffering from sepsis results in preventing, inhibiting, reducing the incidence of acute kidney injury. In some embodiments, administering early apoptotic cells to a subject suffering from sepsis results in preventing, inhibiting, reducing the incidence of lung dysfunction. In some embodiments, administering early apoptotic cells to a subject suffering from sepsis results in preventing, inhibiting, reducing the incidence of liver dysfunction. In some embodiments, administering early apoptotic cells to a subject suffering from sepsis results in preventing, inhibiting, reducing the incidence of hematological aberrations. In some embodiments, administering early apoptotic cells to a subject suffering from sepsis results in preventing, inhibiting, reducing the incidence of a combination of any of cardiovascular dysfunction, acute kidney injury, lung dysfunction, and hematological aberrations.

In some embodiments, treating, preventing, inhibiting, reducing the incidence of, ameliorating, or alleviating sepsis in a subject in need comprises prevention, inhibiting, reducing the incidence of a cytokine storm. In some embodiments, treating, preventing, inhibiting, reducing the incidence of, ameliorating, or alleviating sepsis in a subject in need comprises prevention, inhibiting, reducing the incidence of a chemokine storm. In some embodiments, treating, preventing, inhibiting, reducing the incidence of, ameliorating, or alleviating sepsis in a subject in need comprises prevention, inhibiting, reducing the incidence of a cytokine and chemokine storm.

In some embodiments, administering early apoptotic cells to a subject suffering from sepsis results in preventing, inhibiting, reducing the incidence of a cytokine storm. In some embodiments, administering early apoptotic cells to a subject suffering from sepsis results in preventing, inhibiting, reducing the incidence of a chemokine storm. In some embodiments, administering early apoptotic cells to a subject suffering from sepsis results in preventing, inhibiting, reducing the incidence of a cytokine and chemokine storm.

In some embodiments, treating, preventing, inhibiting, reducing the incidence of, ameliorating, or alleviating sepsis in a subject in need comprises rebalancing the immune response in a subject. In some embodiments, treating, preventing, inhibiting, reducing the incidence of, ameliorating, or alleviating sepsis in a subject in need comprises reducing secretion of pro-inflammatory cytokines. In some embodiments, treating, preventing, inhibiting, reducing the incidence of, ameliorating, or alleviating sepsis in a subject in need comprises reducing secretion of pro-inflammatory cytokines/chemokines and anti-inflammatory cytokines/chemokines.

In some embodiments, administering early apoptotic cells to a subject suffering from sepsis results in rebalancing the immune response in a subject. In some embodiments, administering early apoptotic cells to a subject suffering from sepsis results in reducing secretion of pro-inflammatory cytokines. In some embodiments, administering early apoptotic cells to a subject suffering from sepsis results in reducing secretion of pro-inflammatory cytokines/chemokines and anti-inflammatory cytokines/chemokines.

In some embodiments, treating, preventing, inhibiting, reducing the incidence of, ameliorating, or alleviating sepsis in a subject in need comprises a reduction in mortality of a subject suffering from sepsis. In some embodiments, treating, preventing, inhibiting, reducing the incidence of, ameliorating, or alleviating sepsis in a subject in need comprises improving the survival time in the subject in need.

In some embodiments, treating, preventing, inhibiting, reducing the incidence of, ameliorating, or alleviating sepsis in a subject in need increase the survival time in said subject by greater than 60% compared with a subject not administered apoptotic cells. In some embodiments, treating, preventing, inhibiting, reducing the incidence of, ameliorating, or alleviating sepsis in a subject in need increase the survival time in said subject by greater than 70% compared with a subject not administered apoptotic cells. In some embodiments, treating, preventing, inhibiting, reducing the incidence of, ameliorating, or alleviating sepsis in a subject in need increase the survival time in said subject by greater than 80% compared with a subject not administered apoptotic cells. In some embodiments, treating, preventing, inhibiting, reducing the incidence of, ameliorating, or alleviating sepsis in a subject in need increase the survival time in said subject by greater than 90% compared with a subject not administered apoptotic cells. In some embodiments, treating, preventing, inhibiting, reducing the incidence of, ameliorating, or alleviating sepsis in a subject in need increase the survival time in said subject by greater than 100% compared with a subject not administered apoptotic cells.

In some embodiments, treating, preventing, inhibiting, reducing the incidence of, ameliorating, or alleviating sepsis in a subject in need increase the survival time in said subject by about 50%-100%, compared with a subject not administered apoptotic cells. In some embodiments, treating, preventing, inhibiting, reducing the incidence of, ameliorating, or alleviating sepsis in a subject in need increase the survival time in said subject by about 80%-100%, compared with a subject not administered apoptotic cells. In some embodiments, treating, preventing, inhibiting, reducing the incidence of, ameliorating, or alleviating sepsis in a subject in need increase the survival time in said subject by about 80%, 90%, or 100% compared with a subject not administered apoptotic cells.

In some embodiments, treating, preventing, inhibiting, reducing the incidence of, ameliorating, or alleviating sepsis in a subject in need increase the survival time in said subject by about 100%-2000%, compared with a subject not administered apoptotic cells. In some embodiments, treating, preventing, inhibiting, reducing the incidence of, ameliorating, or alleviating sepsis in a subject in need increase the survival time in said subject by about 200%-300%, compared with a subject not administered apoptotic cells. In some embodiments, treating, preventing, inhibiting, reducing the incidence of, ameliorating, or alleviating sepsis in a subject in need increase the survival time in said subject by greater than 100% compared with a subject not administered apoptotic cells. In some embodiments, treating, preventing, inhibiting, reducing the incidence of, ameliorating, or alleviating sepsis in a subject in need increase the survival time in said subject by greater than 200% compared with a subject not administered apoptotic cells. In some embodiments, treating, preventing, inhibiting, reducing the incidence of, ameliorating, or alleviating sepsis in a subject in need increase the survival time in said subject by greater than 300% compared with a subject not administered apoptotic cells. In some embodiments, treating, preventing, inhibiting, reducing the incidence of, ameliorating, or alleviating sepsis in a subject in need increase the survival time in said subject by greater than 400% compared with a subject not administered apoptotic cells. In some embodiments, treating, preventing, inhibiting, reducing the incidence of, ameliorating, or alleviating sepsis in a subject in need increase the survival time in said subject by greater than 500%, 600%, 700%, 800%, 900%, or 1000% compared with a subject not administered apoptotic cells.

In some embodiments, treating, preventing, inhibiting, reducing the incidence of, ameliorating, or alleviating sepsis in a subject in need increase the survival time in said subject by about 100% compared with a subject not administered apoptotic cells. In some embodiments, treating, preventing, inhibiting, reducing the incidence of, ameliorating, or alleviating sepsis in a subject in need increase the survival time in said subject by about 200% , 300%, 400%, 500%, 600%, 700%, 800%, 900%, or 1000%, compared with a subject not administered apoptotic cells.

In some embodiments, treating, preventing, inhibiting, reducing the incidence of, ameliorating, or alleviating sepsis in a subject in need increase the survival time in said subject by about 100%-1000%, compared with a subject not administered apoptotic cells. In some embodiments, treating, preventing, inhibiting, reducing the incidence of, ameliorating, or alleviating sepsis in a subject in need increase the survival time in said subject by about 100%-500%, compared with a subject not administered apoptotic cells. In some embodiments, treating, preventing, inhibiting, reducing the incidence of, ameliorating, or alleviating sepsis in a subject in need increase the survival time in said subject by about 500%-1000%, compared with a subject not administered apoptotic cells. In some embodiments, treating, preventing, inhibiting, reducing the incidence of, ameliorating, or alleviating sepsis in a subject in need increase the survival time in said subject by about 70%-80%, compared with a subject not administered apoptotic cells. In some embodiments, treating, preventing, inhibiting, reducing the incidence of, ameliorating, or alleviating sepsis in a subject in need increase the survival time in said subject by about 50% compared with a subject not administered apoptotic cells. In some embodiments, treating, preventing, inhibiting, reducing the incidence of, ameliorating, or alleviating sepsis in a subject in need increase the survival time in said subject by about 60% compared with a subject not administered apoptotic cells. In some embodiments, treating, preventing, inhibiting, reducing the incidence of, ameliorating, or alleviating sepsis in a subject in need increase the survival time in said subject by about 70% compared with a subject not administered apoptotic cells. In some embodiments, treating, preventing, inhibiting, reducing the incidence of, ameliorating, or alleviating sepsis in a subject in need increase the survival time in said subject by about 80% compared with a subject not administered apoptotic cells.

In another embodiment, treating, preventing, inhibiting, reducing the incidence of, ameliorating, or alleviating sepsis in a subject in need is compared with a subject experiencing sepsis and not administered apoptotic cells. In another embodiment, treating, preventing, inhibiting, reducing the incidence of, ameliorating, or alleviating sepsis in a subject in need is compared with a subject experiencing sepsis and not administered an apoptotic supernatant.

In some embodiments, administration of apoptotic cells to a subject experiencing sepsis comprises intravenous administration. In some embodiments, administration of apoptotic cells to a subject experiencing sepsis comprising intravenous administration following an initial standard of care treatment with antibiotics, fluids, and vasopressors.

In some embodiments, administration of apoptotic cells to a subject experiencing sepsis comprises administration between 12 - 24 hours post diagnosis of sepsis. In some embodiments, administration of apoptotic cells to a subject experiencing sepsis comprises administration between 12 - 36 hours post diagnosis of sepsis. In some embodiments, administration of apoptotic cells to a subject experiencing sepsis comprises administration between 24 - 36 hours post diagnosis of sepsis. In some embodiments, administration of apoptotic cells to a subject experiencing sepsis comprises administration between 12 - 18 hours post diagnosis of sepsis. In some embodiments, administration of apoptotic cells to a subject experiencing sepsis comprises administration between 18-24 hours post diagnosis of sepsis. In some embodiments, administration of apoptotic cells to a subject experiencing sepsis comprises administration between 18-30 hours post diagnosis of sepsis. In some embodiments, administration of apoptotic cells to a subject experiencing sepsis comprises administration between 24-30 hours post diagnosis of sepsis. In some embodiments, administration of apoptotic cells to a subject experiencing sepsis comprises administration between 24-36 hours post diagnosis of sepsis.

In some embodiments, administration of apoptotic cells to a subject experiencing sepsis comprises administration about 12 hours post diagnosis of sepsis. In some embodiments, administration of apoptotic cells to a subject experiencing sepsis comprises administration about 13, 14, 15, 16, 17, 18, 19, 20, 21, 22, 23, 24, 25, 26, 27, 28, 29, 30, 31, 32, 33, 34, 35, or hours post diagnosis of sepsis. In some embodiments, administration of apoptotic cells to a subject experiencing sepsis comprises administration within 24 hours $\pm$ 6 hours post diagnosis of sepsis.

In some embodiments, the response of a subject suffering sepsis and administered a composition comprising apoptotic cells comprises a dose response. In some embodiments, the response of a subject suffering sepsis and administered a composition comprising an apoptotic cell supernatant comprises a dose response.

*Apoptotic Cells*

Production of apoptotic cells ("ApoCells") for use in compositions as disclosed herein, has been described in WO 2014/087408 and is described in brief in Example 1 below. Apoptotic cells for use in compositions as disclosed herein can be produced in any way that is known in the art. In another embodiment, apoptotic cells for use in compositions disclosed herein are autologous with a subject undergoing therapy. In another embodiment, apoptotic cells for use in compositions disclosed herein are allogeneic with a subject undergoing therapy. In another embodiment, a composition comprising apoptotic cells comprises apoptotic cells as disclosed herein or as is known in art.

A skilled artisan would appreciate that the term "autologous" may encompass a tissue, cell, nucleic acid molecule or polypeptide in which the donor and recipient is the same person.

A skilled artisan would appreciate that the term "allogeneic" may encompass a tissue, cell, nucleic acid molecule or polypeptide that is derived from separate individuals of the same species. In some embodiments, allogeneic donor cells are genetically distinct from the recipient.

Obtaining a mononuclear-enriched cell composition according to the production method disclosed herein may be effected by leukapheresis. A skilled artisan would appreciate that the term "leukapheresis" may encompass an apheresis procedure in which leukocytes are separated from the blood of a donor. The blood of a donor undergoes leukapheresis and thus a mononuclear-enriched cell composition may be obtained according to the production method disclosed herein. It is to be noted, that the use of at least one anticoagulant during leukapheresis is required, as is known in the art, in order to prevent clotting of the collected cells.

The leukapheresis procedure may be configured to allow collection of mononuclear-enriched cell composition according to the production method disclosed herein. Cell collections obtained by leukapheresis may comprise at least 65%, at least 70%, or at least 80% mononuclear cells. Blood plasma from the cell-donor may be collected in parallel to obtain of the mononuclear-enriched cell composition. About 300-600ml of blood plasma from the cell-donor may be collected in parallel to obtain the mononuclear-enriched cell composition according to the production method disclosed herein. Blood plasma collected in parallel to obtain the mononuclear-enriched cell composition according to the production method disclosed herein may be used as part of the freezing and/or incubation medium. Additional detailed methods of obtaining an enriched population of apoptotic cells for use in the compositions as disclosed herein may be found in WO 2014/087408.

In some embodiments, the early apoptotic cells disclosed herein comprise at least 85% mononuclear cells. In further embodiments, the early apoptotic cells disclosed herein contain at least 85% mononuclear cells, 90% mononuclear cells or alternatively over 90% mononuclear cells. In some embodiments, the early apoptotic cells disclosed herein comprise at least 90% mononuclear cells. In some embodiments, the early apoptotic cells disclosed herein comprise at least 95% mononuclear cells.

It is to be noted that while the mononuclear-enriched cell preparation at cell collection may comprise at least 65%, preferably at least 70%, most preferably at least 80% mononuclear cells, the final pharmaceutical population, following the production method, not forming part of the present invention, of the early apoptotic cells for use in the methods disclosed herein, comprises at least 85%, preferably at least 90%, most preferably at least 95% mononuclear cells.

The mononuclear-enriched cell preparation used for production of the composition of the early apoptotic cells for use in the present invention may comprise at least 50% mononuclear cells at cell collection. Disclosed herein is a method for producing the pharmaceutical population wherein the method comprises obtaining a mononuclear-enriched cell preparation from the peripheral blood of a donor, the mononuclear-enriched cell preparation comprising at least 50% mononuclear cells. Disclosed herein is a method for producing the pharmaceutical population wherein the method comprises freezing a mononuclear-enriched cell preparation comprising at least 50% mononuclear cells.

The cell preparation may comprise at least 85% mononuclear cells, wherein at least 40% of the cells in the preparation are in an early-apoptotic state, wherein at least 85% of the cells in the preparation are viable cells. The apoptotic cell preparation may comprise no more than 15% CD15$^{high}$ expressing cells.

A skilled artisan would appreciate that the term "early-apoptotic state" may encompass cells that show early signs of apoptosis without late signs of apoptosis. Examples of early signs of apoptosis in cells include exposure of phosphatidylserine (PS) and the loss of mitochondrial membrane potential. Examples of late events include propidium iodide (PI) admission into the cell and the final DNA cutting. In order to document that cells are in an "early apoptotic" state, PS exposure detection by Annexin-V and PI staining can be used, and cells that are stained with Annexin V but not with PI are considered to be "early apoptotic cells". Cells that are stained by both Annexin-V FITC and PI may be considered to be "late apoptotic cells". Cells that do not stain for either Annexin-V or PI may be considered non-apoptotic viable cells.

A skilled artisan would appreciate that in some embodiments the terms "early apoptotic cells", "apoptotic cell", "Allocetra", "ALC", and "ApoCell", and grammatical variants thereof, may be used interchangeably having all the same qualities and meanings. In some embodiments, as described herein, early apoptotic cells are HLA matched to a recipient of a composition comprising the early apoptotic cells (a subject in need). In some embodiments, as described herein, early apoptotic cells are not matched to a recipient of a composition comprising the early apoptotic cells (a subject in need). In some embodiments, the early apoptotic cells not matched to a recipient of a composition comprising the early apoptotic cells (a subject in need) are irradiated as described herein in detail. In some embodiments, irradiated not matched cells are termed "Allocetra-OTS" or "ALC-OTS".

In some embodiments, apoptotic cells comprise cells in an early apoptotic state. In another embodiment, apoptotic cells comprise cells wherein at least 90% of said cells are in an early apoptotic state. In another embodiment, apoptotic cells comprise cells wherein at least 80% of said cells are in an early apoptotic state. In another embodiment, apoptotic cells comprise cells wherein at least 70% of said cells are in an early apoptotic state. In another embodiment, apoptotic cells comprise cells wherein at least 60% of said cells are in an early apoptotic state. In another embodiment, apoptotic cells comprise cells wherein at least 50% of said cells are in an early apoptotic state.

In some embodiments, the composition comprising apoptotic cells further comprises an anti-coagulant.

In some embodiments, early apoptotic cells are stable. A skilled artisan would appreciate that in some embodiments stability encompasses maintaining early apoptotic cell characteristics over time, for example, maintaining early apoptotic cell characteristics upon storage at about 2-8°C. In some embodiments, stability comprises maintaining early apoptotic cell characteristic upon storage at freezing temperatures, for example temperatures at or below 0°C.

In some embodiments, the mononuclear-enriched cell population for use in the present invention, that may be obtained according to a production method of the early apoptotic cells described herein, undergoes freezing in a freezing medium. In some embodiments, the freezing is gradual. In some embodiments, following collection the cells are maintained at room temperature until frozen. In some embodiments, the cell-preparation undergoes at least one washing step in washing medium following cell-collection and prior to freezing.

As used herein, the terms "obtaining cells" and "cell collection" may be used interchangeably. In some embodiments, the cells of the cell preparation are frozen within 3-6 hours of collection. In some embodiments, the cell preparation is frozen within up to 6 hours of cell collection. In some embodiments, the cells of the cell preparations are frozen within 1, 2, 3, 4, 5, 6, 7, 8 hours of collection. In other embodiments, the cells of the cell preparations are frozen up to 8, 12, 24, 48, 72 hours of collection. In other embodiments, following collection the cells are maintained at 2-8°C until frozen.

Freezing according to the production of an early apoptotic cell population may comprise: freezing the cell preparation at about -18°C to -25°C followed by freezing the cell preparation at about -80°C and finally freezing the cell preparation in liquid nitrogen until thawing. Freezing according to the production of an early apoptotic cell population may comprise: freezing the cell preparation at about -18°C to -25°C for at least 2 hours, freezing the cell preparation at about -80°C for at least 2 hours and finally freezing the cell preparation in liquid nitrogen until thawing. The cells may be kept in liquid nitrogen for at least 8, 10 or 12 hours prior to thawing. The cells of the cell preparation may be kept in liquid nitrogen until thawing and incubation with apoptosis-inducing incubation medium. The cells of the cell preparation may be kept in liquid nitrogen until the day of hematopoietic stem cell transplantation. In examples, the time from cell collection and freezing to preparation of the final population may be between 1-50 days, alternatively between 6-30 days. The cell preparation may be kept in liquid nitrogen for longer time periods, such as at least several months.

The freezing according to the production of an early apoptotic cell population may comprise freezing the cell preparation at about -18°C to -25°C for at least 0.5, 1, 2, 4 hours. The freezing according to the production of an early apoptotic cell population may comprise freezing the cell preparation at about -18°C to -25°C for about 2 hours. The freezing In the production of an early apoptotic cell population may comprise freezing the cell preparation at about -80°C for at least 0.5, 1, 2, 4, 12 hours.

In some embodiments, the mononuclear-enriched cell composition may remain frozen at least 1, 2, 3, 4, 5, 6, 7, 8, 9, 10, 11, 12, 20 months. In some embodiments, the mononuclear-enriched cell composition may remain frozen at least 0.5, 1, 2, 3, 4, 5 years. In certain embodiments, the mononuclear-enriched cell composition may remain frozen for at least 20 months.

In some embodiments, the mononuclear-enriched cell composition is frozen for at least 8, 10, 12, 18, 24 hours. In certain embodiments, freezing the mononuclear-enriched cell composition is for a period of at least 8 hours. In some embodiments, the mononuclear-enriched cell composition is frozen for at least about 10 hours. In some embodiments, the mononuclear-enriched cell composition is frozen for at least about 12 hours. In some embodiments, the mononuclear-enriched cell composition is frozen for about 12 hours. In some embodiments, the total freezing time of the mononuclear-enriched cell composition (at about -18°C to -25°C, at about -80°C and in liquid nitrogen) is at least 8, 10, 12, 18, 24 hours.

The freezing may at least partly induce the early-apoptotic state in the cells of the mononuclear-enriched cell composition. The freezing medium may comprise RPMI 1640 medium comprising L-glutamine, Hepes, Hes, dimethyl sulfoxide (DMSO) and plasma. The plasma in the freezing medium may be an autologous plasma of the donor which donated the mononuclear-enriched cells of the population. The freezing medium may comprise RPMI 1640 medium comprising 2 mM L-glutamine, 10 mM Hepes, 5% Hes, 10% dimethyl sulfoxide and 20% v/v plasma.

The freezing medium may comprise an anti-coagulant. At least some of the media used during the production of an early apoptotic cell population, including the freezing medium, the incubation medium and the washing media may comprise an anti-coagulant. All media used during the production of an early apoptotic cell population which comprise an anti-coagulant may comprise the same concentration of anti-coagulant. In some examples, anti-coagulant is not added to the final suspension medium of the cell population.

Addition of an anti-coagulant at least to the freezing medium can improve the yield of the cell-preparation.

Addition of an anti-coagulant to the freezing medium may improve the yield of the cell-preparation in the presence of a high triglyceride level. As used herein, improvement in the yield of the cell-preparation relates to improvement in at least one of: the percentage of viable cells out of cells frozen, the percentage of early-state apoptotic cells out of viable cells and a combination thereof.

In some embodiments, early apoptotic cells are stable for at least 24 hours. In another embodiment, early apoptotic cells are stable for 24 hours. In another embodiment, early apoptotic cells are stable for more than 24 hours. In another embodiment, early apoptotic cells are stable for at least 36 hours. In another embodiment, early apoptotic cells are stable for 48 hours. In another embodiment, early apoptotic cells are stable for at least 36 hours. In another embodiment, early apoptotic cells are stable for more than 36 hours. In another embodiment, early apoptotic cells are stable for at least 48 hours. In another embodiment, early apoptotic cells are stable for 48 hours. In another embodiment, early apoptotic cells are stable for at least 48 hours. In another embodiment, early apoptotic cells are stable for more than 48 hours. In another embodiment, early apoptotic cells are stable for at least 72 hours. In another embodiment, early apoptotic cells are stable for 72 hours. In another embodiment, early apoptotic cells are stable for more than 72 hours.

A skilled artisan would appreciate that the term "stable" encompasses apoptotic cells that remain PS-positive (Phosphatidylserine-positive) with only a very small percent of PI-positive (Propidium iodide-positive). PI-positive cells provide an indication of membrane stability wherein a PI-positive cells permits admission into the cells, showing that the membrane is less stable. In some embodiments, stable early apoptotic cells remain in early apoptosis for at least 24 hours, for at least 36 hours, for at least 48 hours, or for at least 72 hours. In another embodiment, stable early apoptotic cells remain in early apoptosis for 24 hours, for 36 hours, for 48 hours, or for 72 hours. In another embodiment, stable early apoptotic cells remain in early apoptosis for more than 24 hours, for more than 36 hours, for more than 48 hours, or for more than 72 hours. In another embodiment, stable early apoptotic cells maintain their state for an extended time period.

In some embodiments, an apoptotic cell population is devoid of cell aggregates. In some embodiments, an apoptotic cell population is devoid of large cell aggregates. In some embodiments, an apoptotic cell population has a reduced number of cell aggregates compared to an apoptotic cell population prepared without adding an anticoagulant in a step other than cell collection (leukapheresis) from the donor. In some embodiments, an apoptotic cell population or a composition thereof, comprises an anticoagulant.

In some embodiments, apoptotic cells are devoid of cell aggregates, wherein said apoptotic cells were obtained from a subject with high blood triglycerides. In some embodiments, blood triglycerides levels of the subject are above 150 mg/dL. In some embodiments, an apoptotic cell population is devoid of cell aggregates, wherein said apoptotic cell population is prepared from cells obtained from a subject with normal blood triglycerides. In some embodiments, blood triglycerides levels of the subject are equal to or below 150 mg/dL. In some embodiments, cell aggregates produce cell loss during apoptotic cell production methods.

A skilled artisan would appreciate that the terms "aggregates" or "cell aggregates" may encompass the reversible clumping of blood cells under low shear forces or at stasis. Cell aggregates can be visually observed during the incubation steps of the production of the apoptotic cells. Cell aggregation can be measured by any method known in the art, for example by visually imaging samples under a light microscope or using flow cytometry.

In some embodiments, the anti-coagulant is selected from: heparin, acid citrate dextrose (ACD) Formula A or a combination thereof. In some embodiments, the anti-coagulant is selected from: heparin, acid citrate dextrose (ACD) Formula A or a combination thereof.

Disclosed herein are methods of preparing an early apoptotic cell population and compositions thereof, in which an anticoagulant is added to at least one medium used during preparation of the population. The at least one medium used during preparation of the population may be selected from: the freezing medium, the washing medium, the apoptosis inducing incubation medium, or any combinations thereof.

The anti-coagulant may be selected from: Heparin, ACD Formula A or a combination thereof. It is to be noted that other anti-coagulants known in the art may be used, such as Fondaparinaux, Bivalirudin and Argatroban.

At least one medium used during preparation of the population may contain 5% of ACD formula A solution comprising 10 U/ml heparin. In some examples, anti-coagulant is not added to the final suspension medium of the cell population. As used herein, the terms "final suspension medium" and "administration medium" are used interchangeably having all the same qualities and meanings.

At least one medium used during preparation of the population may comprise heparin at a concentration of between 0.1-2.5 U/ml. At least one medium used during preparation of the population may comprise ACD Formula A at a concentration of between 1%-15% v/v. The freezing medium may comprise an anti-coagulant. The incubation medium may comprise an anti-coagulant. Both the freezing medium and incubation medium may comprise an anti-coagulant.

The heparin in the freezing medium may be at a concentration of between 0.1-2.5 U/ml. The ACD Formula A in the freezing medium may be at a concentration of between 1%-15% v/v. The heparin in the incubation medium may be at a concentration of between 0.1-2.5 U/ml. The ACD Formula A in the incubation medium may be at a concentration of between 1%-15% v/v. The anticoagulant is a solution of acid-citrate-dextrose (ACD) formula A. The anticoagulant added to at least one medium used during preparation of the population is ACD Formula A containing heparin at a concentration of 10 U/ml.

The apoptosis inducing incubation medium used in the production of an early apoptotic cell population may comprise an anti-coagulant. Both the freezing medium and apoptosis inducing incubation medium used in the production of an early apoptotic cell population may comprise an anti-coagulant. Without wishing to be bound by any theory or mechanism, in order to maintain a high and stable cell yield in different cell compositions, regardless of the cell collection protocol, addition of anti-coagulants comprising adding the anticoagulant to both the freezing medium and the apoptosis inducing incubation medium during production of the apoptotic cell population. A high and stable cell yield within the composition may comprise a cell yield of at least 30%, preferably at least 40%, typically at least 50% cells of the initial population of cells used for induction of apoptosis.

Addition of an anti-coagulant both to the incubation medium and freezing medium may result in a high and stable cell-yield between different preparations of the population regardless of cell-collection conditions, such as the timing and/or type of anti-coagulant added during cell collection. Addition of an anti-coagulant both to the incubation medium and freezing medium may result in a high and stable yield of the cell-preparation regardless of the timing and/or type of anti-coagulant added during leukapheresis. Production of the cell-preparation in the presence of a high triglyceride level may result in a low and/or unstable cell-yield between different preparations. Producing the cell-preparation from the blood of a donor having high triglyceride level may result in a low and/or unstable cell-yield of the cell preparation. The term "high triglyceride level" refers to a triglyceride level which is above the normal level of a healthy subject of the same sex and age. The term "high triglyceride level" refers to a triglyceride level above about 1.7 milimole/liter. As used herein, a high and stable yield refers to a cell yield in the population which is high enough to enable preparation of a dose which will demonstrate therapeutic efficiency when administered to a subject. Therapeutic efficiency refers to the ability to treat, prevent or ameliorate an immune disease, an autoimmune disease or an inflammatory disease in a subject. A high and stable cell yield may be a cell yield of at least 30%, possibly at least 40%, typically at least 50% of cells in the population out of cells initially frozen.

In case the cell-preparation is obtained from a donor having a high triglyceride level, the donor will take at least one measure selected from: taking triglyceride-lowering medication prior to donation, such as: statins and/or bezafibrate, fasting for a period of at least 8, 10, 12 hours prior to donation, eating an appropriate diet to reduce blood triglyceride level at least 24, 48, 72 hours prior to donating or any combination thereof.

Cell yield in the population relates to cell number in the composition out of the initial number of cells subjected to apoptosis induction. As used herein, the terms "induction of early apoptotic state" and "induction of apoptosis" may be used interchangeably.

The mononuclear-enriched cell composition may be incubated in incubation medium following freezing and thawing. There is at least one washing step between thawing and incubation. As used herein, the terms "incubation medium" and "apoptosis inducing incubation medium" are used interchangeably. The incubation medium may comprise RPMI 1640 medium supplemented with L-glutamine, Hepes methylprednisolone and plasma. The washing medium may comprise 2 mM L-glutamine, 10 mM Hepes and 10% v/v blood plasma. The blood plasma in in the incubation medium may be derived from the same donor from whom the cells of the cell preparations are derived. The blood plasma may be added to the incubation medium on the day of incubation. In some embodiments, incubation is performed at 37°C and 5% CO2.

The incubation medium may comprise methylprednisolone. The methylprednisolone within the incubation medium may further induces the cells in the mononuclear-enriched cell composition to enter an early-apoptotic state. The cells in the mononuclear-enriched cell composition may be induced to enter an early-apoptotic state both by freezing and incubating in the presence of methylprednisolone. The production of an early apoptotic cell population advantageously allows induction of an early-apoptosis state substantially without induction of necrosis, wherein the cells remain stable at said early-apoptotic state for about 24 hours following preparation.

The incubation medium may comprise methylprednisolone at a concentration of about 10-100 $\mu$g/ml. The incubation medium may comprise methylprednisolone at a concentration of about 40-60 $\mu$g/ml, alternatively about 45-55 $\mu$g/ml. The incubation medium may comprise methylprednisolone at a concentration of 50 $\mu$g/ml.

The incubation may be for about 2-12 hours, possibly 4-8 hours, typically for about 5-7 hours. The incubation may be for about 6 hours. he incubation may be for at least 6 hours. The incubation may be for 6 hours.

The incubation medium may comprise an anti-coagulant. Addition of an anti-coagulant to the incubation medium can improve the yield of the cell-preparation. The anti-coagulant in the incubation medium may be of the same concentration as within the freezing medium. The incubation medium may comprise an anti-coagulant selected from : heparin, ACD Formula A or a combination thereof. The anti-coagulant used in the incubation medium may be ACD Formula A containing heparin at a concentration of 10 U/ml.

The incubation medium may comprise heparin. The heparin in the incubation medium may be at a concentration of between 0.1-2.5 U/ml. The heparin in the incubation medium may be at a concentration of between 0.1-2.5 U/ml, possibly between 0.3-0.7 U/ml, typically about 0.5 U/ml. The heparin in the incubation medium may be at a concentration of about 0.5 U/ml.

The incubation medium may comprise ACD Formula A. The ACD Formula A in the incubation medium may be at a concentration of between 1%-15% v/v. The ACD Formula A in the incubation medium may be at a concentration of between 1%-15% v/v, possibly between 4%-7% v/v, typically about 5% v/v. The ACD Formula A in the incubation medium may be at a concentration of about 5% v/v.

Improvement in the yield of the cell-preparation may comprise improvement in the number of the early-apoptotic viable cells of the preparation out of the number of frozen cells from which the preparation was produced.

Addition of an anti-coagulant to the freezing medium can contribute to a high and stable yield between different preparations of the pharmaceutical population. In preferable examples, addition of an anti-coagulant at least to the freezing medium and incubation medium can result in a high and stable yield between different preparations of the pharmaceutical composition, regardless to the cell collection protocol used.

The freezing medium may comprise an anti-coagulant selected from: heparin, ACD Formula A or a combination thereof. The anti-coagulant used in the freezing medium may be ACD Formula A containing heparin at a concentration of 10 U/ml. The freezing medium may comprise 5% v/v of ACD Formula A solution comprising heparin at a concentration of 10 U/ml.

The freezing medium may comprise heparin. The heparin in the freezing medium may be at a concentration of between 0.1-2.5 U/ml. The heparin in the freezing medium may be at a concentration of between 0.1-2.5 U/ml, possibly between 0.3-0.7 U/ml, typically about 0.5 U/ml. The heparin in the freezing medium may be at a concentration of about 0.5 U/ml.

The freezing medium may comprise ACD Formula A. The ACD Formula A in the freezing medium may be at a concentration of between 1%-15% v/v. The ACD Formula A in the freezing medium may be at a concentration of between 1%-15% v/v, possibly between 4%-7% v/v, typically about 5% v/v. The ACD Formula A in the freezing medium may be at a concentration of about 5% v/v.

Addition of an anti-coagulant to the incubation medium and/or freezing medium can result in a high and stable cell yield within the population regardless of the triglyceride level in the blood of the donor. Addition of an anti-coagulant to the incubation medium and/or freezing medium can result in a high and stable cell yield within the composition the invention when obtained from the blood of a donor having normal or high triglyceride level. Addition of an anti-coagulant at least to the incubation medium, can result in a high and stable cell yield within the composition regardless of the triglyceride level in the blood of the donor. Addition of an anti-coagulant to the freezing medium and incubation medium may result in a high and stable cell yield within the composition regardless of the triglyceride level in the blood of the donor.

The freezing medium and/or incubation medium and/or washing medium may comprise heparin at a concentration of at least 0.1 U/ml, possibly at least 0.3 U/ml, typically at least 0.5 U/ml. The freezing medium and/or incubation medium and/or washing medium may comprise ACD Formula A at a concentration of at least 1% v/v, possibly at least 3% v/v, typically at least 5% v/v.

The mononuclear-enriched cell composition may undergo at least one washing step following cell collection and prior to being re-suspended in the freezing medium and frozen. The mononuclear-enriched cell composition may undergo at least one washing step following freezing and thawing. Washing steps may comprise centrifugation of the mononuclear-enriched cell composition followed by supernatant extraction and re-suspension in washing medium.

The mononuclear-enriched cell composition may undergo at least one washing step between each stage of the production of an early apoptotic cell population. Anti-coagulant may be added to washing media during washing steps throughout the production of an early apoptotic cell population. The mononuclear-enriched cell composition may undergo at least one washing step following incubation. The mononuclear-enriched cell composition may undergo at least one washing step following incubation using PBS. In some cases, anti-coagulant is not added to the final washing step prior to re-suspension of the cell-preparation in the administration medium. In some cases, anti-coagulant is not added to the PBS used in the final washing step prior to re-suspension of the cell-preparation in the administration medium. In certain examples, anti-coagulant is not added to the administration medium.

In some examples, the cell concentration during incubating is about $5 \times 10^6$ cells/ml.

In some examples, the mononuclear-enriched cell composition may be suspended in an administration medium following freezing, thawing and incubating, thereby resulting in the pharmaceutical population. The administration medium may comprise a suitable physiological buffer. Examples of a suitable physiological buffer are: saline solution, Phoshpate Buffered Saline (PBS), Hank's Balanced Salt Solution (HBSS), and the like. The administration medium may comprise PBS. The administration medium may comprise supplements conducive to maintaining the viability of the cells. The mononuclear-enriched cell composition may be filtered prior to administration. The mononuclear-enriched cell composition may be filtered prior to administration using a filter of at least 200$\mu$m.

The mononuclear-enriched cell population may be re-suspended in an administration medium such that the final volume of the resulting cell-preparation is between 100-1000ml, possibly between 200-800ml, typically between 300-600ml.

Cell collection may refer to obtaining a mononuclear-enriched cell composition. Washing steps performed during the production of an early apoptotic cell population may be performed in a washing medium. Washing steps performed up until the incubation step of the production of an early apoptotic cell population may be performed in a washing medium. The washing medium may comprise RPMI 1640 medium supplemented with L-glutamine and Hepes. The washing medium may comprise RPMI 1640 medium supplemented with 2 mM L-glutamine and 10 mM Hepes.

The washing medium may comprise an anti-coagulant. The washing medium may comprise an anti-coagulant selected from: heparin, ACD Formula A or a combination thereof. The concentration of the anti-coagulant in the washing medium may be the same concentration as in the freezing medium. The concentration of the anti-coagulant in the washing medium may be the same concentration as in the incubation medium. The anti-coagulant used in the washing medium may be ACD Formula A containing heparin at a concentration of 10 U/ml.

The washing medium may comprise heparin. The heparin in the washing medium may be at a concentration of between 0.1-2.5 U/ml. The heparin in the washing medium may be at a concentration of between 0.1-2.5 U/ml, possibly between 0.3-0.7 U/ml, typically about 0.5 U/ml. The heparin in the washing medium may be at a concentration of about 0.5 U/ml.

The washing medium may comprise ACD Formula A. The ACD Formula A in the washing medium may be at a concentration of between 1%-15% v/v. The ACD Formula A in the washing medium may be at a concentration of between 1%-15% v/v, possibly between 4%-7% v/v, typically about 5% v/v. The ACD Formula A in the washing medium may be at a concentration of about 5% v/v.

The mononuclear-enriched cell composition may be thawed several hours prior to the intended administration of the population to a subject. The mononuclear-enriched cell composition may be thawed at about 33°C-39°C. The mononuclear-enriched cell composition may be thawed for about 30-240 seconds, preferably 40-180 seconds, most preferably 50-120 seconds.

The mononuclear-enriched cell composition may be thawed at least 10 hours prior to the intended administration of the population, alternatively at least 20, 30, 40 or 50 hours prior to the intended administration of the population. The mononuclear-enriched cell composition may be thawed at least 15-24 hours prior to the intended administration of the population. The mononuclear-enriched cell composition may be thawed at least about 24 hours prior to the intended administration of the population. The mononuclear-enriched cell composition may be thawed at least 20 hours prior to the intended administration of the population. I The mononuclear-enriched cell composition may be thawed 30 hours prior to the intended administration of the population. The mononuclear-enriched cell composition may be thawed at least 24 hours prior to the intended administration of the population. The mononuclear-enriched cell composition may undergo at least one step of washing in the washing medium before and/or after thawing.

In some embodiments, the composition further comprises methylprednisolone. At some embodiments, the concentration of methylprednisolone does not exceed 30μg/ml.

In some embodiments, the apoptotic cells are used at a high dose. In some embodiments, the apoptotic cells are used at a high concentration. In some embodiments, human apoptotic polymorphonuclear neutrophils (PMNs) are used. In some embodiments, a group of cells, of which 50% are apoptotic cells, are used. In some embodiments, apoptotic cells are verified by May-Giemsa-stained cytopreps. In some embodiments, viability of cells are assessed by trypan blue exclusion. In some embodiments, the apoptotic and necrotic status of the cells are confirmed by annexin V/propidium iodide staining with detection by FACS.

In some embodiments, apoptotic cells disclosed herein comprise no necrotic cells. In some embodiments, apoptotic cells disclosed herein comprise less than 1% necrotic cells. In some embodiments, apoptotic cells disclosed herein comprise less than 2% necrotic cells. In some embodiments, apoptotic cells disclosed herein comprise less than 3% necrotic cells. In some embodiments, apoptotic cells disclosed herein comprise less than 4% necrotic cells. In some embodiments, apoptotic cells disclosed herein comprise less than 5% necrotic cells.

In some embodiments, a dose of about $140 \times 10^6$ - $210 \times 10^6$ apoptotic cells are administered. In some embodiments, a dose of about $10\text{-}100 \times 10^6$ apoptotic cells is administered. In some embodiments, a dose of about $20 \times 10^6$ apoptotic cells is administered. In some embodiments, a dose of about $30 \times 10^6$ apoptotic cells is administered. In some embodiments, a dose of about $40 \times 10^6$ apoptotic cells is administered. In some embodiments, a dose of about $50 \times 10^6$ apoptotic cells is administered. In some embodiments, $60 \times 10^6$ apoptotic cells is administered. In some embodiments, a dose of about $60 \times 10^6$ apoptotic cells is administered. In some embodiments, a dose of about $70 \times 10^6$ apoptotic cells is administered. In some embodiments, a dose of about $80 \times 10^6$ apoptotic cells is administered. In some embodiments, a dose of about $90 \times 10^6$ apoptotic cells is administered. In some embodiments, a dose of about $1\text{-}15 \times 10^7$ apoptotic cells is administered. In some embodiments, a dose of about $10 \times 10^7$ apoptotic cells is administered. In some embodiments, a dose of about $15 \times 10^7$ apoptotic cells is administered.

In some embodiments, a dose of $10\times10^6$ apoptotic cells is administered. In another embodiment, a dose of $10\times10^7$ apoptotic cells is administered. In another embodiment, a dose of $10\times10^8$ apoptotic cells is administered. In another embodiment, a dose of $10\times10^9$ apoptotic cells is administered. In another embodiment, a dose of $10\times10^{10}$ apoptotic cells is administered. In another embodiment, a dose of $10\times10^{11}$ apoptotic cells is administered. In another embodiment, a dose of $10\times10^{12}$ apoptotic cells is administered. In another embodiment, a dose of $10\times10^5$ apoptotic cells is administered. In another embodiment, a dose of $10\times10^4$ apoptotic cells is administered. In another embodiment, a dose of $10\times10^3$ apoptotic cells is administered. In another embodiment, a dose of $10\times10^2$ apoptotic cells is administered.

In some embodiments, a high dose of apoptotic cells is administered. In some embodiments, a dose of $35\times10^6$ apoptotic cells is administered. In another embodiment, a dose of $210\times10^6$ apoptotic cells is administered. In another embodiment, a dose of $70\times10^6$ apoptotic cells is administered. In another embodiment, a dose of $140\times10^6$ apoptotic cells is administered. In another embodiment, a dose of $35\text{-}210\times10^6$ apoptotic cells is administered.

In some embodiments, a single dose of apoptotic cells is administered. In some embodiments, multiple doses of apoptotic cells are administered. In some embodiments, 2 doses of apoptotic cells are administered. In some embodiments, 3 doses of apoptotic cells are administered. In some embodiments, 4 doses of apoptotic cells are administered. In some embodiments, 5 doses of apoptotic cells are administered. In some embodiments, 6 doses of apoptotic cells are administered. In some embodiments, 7 doses of apoptotic cells are administered. In some embodiments, 8 doses of apoptotic cells are administered. In some embodiments, 9 doses of apoptotic cells are administered. In some embodiments, more than 9 doses of apoptotic cells are administered. In some embodiments, multiple doses of apoptotic cells are administered.

In some embodiments, the apoptotic cells may be administered by any administration type known in the art including intravenous, subcutaneous, intranodal, intratumoral, intrathecal, intrapleural, intraperitoneal and directly to the thymus.

In some embodiments, the apoptotic cells are prepared from cells obtained from a subject other than the subject that will receive said apoptotic cells. The apoptotic cells may be prepared via an additional step that is useful in overcoming rejection of allogeneic donor cells, including one or more steps described in U.S. Patent Application 20130156794

Disclosed herein are methods for producing a population of mononuclear apoptotic cell comprising a decreased percent of non-quiescent non-apoptotic viable cells; a suppressed cellular activation of any living non-apoptotic cells; or a reduced proliferation of any living non-apoptotic cells; or any combination thereof, said method comprising the following steps, obtaining a mononuclear-enriched cell population of peripheral blood; freezing said mononuclear-enriched cell population in a freezing medium comprising an anticoagulant; thawing said mononuclear-enriched cell population; incubating said mononuclear-enriched cell population in an apoptosis inducing incubation medium comprising methylprednisolone at a final concentration of about 10-100 $\mu$g/mL and an anticoagulant; resuspending said apoptotic cell population in an administration medium; and inactivating said mononuclear-enriched population, wherein said inactivation occurs following apoptotic induction, wherein said method produces a population of mononuclear apoptotic cell comprising a decreased percent of non-quiescent non-apoptotic cells; a suppressed cellular activation of any living non-apoptotic cells; or a reduced proliferation of any living non-apoptotic cells; or any combination thereof.

The methods may comprise the step of irradiating a population of apoptotic cells derived from a subject prior to administration of the population of apoptotic cells to the same subject (autologous ApoCells). The methods may comprise the step of irradiating apoptotic cells derived from a subject prior to administration of the population of apoptotic cells to a recipient (allogeneic ApoCells).

In some embodiments, cells are irradiated in a way that will decrease proliferation and/or activation of residual viable cells within the apoptotic cell population. In some embodiments, cells are irradiated in a way that reduces the percent of viable non-apoptotic cells in a population. In some embodiments, the percent of viable non-apoptotic cells in an inactivated early apoptotic cell population is reduced to less than 50% of the population. In some embodiments, the percent of viable non-apoptotic cells in an inactivated early apoptotic cell population is reduced to less than 40% of the population. In some embodiments, the percent of viable non-apoptotic cells in an inactivated early apoptotic cell population is reduced to less than 30% of the population. In some embodiments, the percent of viable non-apoptotic cells in an inactivated early apoptotic cell population is reduced to less than 20% of the population. In some embodiments, the percent of viable non-apoptotic cells in an inactivated early apoptotic cell population is reduced to less than 10% of the population. In some embodiments, the percent of viable non-apoptotic cells in an inactivated early apoptotic cell population is reduced to 0% of the population.

In another embodiment, the irradiated apoptotic cells preserve all their early apoptotic-, immune modulation-, stability-properties. In another embodiment, the irradiation step uses UV radiation. In another embodiment, the radiation step uses gamma radiation. In another embodiment, the apoptotic cells comprise a decreased percent of living non-apoptotic cells, comprise a preparation having a suppressed cellular activation of any living non-apoptotic cells present within the apoptotic cell preparation, or comprise a preparation having reduced proliferation of any living non-apoptotic cells present within the apoptotic cell preparation, or any combination thereof.

In some embodiments, irradiation of apoptotic cells does not increase the population of dead cells (PI+) compared with apoptotic cells not irradiated. In some embodiments, irradiation of apoptotic cells does not increase the population of dead cells (PI+) by more than about 1% compared with apoptotic cells not irradiated. In some embodiments, irradiation of apoptotic cells does not increase the population of dead cells (PI+) by more than about 2% compared with apoptotic cells not irradiated. In some embodiments, irradiation of apoptotic cells does not increase the population of dead cells (PI+) by more than about 3% compared with apoptotic cells not irradiated. In some embodiments, irradiation of apoptotic cells does not increase the population of dead cells (PI+) by more than about 4% compared with apoptotic cells not irradiated. In some embodiments, irradiation of apoptotic cells does not increase the population of dead cells (PI+) by more than about 5% compared with apoptotic cells not irradiated. In some embodiments, irradiation of apoptotic cells does not increase the population of dead cells (PI+) by more than about 6% compared with apoptotic cells not irradiated. In some embodiments, irradiation of apoptotic cells does not increase the population of dead cells (PI+) by more than about 7% compared with apoptotic cells not irradiated. In some embodiments, irradiation of apoptotic cells does not increase the population of dead cells (PI+) by more than about 8% compared with apoptotic cells not irradiated. In some embodiments, irradiation of apoptotic cells does not increase the population of dead cells (PI+) by more than about 9% compared with apoptotic cells not irradiated. In some embodiments, irradiation of apoptotic cells does not increase the population of dead cells (PI+) by more than about 10% compared with apoptotic cells not irradiated. In some embodiments, irradiation of apoptotic cells does not increase the population of dead cells (PI+) by more than about 15% compared with apoptotic cells not irradiated. In some embodiments, irradiation of apoptotic cells does not increase the population of dead cells (PI+) by more than about 20%, 25%, 30%, 35%, 40%, 45%, or 50% compared with apoptotic cells not irradiated.

In some embodiments, a cell population comprising a reduced or non-existent fraction of living non-apoptotic cells may in one embodiment provide a mononuclear early apoptotic cell population that does not have any living / viable cells. In some embodiments, a cell population comprising a reduced or non-existent fraction of living non-apoptotic cells may in one embodiment provide a mononuclear apoptotic cell population that does not elicit GVHD in a recipient.

In some embodiments, use of irradiated ApoCells removes the possible graft versus leukemia effect use of an apoptotic population (that includes a minor portion of viable cells) may cause, demonstrating that the effects shown here in the Examples (See Example 8) result from the apoptotic cells and not from a viable proliferating population of cells with cellular activity, present within the apoptotic cell population.

The methods, not forming part of the present invention, may comprise the step of irradiating apoptotic cells derived from WBCs from a donor prior to administration to a recipient. Cells may be irradiated in a way that will avoid proliferation and/or activation of residual viable cells within the apoptotic cell population. In another embodiment, the irradiated apoptotic cells preserve all their early apoptotic-, immune modulation-, stability-properties. For example, the irradiation step uses UV radiation. For example, the radiation step uses gamma radiation. In another embodiment, the apoptotic cells comprise a decreased percent of living non-apoptotic cells, comprise a preparation having a suppressed cellular activation of any living non-apoptotic cells present within the apoptotic cell preparation, or comprise a preparation having reduced proliferation of any living non-apoptotic cells present within the apoptotic cell preparation, or any combination thereof.

In some embodiments, apoptotic cells comprise a pooled mononuclear apoptotic cell preparation. In some embodiments, a pooled mononuclear apoptotic cell preparation comprises mononuclear cells in an early apoptotic state, wherein said pooled mononuclear apoptotic cells comprise a decreased percent of living non-apoptotic cells, a preparation having a suppressed cellular activation of any living non-apoptotic cells, or a preparation having reduced proliferation of any living non-apoptotic cells, or any combination thereof. In another embodiment, the pooled mononuclear apoptotic cells have been irradiated. In another embodiment, disclosed herein is a pooled mononuclear apoptotic cell preparation that in some embodiments, originates from the white blood cell fraction (WBC) obtained from donated blood.

In some embodiments, the apoptotic cell preparation is irradiated. In another embodiment, said irradiation comprises gamma irradiation or UV irradiation. In yet another embodiment, the irradiated preparation has a reduced number of non-apoptotic cells compared with a non-irradiated apoptotic cell preparation. In another embodiment, the irradiated preparation has a reduced number of proliferating cells compared with a non-irradiated apoptotic cell preparation. In another embodiment, the irradiated preparation has a reduced number of potentially immunologically active cells compared with a non-irradiated apoptotic cell population.

Pooled blood may comprise 3rd party blood not matched between donor and recipient.

A skilled artisan would appreciate that the term "pooled" may encompass blood collected from multiple donors, prepared and possibly stored for later use. This combined pool of blood may then be processed to produce a pooled mononuclear apoptotic cell preparation. In another embodiment, a pooled mononuclear apoptotic cell preparation ensures that a readily available supply of mononuclear apoptotic cells is available. Cells may be pooled just prior to the incubation step wherein apoptosis is induced. Cells may be pooled following the incubation step at the step of resuspension, cells may be pooled just prior to an irradiation step, cells may be pooled following an irradiation step, or cells may be pooled at any step in the methods of preparation.

In some embodiments, a pooled apoptotic cell preparation is derived from cells present in between about 2 and 25 units of blood. In another embodiment, said pooled apoptotic cell preparation is comprised of cells present in between about 2-5, 2-10, 2-15, 2-20, 5-10, 5-15, 5-20, 5-25, 10-15, 10-20, 10-25, 6-13, or 6-25 units of blood. In another embodiment, said pooled apoptotic cell preparation is comprised of cells present in about 2, 3, 4, 5, 6, 7, 8, 9, 10, 11, 12, 13, 14, 15, 16, 17, 18, 19, 20, 21, 22, 23, 24 or 25 units of blood. The number of units of blood needed is also dependent upon the efficiency of WBC recovery from blood. For example, low efficiency WBC recovery would lead to the need for additional units, while high efficiency WBC recovery would lead to fewer units needed. In some embodiments, each unit is a bag of blood. In another embodiment, a pooled apoptotic cell preparation is comprised of cells present in at least 25 units of blood, at least 50 units of blood, or at least 100 units of blood.

In some embodiments, the units of blood comprise white blood cell (WBC) fractions from blood donations. In another embodiment, the donations may be from a blood center or blood bank. In another embodiment, the donations may be from donors in a hospital gathered at the time of preparation of the pooled apoptotic cell preparation. Units of blood comprising WBCs from multiple donors may be saved and maintained in an independent blood bank created for the purpose of compositions as disclosed herein. A blood bank developed for the purpose of compositions and methods thereof as disclosed herein, can supply units of blood comprising WBC from multiple donors and comprises a leukapheresis unit.

In some embodiments, the units of pooled WBCs are not restricted by HLA matching. Therefore, the resultant pooled apoptotic cell preparation comprises cell populations not restricted by HLA matching. Accordingly, in certain embodiments a pooled mononuclear apoptotic cell preparation comprises allogeneic cells.

An advantage of a pooled mononuclear apoptotic cell preparation that is derived from pooled WBCs not restricted by HLA matching, is a readily available source of WBCs and reduced costs of obtaining WBCs.

In some embodiments, pooled blood comprises blood from multiple donors independent of HLA matching. In another embodiment, pooled blood comprises blood from multiple donors wherein HLA matching with the recipient has been taken into consideration. For example, wherein 1 HLA allele, 2 HLA alleles, 3 HLA alleles, 4 HLA alleles, 5 HLA alleles, 6 HLA alleles, or 7 HLA alleles have been matched between donors and recipient. In another embodiment, multiple donors are partially matched, for example some of the donors have been HLA matched wherein 1 HLA allele, 2 HLA alleles, 3 HLA alleles, 4 HLA alleles, 5 HLA alleles, 6 HLA alleles, or 7 HLA alleles have been matched between some of the donors and recipient.

In certain embodiments, some viable non-apoptotic cells (apoptosis resistant) may remain following the induction of apoptosis step described below (Example 1). The presence of these viable non-apoptotic cells is, in some embodiments, is observed prior to an irradiation step. These viable non-apoptotic cells may be able to proliferate or be activated. In some embodiments, the pooled mononuclear apoptotic cell preparation derived from multiple donors may be activated against the host, activated against one another, or both.

In some embodiments, an irradiated cell preparation as disclosed herein has suppressed cellular activation and reduced proliferation compared with a non-irradiated cell preparation. In another embodiment, the irradiation comprises gamma irradiation or UV irradiation. In another embodiment, an irradiated cell preparation has a reduced number of non-apoptotic cells compared with a non-irradiated cell preparation. In another embodiment, the irradiation comprises about 15 Grey units (Gy). In another embodiment, the irradiation comprises about 20 Grey units (Gy). In another embodiment, the irradiation comprises about 25 Grey units (Gy). In another embodiment, the irradiation comprises about 30 Grey units (Gy). In another embodiment, the irradiation comprises about 35 Grey units (Gy). In another embodiment, the irradiation comprises about 40 Grey units (Gy). In another embodiment, the irradiation comprises about 45 Grey units (Gy). In another embodiment, the irradiation comprises about 50 Grey units (Gy). In another embodiment, the irradiation comprises about 55 Grey units (Gy). In another embodiment, the irradiation comprises about 60 Grey units (Gy). In another embodiment, the irradiation comprises about 65 Grey units (Gy). In another embodiment, the irradiation comprises up to 2500 Gy. In another embodiment, an irradiated pooled apoptotic cell preparation maintains the same or a similar apoptotic profile, stability and efficacy as a non-irradiated pooled apoptotic cell preparation.

In some embodiments, a pooled mononuclear apoptotic cell preparation as disclosed herein is stable for up to 24 hours. In another embodiment, a pooled mononuclear apoptotic cell preparation is stable for at least 24 hours. In another embodiment, a pooled mononuclear apoptotic cell preparation is stable for more than 24 hours. In yet another embodiment, a pooled mononuclear apoptotic cell preparation as disclosed herein is stable for up to 36 hours. In still another embodiment, a pooled mononuclear apoptotic cell preparation is stable for at least 36 hours. In a further embodiment, a pooled mononuclear apoptotic cell preparation is stable for more than 36 hours. In another embodiment, a pooled mononuclear apoptotic cell preparation as disclosed herein is stable for up to 48 hours. In another embodiment, a pooled mononuclear apoptotic cell preparation is stable for at least 48 hours. In another embodiment, a pooled mononuclear apoptotic cell preparation is stable for more than 48 hours.

Disclosed herein are methods of producing the pooled cell preparation comprising an irradiation step which may preserve the early apoptotic, immune modulation, and stability properties observed in an apoptotic preparation derived from a single match donor wherein the cell preparation may not include an irradiation step. In another embodiment, a pooled mononuclear apoptotic cell preparation as disclosed herein does not elicit a graft versus host disease (GVHD) response.

Irradiation of the cell preparation is considered safe in the art. Irradiation procedures are currently performed on a routine basis to donated blood to prevent reactions to WBC.

In another embodiment, the percent of apoptotic cells in a pooled mononuclear apoptotic cell preparation as disclosed herein is close to 100%, thereby reducing the fraction of living non-apoptotic cells in the cell preparation. In some embodiments, the percent of apoptotic cells is at least 40%. In another embodiment, the percent of apoptotic cells is at least 50%. In yet another embodiment, the percent of apoptotic cells is at least 60%. In still another embodiment, the percent of apoptotic cells is at least 70%. In a further embodiment, the percent of apoptotic cells is at least 80%. In another embodiment, the percent of apoptotic cells is at least 90%. In yet another embodiment, the percent of apoptotic cells is at least 99%. Accordingly, a cell preparation comprising a reduced or non-existent fraction of living non-apoptotic cells may in one embodiment provide a pooled mononuclear apoptotic cell preparation that does not elicit GVHD in a recipient.

Alternatively, in another embodiment, the percentage of living non-apoptotic WBC is reduced by specifically removing the living cell population, for example by targeted precipitation. In another embodiment, the percent of living non-apoptotic cells may be reduced using magnetic beads that bind to phosphatidylserine. In another embodiment, the percent of living non-apoptotic cells may be reduced using magnetic beads that bind a marker on the cell surface of non-apoptotic cells but not apoptotic cells. In another embodiment, the apoptotic cells may be selected for further preparation using magnetic beads that bind to a marker on the cell surface of apoptotic cells but not non-apoptotic cells. In yet another embodiment, the percentage of living non-apoptotic WBC is reduced by the use of ultrasound.

In one embodiment, the apoptotic cells are from pooled third party donors.

In some embodiments, a pooled cell preparation comprises at least one cell type selected from: lymphocytes, monocytes or natural killer cells. In another embodiment, a pooled cell preparation comprises an enriched population of mononuclear cells. In some embodiments, a pooled mononuclear is a mononuclear enriched cell preparation comprises cell types selected from: lymphocytes, monocytes or natural killer cells. In another embodiment, the mononuclear enriched cell preparation comprises no more than 15%, alternatively no more than 10%, typically no more than 5% polymorphonuclear leukocytes, also known as granulocytes (i.e., neutrophils, basophils and eosinophils). In another embodiment, a pooled mononuclear cell preparation is devoid of granulocytes.

In another embodiment, the pooled mononuclear enriched cell preparation comprises no more than 15%, alternatively no more than 10%, typically no more than 5% CD15$^{high}$ expressing cells. In some embodiments, a pooled apoptotic cell preparation comprises less than 15% CD15 high expressing cells.

In some embodiments, the pooled mononuclear enriched cell preparation disclosed herein comprises at least 80% mononuclear cells, at least 85% mononuclear cells, alternatively at least 90% mononuclear cells, or at least 95% mononuclear cells. According to some embodiments, the pooled mononuclear enriched cell preparation disclosed herein comprises at least 85% mononuclear cells.

In another embodiment, any pooled cell preparation that has a final pooled percent of mononuclear cells of at least 80% is considered a pooled mononuclear enriched cell preparation as disclosed herein. Thus, pooling cell preparations having increased polymorphonuclear cells (PMN) with cell preparations having high mononuclear cells with a resultant "pool" of at least 80% mononuclear cells comprises a preparation as disclosed herein. According to some embodiments, mononuclear cells comprise lymphocytes and monocytes.

A skilled artisan would appreciate that the term "mononuclear cells" may encompass leukocytes having a one lobed nucleus. In another embodiment, a pooled apoptotic cell preparation as disclosed herein comprises less than 5% polymorphonuclear leukocytes.

In some embodiments, the apoptotic cells are T-cells. In another embodiment, the apoptotic cells are derived from the CAR T-cell population.

Surprisingly, the apoptotic cells reduce production of cytokines associated with the cytokine storm including IL-6, and interferon-gamma (IFN-γ), alone or in combination, while the effectiveness of CAR T-cell therapy was maintained (Example 2).

In another embodiment, the effect of apoptotic cells on cytokine expression levels in macrophages, DCs, or a combination thereof, results in reduction of CRS. In another embodiment, the effect of apoptotic cells on cytokine expression levels in macrophages, DCs, or a combination thereof, results in reduction of severe CRS. In another embodiment, the effect of apoptotic cells on cytokine expression levels in macrophages, DCs, or a combination thereof, results in suppression of CRS. In another embodiment, the effect of apoptotic cells on cytokine expression levels in macrophages, DCs, or a combination thereof, results in suppression of severe CRS. In another embodiment, the effect of apoptotic cells on cytokine expression levels in macrophages, DCs, or a combination thereof, results in inhibition of CRS. In another embodiment, the effect of apoptotic cells on cytokine expression levels in macrophages, DCs, or a combination thereof, results in inhibition of severe CRS. In another embodiment, the effect of apoptotic cells on cytokine expression levels in macrophages, DCs, or a combination thereof, results in prevention of CRS. In another embodiment, the effect of apoptotic cells on cytokine expression levels in macrophages, DCs, or a combination thereof, results in prevention of severe CRS.

In another embodiment, the apoptotic cells trigger death of T-cells, but not via changes in cytokine expression levels.

In another embodiment, apoptotic cells antagonize the priming of macrophages and dendritic cells to secrete cytokines that would otherwise amplify the cytokine storm. In another embodiment, apoptotic cells increase Tregs which suppress the inflammatory response and/or prevent excess release of cytokines.

In some embodiments, administration of apoptotic cells inhibits one or more pro-inflammatory cytokines. In some embodiments, the pro-inflammatory cytokine comprises IL-1beta, IL-6, TNF-alpha, or IFN-gamma, or any combination thereof. In some embodiments, inhibition of one or more pro-inflammatory cytokines comprises downregulation of pr0-inflammatory cytokines, wherein a reduced amount of one or more pro-inflammatory cytokines is secreted.

In another embodiment, administration of apoptotic cells promotes the secretion of one or more anti-inflammatory cytokines. In some embodiments, the anti-inflammatory cytokine comprises TGF-beta, IL10, or PGE2, or any combination thereof.

In some embodiments, administration of apoptotic cells inhibits one or more pro-inflammatory cytokine and inhibits on or more anti-inflammatory cytokine. In some embodiments, inhibition of one or more pro-inflammatory cytokine and one or more anti-inflammatory cytokine comprises downregulation of the one or more pro-inflammatory cytokines followed by downregulation of one or more anti-inflammatory cytokine, wherein a reduced amount of the one or more pro-inflammatory cytokines and the one or move anti-inflammatory cytokine is secreted. A skilled artisan would appreciate that apoptotic cells may therefore have a beneficial effect on aberrant innate immune response, with downregulation of both anti- and pro-inflammatory cytokines. In some embodiments, this beneficial effect may follow recognition of PAMPs and DAMPs by components of the innate immune system.

In another embodiment, administration of apoptotic cells inhibits dendritic cell maturation following exposure to TLR ligands. In another embodiment, administration of apoptotic cells creates potentially tolerogenic dendritic cells, which in some embodiments, are capable of migration, and in some embodiments, the migration is due to CCR7. In another embodiment, administration of apoptotic cells elicits various signaling events which in one embodiment is TAM receptor signaling (Tyro3, Axl and Mer) which in some embodiments, inhibits inflammation in antigen-presenting cells.

In some embodiments, Tyro-3, Axl, and Mer constitute the TAM family of receptor tyrosine kinases (RTKs) characterized by a conserved sequence within the kinase domain and adhesion molecule-like extracellular domains. In another embodiment, administration of apoptotic cells activates signaling through MerTK. In another embodiment, administration of apoptotic cells activates the phosphatidylinositol 3-kinase (PI3K)/AKT pathway, which in some embodiments, negatively regulates NF-κB. In another embodiment, administration of apoptotic cells negatively regulates the inflammasome which in one embodiment leads to inhibition of pro-inflammatory cytokine secretion, DC maturation, or a combination thereof. In another embodiment, administration of apoptotic cells upregulates expression of anti-inflammatory genes such as *Nr4a, Thbs1,* or a combination thereof. In another embodiment, administration of apoptotic cells induces a high level of AMP which in some embodiments, is accumulated in a Pannexin1-dependent manner. In another embodiment, administration of apoptotic cells suppresses inflammation.

In some embodiments, the composition comprising the early apoptotic cells, as described herein, includes the composition in combination with an antibody. In some embodiments, the antibody is directed against a tumor cell antigen. In another embodiment, the antibody is directed against CD20. In another embodiment, the antibody is rituximab (Rtx).

In some embodiments, early apoptotic cells and an antibody are comprised in the same composition. In some embodiments, early apoptotic cells and an antibody are comprised in different compositions. In some embodiments, administration of a combination of early apoptotic cells and an antibody, or composition(s) thereof are concurrent. In some embodiments, administration of a combination of early apoptotic cells and an antibody, or composition(s) thereof comprises administration of apoptotic cells or a composition thereof, prior to the antibody. In some embodiments, administration of a combination of early apoptotic cells and an antibody, or composition(s) thereof comprises administration of apoptotic cells or a composition thereof, following administration of the antibody.

In another embodiment, the antibody is Trastuzumab (Herceptin; Genentech): humanized IgG1, which is directed against ERBB2. In another embodiment, the antibody is Bevacizumab (Avastin; Genentech/Roche): humanized IgG1, which is directed against VEGF. In another embodiment, the antibody is Cetuximab (Erbitux; Bristol-Myers Squibb): chimeric human-murine IgG1, which is directed against EGFR. In another embodiment, the antibody is Panitumumab (Vectibix; Amgen): human IgG2, which is directed against EGFR. In another embodiment, the antibody is Ipilimumab (Yervoy; Bristol-Myers Squibb): IgG1, which is directed against CTLA4.

In another embodiment, the antibody is Alemtuzumab (Campath; Genzyme): humanized IgG1, which is directed against CD52. In another embodiment, the antibody is Ofatumumab (Arzerra; Genmab): human IgG1, which is directed against CD20. In another embodiment, the antibody is Gemtuzumab ozogamicin (Mylotarg; Wyeth): humanized IgG4, which is directed against CD33. In another embodiment, the antibody is Brentuximab vedotin (Adcetris; Seattle Genetics): chimeric IgG1, which is directed against CD30. In another embodiment, the antibody is 90Y-labelled ibritumomab tiuxetan (Zevalin; IDEC Pharmaceuticals): murine IgG1, which is directed against CD20. In another embodiment, the antibody is 131I-labelled tositumomab (Bexxar; GlaxoSmithKline): murine IgG2, which is directed against CD20.

In another embodiment, the antibody is Ramucirumab, which is directed against vascular endothelial growth factor receptor-2 (VEGFR-2). In another embodiment, the antibody is ramucirumab (Cyramza Injection, Eli Lilly and Company), blinatumomab (BLINCYTO, Amgen Inc.), pembrolizumab (KEYTRUDA, Merck Sharp & Dohme Corp.), obinutuzumab (GAZYVA, Genentech, Inc.; previously known as GA101), pertuzumab injection (PERJETA, Genentech, Inc.), or denosumab (Xgeva, Amgen Inc.). In another embodiment, the antibody is Basiliximab (Simulect; Novartis). In another embodiment, the antibody is Daclizumab (Zenapax; Roche).

In another embodiment, the antibody administered in combination with apoptotic cells is directed to a tumor or cancer antigen or a fragment thereof, that is described herein and/or that is known in the art. In another embodiment, the antibody is directed to a tumor-associated antigen. In another embodiment, the antibody is directed to a tumor-associated antigen or a fragment thereof that is an angiogenic factor.

***Compositions***

As used herein, the terms "composition" and pharmaceutical composition" may in some embodiments, be used interchangeably having all the same qualities and meanings. In some embodiments, disclosed herein is a pharmaceutical composition for the treatment of a condition or disease as described herein.

In another embodiment, the composition disclosed herein comprises apoptotic cells and a pharmaceutically acceptable excipient.

In another embodiment, apoptotic cells comprised in the composition are pooled third party donor cells.

In some embodiments, the composition further comprises an additional agent. In some embodiments, the composition further comprises an antibody or a functional fragment thereof. In some embodiments, the composition further a RtX antibody or a functional fragment thereof. In some embodiments, apoptotic cells and an antibody or a functional fragment thereof are comprised in separate compositions. In some embodiments, apoptotic cells and an antibody or a functional fragment thereof may be comprised in the same composition.

A skilled artisan would appreciate that a "pharmaceutical composition" may encompass a preparation of one or more of the active ingredients described herein with other chemical components such as physiologically suitable carriers and excipients. The purpose of a pharmaceutical composition is to facilitate administration of a compound to an organism.

In some embodiments, a pharmaceutical composition comprises an early apoptotic cell population as described herein, and a pharmaceutically acceptable excipient.

A skilled artisan would appreciate that the phrases "physiologically acceptable carrier", "pharmaceutically acceptable carrier", "physiologically acceptable excipient", and "pharmaceutically acceptable excipient", may be used interchangeably may encompass a carrier, excipient, or a diluent that does not cause significant irritation to an organism and does not abrogate the biological activity and properties of the administered active ingredient.

A skilled artisan would appreciate that an "excipient" may encompass an inert substance added to a pharmaceutical composition to further facilitate administration of an active ingredient. In some embodiments, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

In some embodiments, compositions are administered at the same time. In an alternative embodiment, compositions are administered at different times. In another embodiment, compositions comprising apoptotic cells are administered prior to infusion or genetically modified immune cells or receptors thereof. In another embodiment, compositions comprising apoptotic cells are administered prior to cytotoxic T-cell infusion. In another embodiment, compositions comprising apoptotic cells are administered prior to natural killer cell infusion. In another embodiment, compositions comprising apoptotic cells are administered prior to dendritic infusion. In another embodiment, compositions comprising apoptotic cells are administered prior to infusion of a genetically modified T-cell receptor.

In another embodiment, compositions comprising apoptotic cells are administered about 24 hours prior to genetically modified immune cell or receptor thereof infusion.

In some embodiments, compositions are administered at the same time. In an alternative embodiment, compositions are administered at different times. In another embodiment, compositions comprising apoptotic cells are administered prior to administration of an antibody or fragment thereof, or a composition comprising an antibody or fragment thereof.

In another embodiment, compositions comprising apoptotic cells are administered about 2 hours, 4 hours, 6 hours, 8 hours, 10 hours, 12 hours, 14 hours, 16 hours, 18 hours 20 hours, 22 hours, 24 hours, 36 hours, 48 hours, 60 hours, or 72 hours prior to an antibody or fragment thereof, or composition comprising the antibody or fragment thereof.

In another embodiment, compositions comprising apoptotic cells are administered about 1 day, 2 days, 3 days, 4 days, 5 days, 6 days, 7 days, 8 days, 9 days, 10 days, 11 days, 12 days, 13 days 14 days, or 15 days prior to an antibody or fragment thereof, or composition comprising the antibody or functional fragment thereof.

In another embodiment, compositions comprising apoptotic cells are administered after infusion of an antibody or fragment thereof, or composition comprising the antibody or fragment thereof. In another embodiment, composition comprising apoptotic cells are administered after an antibody or fragment thereof, or composition comprising the antibody or fragment thereof. In another embodiment, compositions comprising apoptotic cells are administered about 24 hours after an antibody or fragment thereof, or composition comprising the antibody or fragment thereof. In another embodiment, compositions comprising apoptotic cells are administered after administration of an antibody or fragment thereof, or composition comprising the antibody or fragment thereof. In another embodiment, compositions comprising apoptotic cells are administered about 2 hours, 4 hours, 6 hours, 8 hours, 10 hours, 12 hours, 14 hours, 16 hours, 18 hours 20 hours, 22 hours, 24 hours, 36 hours, 48 hours, 60 hours, or 72 hours after administration of an antibody or fragment thereof, or composition comprising the antibody or fragment thereof.

In another embodiment, compositions comprising apoptotic cells are administered about 1 day, 2 days, 3 days, 4 days, 5 days, 6 days, 7 days, 8 days, 9 days, 10 days, 11 days, 12 days, 13 days 14 days, or 15 days after an antibody or fragment thereof, or composition comprising the antibody or functional fragment thereof.

In some embodiments, a composition comprising apoptotic cells is administered in combination with an additional agent. In some embodiments, the additional agent is an antibody.

In some embodiments, the composition as disclosed herein comprises a therapeutic composition. In some embodiments, the composition as disclosed herein comprises a therapeutic efficacy.

In some embodiments, a composition as disclosed herein is administered once. In another embodiment, the composition is administered twice. In another embodiment, the composition is administered three times. In another embodiment, the composition is administered four times. In another embodiment, the composition is administered at least four times. In another embodiment, the composition is administered more than four times.

In some embodiments, the composition as disclosed herein is a therapeutic composition. In another embodiment, the composition as disclosed herein has therapeutic efficacy.

*Formulations*

Pharmaceutical compositions disclosed herein comprising early apoptotic cell populations, can be conveniently provided as sterile liquid preparations, e.g., isotonic aqueous solutions, suspensions, emulsions, dispersions, or viscous compositions, which may be buffered to a selected pH, Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions can be prepared by incorporating the early apoptotic cell population described herein in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. According to the disclosure herein, however, any vehicle, diluent, or additive used would have to be compatible with the genetically modified immunoresponsive cells or their progenitors.

The compositions or formulations described herein can be isotonic, i.e., they can have the same osmotic pressure as blood and lacrimal fluid. The desired isotonicity of the compositions as disclosed herein may be accomplished using sodium chloride, or other pharmaceutically acceptable agents such as dextrose, boric acid, sodium tartrate, propylene glycol or other inorganic or organic solutes. Sodium chloride may be preferred particularly for buffers containing sodium ions.

Viscosity of the compositions, if desired, can be maintained at the selected level using a pharmaceutically acceptable thickening agent. Methylcellulose may be preferred because it is readily and economically available and is easy to work with.

Other suitable thickening agents include, for example, xanthan gum, carboxymethyl cellulose, hydroxypropyl cellulose, carbomer, and the like. The preferred concentration of the thickener will depend upon the agent selected. The important point is to use an amount that will achieve the selected viscosity. Obviously, the choice of suitable carriers and other additives will depend on the exact route of administration and the nature of the particular dosage form, e.g., liquid dosage form (e.g., whether the composition is to be formulated into a solution, a suspension, gel or another liquid form, such as a time release form or liquid-filled form).

Those skilled in the art will recognize that the components of the compositions or formulations should be selected to be chemically inert and will not affect the viability or efficacy of the early apoptotic cell populations as described herein. This will present no problem to those skilled in chemical and pharmaceutical principles, or problems can be readily avoided by reference to standard texts or by simple experiments (not involving undue experimentation), from this disclosure and the documents cited herein.

One consideration concerning the therapeutic use of genetically modified immunoresponsive cells disclosed herein is the quantity of cells necessary to achieve an optimal effect. The quantity of cells to be administered will vary for the subject being treated. In a some embodiments, between $10^4$ to $10^{10}$, between $10^5$ to $10^9$, or between $10^6$ and $10^8$ genetically modified immunoresponsive cells disclosed herein are administered to a human subject. More effective cells may be administered in even smaller numbers. In some embodiments, at least about $1 \times 10^8$, $2 \times 10^8$, $3 \times 10^8$, $4 \times 10^8$, and $5 \times 10^8$ genetically modified immunoresponsive cells disclosed herein are administered to a human subject. The precise determination of what would be considered an effective dose may be based on factors individual to each subject, including their size, age, sex, weight, and condition of the particular subject. Dosages can be readily ascertained by those skilled in the art from this disclosure and the knowledge in the art.

The skilled artisan can readily determine the amount of cells and optional additives, vehicles, and/or carrier in compositions and to be administered. Typically, any additives (in addition to the active cell(s) and/or agent(s)) are present in an amount of 0.001 to 50% (weight) solution in phosphate buffered saline, and the active ingredient is present in the order of micrograms to milligrams, such as about 0.0001 to about 5 wt %. In another embodiment, about 0.0001 to about 1 wt %. In still another embodiment, about 0.0001 to about 0.05 wt% or about 0.001 to about 20 wt %. In a further embodiment, about 0.01 to about 10 wt %. In another embodiment, about 0.05 to about 5 wt %. Of course, for any composition to be administered to an animal or human, and for any particular route of administration, it is preferred to determine therefore: toxicity, such as by determining the lethal dose (LD) and LD50 in a suitable animal model e.g., rodent such as mouse; and, the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

*Uses*

In some embodiment, "treating" comprises therapeutic treatment and "preventing" comprises prophylactic or preventative measures, wherein the object is to prevent or lessen the targeted pathologic condition or disorder as described hereinabove. Thus, in some embodiments, treating may include directly affecting or curing, suppressing, inhibiting, preventing, reducing the severity of, delaying the onset of, reducing symptoms associated with the disease, disorder or condition, or a combination thereof. Thus, in some embodiments, "treating," "ameliorating," and "alleviating" refer inter alia to delaying progression, expediting remission, inducing remission, augmenting remission, speeding recovery, increasing efficacy of or decreasing resistance to alternative therapeutics, or a combination thereof. In some embodiments, "preventing" refers, inter alia, to delaying the onset of symptoms, preventing relapse to a disease, decreasing the number or frequency of relapse episodes, increasing latency between symptomatic episodes, or a combination thereof. In some embodiments, "suppressing" or "inhibiting", refers inter alia to reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

A skilled artisan would appreciate that the term "antigen recognizing receptor" may encompass a receptor that is capable of activating an immune cell (e.g., a T-cell) in response to antigen binding. Exemplary antigen recognizing receptors may be native or endogenous T-cell receptors or chimeric antigen receptors in which a tumor antigen-binding domain is fused to an intracellular signaling domain capable of activating an immune cell (e.g., a T-cell).

A skilled artisan would appreciate that the term "disease" may encompass any condition or disorder that damages or interferes with the normal function of a cell, tissue, or organ. Examples of diseases include neoplasia or pathogen infection of cell.

A skilled artisan would appreciate that the term "pathogen" may encompass a virus, bacteria, fungi, parasite or protozoa capable of causing disease.

A skilled artisan would appreciate that the term "tumor antigen" or "tumor associated antigen" may encompass an antigen (e.g., a polypeptide) that is uniquely or differentially expressed on a tumor cell compared to a normal or non- IS neoplastic cell. With reference to the compositions disclosed herein, a tumor antigen includes any polypeptide expressed by a tumor that is capable of activating or inducing an immune response via an antigen recognizing receptor (e.g., CD 19, MUCI) or capable of suppressing an immune response via receptor-ligand binding (e.g., CD47, PD-L1/L2, B7.1/2).

A skilled artisan would appreciate that the term "virus antigen" may encompass a polypeptide expressed by a virus that is capable of inducing an immune response.

The compositions as disclosed herein are envisioned to either comprise the active ingredient or specified step, consist of the active ingredient or specified step, or consist essentially of the active ingredient or specified step.

A skilled artisan would appreciate that the term "treatment" may encompass clinical intervention in an attempt to alter the disease course of the individual or cell being treated, and can be performed either for prophylaxis or during the course of clinical pathology. Therapeutic effects of treatment include preventing occurrence or recurrence of disease, alleviation of symptoms, diminishment of any direct or indirect pathological consequences of the disease, preventing metastases, decreasing the rate of disease progression, amelioration or palliation of the disease state, and remission or improved prognosis. By preventing progression of a disease or disorder, a treatment can prevent deterioration due to a disorder in an affected or diagnosed subject or a subject suspected of having the disorder, but also a treatment may prevent the onset of the disorder or a symptom of the disorder in a subject at risk for the disorder or suspected of having the disorder.

A skilled artisan would appreciate that the term "subject" may encompass a vertebrate, in some embodiments, to a mammal, and in some embodiments, to a human. Subject may also refer, in some embodiments, to domesticated such as cows, sheep, horses, cats, dogs and laboratory animals such as mice, rats, gerbils, hamsters, etc.

A skilled artisan would recognize that an "effective amount" (or, "therapeutically effective amount") may encompass an amount sufficient to effect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the antigen-binding fragment administered.

The modified cells can be administered in any physiologically acceptable vehicle, normally intravascularly, although they may also be introduced into bone or other convenient site where the cells may find an appropriate site for regeneration and differentiation (e.g., thymus). Usually, at least $1x10^5$ cells will be administered, eventually reaching $1x10^{10}$ or more. Genetically modified immunoresponsive cells disclosed herein may comprise a purified population of cells. Those skilled in the art can readily determine the percentage of genetically modified immunoresponsive cells in a population using various well-known methods, such as fluorescence activated cell sorting (FACS). In some embodiments, ranges of purity in populations comprising genetically modified immunoresponsive cells are about 50 to about 55%, about 55 to about 60%, and about 65 to about 70%. In other embodiments, the purity is about 70 to about 75%, about 75 to about 80%, about 80 to about 85%. In further embodiments, the purity is about 85 to about 90%, about 90 to about 95%, and about 95 to about 100%. Dosages can be readily adjusted by those skilled in the art (e.g., a decrease in purity may require an increase in dosage). The cells can be introduced by injection, catheter, or the like. If desired, factors can also be included, including interleukins, e.g. IL-2, IL-3, IL-6, IL-1 1, IL7, IL12, ILIS, IL21, as well as the other interleukins, the colony stimulating factors, such as G-, M- and GM-CSF, interferons, e.g. gamma-interferon and erythropoietin.

Compositions include pharmaceutical compositions comprising genetically modified immunoresponsive cells or their progenitors and a pharmaceutically acceptable carrier. Administration can be autologous or heterologous. For example, immunoresponsive cells, or progenitors can be obtained from one subject, and administered to the same subject or a different, compatible subject. Peripheral blood derived immunoresponsive cells disclosed herein or their progeny (e.g., *in vivo, ex vivo* or *in vitro* derived) can be administered via localized injection, including catheter administration, systemic injection, localized injection, intravenous injection, or parenteral administration. When administering a therapeutic composition as disclosed herein (e.g., a pharmaceutical composition containing a genetically modified immunoresponsive cell), it will generally be formulated in a unit dosage injectable form (solution, suspension, emulsion.

In some embodiments, the subject is a human subject. In some embodiments, the subject is a child. In some embodiments, the subject is an adult. In some embodiments, the subject is animal mammal.

In some embodiments, the early apoptotic cell population comprises a stable population cell, wherein said cell population is stable for greater than 24 hours. Stable populations of early apoptotic cells have been described in detail above. In some embodiments, the early apoptotic cell population comprises a population of cells devoid of cell aggregates. Early apoptotic cell populations devoid of aggregates and methods of making them have been described in detail above.

In some embodiments, the composition comprises an autologous early apoptotic cell population. In some embodiments, the composition comprises an allogeneic early apoptotic cell population.

In some embodiments, administration of the early apoptotic cell populations or compositions thereof comprise administering a single infusion of said apoptotic cell population or composition thereof. In some embodiments, a single infusion may be administered as a prophylactic to a subject predetermined to be at risk for a cancer or tumor. In some embodiments, a single infusion may be administered to a subject having a cancer or tumor on a regular basis as a part of the subject therapeutic treatment. In some embodiments, a single infusion may be administered as a prophylactic to a subject having a cancer or tumor in order to prevent, reduce the risk of, or delay the appearance of metastatic cancer.

In some embodiments, administration of early apoptotic cell populations or compositions thereof comprises administering multiple infusions of said apoptotic cell population or composition thereof.

In some embodiments, multiple infusions comprise at least two infusions. In some embodiments, multiple infusions comprise 2 infusions. In some embodiments, multiple infusions comprise more than 2 infusions. In some embodiments, multiple infusions comprise at least 3 infusions. In some embodiments, multiple infusions comprise 3 infusions. In some embodiments, multiple infusions comprise more than 3 infusions. In some embodiments, multiple infusions comprise at least 4 infusions. In some embodiments, multiple infusions comprise 4 infusions. In some embodiments, multiple infusions comprise more than 4 infusions. In some embodiments, multiple infusions comprise at least 5 infusions. In some embodiments, multiple infusions comprise 5 infusions. In some embodiments, multiple infusions comprise more than 5 infusions. In some embodiments, multiple infusions comprise at least six infusions. In some embodiments, multiple infusions comprise 6 infusions. In some embodiments, multiple infusions comprise more than 6 infusions. In some embodiments, multiple infusions comprise at least 7 infusions. In some embodiments, multiple infusions comprise 7 infusions. In some embodiments, multiple infusions comprise more than 7 infusions. In some embodiments, multiple infusions comprise at least 8 infusions. In some embodiments, multiple infusions comprise 8 infusions. In some embodiments, multiple infusions comprise more than 8 infusions. In some embodiments, multiple infusions comprise at least nine infusions. In some embodiments, multiple infusions comprise 9 infusions. In some embodiments, multiple infusions comprise more than 9 infusions. In some embodiments, multiple infusions comprise at least 10 infusions. In some embodiments, multiple infusions comprise 10 infusions. In some embodiments, multiple infusions comprise more than 10 infusions.

In some embodiments, multiple infusions comprise smaller amounts of early apoptotic cell, wherein the total dosage of cells administered is the sum of the infusions.

In some embodiments, multiple infusions are administered over a period of hours. In some embodiments, multiple infusions are administered over a period of days. In some embodiments, multiple infusions are administered over a period of hours, wherein there is at least 12 hours between infusions. In some embodiments, multiple infusions are administered over a period of hours, wherein there is at least 24 hours between infusions. In some embodiments, multiple infusions are administered over a period of hours, wherein there is at least a day between infusions. In some embodiments, multiple infusions are administered over a period of hours, wherein there is at least two days between infusions. In some embodiments, multiple infusions are administered over a period of hours, wherein there is at least three days between infusions. In some embodiments, multiple infusions are administered over a period of hours, wherein there is at least four days between infusions. In some embodiments, multiple infusions are administered over a period of hours, wherein there is at least five days between infusions. In some embodiments, multiple infusions are administered over a period of hours, wherein there is at least six days between infusions. In some embodiments, multiple infusions are administered over a period of hours, wherein there is at least seven days between infusions. In some embodiments, multiple infusions are administered over a period of hours, wherein there is at least a week between infusions. In some embodiments, multiple infusions are administered over a period of hours, wherein there is at least two weeks between infusions.

In some embodiments, the amount of cells in multiple infusions is essentially equivalent one to the other. In some embodiments, the amount of cells in multiple infusions is different one to the other.

In some embodiments, an additional chemotherapeutic agent or an immune modulator is administered to said subject.

In some embodiments, an immune modulator comprises an antibody or a functional fragment thereof. In some embodiments, an antibody or functional fragment thereof comprises a monoclonal antibody, a single chain antibody, an Fab fragment, an F(ab')2 fragment, or an Fv fragment.

In some embodiments, disclosed herein are active fragments of any one of the polypeptides or peptide domains disclosed herein. A skilled artisan would appreciate that the term "a fragment" may encompass at least 5, 10, 13, or 15 amino acids. In other embodiments, a fragment is at least 20 contiguous amino acids. Fragments disclosed herein can be generated by methods known to those skilled in the art or may result from normal protein processing (e.g., removal of amino acids from the nascent polypeptide that are not required for biological activity or removal of amino acids by alternative mRNA splicing or alternative protein processing events).

The terms "antibody" and "immunoglobulin" are used interchangeably in the broadest sense and specifically refer to a polyclonal antibody, a monoclonal antibody, or any fragment thereof, which retains the binding activity of the antibody. In certain embodiments, compositions disclosed herein comprise use of a chimeric antibody, a humanized antibody, or a human antibody.

In some embodiments, the term "antibody" refers to intact molecules as well as functional fragments thereof, such as Fab, F(ab')2, and Fv that are capable of specifically interacting with a desired target as described herein, for example, binding to phagocytic cells. In some embodiments, the antibody fragments comprise:

(1) Fab, the fragment which contains a monovalent antigen-binding fragment of an antibody molecule, which can be produced by digestion of whole antibody with the enzyme papain to yield an intact light chain and a portion of one heavy chain;
(2) Fab', the fragment of an antibody molecule that can be obtained by treating whole antibody with pepsin, followed by reduction, to yield an intact light chain and a portion of the heavy chain; two Fab' fragments are obtained per antibody molecule;

(3) (Fab')2, the fragment of the antibody that can be obtained by treating whole antibody with the enzyme pepsin without subsequent reduction; F(ab')2 is a dimer of two Fab' fragments held together by two disulfide bonds;

(4) Fv, a genetically engineered fragment containing the variable region of the light chain and the variable region of the heavy chain expressed as two chains; and

(5) Single chain antibody ("SCA"), a genetically engineered molecule containing the variable region of the light chain and the variable region of the heavy chain, linked by a suitable polypeptide linker as a genetically fused single chain molecule.

Methods of making these fragments are known in the art. (See for example, Harlow and Lane, Antibodies: A Laboratory Manual, Cold Spring Harbor Laboratory, New York, 1988).

The antibody fragments may be prepared by proteolytic hydrolysis of the antibody or by expression in *E. coli* or mammalian cells (e.g. Chinese hamster ovary cell culture or other protein expression systems) of DNA encoding the fragment.

Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647. See also Porter, R. R., Biochem. J., 73: 119-126, 1959. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al., Proc. Nat'l Acad. Sci. USA 69:2659-62, 1972. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as *E. coli.* The recombinant host cells synthesize a single polypeptide chain with a linker peptide bridging the two V domains. Methods for producing sFvs are described, for example, by Whitlow and Filpula, Methods, 2: 97-105, 1991; Bird et al., Science 242:423-426, 1988; Pack et al., Bio/Technology 11:1271-77, 1993; and Ladner et al., U.S. Pat. No. 4,946,778.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry, Methods, 2: 106-10, 1991.

In some embodiments, the antibodies or fragments as described herein may comprise "humanized forms" of antibodies. In some embodiments, the term "humanized forms of antibodies" refers to non-human (e.g. murine) antibodies, which are chimeric molecules of immunoglobulins, immunoglobulin chains or fragments thereof (such as Fv, Fab, Fab', F(ab'). sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al.,Mol. Biol., 222:581 (1991)]. The techniques of Cole *et al.* and Boerner *et al.* are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human can be made by introducing of human immunoglobulin loci into transgenic animals, e.g. mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

In some embodiments, the immune modulator comprises an anti-CD20 monoclonal antibody. In some embodiments, the antiCD20 monoclonal antibody is Rituximab. Rituximab is commercially available and sold under the name Rituxan®, marketed jointly by Biogen and Genentech USA, Inc.

In some embodiments, the composition disclosed herein comprises a first-line therapy.

A skilled artisan would appreciate that the term "first-line therapy" may encompass the first treatment given for a disease. It is often part of a standard set of treatments, such as surgery followed by chemotherapy and radiation. When used by itself, first-line therapy is the one accepted as the best treatment. If it doesn't cure the disease or it causes severe side effects, other treatment may be added or used instead. Also called induction therapy, primary therapy, and primary treatment.

In some embodiments, the composition disclosed herein comprises an adjuvant therapy.

A skilled artisan would appreciate that the term "adjuvant therapy" may encompass a treatment that is given in addition to the primary or initial treatment.

The skilled artisan would appreciate that the term "comprising" is intended to mean that the system includes the recited elements, but not excluding others which may be optional. For example a composition comprising early apoptotic cells this population of cells. Further, the term "consisting essentially of" may encompass a composition that includes the recited elements, for example a composition consisting essentially or early apoptotic cells but exclude other elements that may have an essential significant effect on the performance of the composition. Thus, such a composition may still include a pharmaceutically acceptable excipient that does not comprise an essential activity in treating cancer. Further, "consisting of" encompasses excluding more than traces of other elements. Thus, such a composition consisting of early apoptotic cells would not include more than traces of other elements as disclosed herein.

In some embodiments, the compositions as disclosed herein comprise the various components or steps. However, in another embodiment, the compositions as disclosed herein consist essentially of the various components or steps, where other components or steps may be included. In another embodiment, the compositions as disclosed herein consist of the various components or steps.

In some embodiments, the term "comprise" may encompass the inclusion of other components of the composition which affect the efficacy of the composition that may be known in the art.

A skilled artisan would appreciate that the term "about", may encompass a deviance of between 0.0001-5% from the indicated number or range of numbers. Further, it may encompass a deviance of between 1 -10% from the indicated number or range of numbers. In addition, it may encompass a deviance of up to 25% from the indicated number or range of numbers.

A skilled artisan would appreciate that the singular form "a", "an" and "the" include plural references unless the context clearly dictates otherwise. For example, the term "an agent" or "at least an agent" may include a plurality of agents, including mixtures thereof.

Throughout this application, various embodiments disclosed herein may be presented in a range format.. Accordingly, the description of a range should be considered to have specifically disclosed all the possible sub ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicated number and a second indicated number and "ranging/ranges from" a first indicated number "to" a second indicated number are used herein interchangeably and are meant to include the first and second indicated numbers and all the fractional and integral numerals there between.

The following examples are presented in order to more fully illustrate embodiments disclosed herein.

**EXAMPLES**

***EXAMPLE 1: Apoptotic Cell Production***

***Objective:*** To produce early-apoptotic cells.

***Methods:*** Methods of making populations of early-apoptotic cells have been well documented in International Publication No. WO 2014/087408 and United States Application Publication No. US2015/0275175-A1, see for example, the Methods section preceding the Examples at "Early apoptotic cell population Preparation" and "Generation of apoptotic cells" (paragraphs [0223] through [0288]), and (Reference) Examples 11, 12, 13, and 14.

The flow chart presented in **Figure 1** provides an overview of one embodiment of the steps used during the process of producing a population of early apoptotic cells, wherein anticoagulants were included in the thawing and induction of apoptosis steps. As is described in detailed in Reference Example 14 of International Publication No. WO 2014/087408 and United States Application Publication No. US US-2015-0275175-A1, early apoptotic cell populations were prepared wherein anti-coagulants were added at the time of freezing, or at the time of incubation, or at the time of freezing and at the time of incubation. The anticoagulant used was acid-citrate dextrose, NIH Formula A (ACD formula A) was supplemented with 10 U/ml heparin to a final concentration of 5% ACD of the total volume and 0.5 U/ml heparin.

Briefly: The cells were collected and then frozen with addition of 5% anticoagulant citrate dextrose formula A and 10U/ml heparin (ACDhep) to the freezing media. Thawing, incubation in an apoptosis induction media containing 5% ACDhep, and final product preparation were performed in a closed system.

Apoptosis and viability analysis, potency assay, and cell population characterization were performed in each experiment. In order to establish consistence in production of the early apoptotic cell product, the final product (FP) of initial batches of apoptotic cells were stored at 2-8°C and examined at t0, t24h, t48h and t72h. At each point apoptosis analysis, short potency assay (Applicants CD14+ frozen cells), trypan blue measurement and cell population characterization were performed. The FP was tested for cell count to assess average cell loss during storage and apoptosis and viability analysis.

The methods sections cited above and Example 11 of International Publication No. WO 2014/087408 and United States Application Publication No. US US-2015-0275175-A1 provide details of preparing other embodiment of apoptotic cell populations in the absence of anti-coagulants.

***Methods of preparing irradiated apoptotic cells:*** Similar methods were used to prepare an inactivated apoptotic cell population, wherein a mononuclear early apoptotic cell population comprises a decreased percent of non-quiescence non-apoptotic cells, or a population of cells having a suppressed cellular activation of any living non-apoptotic cells, or a population of cells having a reduced proliferation of any living non-apoptotic cells, or any combination thereof.

Briefly, an enriched mononuclear cell fraction was collected via leukapheresis procedure from healthy, eligible donors. Following apheresis completion, cells were washed and resuspended with freezing media comprising 5% Anticoagulant Citrate Dextrose Solution-Formula A (ACD-A) and 0.5U\ml heparin. Cell were then gradually frozen and transferred to liquid nitrogen for long term storage.

For preparation of irradiated ApoCells, cryopreserved cells were thawed, washed and resuspended with apoptosis induction media comprising 5% ACD-A, 0.5U\ml heparin sodium and $50\mu g$/ml methylprednisolone. Cells were then incubated for 6 hours at 37°C in 5% $CO_2$. At the end of incubation, cells were collected, washed and resuspended in Hartmann's solution using a cell processing system (Fresenius Kabi, Germany). Following manufacturing completion, ApoCell were irradiated at 4000 cGy using g-camera at the radiotherapy unit, Hadassah Ein Kerem. Apoptosis and viability of ApoCell determined using AnnexinV and PI (MBL, MA, USA) staining ($\geq$ 40% and $\leq$ 15%, respectively) via Flow cytometer. Results analyzed using FCS express software. This irradiated APOcell population is considered to include early apoptotic cells, wherein any viable cells present have suppressed cellular activity and reduced or no proliferation capabilities. In certain cases, the Apocell population has no viable non-apoptotic cells. ***Results:***

The stability of the FP produced with inclusion of anticoagulant at freezing and incubation (apoptotic induction) and then stored at 2-8°C are shown below in Table 3.

**Table 3: Cell count\*-** performed using a MICROS 60 hematology analyzer.

| FP Time point | Cell concentration (x10⁶cells\ml) | % of cell loss |
|---|---|---|
| t0 | 20.8 | NA |
| t24h | 20.0 | -3.85 |
| t48h | 20.0 | -3.85 |

(continued)

| FP Time point | Cell concentration (x10^6cells\ml) | % of cell loss |
|---|---|---|
| t72h | 19.7 | -5.3 |

| * Results Representative of 6 (six) experiments. |
|---|

When manufacturing the cells without including an anticoagulant in the induction medium, cells were stable for 24 hours and less stable thereafter. Use of anticoagulants unexpectedly extended the stability of the apoptotic cell population for at least 72 hours, as shown in Table 3.

**Table 4: Trypan blue measurement**

| FP Time point | trypan blue positive cells (%) |
|---|---|
| t0 | 3.0 |
| t24h | 5.9 |
| t48h | 5.2 |
| t72h | 6.5 |

The results of Table 4 show viability of the FP remained high for at least 72 hours.

**Table 5: Apoptosis analysis-** (AnPI staining) performed using Flow Cytometry

| FP Time point | 1.5mM Ca | | |
|---|---|---|---|
| | An-PI- (%) | An+PI- (%) | An+PI+ (%) |
| t0 | 44.3 | 50.9 | 4.8 |
| t24h | 39.0 | 55.9 | 5.1 |
| t48h | 34.8 | 60.1 | 5.1 |
| t72h | 33.4 | 60.5 | 6.1 |

The results of Table 5 show that the percent apoptotic cells versus necrotic cells was maintained over at extended time period of at least 72 hours post preparation of the cells, as was the percentage of early apoptotic cells.

Inclusion of anticoagulants both at the time of freezing and during induction of apoptosis resulted in the most consistently high yield of stable early-apoptotic cells (average yield of early apoptotic cells 61.3±2.6% % versus 48.4 ±5.0%, wherein 100% yield is based on the number of cells at freezing). This high yield was maintained even after 24 hours storage at 2-8°C.

Next a comparison was made between the inclusion of the anticoagulant at freezing or thawing or both, wherein percent (%) recovery was measured as well as stability. Anticoagulant was included in the apoptotic incubation mix for all populations. Table 6 presents the results of these studies.

**Table 6:** Yield and stability comparison of final products (FP) manufactured from cells collected, with ("+") or without ("-") addition of anticoagulant during freezing ("F") and thawing ("Tha")

| Donor ID | # of Collect ed Cells (×10^9, 100%) | % Cell Recovery in Final Product of Collected Cells | | | | | | | |
|---|---|---|---|---|---|---|---|---|---|
| | | FP t0 | | | | FP t24h* | | | |
| | | **F-/Tha-** | **F-/Tha+** | **F+/Tha +** | **F+/Tha -** | **F-/Tha-** | **F-/Tha+** | **F+/Tha +** | **F+/Tha-** |
| *1* | 13.3 | 52.1 | 53.4 | 62.5 | 62 | 52.1 | 48.9 | 62.5 | 62 |
| *2* | 13.6 | 50.5 | 36.7 | 53.5 | 63.5 | 47.6 | 36.7 | 53.1 | 59.7 |
| *3* | 15.0 | 42.7 | 42 | 53.6 | 58.4 | 42.7 | 41.7 | 53.6 | 57.8 |
| *Avg* | 14.0 | 48.4±5.0 | 44.0±8.5 | 56.5±5. 2 | 61.3±2.6 | 47.5±4.7 | 42.4±6.1 | 56.4±5. 3 | 59.8±2.1 |

Additional population analysis comparisons of early apoptotic cell populations (batches of cells) prepared with and without anti-coagulant added, show the consistency of these results.

**Table 7: Cell population analysis comparison between batches prepared with and without anticoagulant**

| Test | Specification | At Thawing | | ApoCell Time 0 h | | ApoCell Time 24 h Storage | |
|---|---|---|---|---|---|---|---|
| | | w\o ACDhep | +ACDhep | w\o ACDhep | +ACDhep | w\o ACDhep | +ACDhep |
| Change in Total Cell Count Percent change (min-max) | >35.0% | 85.5 (79.5-92.5) | 82.8 (67.7-96.4) | 49.9 (46.6-52.3) | 66.7 (62.5-71.2) | 49.0 46.6-50.3) | 66.7 (62.5-71.2) |
| Changes in ApoCell Percent change Range (min-max) | 90.0±10.0 % | | | 100 | 100 | 98.2 (96.2-100) | 100 |
| Cell viability PI exclusion Percent viable Range (min-max) | > 85.0% | 98.0 (97.4-98.4) | 96.0 (91.9-98.1) | 98.5 (97.9-99.2) | 94.6 (93.5-95.5) | 97.7 (96.4-98.6) | 94.5 (93.4-95.1) |
| Identity/ Purity Analysis of cell phenotype Average (%) (maximal calculated range) | CD3 (T cells): 71.9 (50.0-85.0) | 75.7 (71.6-81.4) | 66.5 (60.1-70.1) | 73.3 (70.3-78.3) | 62.8 (61.1-65.3) | 71.6 (61.5-79.1) | 64.2 (61.6-68.1) |
| | ApoCell CD3: 71.6 (50.0-85.0) | | | | | | |
| | CD19 (B cells): 9.3 (3.0-15.0) | 7.5 (4.0-11.1) | 9.8 (8.6-12.0) | 9.0 (7.6-10.2) | 9.9 (9.3-10.2) | 9.5 (8.6-10.3) | 9.7 (9.2-10.4) |
| | ApoCell CD19: 9.5 (4-15) | | | | | | |
| | CD14 (monocytes): 10.1 (2.5-22.0) | 9.8 (6.4-13.0) | 14.0 (8.8-22.1) | 11.6 (10.2-13.3) | 15.4 (8.2-19.3) | 9.3 (4.8-17.2) | 16.1 (9.0-20.4) |
| | ApoCell CD14: 10.6 (2.5- | | | | | | |

(continued)

| Test | Specification | At Thawing | | ApoCell Time 0 h | | ApoCell Time 24 h Storage | |
|---|---|---|---|---|---|---|---|
| | | w\o ACDhep | +ACDhep | w\o ACDhep | +ACDhep | w\o ACDhep | +ACDhep |
| | 22.0) | | | | | | |
| | CD15 high (granulocytes): 0.4 (0-6.0) | 0.2 (0-0.3) | 0.46 (0.18-0.69) | 0.2 (0.1-0.4) | 0.083 (0.08-0.09) | 0.1 (0.1-0.2) | 0.09 (0.07-0.1) |
| | ApoCell CD15high: 0.2 (0-2.0) | | | | | | |
| | CD 56 (NK): 7.2 (1.5-22.0) | 7.4 (2.4-11.0) | 10.1 (6.6-14.2) | 4.7 (2.7-8.0) | 11.2 (7.2-14.2) | 4.9 (2.2-9.2) | 10.0 (6.4-13.0) |
| | ApoCell CD56: 5.2 (1.5-15.0) | | | | | | |

*Percentage of final product cells (yield) in the presence or absence of anticoagulants.* Similar to the results presented above at Table 3, the data presented in Table 6 demonstrates that early apoptotic cells manufactured from cells frozen in the presence of anticoagulant had a beneficial effect on average yield of fresh final product (FP t0) as compared to cells frozen without anticoagulant. The beneficial effect was seen when anticoagulant was used while freezing only (61.3 $\pm$2.6% versus 48.4$\pm$5.0%), or both freezing and thawing (56.5$\pm$5.2% versus 48.4$\pm$5.0%). The beneficial effect was less significant when anticoagulant was used upon thawing only (44.0$\pm$8.5% versus 48.4$\pm$5.0%). These were non-high triglyceride samples.

*Effect of anticoagulants on aggregation.* No cell aggregations were seen in these 3 non-high triglyceride samples, or in 21 additional samples (data not shown). However, in 41 other non-high triglyceride samples manufactured without anticoagulants (data not shown), mild aggregates were seen in 10 (24.4%) and severe aggregates in 5 (12.2%); thus, anticoagulants avoid completely cell aggregates.

*Effect of anticoagulants on stability.* Fresh FPs manufactured with- or without anticoagulants were stored at 2-8°C for 24 hours to determine whether addition of ACDhep to the manufacturing procedure impairs the stability of the FP. Cells were sampled following 24 hours of storage and yield was calculated In cell count. Similar to the results shown in Table 3 for extended time periods (up to 72 hours), Table 6 shows that the beneficial effect was kept and observed when anticoagulant was used while freezing only (59.8$\pm$2.1% versus 47.5$\pm$4.7%), or both freezing and thawing (56.4$\pm$5.3% versus 47.5$\pm$4.7%). The beneficial effect was less significant when anticoagulant was added only upon thawing (42.4 $\pm$6.1% versus 47.5$\pm$4.7%). These were all non-high triglyceride samples. These results show minimal cell loss following 24 hours of FP storage in all treatments with significant advantage to cells treated with anticoagulant during both freezing and thawing. Average loss of cells treated with anticoagulant during freezing only was 2.3$\pm$3.2% compared to 1.9$\pm$3.3% without anticoagulants, upon thawing only was 3.0$\pm$4.7 compared to 1.9$\pm$3.3% without anticoagulants, and 0.2$\pm$0.4% compared to 1.9$\pm$3.3% without anticoagulants when cells were both frozen and thawed with ACDhep. In summary, the beneficial effect of anticoagulants on yield was kept for at least 24 hours.

The characteristics of a representative cell population of the FP are shown below in Table 8.

**Table 8**: Characterization of the cell population of fresh (t0) FP manufactured from cells collected with ("+") or without ("-") addition of anticoagulant during freezing ("F") and thawing ("Tha") procedures.*

| D on or ID | FP t0 | | | | | | | | | | | | | | | | | | | | |
|---|---|---|---|---|---|---|---|---|---|---|---|---|---|---|---|---|---|---|---|---|
| | F-/Tha- | | | | | F-/Tha+ | | | | | F+/Tha+ | | | | | F+/Tha- | | | | |
| | C D 3+ ( % ) | C D1 9+ (%) | C D5 6+ (%) | C D1 4+ (%) | C D1 5+ (%) | C D 3+ (%) | C D1 9+ (%) | C D5 6+ (%) | C D1 4+ (%) | C D1 5+ (%) | C D 3+ ( % ) | C D1 9+ (%) | C D5 6+ (%) | C D1 4+ (%) | C D1 5+ (%) | C D 3+ ( % ) | C D1 9+ (%) | C D5 6+ (%) | C D1 4+ (%) | C D1 5+ (%) |
| 1-3 | 62 .2 ± 6. 1 | 5.6 ± o.7 | 9.8 ± 0.9 | 13. 5 ± 1.1 | 0± 0 | 61 ±6 .1 | 8.6 ± 0.4 | 8.6 ± 0.9 | 14. 1 ± 1.1 | 0± 0 | 63 .9 ± 5. 8 | 7.4 ± 0.6 | 9.4 ± 0.8 | 13. 3 ± 1.9 | 0± 0 | 61 .9 ± 6. 0 | 11. 5 ± 1.1 | 10. 1 ± 1.0 | 14. 3 ± 1.3 | 0± 0 |

*Induction of apoptosis was performed using a medium containing anticoagulant for all batches.

The results of Table 8 show the cell characteristics of the final products (FP) manufactured with or without anticoagulant at freezing and thawing. Batches were sampled, stained for mononuclear markers, and analyzed via flow cytometry to determine the cell distribution in each sample and to examine whether the addition of anticoagulant affected the cell population. As presented in Table 7, there were no significant differences detected in cell populations manufactured with or without anticoagulants at freezing or thawing. The average T cell population (CD3+ cells) in fresh FP was 62.3±1.2% between treatments compared to 62.9±1.1% before freezing; the average B cell population (CD19+ cells) was 8.3±2.5% between treatments compared to 3.1±0.8% before freezing; the average natural killer cell population (CD56+ cells) was 9.5±0.7% between treatments compared to 12.9±0.5% before freezing; the average monocyte cell population (CD14+ cells) was 13.8±0.5% between treatments compared to 17.5±0.3% before freezing; and the average granulocyte population (CD15+ cells) was 0.0% in the fresh FP compared to 0.35±0.2% at freezing.

The potency of the early apoptotic population was also examined.

**Table 9**: Potency analysis of fresh (t0) FP manufactured from cells with ("+") or without ("−") addition of anticoagulant during freezing ("F") and thawing ("Tha") procedures.

| Donor ID # / Treatment | FP t0 | | | | | | | |
|---|---|---|---|---|---|---|---|---|
| | F–/Tha– | | F–/Tha+ | | F+/Tha+ | | F+/Tha– | |
| Median fluorescence | DR | CD86 | DR | CD86 | DR | CD86 | DR | CD86 |
| DCs 1:2 Early apoptotic cell population +LPS | 3% | 28% | *4% up from LPS* | 24% | 5% | 24% | 9% | 15% |
| DCs 1:4 Early apoptotic cell population +LPS | 4% | 38% | 6% | 35% | 6% | 34% | 6% | 24% |
| DCs 1:8 Early apoptotic cell population +LPS | 13% | Not done | 10% | 45% | 15% | 54% | 8% | 48% |

The results presented in Table 9 are from a potency assay performed to determine the ability of each final product to enhance a tolerogenic state in immature dendritic cells (iDCs) following stimulation with (LPS). The tolerogenic effect was determined by assessing downregulation of co-stimulatory molecule HLA-DR and CD86 expression on iDCs following interaction with the early apoptotic cell populations and different treatments leading to LPS upregulation. The analysis was performed on DCsign+ cells. Results represent the percent delay in maturation following interaction with early apoptotic cell population and following addition of LPS versus LPS-induced maturation. The experiment tested the potency of fresh FP (t0) manufactured with- or without anticoagulant. Results presented in Table 9 show that apoptotic cells manufactured with or without anticoagulant enhance the tolerance effect of both co-stimulatory markers in a dose-dependent manner.

The early apoptotic cells produced herein were from non-high triglyceride samples. This consistent high yield of stable early apoptotic cells was produced even in the cases when the donor plasma is high in triglycerides (*See for example,* Examples 12 and 13 of International Publication No. WO 2014/087408 and United States Application Publication No. US US-2015-0275175-A1). Note that anti-coagulants were not added to the PBS media used for formulation of the final early apoptotic cell dose for infusion.

*Summary*

The objective of this study was to produce a stable, high yield early apoptotic cell population. The rational for use of anticoagulants was that aggregates were seen first in patients with high-triglycerides, but later in a significant portion of other patients. A concern here was the disclosure in United States Patent No. US 6,489,311 that the use of anticoagulants prevented cell apoptosis.

In short, with minimal impact on the composition, viability, stability, and the apoptotic nature of the cells, there was a significant improvement of at least 10-20% in the number of collected cells in the final product (Yield) when anticoagulant was added. In this study an up to 13% increase in yield was shown, which represents 26.8% augmentation in yield in controlled conditions but in real GMP conditions it went up to 33% and more augmentations in cell number then can be produced in a single collection. This effect is crucial, since it may avoid the need for a second apheresis from a donor.

This effect was surprising because the anticipated impact was expected to be dissolution of mild aggregates. It had been hypothesize that thawing cells with anticoagulant reduced the amount of aggregates. When formed, these aggregates eventually lead to massive cell loss. Cells collected and frozen without anticoagulant demonstrated aggregate formation at thawing, immediately after wash. Furthermore, a high level of aggregates was also detected in cells that were frozen without anticoagulant and resuspended with media containing anticoagulant. No aggregates were seen in cells that were both frozen and resuspended with media containing anticoagulant. Taken together, it was conclude that the addition of anticoagulants during freezing and apoptosis induction is of high importance, and did not appear to negatively impact the induction of early apoptosis on the cell population.

Recovery of early apoptotic cells was further tested, for example, following 24 hours of storage at 2-8°C, for stability purposes, during which an average cell loss of 3-4.7% was measured, regardless of manufacturing conditions, with favorable results for cells that were both frozen and thawed with media containing anticoagulant ($0.2\pm0.4\%$ cell loss following 24 hours of FP storage), suggesting that addition of anticoagulant is critical during freezing and thawing, but once finally formulated, the early apoptotic cell population is stable. Extended time point studies showed this stability to at least 72 hours.

Apoptosis and viability, as well as cell composition of the FP product were not significantly affected by the addition of anticoagulant at the freezing and/or thawing stage. Values measured from a wide variety of characteristics were similar, indicating the ACDhep did not change the early apoptotic cell characteristics and the final product met the acceptance criteria of $\geq40\%$ apoptotic cells.

The assay used to test apoptotic cells potency was based on immature dendritic cells (iDCs), DCs that are characterized by functions such as phagocytosis, antigen presentation, and cytokine production.

The HLA-DR (MHC class II) membrane molecule and co-stimulatory molecule CD86 were selected as markers to detect the tolerogenic effects of antigen-presenting cells (APCs). Using flow cytometry, changes in expression of HLA-DR and CD86 on iDCs were measured following stimulation with LPS, as well as in the presence of the early apoptotic cell population manufactured with- or without anticoagulant and stimulated with LPS. Early apoptotic cell populations were offered to DCs in ascending ratios of 1:2, 1:4, and 1:8 iDCs : early apoptotic cell population. As presented in Table 6, it was shown that early apoptotic cell population enhanced the tolerogenic effect over stimulated DCs in a dose-dependent manner, with slightly better results for early apoptotic cell population manufactured with anticoagulant both at freezing and apoptosis induction.

Taken together, it was concluded that addition of anticoagulant to both freezing and apoptosis media is of high importance to increase cell recovery and avoid massive cell loss due to aggregates, and to avoid in many cases a second round of apheresis from a donor. It was shown that all cells met acceptance criteria for the validated FP, indicating that the addition of anticoagulant does not impair the FP.

*EXAMPLE 2: Effect Of Apoptotic Cells On Cytokine Release In An*

*In Vitro Cytokine Storm Model*

*Objective:* Test the effect of apoptotic cells on the level of cytokine storm markers (cytokines IL-6, IL-10, MIP-1$\alpha$, IL-8, TNF-$\alpha$, MIP-1$\beta$, MCP-1, and IL-9) in a cytokine storm induced in an LPS-Sterile model of macrophage activation syndrome.

*Methods:*

*Cell lines and culturing reagents*

The human lymphoma cell line Raji (eCACC, UK, access no. 85011429), the human cervical adenocarcinoma cell line HeLa (ATCC, USA, number: CCL-2) and HeLa-CD19 (ProMab, USA, cat. no. PM-Hela-CD19) were cultured in RPMI 1640 (Gibco, ThermoFisher Scientific, USA, cat. no. 31870-025) supplemented with 10% FBS (Gibco, Thermo-Fisher Scientific, South America, cat. no. 12657-029), 2 mM GlutaMAX (Gibco, ThermoFisher Scientific, USA, cat. no. 35050-038), and 100 U/ml Penicillin + 100 U/ml Streptomycin (Gibco, ThermoFisher Scientific, USA, cat. no. 15140-122), henceforth referred to as "Complete Medium". HeLa-CD19 medium was further supplemented with 1 $\mu$g/ml puromycin (Sigma-Aldrich, USA, cat. no. P9620), as the selective antibiotics, during standard culturing.

All cells were kept in sub-confluent conditions. Raji cells were maintained in a concentration range of $0.3 \times 10^6$-$2 \times 10^6$ cell/ml. HeLa and HeLa-CD19 cells were passaged when receptacle was filled to 90% confluence.

Primary monocytes were isolated from blood donations buffy coats (Sheba Medical Center, Israel). First, peripheral blood mononuclear cells (PBMCs) were isolated on a Ficoll density gradient (Ficoll-Paque PLUS, GE Healthcare, UK, cat. no. 17-1440-03). Upon centrifugation (800x g, 2-8°C, 20 min. with break 0), the interphase containing the PBMCs were transferred to a fresh test tube and washed with RPMI-1640 (Lonza, Switzerland, cat. no. BE12-918F) supplemented with 2 mM L-glutamine (Lonza, Switzerland, cat. no. BE17-605E) and 10 mM Hepes (Lonza, Switzerland, cat. no. BE17-737B), henceforth "Wash Medium", and centrifuged (650x g, 2-8°C, 10 min.). Pelleted cells were re-suspended in "Wash Medium" to a concentration of $15 \times 10^6$ cell/ml. Cells were seeded as a 0.9 ml drop at the center of a 35-mm plate (Corning, USA, cat. no. 430165). Plates were incubated for 1.5h in a humidified incubator (37°C, 5% $CO_2$), allowing monocytes to adhere, and then washed three times with pre-warmed PBS (Lonza, Switzerland, cat. no. BE17-516F), removing other cell types. After washing, cells were cultured in 2 ml RPMI 1640 (Gibco, ThermoFisher Scientific, USA, cat. no. 31870-025) supplemented with 10% FBS (Gibco, ThermoFisher Scietific, South America, cat. no. 12657-029), 2 mM GlutaMAX (Gibco, ThermoFisher Scientific, USA, cat. no. 35050-038), and 100 U/ml Penicillin + 100 U/ml Streptomycin (Gibco, ThermoFisher Scientific, USA, cat. no. 15140-122), aka "Complete Medium".

All cell lines were cultured in a humidified incubator at 37°C and containing 5% $CO_2$.

In brief, and following manufacturer's guidelines, target cells (HeLa or HeLa-CD19) were cultured alone or in conjunction with monocytes. After target cells adhered to the plate (6h-overnight), cultures were exposed to y x10$^6$ ApoCells cells for 1h, after which these cells were washed off by 4-5 washes of RPMI. Removal of ApoCells cells was confirmed visually under a light microscope. 10 ng/ml LPS (Sigma-Aldrich, USA, cat. no. L4391) was introduced to the co-culture and incubated for 1h. After incubation, LPS was removed by 3-5 washing cycles with RPMI. Viable CD19-CAR T cells or naïve T cells were added at the designated E/T ratio(s) and incubated for 4h. To collect media, plates were centrifuged at 250x g, 2-25°C, 4 min. (Centrifuge 5810 R, Eppendorf, Germany) to sediment cells. 50 $\mu$l of supernatant medium from each well was transferred to a fresh flat-bottom 96-well microplate well (Corning, USA, cat. no. 3596) and 50 $\mu$l CytoTox 96 Reagent was added to each well. Plates were incubated in the dark at room temperature for 30 min., after which the reaction was terminated by addition of 50 $\mu$l Stop Solution per well. Absorbance was read at 492 nm using Infinite F50 (Tecan, Switzerland) and captured using Magellan F50 software. Data analysis and graph generation was performed using Microsoft Excel 2010.

Analysis of cytokine release was performed using Liminex technology following incubation with apoptotic cells or incubation with supernatant from apoptotic cells.

*Results:* **Figures 4A** through **4H** show that there was a significant reduction in the levels of cytokine storm markers IL-10, IL-6, MIP-1$\alpha$, IL-8, TNF-$\alpha$, MIP-1$\beta$, MCP-1, and IL-9 which were induced by LPS in an *in vitro* model of macrophage activation syndrome. While administration of ApoCells to achieve a macrophage:Apocell ratio of 1:8 resulted in significantly decreased levels of both IL-10, IL-6, MIP-1$\alpha$, IL-8, TNF-$\alpha$, MIP-1$\beta$, MCP-1, and IL-9 released into the medium **(Figures 4A, 4B, 4C, 4D, 4E, 4F, 4G, 4H),** administration of ApoCells to achieve a macrophage: ApoCell ratio of 1:16 actually inhibited or nearly inhibited the release of cytokines IL-10, IL-6, MIP-1$\alpha$, IL-8, TNF-$\alpha$, MIP-1$\beta$, MCP-1, and IL-9 in this model.

Addition of apoptotic cells resulted in the inhibition of at least 20 pro-inflammatory cytokine and chemokines induced in macrophage activating, a sample of the results are shows in **Figures 4A-4H.** The common mechanism for pro-inflammatory cytokine and chemokine release is NF-$\kappa$B inhibition.

The inhibition of release of pro-inflammatory cytokines and chemokines appears to be specific, as examination of cytokine IL-2R (IL-2 receptor) levels under similar conditions showed that IL-2R levels released was not influenced in the same manner at the pro-inflammatory cytokines. **(Figure 4I).** Addition of apoptotic cells increased the release of IL-2R at 1:4 and 1:8 ratios. Further, **Figure 4J** shows that apoptotic cells had no influence on release of IL-2 over a 24 hour time period. Activation of the IL-2 receptor is considered to have an essential role in key functions of the immune system including tolerance.

*Conclusion:* Addition of early apoptotic cells in a cytokine storm model of macrophage activation syndrome in the presence of cancer and CAR-19, resulted in significant reduction and, surprisingly even prevention of pro-inflammatory cytokines, for example IL-10, IL-6, MIP-1$\alpha$, IL-8, TNF-$\alpha$, MIP-1$\beta$, MCP-1, and IL-9, while increasing or not affecting cytokine IL-2R levels. Thus, the results here show that while pro-inflammatory cytokines were reduced by incubation with apoptotic cells, IL-2 and IL-2R were not influenced in the same manner with incubation of early apoptotic cells. Thus, the T-cell associated cytokines are not influenced by the CAR T-cell therapy + apoptotic cells, whereas the innate immunity cytokines, for example those released from monocytes, macrophages, and dendritic cells are.

**REFERENCE EXAMPLE 3: Effect of Apoptotic Cells on Cytokine Storm Without a Negative Effect on The CAR-T Cell Efficacy**

*Objective:* Test the effect of apoptotic cells or supernatants derived from apoptotic cells on cytokine storm marker cytokines and CAR T-cell efficacy on tumor cells.

*Methods:*

*T4+ CAR T-cells*

A solid tumor model (van der Stegen et al., 2013 *ibid*) reported to induce cytokine storms in mice was utilized. In this model, T cells were engineered with a chimeric antigen receptor (CAR) targeting certain ErbB dimers (T4[+] CAR-T cells), which are often highly up-regulated in specific solid tumors such as head and neck tumors and ovarian cancers. T-cells were isolated from PBMC separated from peripheral blood using CD3 micro-beads. Vectors containing the chimeric T4+ receptor were constructed and transducer into the isolated T-cells, resulting in T4+ CAR T-cells. For the experiments performed herein, T4+ CAR T-cells were purchased from Creative Biolabs (NY USA) or Promab Biotechnologies (CA USA). **Figure 5** presents flow cytometry curves verifying the surface expression of $4\alpha\beta$ chimeric receptor on the T4+ CAR T-cells using an anti-CD124 monoclonal antibody (Wilkie et al., *ibid*). In addition, a PCR procedure was performed and verified the presence of the vector in transduced T cells.

*SKOV3-luc cells*

SKOV3-luc ovarian adenocarcinoma tissue culture cells were purchased from Cell BioLabs (cat. #AKR-232). SKOV3-luc highly express ErbB receptors and are a target for the T4[+] CAR-T cells (van der Stegen et al., 2013, *ibid*). These cells had been further manipulated to constitutively express the firefly luciferase gene, allowing tracking of cell proliferation *in vitro* and tumor growth and recession *in vivo.*

*Apoptotic cells*

Apoptotic cells were prepared as per Example 1.

*Apoptotic cell supernatants*

Eight (8) million apoptotic cells per seeded per well in a 12-well plate. After 24 hours the cells were centrifuge (290g, 4 degrees Celsius, 10 minutes). Supernatant was collected and frozen in aliquots at -80 degrees until use. Different numbers of cells are used to make supernatants. Some aliquots contain concentrated supernatants.

*Monocyte isolation*

PBMCs were isolated using Ficoll (GE healthcare, United Kingdome) from peripheral blood\ buffy coat obtained from healthy, eligible donors. Cells were brought to a concentration of $15\times10^6$ cells\ml in RPMI1640 (Gibco, Thermo Fisher Scientific, MA, USA) and seeded in a 0.9ml drop in the middle of 35mm plates (Corning, NY, USA). Plates were then incubated at 37°C in 5% $CO_2$ for 1 hour. At the end of incubation, cells were washed three times with PBS (Biological industries, Beit Haemek, Israel) and adhesion was determined using a light microscope. Cells were then incubated with complete media (RPMI1640+ 10% heat inactivated FBS+ 1% Glutamax+ 1% PenStrep, all from Gibco).
An alternative method of monocyte isolation was also used wherein human mononuclear cells were isolated from heparinized peripheral blood by density gradient centrifugation. The isolated mononuclear cells then were separated into monocyte, B-cell and T-cell populations by positively selecting monocytes as the CD14+ fraction by magnetic bead separation (Miltenyi Biotec., Auburn, CA, USA), positively selecting B-cells as the CD22+ fraction, and negatively selecting T-cells as the CD14-CD22- fraction. Purity was greater than 95 percent for monocytes.
For macrophage differentiation, at the end of adhesion, cells were washed three times with PBS then incubated with RPMI1640+ 1% Glutamax+ 1% PenStrep and 10% heat inactivated human AB serum (Sigma, MO, USA). Cells were incubated at 37°C and 5% for 7-9 days, with media exchange at day 3 and day 6. Differentiation was determined by morphology via light microscope.

*Supernatant from apo+ monocytes*

CD14+ monocytes were cultured with apoptotic cells as prepared above at a ratio of 1:16, for 24h. The number of monocytes was: 0.5 million cells per well in a 12-well plate and the number of apoptotic cells was: 8 million cells per well in a 12-well plate. After incubation for 24 hours the cells were centrifuge (290g, 4 degrees Celsius, 10 minutes). Supernatant was collected and frozen in aliquots at -80 degrees until use. Similar procedures could be performed at different ratios of monocytes:apoptotic cells and/or using other sources of cells, such as macrophages and dendritic cells.

*In vitro culturing conditions*

Initial experiments were performed by incubating SKOV3-luc cancer cells with apoptotic cells, or apoptotic supernatants, for 1 hour followed by co-culturing with T4+ CAR T-cells (+/monocytes-macrophages) for 48 hours.

In order to simulate *in vivo* conditions, $1x10^5$ THP-1 cells/ml (HTCC USA), or monocytes or macrophages or dendritic cells, will be differentiated with 200 nM (123.4 ng/ml) phorbol myristate acetate (PMA) for 72 hrs and will then be cultured in complete medium without PMA for an additional 24h. Next, cancer or tumor cells - for example SKOV3-luc cells will be plated in a 24-well plate at $5x10^5$ SKOV3-luc cells/well on the differentiated THP-1 cells. Following initial culturing of the cancer or tumor cells, $4x10^5$-$8x10^5$ apoptotic cells (ApoCell) will be added to the culture for 1-3h to induce an immunotolerant environment. The ratio of cancer cell to ApoCell will be optimized for each cell type. After washing, the co-culture will be treated with 10 ng/ml LPS after which $1x10^6$ T4$^+$ CAR T cells (or a quantity to be determined by an effector/target (E/T) ratio graph) will be added. The ratios of tumor cells and T4+ CAR T-cells will be varied in order to generate effector/target (E/T) ratio graphs for each tumor or cancer cell type.

To assay for SKOV3 cancer cell cytotoxicity, lysates were prepared and luciferase activity was determined after the 48 hour incubation period. Additional experiments will be performed assaying for cancer or tumor cell cytotoxicity for the other cancer cell types and at intervals within the 48h incubation time period. Alternatively, Promega's CytoTox 96 Non-Radioactive Cytotoxicity Assay (Promega, cat #G1780) will be used.

*Lysate Preparation*

SKOV3-luc cell lysates were prepared by washing the SKOV3-luc monolayer with PBS to remove any residual serum and adding 70 μl CCLR lysis buffer x1/well (for 24-well plates). Detachment was further enhanced by physical scraping of well bottoms. Following vortexing for 15 seconds, lysates were centrifuged at 12,000g for 2 minutes at 4°C. Supernatants were collected and stored at -80°C.

*In vitro Luciferase Activity*

To detect luciferase activity in SKOV3-luc cells in culture, Luciferase Assay System (Promega, cat. #E1501) was used. Calibration of this kit with the luminometer reader (Core Facility, Faculty of Medicine, Ein Kerem, Hebrew University of Jerusalem) was done by using QuantiLum recombinant luciferase (Promega, cat. #E170A). 612 ag - 61.2 μg ($10^{-20}$-$10^{-9}$ moles) was used to determine detection range and following manufacturer's guidelines. In brief, each rLuciferase quantity in 20 μl volume was placed in a well of black 96-well plates (Nunc). Each quantity was done in triplicate. 100 μl LAR (luciferin substrate from Luciferase Assay System kit) was added to each well and read immediately with a 10 second exposure.

For luciferase activity reading, lysates were thawed on ice and 20 μl samples were placed in a black 96-well plate (Nunc). Each sample was read in duplicate. 100 μl LAR was added and luminescence was read for 10 second exposure period every 2.5 minutes for 25 minutes and every 40 seconds for the ensuing 10 minutes.

*Cytokine Analysis*

Initial assays for IL-2, IL-2 receptor (IL-2R), IL-6, IL-1α, IL-4, IL-2, TNF-α were performed. To assay for cytokine release reduction of IL-2, IL-2 receptor (IL-2R), IL-6, IL-1α, IL-4, IL-2, TNF-α as well as other cytokines, supernatants were be collected and examined for selected cytokine using Luminex MagPix reader and ELISA assays.

***Results:***

**SKOV3-luc growth**

SKOV3-luc growth was followed using luciferase activity as an indicator, to determine target SKOV3-luc cell number in future experiments. $3.8x10^4$-$3.8x10^5$ SKOV3-luc cells/well were plated in 24-well plates (Corning) and luciferase activity was monitored daily for 3 days. $1.9x10^5$ cells/well or higher cell number plated reach confluence and present growth saturation indicated by luciferase activity 2 days after plating **(Figure 6)**. Note that $3.8x10^4$-$1.1x10^5$ SKOV3-luc cells/well were still in the linear or exponential growth phase three days after plating **(Figure 6,** plots orange, turquoise and purple). Negative control ($3.8x10^5$ SKOV3-luc cells without LAR substrate) displayed only background-level reading and demonstrates that bioluminescent readings from SKOV3-luc cells result from luciferase activity.

*Verification of T4$^+$ CAR-T cell activity against SKOV3-luc tumor cells*

To corroborate the T4$^+$ CAR-T cell activity, monolayers of SKOV3-luc were exposed to either 1,000,000 (one million) T4$^+$ CAR-T cells or to 1,000,000 (one million) non-transduced T cells. After 24h incubation, T4$^+$ CAR-T cells reduced SKOV3-luc proliferation by 30% compared to the non-transduced T cell control **(Figure 7)**, showing anti-tumor activity of the T4$^+$ CAR-T cells.

*Activity of stand-alone T4+ CAR-T cells against SKOV3-luc tumor cells was compared to activity post exposure to Apoptotic Cells*

Apoptotic cells (ApoCell) and apoptotic cell supernatants (ApoSup and ApoMon Sup) were tested to determine if they interfere with T4+ CAR-T cell anti-tumor activity. The SKOV3-luc tumor cells were incubate with Apoptotic Cells for one hour, followed by the addition of T4+ CAR-T cells (500,000, five hundred thousands) or T4+ non-transduced T cells (500,000, five hundred thousands) (ratio of 1:2 T4$^+$ CAR-T cells to Apoptotic Cells). The tumor cell/Apoptotic cell/T4$^+$ CAR T-cells were then co-cultured for 48h. The control SKOV3-luc tumor cells were co-cultured with T4+ CAR-T cells and Hartman solution (the vehicle of Apoptotic Cells), but without Apoptotic Cells, for 48h.
The results showed that after 48h incubation, T4+ CAR-T cells anti-tumor activity was superior to incubation with non-transduced T cells. Similar incubations were performed with apoptotic cells or apoptotic cell supernatants. Surprisingly, T4+ CAR T-cell anti-tumor activity was comparable with or without exposure to apoptotic cells or apoptotic cell supernatants. **(Figure 8).**

*Effect of Apoptotic Cells on amelioration, reduction or inhibition of cytokine storms resulting from CAR-T treatment*

The effect of apoptotic cells to reduce cytokine storms was examined next. IL-6 is a prototype pro-inflammatory cytokine that is released in cytokine storms (Lee DW et al. (2014) Blood 124(2): 188-195) and is often used as a marker of a cytokine storm response.
Cultures were established to mimic an *in vivo* CAR T-cell therapy environment. SKOV3-luc tumor cells were cultured in the presence of human monocyte-macrophages and T4+ CAR T-cells. The concentration of Il-6 measured in the culture media was approximately 500-600 pg/ml. This concentration of IL-6 is representative of a cytokine storm.
Unexpectedly, IL-6 levels measured in the cultured media of SKOV3-luc tumor cells, human monocyte-macrophages, T4+ CAR-T cells, wherein the tumor cells had been previously incubated with apoptotic cells for one hour (ratio of 1:2 T4+ CAR-T cells to Apoptotic Cells) were dramatically reduced. Similarly, IL-6 levels measured in the cultured media of SKOV3-luc tumor cells, human monocyte-macrophages, T4+ CAR-T cells, wherein the tumor cells had been previously incubated with apoptotic cell supernatants for one hour, were also dramatically reduced. This reduction in concentration of IL-6 is representative of a decrease in the cytokine storm **(Figure 9).**
It was concluded that unexpectedly, apoptotic cells and apoptotic supernatants do not abrogate the effect of CAR-T cells on tumor cell proliferation while at the same time they down regulating pro-inflammatory cytokines such as IL-6, which was been described as a major cytokine leading to morbidity.

*Analysis using a wider range of cytokines*

To further evaluate the effect on a possible wider range and levels of cytokines that are not generated during experimental procedures but do appear in clinical settings during a human cytokine storm, LPS (10 ng/ml) was added to the SKOV3-luc culture conditions outlined above. The addition of LPS is expected to exponentially increase the cytokine storm level. As expected, the addition of LPS increased the cytokine storm effect and as a result IL-6 levels increased to approximately 30,000 pg/ml. Other cytokines known to be expressed in high levels during a cytokine storm showed elevated levels, for example: TNF-$\alpha$ (250-300 pg/ml), IL-10 (200-300 pg/ml), IL1-alpha (40-50 pg/ml) and IL-18 (4-5 pg/ml). As shown in **Figure 10,** exposure to apoptotic cells dramatically reduced the levels of IL-6 even during the exponential state of the cytokine storm to almost normal levels that may be seen in clinical settings, and is not always seen in experimental procedures with CAR T-cells. This effect was similar across the other pro-inflammatory cytokines TNF-alpha☐ IL☐ ☐ ☐ ☐ ☐IL☐ ☐ alpha, IL-1β, ☐ and IL-18, which showed a reduction of between 20-90%. Similar results were found when using apoptotic cell supernatants in place of the apoptotic cells.

*Effect of Apoptotic Cells on IL-2 and IL-2R*

The concentration of IL-2 measured in culture supernatants following incubation of SKOV3-luc cells with T4+ CAR T-cells was 1084 pg/ml. Surprisingly, when SKOC3-luc cells were first incubated with apoptotic cells and then T4+ CAR T-cells the concentration of IL-2 increased to 1190 pg/ml. Similarly, the concentration of IL-2R measured in culture supernatants following incubation of SKOV3-luc cells with T4+ CAR T-cells was 3817 pg/ml. Surprisingly, when SKOC3-luc cells were first incubated with apoptotic cells and then T4+ CAR T-cells the concentration of IL-2R increased to 4580 pg/ml. In SKOV3-luc alone the concentration of Il-2 was 3.2 pg/ml and with the addition of apoptotic cells the concentration was 10.6 pg/ml. In SKOV3-luc alone the concentration of Il-2R was 26.3 pg/ml and with the addition of apoptotic cells the concentration was 24.7 pg/ml.

**Conclusion**

CAR-T cell therapy has been documented to cause cytokine storms in a significant number of patients. These results demonstrate that apoptotic cells and apoptotic cell supernatants surprisingly decreased cytokine storms cytokine markers without affecting CAR-T cell efficacy against tumor cells. Moreover, it appears that apoptotic cells increase cytokine IL-2, which may increase duration of CAR T-cell therapy by maintaining or increasing CAR T-cell proliferation.

*REFERENCE EXAMPLE 4: Apoptotic Cell Therapy Prevents Cytokine Storms in Mice Administered CAR T-Cell Therapy*

*Objective:* Test the *in vivo* effect of apoptotic cells or apoptotic cell supernatants in a solid tumor model (SKOV3 ovarian adenocarcinoma), in order to determine T4+ CAR T-cell efficacy and the level of cytokine storm marker cytokines.

**Materials and Methods**

*In vitro studies*

*In vitro* methods including methods of making, culturing, and analyzing the results described above and relevant for use of T4+ CAR T-cells that recognize the ErbB target antigen (referred to herein as "T4+ CAR T-cells", SKOV3-luc cells, apoptotic cells, apoptotic supernatants, monocytes, macrophages, and the various assays, have all been described above in Example 1. The same methods were used herein.

*In vivo studies*

*Mice*

7-8 week old SCID-beige mice and NSGS mice were purchased from Harlan (Israel) and kept in the SPF animal facility in Sharett Institute.

SKOV3-luc tumor cells ($1 \times 10^6$ or $2 \times 10^6$) are inoculated into SCID beige mice or NSGS mice, by either i.p. in PBS or s.c. in 200 ml Matrigel (BD Biosciences). Tumor engraftment is confirmed by bioluminescence imaging (BLI) at about 14-18 days post injection, and mice are sorted into groups with similar signal intensity prior to T-cell administration.

Mice will receive $30 \times 10^6$ apoptotic cells either 24 hours prior to administration of T4+ CAR T-cells or concurrent with administration of T4+ CAR T-cells ($10\text{-}30 \times 10^6$ T4+ CAR T-cells). Tumor growth will be followed by bioluminescence imaging (BLI) and circulating cytokine levels will be determined by Luminex.

*In vivo Luciferase assay*

Tumor growth was monitored weekly through firefly luciferase activity. In brief, 3 mg D-luciferin (E1605. Promega, USA)/mouse (100 μl of 30 mg/ml D-luciferin) was injected i.p. into isoflurane-anesthetized mice and ventral images were acquired 10 minutes after injection using IVIS Imaging System and Live Image image capture software (both from Perkin Elmer, USA).

Image acquisition parameters were chosen for each image session by imaging mice that received $0.5 \times 10^6$

SKOV3-luc cells/mouse, 5 minutes post D-luciferin injection the "auto" option. Capture parameters were set for binning 4, F/stop 1.2 and exposure of 2-4 minutes using the 24x lens. Data analysis and quantification was performed with the Live Image software and graphs were generated using Microsoft's Excel program.

*In vivo Cytotoxicity*

To assess *in vivo* toxicity of T-cells, organs are collected from mice, formalin fixed, and subjected to histopathologic analysis.

*Cytokine analysis*

Supernatants and sera are analyzed using Luminex MagPix reader and/or ELISA kits, cytometric bead arrays (Th1/Th2/Th17; BD Biosciences) as described by the manufacturers. For example, analysis may be for pro-inflammatory cytokine, which in one case would be IL-6, though, in some embodiments, any of the cytokines listed in Tables 1 and 2 or known in the art may be analyzed herein.

**Results**

*Calibrating SKOV3-luc tumors* in vivo

$0.5 \times 10^6$, $1 \times 10^6$ or $4.5 \times 10^6$ SKOV3-luc cells were injected i.p. to SCID beige mice and bioluminescence imaging (BLI) was conducted weekly in order to follow tumor growth, as described in the Methods (data not shown).

*Clinical score of mice*

Mice displayed no clinical symptoms for the initial 4 weeks. However, 28 days post SKOV3-luc injection, the mice that received the high dose ($4.5 \times 10^6$; purple line) began to lose weighed steadily **(Figure 11A)** and the overall appearance of the mice deteriorated, manifested in lethargy, abnormal pacing and general loss of activity. This group was culled at the day 39, and an abdominal autopsy was performed to expose tumor appearance and size **(Figure 11B).** SKOV3-luc tumors were large, solid, vascularized and displayed a whitish shining complexion. One large tumor predominated on the side of the injection (left) either caudal or rostral in the abdominal cavity. This tumor encompassed approximately 25-75% of the cavity and clearly pressed and disturbed the intestines. Smaller foci were also observed at various locations within the abdominal cavity. Tumors were contained within the abdominal cavity and no other tumors were observed in any other part of the body in any mice. Mice receiving low ($0.5 \times 10^6$) or medium ($1 \times 10^6$) dose of SKOV3-luc cease gaining weight 40 days after SKOV3-luc injection and began to steadily lose weighed. Experiment was terminated 50 days after SKOV3-luc injection.

*SKOV3-luc Tumor Kinetics*

PBS was injected to control for SKOV3-luc cells and these mice did not exhibit any luciferase activity throughout the experiment **(Figure 12,** Left panel). Tumor detection and growth was dose-dependent. Lower dose ($0.5 \times 10^6$ SKOV3-luc cells) began to display tumors 25 days post-injection (4/5 animals), medium dose ($1 \times 10^6$) injections showed tumors at 18 days post-injection (4/5 animals), whereas at higher dose ($4.5 \times 10^6$) tumors were detected as early as 11 days post-injection in 3/5 animals and by day 18 all animals displayed well-established tumors **(Figure 12**

**and Figures 13A-13D).**

*CAR T-cell therapy induces cytokine release syndrome*

Three groups of tumor-free mice as well as mice with tumors are administered (i.p. or directly into the tumor) increasing doses of T4+ CAR T-cells ($3 \times 10^6$, $10 \times 10^6$ or $30 \times 10^6$). At the highest dose, tumor-free mice and mice with tumors demonstrate subdued behavior, piloerection, and reduced mobility within 24 h, accompanied by rapid weight loss followed by death within 48 hrs. At least Human interferon-gamma and mouse IL-6 are detectable in blood samples from the mice given the highest dose of CAR T-cells. Animals that receive a high dose of CAR T-cells directed to a different tumor antigen do not exhibit weight loss or behavioral alterations.

*Administration of apoptotic cells inhibits or reduces the incidence of cytokine release syndrome induced by CAR T-cell therapy*

One group of mice given the highest dose of CAR T-cells is concomitantly administered $2.10 \times 10^8$/kg apoptotic cells, which was previously demonstrated to be a safe and effective dose. Mice receiving human CAR T + apoptotic cells have significantly lowered levels of mouse IL-6, lower weight loss, and reduced mortality.

***REFERENCE EXAMPLE 5: Effect of Combination Immune Therapy on in vitro Diffuse Tumor Models***

*Objective:* Test the effect of apoptotic cells or supernatants derived from apoptotic cells in a diffuse tumor model where the cancer is widely spread and not localized or confined, in order to determine CAR T-cell efficacy on the cancer cells and the level of cytokine storm marker cytokines.

*Methods:*

*CD19+ T4+ CAR T-cells ("CD19+ CAR T-cells")*

CD19-specific CAR-T cells were purchased from ProMab (Lot # 012916). The T cells were 30% positive for CAR (according to manufacturer's FACS data - Fab staining). Briefly, cells were thawed into AimV + 5% heat-inactivated FBS, centrifuged (300g, 5 minutes, room-temperature), and resuspended in AimV. On day 6 of the experiment $20 \times 10^6$ cells were injected IV per mouse (70% AnnexinPI negative, of which 30% CAR positive).

Recombinant HeLa cells expressing CD19 will be used as a control cell-type that also expresses CD19 on their cell surface.

*CD123+ CAR T-cells*

T4+ CAR T-cells will also be engineered with a CAR targeting CD123 epitopes (referred to herein as "CD123+ CAR T-cells").

*Raji cells, CD19 expressing HeLa cells, and CD123 expressing leukemic cells*

Raji cells Raji cells were purchased from ECACC (Cat. #: 85011429), and routinely cultured in complete medium (RPMI-1640 supplemented with 10% H.I. FBS, 1% Glutamax, 1% Penicillin / Streptomycin), and maintained at a concentration of $3 \times 10^5$ - $3 \times 10^6$ cells/ml. On day 1 of the experiment $0.1 \times 10^6$ cells were injected IV per mouse.

Similarly, CD19 expressing HeLa cells will be generated in the laboratory and used as a target for CD19+ CAR T-cells. CD123 expressing leukemic cells will be used as targets for CD123+ CAR T-cells. In addition, primary cancer cells will be utilized as a target for CAR T-cells.

HeLa cells expressing CD19 were prepared using methods known in the art. Cells will be cultured as is well known in the art.

CD123 is a membrane biomarker and a therapeutic target in hematologic malignancies. CD123 expressing leukemic cells, for example leukemic blasts and leukemic stem cells will be cultured as is known in the art.

*Apoptotic cells, Apoptotic cell supernatants and monocyte isolation,* will be prepared as described in Example 1. Early apoptotic cells produced were at least 50% annexin V-positive and less than 5% PI-positive cells.

*Macrophages* were generated from CD14 positive cells by adherence.

*Dendritic cells* were CD14 derived grown in the presence of IL4 and GMCSF.

*Flow-cytometry.* The following antibodies were used :hCD19-PE (eBiosciences, Cat. # 12-0198-42); mIgG1-PE (eBiosciences, Cat. # 12-0198-42); hCD3-FITC (eBiosciences, Cat. # 11-0037-42); mIgG2a-FITC (eBiosciences, Cat. # 11-4724-82). Acquisition was performed using FACS Calibur, BD.

*Naïve T cells.* Naïve T cells were isolated from Buffy coat using magnetic beads (BD), and cryopreserved in 90% human AB serum and 10% DMSO. Thawing and injection was identical to the CAR-T cells.

*In vitro culturing conditions*

*Cell lines and culturing reagents*

The human lymphoma cell line Raji (eCACC, UK, access no. 85011429), the human cervical adenocarcinoma cell line HeLa (ATCC, USA, number: CCL-2) and HeLa-CD19 (ProMab, USA, cat. no. PM-Hela-CD19) were cultured in RPMI 1640 (Gibco, ThermoFisher Scientific, USA, cat. no. 31870-025) supplemented with 10% FBS (Gibco, Thermo-

Fisher Scietific, South America, cat. no. 12657-029), 2 mM GlutaMAX (Gibco, ThermoFisher Scientific, USA, cat. no. 35050-038), and 100 U/ml Penicillin + 100 U/ml Streptomycin (Gibco, ThermoFisher Scientific, USA, cat. no. 15140-122), henceforth referred to as "Complete Medium". HeLa-CD19 medium was further supplemented with 1 $\mu$g/ml puromycin (Sigma-Aldrich, USA, cat. no. P9620), as the selective antibiotics, during standard culturing.

All cells were kept in sub-confluent conditions. Raji cells were maintained in a concentration range of $0.3\times10^6$-$2\times10^6$ cell/ml. HeLa and HeLa-CD19 cells were passaged when receptacle was filled to 90% confluence.

Primary monocytes were isolated from blood donations buffy coats (Sheba Medical Center, Israel). First, peripheral blood mononuclear cells (PBMCs) were isolated on a Ficoll density gradient (Ficoll-Paque PLUS, GE Healthcare, UK, cat. no. 17-1440-03). Upon centrifugation (800x g, 2-8°C, 20 min. with break 0), the interphase containing the PBMCs were transferred to a fresh test tube and washed with RPMI-1640 (Lonza, Switzerland, cat. no. BE12-918F) supplemented with 2 mM L-glutamine (Lonza, Switzerland, cat. no. BE17-605E) and 10 mM Hepes (Lonza, Switzerland, cat. no. BE17-737B), henceforth "Wash Medium", and centrifuged (650x g, 2-8°C, 10 min.). Pelleted cells were re-suspended in "Wash Medium" to a concentration of $15\times10^6$ cell/ml. Cells were seeded as a 0.9 ml drop at the center of a 35-mm plate (Corning, USA, cat. no. 430165). Plates were incubated for 1.5h in a humidified incubator (37°C, 5% $CO_2$), allowing monocytes to adhere, and then washed three times with pre-warmed PBS (Lonza, Switzerland, cat. no. BE17-516F), removing other cell types. After washing, cells were cultured in 2 ml RPMI 1640 (Gibco, ThermoFisher Scientific, USA, cat. no. 31870-025) supplemented with 10% FBS (Gibco, ThermoFisher Scietific, South America, cat. no. 12657-029), 2 mM GlutaMAX (Gibco, ThermoFisher Scientific, USA, cat. no. 35050-038), and 100 U/ml Penicillin + 100 U/ml Streptomycin (Gibco, ThermoFisher Scientific, USA, cat. no. 15140-122), aka "Complete Medium".

All cell lines were cultured in a humidified incubator at 37°C and containing 5% $CO_2$.

CD19-CAR T cells (ProMab, USA, cat. no. FMC63) were delivered either in AIM-V medium or frozen. Cryopreserved CAR T cells for *in vitro* experiments were thawed on the day of the experiment in a 35-38°C bath and immediately immersed in pre-warmed AIM V medium (Gibco, ThermoFisher Scientific, USA, cat. no. 12055-091) supplemented with 5% FBS (Gibco, South America, cat. no. 12657-029). DMSO was removed by centrifuging the cells (300x g, room temperature, 5 min.) and re-suspending in pre-warmed AIM V medium. Concentration and viability of CD19-CAR+ cell population was determined by anti-FLAG (BioLegend, USA, cat. no. 637310) staining and by Annexin V and PI staining (MEBCYTO Apoptosis kit, MBL, USA, cat. no. 4700) read with FACSCalibur flow cytometer (BD, USA).

For Naïve T cell isolation, PBMCs were extracted either from leukapheresis fractions collected from informed consenting eligible donors at Hadassah Medical Center (Ein Kerem Campus, Jerusalem, Israel) using a Cobe SpectraTM apheresis apparatus (Gambro BCT, USA) according to Leuakapheresis Unit's SOP or from buffy coats (Sheba Medical Center, Israel) loaded on a Ficoll density gradient and centrifuged 800x g, 2-8°C, 20 min. T cells were isolated from the positive fraction using MagniSort Human CD3 Positive Selection Kit (eBioscience, USA, cat. no. 8802-6830-74) following manufacturer's guidelines. T cells were cryopreserved in "Complete Medium" (defined above) containing an additional 20% FBS (Gibco, ThermoFisher Scietific, South America, cat. no. 12657-029) and 5% DMSO (CryoSure-DMSO, WAK-Chemie Medical GmbH, Germany, cat. no. WAK-DMSO-70) and thawed on the day of experiment parallel to the CD19-CAR T cells.

*LDH cytotoxicity assay*

Lactate dehydrogenase (LDH), a stable cytosolic enzyme, is released by cells undergoing lysis in a correlative manner. Hence, LDH levels in the medium can be used to quantify cytotoxic activity. CytoTox 96 Non-Radioactive Cytotoxicity Assay (Promega, USA, cat. no. G1780) is a colorimetric assay to quantify LDH levels in the medium. A tetrazolium salt substrate (iodonitro-tetrazolium violet, INT) is introduced to the medium in excess and LDH converts the substrate into a red formazan product. The amount of red color formed is directly proportional to the number of cells lysed.

In brief, and following manufacturer's guidelines, target cells (HeLa or HeLa-CD19) were cultured alone or in conjunction with monocytes. After target cells adhered to the plate (6h-overnight), cultures were exposed to y $x10^6$ ApoCells cells for 1h, after which these cells were washed off by 4-5 washes of RPMI. Removal of ApoCells cells was confirmed visually under a light microscope. 10 ng/ml LPS (Sigma-Aldrich, USA, cat. no. L4391) was introduced to the co-culture and incubated for 1h. After incubation, LPS was removed by 3-5 washing cycles with RPMI. Viable CD19-CAR T cells or naïve T cells were added at the designated E/T ratio(s) and incubated for 4h. To collect media, plates were centrifuged at 250x g, 2-25°C, 4 min. (Centrifuge 5810 R, Eppendorf, Germany) to sediment cells. 50 $\mu$l of supernatant medium from each well was transferred to a fresh flat-bottom 96-well microplate well (Corning, USA, cat. no. 3596) and 50 $\mu$l CytoTox 96 Reagent was added to each well. Plates were incubated in the dark at room temperature for 30 min., after which the reaction was terminated by addition of 50 $\mu$l Stop Solution per well. Absorbance was read at 492 nm using Infinite F50 (Tecan, Switzerland) and captured using Magellan F50 software. Data analysis and graph generation was performed using Microsoft Excel 2010.

*Flow cytometry cytotoxicity assay*

HeLa-CD19 (target) and HeLa (control) cells were pre-stained with 5 $\mu$M carboxyfluorescein succinimidyl ester (CFSE, Life Technologies, USA, cat. no. C1157), mixed together, and plated on either fresh plates or on plates populated with isolated primary monocyte. After target cells adhere to the plate (6h-overnight), cultures were exposed to y x10[6] ApoCells cells for 1h. Plates were washed with RPMI 3-5 times and visually verified that suspended ApoCells cells were washed off. 10 ng/ml LPS was introduced to the co-culture and incubated for 1h, after which LPS was removed by 3-5 washing cycles with RPMI. Viable CD19-CAR T cells were then added to the co-cultures as indicated by specific E/T ratio(s) and incubated for 4h. After incubation, cells were harvested by adding trypsin-EDTA (Biological Industries, Israel, cat. no. 03-052-1B) and incubating for 4 min. at 37°C. To terminate the enzymatic activity, two- to four-fold volume of "complete medium" was added. Cells were collected, centrifuged at 200x g for 5 min. at room temperature and re-suspended in 100 $\mu$l RPMI (Gibco, ThermoFisher Scientific, USA, cat. no. 15140-122). Staining ensued first against anti-CD19 (eBioscience, USA, cat. no. 12-0198-42), incubated in dark for 30 min. at room temperature. After centrifugation (290x g, 1 min., 2-8°C) and re-suspended in 300 $\mu$l RPMI, cells were stained against anti-7AAD (eBioscience, USA, cat. no. 00-6993-50). Analysis was gated on 7ADD-negative cells (live cells), where live target cell (HeLa-CD19) and live control cells (HeLa) was calculated. Percent survival was calculated by dividing percent live target cells by percent live control cells. To correct for variation in starting cell numbers and spontaneous target cell death, percent survival was divided by the ratio of percent target cells to percent control cells cultured without effector cells (CD19-CAR T cells). Finally, percent cytotoxicity was determined by subtracting the corrected survival percentage from 100% [2].

Initial experiments are performed by incubating Raji cancer cells with CD19+ CAR T-cells (+/- monocytes-macrophages) for 48 hours in order to determine optimal ratios of CD19+ CAR T-cells to target Raji cancer cells, beginning with 5x10[4] Raji cells/well in a 96-well plate. An effector/target (E/T) ratio plate is constructed based on the results.

Combination immunotherapy experiments are performed by incubating the Raji cancer cells with apoptotic cells, or apoptotic supernatants, for 1 hour followed by co-culturing with CD19+ CAR T-cells (+/- monocytes-macrophages) for 48 hours.

In order to simulate *in vivo* conditions, 1x10[5] THP-1 cells/ml will be differentiated with 200 nM (123.4 ng/ml) phorbol myristate acetate (PMA) for 72 hrs and will then be cultured in complete medium without PMA for an additional 24h. Next, Raji cancer cells will be plated in a 24-well plate at 5x10[5] Raji cells/well on the differentiated THP-1 cells.

Following initial culturing of the Raji cancer cells, 4x10[5]-8x10[5] apoptotic cells (ApoCell) will be added to the culture for 1-3h to induce an immunotolerant environment. The ratio of cancer cell to ApoCell will be optimized for each cell type. After washing, the co-culture will be treated with a pre-determined number of CD19[+] CAR-T cells based on the E/T ratio graph. In certain experiments, 10 ng/ml LPS will be added to the culture media prior to addition of the CD19+ CAR T-cells. In other experiments, interferon $\gamma$ (IFN-$\gamma$) will be added to the culture media prior to addition of the CD19+ CAR T-cells. The addition of LPS or IFN-$\gamma$ is expected to exponentially increase the cytokine storm level.

To assay for Raji cancer cell cytotoxicity, lysates are prepared and viability is determined after the 48 hour incubation period. Additional experiments will be performed assaying for Raji cell cytotoxicity at intervals within the 48h incubation time period. Alternatively, Promega's CytoTox 96 Non-Radioactive Cytotoxicity Assay (Promega, cat #G1780) will be used.

Similar experiments are run with CD19 expressing HeLa cells and CD19+ CAR T-cells.

Similar experiments are run with CD123 expressing leukemic cells and CD123+ CAR T-cells.

*Cytokine Analysis*

Initial cytokine assays examine the levels of MIP1a, IL-4, IL-2, IL-2R, IL-6, IL8, IL-9, IL-10, IL-13, IL-15, INF-$\gamma$, GMCSF, TNF-$\alpha$, in the culture supernatant.

Additional cytokine assays examine the level of cytokines IL-10, IL-1$\beta$, IL-2, IP-10, IL-4, IL-5, IL-6, IFN$\alpha$, IL-9, IL-13, IFN-$\gamma$, IL-12p70, GM-CSF, TNF-$\alpha$, MIP-1$\alpha$, MIP-1$\beta$, IL-17A, IL-15/IL-15R, or IL-7, or any combination thereof.

Cultures were established to mimic an *in vivo* CAR T-cell therapy environment. Raji Burkett Lymphoma cells were cultured in the presence of human monocyte-macrophages, LPS and CD19+ CAR T-cells without and with the addition of apoptotic cells.

Raji cells were incubated in the presence of monocytes and LPS, followed by addition of Naive T-cells (Raji + Naive T), CD19+ CAR T-cells (Raji + CAR T), CD19+ CAR T-cells and apoptotic cells (ApoCell) at a ratio of 1:8 CAR T-cells:ApoCells (Raji + CAR T+ApoCell 1:8), CD19+ CAR T-cells and apoptotic cells (ApoCell) at a ratio of 1:32 CAR T-cells:ApoCells (Raji + CAR T+ApoCell 1:32), and CD19+ CAR T-cells and apoptotic cells (ApoCell) at a ratio of 1:64 CAR T-cells:ApoCells (Raji + CAR T+ApoCell 1:64). Concentration measurements were made following GM-CSF and TNF-$\alpha$ (TNF-a).

To assay for cytokine release reduction of IL-6, IL-8, and IL-13, as well as other cytokines, supernatants will be collected and examined for selected cytokine using Luminex MagPix reader and ELISA assays. Cytokines (mouse or human) may be evaluated by Luminex technology using MAPIX system analyzer (Merck Millipore)) and MILIPLEX analysis software (Merck Millipore). Mouse IL-6Rα, MIG (CXCL9) and TGF-β1 were evaluated by Quantikine ELISA (R&D systems).

*Tissue Analysis*

Bone marrow and liver were evaluated using flow cytometry and immunohistochemistry. Upon sacrifice liver and bone marrow were collected for histopathological analysis. Tissues were fixed in 4% formalin for 48h at room temperature, and then submitted to the animal facility at the Hebrew University for processing. Bones were decalcified prior to processing. Paraffin sections were stained for Hematoxylin and Eosin, and CD19.

*IFN-γ Effect*

IFN-γ effect is evaluated both by STAT1 phosphorylation and biological products.

***Results:***

*Calibrating cell number for Cytotoxicity assay*

To determine the number of Raji cells to be used in the *in vitro* model, sensitivity limits of the cytotoxicity assay was assessed. $5 \times 10^4$-$20 \times 10^4$ Raji cells/well were plated in a 96-well plate, in quadruplicate. Lysis was performed on one set of quadruplicate to be compared with cells that are still completely viable. Lysis was momentary, adding the lysis solution immediately prior to centrifugation to simulate partial cell cytotoxicity. Indeed, all cell quantities exhibited readings well above viable cells, with the $5 \times 10^4$ cell number producing the greatest relative reading **(Figure 14;** extrapolation of data). Therefore, subsequent experiments will be using this cell number as default, unless otherwise required by experimental deign.

*Verification of CD19⁺ CAR-T cell activity against Raji Burkett Lymphoma cells*

To corroborate the CD19⁺ CAR T-cell activity, monolayers of Raji cancer cells are exposed to either 1,000,000 (one million) CD19⁺ CAR-T cells or to 1,000,000 (one million) non-transduced T cells. After 24h incubation, CD19⁺ CAR-T cells reduce Raji cancer cell proliferation, showing anti-tumor activity of the CD19⁺ CAR-T cells.

*Activity of stand-alone CD19+ CAR-T cells against Raji Burkett Lymphoma cells was compared to activity post exposure to Apoptotic Cells*

Apoptotic cells (ApoCell) and apoptotic cell supernatants (ApoSup and ApoMon Sup) are tested to determine if they interfere with CD19+ CAR-T cell anti-tumor activity. The Raji Burkett Lymphoma cells are incubate with Apoptotic Cells for one hour, followed by the addition of CD19+ CAR-T cells (500,000, five hundred thousands) or CD19+ non-transduced T cells (500,000, five hundred thousands) (ratio of 1:2 CD19⁺ CAR-T cells to Apoptotic Cells). The tumor cell/Apoptotic cell/CD19⁺ CAR T-cells are then co-cultured for 48h. The control Raji Burkett Lymphoma cells are co-cultured with CD19+ CAR-T cells and Hartman solution (the vehicle of Apoptotic Cells), but without Apoptotic Cells, for 48h.

The results are showing that after 48h incubation, CD19+ CAR-T cells anti-tumor activity was superior to incubation with non-transduced T cells. Similar incubations will be performed with apoptotic cell supernatants. Surprisingly, CD19+ CAR T-cell anti-tumor activity is comparable with or without exposure to apoptotic cells or apoptotic cell supernatants.

No negative effect of apoptotic cells on CAR-modified T cells against CD19 both in vitro was seen with comparable E/T ratio results of CAR T in the presence or absence of apoptotic cells.

*Verification of CD19⁺ CAR-T cell activity against HeLa Leukemia cells*

HeLa cells are specific CD19 expressing cells, which renders them susceptible to CAR CD19⁺ T-cell activity. In addition, in contrast to Raji cells, which are a non-adherent cell line, HeLa cells are adherent.

To corroborate the CD19⁺ CAR T-cell activity, monolayers of HeLa cancer cells were exposed to either 1,000,000 (one million) CD19⁺ CAR-T cells or to 1,000,000 (one million) non-transduced T cells. After 24h incubation, CD19⁺ CAR-T cells reduce HeLa cancer cell proliferation, showing anti-tumor activity of the CD19⁺ CAR-T cells **(Figure 15** CD19⁺ + RPMI and CD19⁺ + CAR T-19 cells).

*Activity of stand-alone CD19+ CAR-T cells against CD19+HeLa cells was compared to activity post exposure to Apoptotic Cells*

Apoptotic cells (ApoCell) were tested to determine if they interfere with CD19+ CAR-T cell anti-tumor activity. The HeLa cells were incubated with Apoptotic Cells for one hour, followed by the addition of CD19+ CAR-T cells (500,000, five hundred thousand) or CD19+ non-transduced T cells (Naive T cells; 500,000, five hundred thousand) (ratio of 1:2 CD19+ CAR-T cells to Apoptotic Cells). The tumor cell/Apoptotic cell/CD19+ CAR T-cells were then co-cultured for 48h. The control HeLa cells were co-cultured with CD19+ CAR-T cells and RPMI (the vehicle of Apoptotic Cells), but without Apoptotic Cells, for 48h. The CD19+CAR-T cell: HeLa cell ratio (E/T ratio) ranged from 5-20 **(Figure 15).**

**Figure 15** shows that after 48h incubation, CD19+ CAR-T cells anti-tumor activity was superior to incubation with non-transduced T cells (Naive cells) or buffer alone. Similar incubations were performed with apoptotic cells. Surprisingly, CD19+ CAR T-cell anti-tumor activity was comparable with or without exposure to apoptotic cells. Similar experiments are performed using apoptotic cell supernatants. **Figure 15** shows the same *in vitro* cytotoxicity effect of CAR T-CD19 therapy with or without the addition of ApoCells.

No negative effect of the apoptotic cells on CAR-modified T cells against CD19+HeLa cells was observed at comparable E/T ratios in the presence or absence of apoptotic cells.

Thus, the same *in vitro* cytotoxic effect of the CD19+CAR T-cells was observed with or without the addition of early apoptotic cells.

*Effect of Apoptotic Cells on amelioration, reduction or inhibition of cytokine storms resulting from CAR-T treatment*

Cytokines IL-8 and IL-13 are measured in the culture media prior to and following addition of CD19+ CAR T-cells and are showing a concentration consistent with a cytokine storm. Addition of apoptotic cells or apoptotic cell supernatant is showing a reduction of IL-8 and IL-13 concentrations in the media.

*Analysis using a wider range of cytokines*

To further evaluate the effect on a possible wider range and levels of cytokines that are not generated during experimental procedures but do appear in clinical settings during a human cytokine storm, LPS (10 ng/ml) was added to the Raji cell culture conditions outlined above in the presence of cancer and CAR-19. The addition of LPS was expected to exponentially increase the cytokine storm level. Exposure to apoptotic cells is dramatically reduced the levels of cytokines. The results presented in **Figure 16** and **Figure 17** show that while addition of CD19+ CAR T-cell greatly increases cytokine concentration (pg/ml) of GM-CSF and TNF-$\alpha$ in the culture medium, there is a significant decrease of both GM-CSF and TNF-$\alpha$ in the presence of apoptotic cells. The decrease in the cytokine concentration is dose dependent with respect to apoptotic cell ratio of CAR T-cells to apoptotic cells.

*Conclusion:*

Apoptotic cells were able to down regulate cytokine markers of cytokine storm associated with CAR T-cell clinical procedures. Significantly, the apoptotic cells did not show an effect on the tumor activity of the CAR T-cells. Apoptotic cells decreased pro-inflammatory cytokines that originated from innate immunity and inhibit IFN-$\gamma$ effect without harming IFN-$\gamma$ levels and CAR-T cytotoxicity.

### REFERENCE EXAMPLE 6: Apoptotic Cell Therapy Prevents Cytokine Storms in A Diffuse Cancer in vivo Model Administered Car T-Cell Therapy

*Objective:* Test the *in vivo* effect of apoptotic cells or supernatants derived from apoptotic cells in a diffuse tumor model, in order to determine CAR T-cell efficacy on the cancer cells and the level of cytokine storm marker cytokines.

**Materials and Methods**

**In vitro studies**

See methods described in Reference Example 5 for *in vitro* studies.

*Cells and cell culture*

Raji Burkitt lymphoma cells (Sigma-Aldrich cat. # 85011429) were cultured as per the manufacture's guidelines.

CD19+ CAR T-cells, cell cultures, apoptotic cells, apoptotic cell supernatants, monocyte isolation, and *in vitro* measurements are as above for Examples. Early apoptotic cells produced were least 50% annexin V-positive and less than 5% PI-positive cells.

### *In vivo studies*

*Mice*

7-8 week old SCID beige mice were purchased from Envigo (formerly known as Harlan). Mice were kept in an SPF free animal facility in compliance with institutional IACUC guidelines. During the course of the experiments the mice were monitored daily, and weighted 3 times a week. Mice showing hind limb paralysis were sacrificed. Upon sacrifice bone marrow and liver were collected for FACS analysis and histological processing, and sera were frozen at -80°C for cytokine profiling. *In vivo experiments*

SCID beige mice (C.B-17/IcrHsd-Prkdc-SCID-Lyst-bg, Harlan, Israel) were housed in SPF conditions at The Authority for Animal Facilities (AAF), The Hebrew University of Jerusalem (Ein Kerem Campus, Israel) and following the Association for Assessment and Accreditation of Laboratory Animal Care (AAALAC). The studies were approved by The Hebrew University Ethics Committee for Animal Experiments, and animal suffering was minimized as possible.

**(Figure 18A)** For the disseminating tumor model, 7-8 week female SCID beige mice were injected i.v. with $1\times10^5$ Raji cells suspended in 200 $\mu$l RPMI (Gibco, , ThermoFisher Scientific, USA, cat. no. 15140-122) per mouse (day 1). On day 6, mice of pertinent groups were inoculated i.v. with $30\times10^6$ cells ApoCells in 200 $\mu$l Hartmann's solution Lactated Ringer's Injection, Teva Medical, Israel, cat. no. AWN2324) per mouse. On day 6, mice of relevant groups were inoculated i.v. with $10\times10^6$ viable CD19-CAR T cells or naïve T cells in 200 $\mu$l AIM V per mouse. Control mice received equal volume of RPMI for each treatment.

Mice were examined for clinical indications and weighed twice a week and were sacrificed upon development of hind limb paralysis. Pathological samples of bone and liver were prepared by the Animal Facility Unit of The Hebrew University of Jerusalem and stained against human CD20 (Cell Marque, USA, clone L26, cat. no. 120M-84), to detect Raji cells, and against human CD3 (Cell Signaling Technology, USA, cat. no. 85061), to detect human T cells.

In certain experiments, LPS will be administered to the animal subject prior to addition of the CD19+ CAR T-cells. In other experiments, interferon-$\gamma$ (IFN-$\gamma$) will be administered prior to addition of the CD19+ CAR T-cells. The addition of LPS or IFN-$\gamma$ is expected to exponentially increase the cytokine storm level.

Cytokine assays examine the level of cytokines including IL-10, IL-1$\beta$, IL-2, IP-10, IL-4, IL-5, IL-6, IFN$\alpha$, IL-9, IL-13, IFN-$\gamma$, IL-12p70, GM-CSF, TNF-$\alpha$, MIP-1$\alpha$, MIP-1$\beta$, IL-17A, IL-15/IL-15R, or IL-7, or any combination thereof. Cytokines (mouse or human) are evaluated by Luminex technology using MAPIX system analyzer (Merck Millipore)) and MILIPLEX analysis software (Merck Millipore). Mouse IL-6R$\alpha$, MIG (CXCL9) and TGF-$\beta$1 are evaluated by Quantikine ELISA (R&D systems).

*Tissue Analysis*

Bone marrow and liver are evaluated using flow cytometry and immunohistochemistry. Upon sacrifice liver and bone marrow were collected for histopathological analysis. Tissues were fixed in 4% formalin for 48h at room temperature, and then submitted to the animal facility at the Hebrew University for processing. Bones were decalcified prior to processing. Paraffin sections were stained for Hematoxylin and Eosin, and CD19.

*IFN-$\gamma$ Effect*

IFN-$\gamma$ effect is evaluated both by STAT1 phosphorylation and biological products.

**Results**

*CAR T-cell therapy induces cytokine release syndrome*

Three groups of tumor-free mice as well as mice with tumors are administered (i.p. or directly into the tumor) increasing doses of CD19+ CAR T-cells ($3\times10^6$, $10\times10^6$ or $30\times10^6$). At the highest dose, tumor-free mice and mice with tumors demonstrate subdued behavior, piloerection, and reduced mobility within 24 h, accompanied by rapid weight loss followed by death within 48 hrs. Human interferon-gamma, and mouse IL-6 , IL-8, and IL-13 are detectable in blood samples from the mice given the highest dose of CD19+ CAR T-cells. Animals that receive a high dose of CD19+ CAR T-cells directed to a different tumor antigen do not exhibit weight loss or behavioral alterations.

*Administration of apoptotic cells inhibits or reduces the incidence of cytokine release syndrome induced by CAR T-cell therapy*

One group of mice given the highest dose of CD19+ CAR T-cells is concomitantly administered $2.10 \times 10^8$/kg apoptotic cells, which was previously demonstrated to be a safe and effective dose. Mice receiving human CD19+ CAR T + apoptotic cells have significantly lowered levels of at least one mouse pro-inflammatory cytokines, lower weight loss, and reduced mortality.

*Administration of apoptotic cells in combination with CAR T-cell administration did not affect CAR T-cell anti-tumor activity*

**Figure 18B** shows that the expected death of SCID mice injected with CD19+ Raji cells without administration of CD19+CAR T-cells was 18-21 days. Forty percent (40%) of the mice who received CD19+CAR T-cells survived to at least day 30 **(Figure 18** dash-dot-dash line and dash-double dot-dash line). The percentage of survivors was independent of the addition of apoptotic cells **(Figure 18)**. The surviving mice were sacrifice on day 30.

Conclusion: There was comparable survival and no negative effect of apoptotic cells on CAR-modified T cells against CD19 *in vivo.*

Significant down regulation (p<0.01) of pro-inflammatory cytokines including, IL-6, IP-10, TNF-a, MIP-1α, MIP-1β was documented. IFN-γ was not downregulated but its effect on macrophages and dendritic cells was inhibited both at the level of phosphorylated STAT1 and IFN-γ-induced expression of CXCL10 and CXCL9.

*Conclusion:*

Apoptotic cells decrease pro-inflammatory cytokines that originate from innate immunity and inhibit IFN-γ effect without harming IFN-γ levels and CAR-T cytotoxicity.

### REFERENCE EXAMPLE 7: Apoptotic cell therapy prevents cytokine storms in a solid tumor cancer in vivo model administered CAR T-cell therapy

*Objective:* Test the *in vivo* effect of apoptotic cells or supernatants derived from apoptotic cells in a solid tumor model, in order to determine CAR T-cell efficacy on the cancer cells and the level of cytokine storm marker cytokines.

**Materials and Methods**

*In vitro studies*

*Cells and cell culture*

CD19+ CAR T-cells, Second generation CAR-T-CD19 cells containing TMCD28 were used, cell cultures, apoptotic cells, apoptotic cell supernatants, monocyte isolation, and *in vitro* measurements were as above for Examples 2 & 4 & 6. Early apoptotic cells produced were least 50% annexin V-positive and less than 5% PI-positive cells, as described in detail in Example 1.

*In vivo studies*

*Mice*

7-8 week old SCID-beige mice and NSGS mice were purchased from Harlan (Israel) and kept in the SPF animal facility in Sharett Institute.

SCID beige mice or NSGS mice were inoculated with CD19 expressing Hela cells, that can adhere to the peritoneum, in order to form solid intra-peritoneal tumors. Mice were sorted into groups prior to T-cell administration.

Six days post i.v. inoculation, mice were administered $10 \times 10^6$ CD19+ CAR T-cells with and without apoptotic cell (ApoCell) preconditioning on day 5. Mice receiving pre-conditioning were administered $5 \times 10^6$ or $30 \times 10^6$ ApoCells. Tumors were surveyed weekly and circulating cytokine levels were monitored weekly and determined by the Luminex system. 25 mouse cytokines and 32 human cytokines were evaluated using the Luminex technology. Upon termination of the experiment, mice were culled and organs (bone marrow, liver and spleen) were examined (by FACS and immunohistochemistry) for the presence/size of tumors.

Cytokine assays examined the level of cytokines including GM-CSF, IFNγ, IL-1β, IL-10, IL-12p70, IL-13, IL-15,

**EP 3 883 584 B1**

IL-17A, IL-2, IL-4, IL-5, IL-6, IL-7, IL-9, MIP-1α, TNFα, MIP-1β, IFNα, and IP-10. Cytokines (mouse or human) were evaluated by Luminex technology using MAPIX system analyzer (Mereck Millipore)) and MILIPLEX analysis software (Merck Millipore). Mouse IL-6Rα, MIG (CXCL9) and TGF-β1 were evaluated by Quantikine ELISA (R&D systems).

*Tissue Analysis*

Bone marrow and liver are evaluated using flow cytometry and immunohistochemistry.

*IFN-γ Effect*

IFN-γ effect was evaluated both by STAT1 phosphorylation and biological products.

**Results**

*CAR T-cell therapy induces cytokine release syndrome*

Three groups of tumor-free mice as well as mice with tumors were administered (i.p. or directly into the tumor) increasing doses of CD19+ CAR T-cells ($3x10^6$, $10 x10^6$ or $30x10^6$). At the highest dose, tumor-free mice and mice with tumors demonstrate subdued behavior, piloerection, and reduced mobility within 24 h, accompanied by rapid weight loss followed by death within 48 hrs.

**Figures 19A-19C** graphically show the increased levels of IL-6, IP-10 and surprisingly even TNF-α cytokine release from tumors even before the presence of CAR T-cells. **Figures 19A-19C** show that unexpectedly IL-6, IP-10, and TNF-α were increased by the presence of cancer cells even without CAR T-cell therapy. In the presence of CAR T-Cell therapy (Hela-CAR T-cell CD-19) the release of cytokines was significantly augmented. These results show that the tumor itself releases pro-inflammatory cytokines.

In order to evaluate the benefit of the addition of early apoptotic cells, cytokines GM-CSF, IFNγ, IL-1β, IL-10, IL-12p70, IL-13, IL-15, IL-17A, IL-2, IL-4, IL-5, IL-6, IL-7, IL-9, MIP-1α, TNFα, MIP-1β, IFNα, and IP-10 were measured in three experiments, wherein the results showed that macrophage associated cytokines were down-regulated in the presence of ApoCell administration, while T-cell associated cytokine levels were not significantly changed (Table 10).

**Table 10:** Cytokine levels from an intra-peritoneum *in vivo* model that contained CD19 expressing Hela cells solid tumor, +/- CAR T-cell CD19 therapy, and +/- ApoCell

| Pg/ml | Before Tumor Car or Apo | After tumor + CAR | After tumor, CAR, +with ApoCell |
|---|---|---|---|
| GM-CSF | 4±2 | 88±10 | 12±4 |
| IFNγ | 4±1 | 5±8 | 5±21 |
| IL-1β | 8±3 | 14±6 | 16±8 |
| IL-10 | 76±13 | 222±44 | 36±22 |
| IL-12p70 | 5±1 | 188±22 | 12±11 |
| IL-13 | 6±2 | 8±1 | 8±4 |
| IL-15 | 4±2 | 6±2 | 8±2 |
| IL-2 | 4±2 | 26±2 | 29±2 |
| IL-4 | 1±2 | 16±4 | 18±6 |
| IL-6 | 24±6 | 820±56 | 74±12 |
| MIP-1α | 8±5 | 99±13 | 18±8 |
| TNFα | 6±2 | 760±33 | 17±15 |
| MIP-1β | 7±1 | 144±21 | 21±10 |
| IFNα | 74±12 | 68±26 | 71±14 |
| IP-10 | 8±4 | 188±33 | 21±16 |

Table 10 shows cytokine measurement twenty-four (24) hours after CAR T-Cell administration +/- ApoCells. Resultant cytotoxicity from CAR T-cell therapy elevated cytokines including GM-CSF, IL-10, IL-12p70, IL-6, MIP-1α,

**60**

TNF$\alpha$, MIP-1$\beta$, and IP-10, the levels of which were significantly down regulated (p<0.05-0.0001) in the presence of ApoCells. These cytokines are mainly associated with macrophages. In contrast, the levels of cytokines associated with T-cells such as IL-2, IL-4, IL-13, and IL 15 were not changed significantly.

The results presented in **Figures 19A-C** and Table 10, illustrate that the CRS in the context of cancer and CAR has several ingredients: a tumor that can secrete cytokines; an innate immunity that respond to tumor and to CAR and to other factors; and that CAR T-cells that secrete cytokines causes death that influence innate immunity. ApoCells are interacting with innate immunity, mainly macrophages, monocytes and dendritic cells, to down regulate the response of these macrophages, monocytes and dendritic cells without interacting with T cells or CAR T cells.

Animals that received a high dose of CD19+ CAR T-cells directed to a different tumor antigen do not exhibit weight loss or behavioral alterations.

*Administration of apoptotic cells inhibits or reduces the incidence of cytokine release syndrome induced by CAR T-cell therapy*

One group of mice given the highest dose of CD19+ CAR T-cells was concomitantly administered $2.10 \times 10^8$/kg apoptotic cells, which was previously demonstrated to be a safe and effective dose. Apoptotic cells had no negative effect *in vitro* or *in vivo* on CAR-modified T cells with specificity against CD19. There were comparable E/T ratios for CAR T-cells in the presence/absence of apoptotic cells *in vitro,* and comparable survival curves *in vivo* (Data not shown).

Mice receiving human CD19+ CAR T + apoptotic cells had significantly lowered levels of at least one mouse pro-inflammatory cytokines, lower weight loss, and reduced mortality.

No negative effect of apoptotic cells on CAR-modified T cells against CD19 *in vivo* was seen with comparable E/T ratio results of CAR T in the presence or absence of apoptotic cells, and a comparable survival curve in vivo.

Significant down regulation (p<0.01) of pro-inflammatory cytokines including, IL-6, IP-10, TNF-a, MIP-1$\alpha$, MIP-1$\beta$ was documented (Data not shown). IFN-$\gamma$ was not downregulated but its effect on macrophages and dendritic cells was inhibited both at the level of phosphorylated STAT1 and IFN-$\gamma$-induced expression of CXCL10 and CXCL9 (Data not shown.

*Conclusion:*

. CRS evolves from several factors, including tumor biology, interaction with monocytes/macrophages/dendritic cells, and as a response to the CAR T cell effect and expansion. Apoptotic cells decrease pro-inflammatory cytokines that originate from innate immunity and inhibit the IFN-g effect on monocyte/macrophages/ dendritic cells without harming IFN-$\gamma$ levels or CAR-T cytotoxicity. Thus, apoptotic cells decreased pro-inflammatory cytokines that originate from innate immunity and inhibit IFN-$\gamma$ effect without harming IFN-$\gamma$ levels and CAR-T cytotoxicity. These results support the safe use of ApoCells for the prevention of CRS in clinical studies using CAR-T cell therapy.

***REFERENCE EXAMPLE 8: Apoptotic cells downregulate Cytokine Release Syndrome (CRS) and increases CAR-T-cell efficacy***

*Objective:* To test the effects of early apoptotic cells on cytokines and CAR T cell cytotoxicity over an extended time period. To demonstrate the *in vivo* efficacy of CD19-CAR T-cells. To demonstrate the synergistic effect of early apoptotic cells and CD19-CAR T-cells.

*Methods:* CD19-expressing HeLa cells (ProMab) were used alone or after co-incubation with human macrophages for *in vitro* and intraperitoneal experiments in mice. Raji was used *in vivo* for leukemia induction. LPS and IFN-g were used to trigger additional cytokine release. Second generation, CD28-bearing, CD19-specific CAR-modified cells were used (either ProMab or produced using a retronectin manufacturing protocol or a polybrene manufacturing protocol) for anti-tumor effect against CD19-bearing cells. Cytotoxicity assay was examined *in vivo* (7-AAD flow cytometry) and *in vitro* (survival curves; tumor load in bone marrow and liver, flow cytometry and immunohistochemistry). CRS occurred spontaneously or in response to LPS and IFN-g. Mouse IL-10, IL-1$\beta$, IL-2, IP-10, IL-4, IL-5, IL-6, IFN-a, IL-9, IL-13, IFN-g, IL-12p70, GM-CSF, TNF-a, MIP-1a, MIP-1$\beta$, IL-17A, IL-15/IL-15R, IL-7, and 32 human cytokines were evaluated (Luminex technology, MAPIX system; MILLIPLEX Analyst, Merck Millipore). Mouse IL-6Ra, MIG (CXCL9), and TGF-$\beta$1 were evaluated (Quantikine ELISA, R&D systems). IFN-$\gamma$ effect was evaluated (STAT1 phosphorylation, biological products). Human macrophages and dendritic cells were generated from monocytes. Early apoptotic cells were produced generally as presented in Example 1 above; $\geq$40% of cells were Annexin V-positive; $\leq$15% were PI-positive.

*Mice:* SCID-Bg mice (female, 7-8 wk) were injected with 2 consecutive doses of $0.25 \times 10^6$ HeLa-CD19 cells, intra-peritoneally (i.p) on days 1 and 2 of experiment. On day 9 mice received i.p. dose of $10 \times 10^6$ 4000-rad (cGy) irradiated ApoCell (using a cell processing system) and an i.p. dose of a population of $10 \times 10^6$ CAR-T cells comprising either $0.5 \times 10^6$ CAR-T positive cells or $2.2 \times 10^6$ CAR-T positive cells, on the following day. As control, mice received $10 \times 10^6$ activated

mononuclear cells or Mock-T cells. Mice were kept in an SPF animal facility in compliance with institutional IACUC guidelines. Mice were weighted twice a week and monitored daily for clinical signs and peritonitis. End point was defined as severe peritonitis manifested as enlarged and tense abdomen, lethargy, reduced mobility or increased respiratory effort. Survival analysis was performed according to the Kaplan-Meier method.

*Results:* Significant downregulation (p<0.01) of pro-inflammatory cytokines, including IL-6, IP-10, TNF-a, MIP-1a, MIP-1β, was documented (Data not shown). IFN-g was not downregulated, but its effect on macrophages and dendritic cells was inhibited at the level of phosphorylated STAT1 (Data not shown). IFN-$\gamma$ induced expression of CXCL10 and CXCL9 in macrophages was reduced (Data not shown).

In the experiment wherein $0.5 \times 10^6$ CAR-T positive cells were used, 2 mice per group were sacrificed on day 17 and 21. HeLa-CD19 treated mice showed peritonitis manifested as blood accumulation in the peritoneum, enlarged spleen and tumor loci (Data not shown). Mice treated with control MNCs had a little bit less blood in peritoneum and less tumor loci. Mice treated with CAR-T or with CAR-T and ApoCell had no signs of peritonitis. This observation correlated to the survival curve presented in **Figure 20A.**

In the experiment wherein $2.2 \times 10^6$ CAR-T positive cells were used, the same pattern of effect as seen with four-fold fewer CAR T-cells was observed **(Figure 20B).** CAR-T treatment prolonged survival of mice with peritoneal HeLa-CD19 (p=0.0002). The effect was more significant in this experiment, probably due to the higher number of infused CAR T cells (2.2 versus $0.5 \times 10^6$ CAR-T positive cells). Even with the more significant and prolonged effect, the early apoptotic cells had a synergistic effect and prolonged survival (p= 0.0032). E/T ratios for CAR T were comparable in the presence/absence of apoptotic cells *in vitro.* Surprisingly, CAR T cell therapy given in the presence of apoptotic cells ameliorated survival of mice with a significant and reproducible addition of at least 12 days (p<0.0032, **Figures 20A** and **20B)** in comparison to CAR therapy alone.

*Conclusion:* CAR-T cell treatment prolonged survival of mice with peritoneal HeLa-CD19 cells. Administration of irradiated early apoptotic cells one day before CAR-T had a synergistic effect and prolonged mice survival for more than 10 days (p<0.044, log-rank test), compared to CAR-T cell treatment alone.

The irradiated apoptotic cell infusion had a dramatic synergistic effect to CD19-specific CAR T cells in treating CD19-bearing Hela cells in SCID mice. In this example, similar results were observed to the results presented in Examples 5 and 6. By using irradiated apoptotic cells in this Example, compared with Examples 5 and 6, the possibility of a "graft versus leukemia effect" has been removed. Thus, this surprising synergistic effect appears to be mediated via the irradiated apoptotic cells provided.

CRS evolves from several factors, including tumor biology, interaction with monocytes/macrophages/dendritic cells, and as a response to the CAR T cell effect and expansion. Apoptotic cells decrease pro-inflammatory cytokines originating from innate immunity, and inhibit the IFN-$\gamma$ effect on monocytes/macrophages/dendritic cells without harming IFN-$\gamma$ levels or CAR-T cytotoxicity, and with significant increase in CAR-T cell efficacy. Unexpectedly, treatment with irradiated apoptotic cells complements CAR-T cell therapy, effectively extending the anti-cancer effect of the CAR-T cell therapy.

### REFERENCE EXAMPLE 9: Effect of Apoptotic Cells Treatment on a non-Solid Tumor Model

*Objective:* To test the effect of apoptotic cells on a non-solid tumor model where the cancer is widely spread and not localized or confined, in order to determine apoptotic cells efficacy on the survival in cancer.

### *Methods:*

### *Raji cells*

Raji cells were purchased from ECACC (Cat. #: 85011429), and routinely cultured in complete medium (RPMI-1640 supplemented with 10% H.I. FBS, 1% Glutamax, 1% Penicillin / Streptomycin), and maintained at a concentration of $3\times10^5$ - $3\times10^6$ cells/ml.

*Apoptotic cells* were prepared as described in Example 1. Early apoptotic cells produced were at least 50% annexin V-positive and less than 5% PI-positive cells.

### *Non-solid (diffuse) tumor model*

SCID mice received a single IV injection of $10^5$ Raji cells on day 1 of the experiment. A control group of SCID mice received a single IV injection of saline solution. (3 cohorts were tested; leukemia was induced in 2 cohorts using Raji cells, and 1 cohort was maintained as a control.)

*Solid tumor model*

SCID mice will receive a single investigational product (IP; Allocetra-OTS) injection of $10^5$ Raji cells on day 1 of the experiment, wherein control groups will receive a single investigational product (IP; Allocetra-OTS) injection of saline solution.

*Apoptotic cell treatment*

In a preliminary study, mice received an infusion of early apoptotic cells 6 days after the infusion of Raji cells. In later studies, the mice from one of the leukemic cohorts above received 3 infusions of early apoptotic cells (30 x $10^6$ cells) starting 6 days after the infusion of Raji cells.

***Results***

SCID mice have no T-cells and therefore no ability to recover from leukemia without therapy.

Surprising, in the preliminary study and as shown in **Figure 21,** the apoptotic cell infusion (APO) 6 days after the infusion of Raji, significantly prolonged tumor free death in SCID injected with CD19+ Raji, compared with mice that did not receive an apoptotic cell infusion (NO APO).

In the leukemic (NO APO) cohort, 70% of mice receiving Raji cells survived through their lifespan, compared to 94% of mice receiving both Raji cells and apoptotic cells (n=51 animals in total, p<0.001). As expected, 100% of control mice survived through their expected lifespan **(Figure 22A).** In the leukemic cohort, 9% of mice receiving Raji cells and no apoptotic cells survived through up to 12% above the expected lifespan, compared to 47% of mice receiving both Raji cells and apoptotic cells **(Figure 22B).** No mice receiving Raji cells and no apoptotic cells survived through greater than 30% of the expected lifespan, compared to 41% of mice receiving both Raji cells and apoptotic cells **(Figure 22C).** No mice receiving Raji cells and no apoptotic cells attained complete remission, compared to 10% of mice receiving both Raji cells and apoptotic cells **(Figure 22D).**

*Conclusion:*

Administration of an apoptotic cell infusion maintained and increased the lifespan of leukemic mice, wherein in certain instances mice administered early apoptotic cells attained complete remission (Figures **2 and 3A-3D).**

***REFERENCE EXAMPLE 10: Effect of Combined Apoptotic Cell and anti-CD20 mAb Treatment on a Diffuse Tumor Model***

***Objective:*** To test the effect of administering a combination of early apoptotic cells and anti-CD20 mAb on a diffuse (non-solid) tumor model, wherein the cancer is widely spread and not localized or confined, in order to determine the efficacy on survival of this combination therapy.

Raji cells, apoptotic cells, non-solid (diffuse) tumor model, solid tumor model, and apoptotic cell treatment were as described in Examples 1 and 9 above.

*anti-CD20 mAb*

Commercially available anti-CD20 mAb was acquired from Roche.

*anti-CD20 mAb treatment*

Mice received an IV infusion of 5 mg of anti-CD20 mAb.

*Combined Apoptotic cell and anti-CD20 mAb treatment*

Starting at day 6 following Raji cell administration, mice received three IV infusions of 30x$10^6$ apoptotic cells each. In addition, mice received an IV infusion of 5 mg of anti-CD20 mAb.

***Results***

100% of mice receiving Raji cells, Raji cells + anti-CD20 mAb, and Raji cells + antiCD20 + apoptotic cells survived through the expected lifespan of leukemic mice, compared to 86% of mice receiving both Raji cells and apoptotic cells (n=28 animals in total, p<0.0002) **(Figure 23A).** No mice receiving Raji cells survived longer than 24% above the expected lifespan, compared to 29% of mice receiving both Raji cells + apoptotic cells, and 100% of mice receiving either Raji cells + anti-CD20 mAb or Raji cells + antiCD20 + apoptotic cells **(Figure 23B).** No mice receiving Raji cells survived longer than 59% above the expected lifespan, compared to 29% of mice receiving both Raji cells + apoptotic cells, 57% of mice receiving Raji cells + anti-CD20 mAb, and 100% of mice receiving Raji cells + antiCD20 + apoptotic cells **(Figure 23C).** No mice receiving Raji cells survived longer than 76% above the expected lifespan, compared to 29% of mice receiving both Raji cells + apoptotic cells, 14% of mice receiving Raji cells + anti-CD20 mAb, and 85% of mice receiving Raji cells + antiCD20 + apoptotic cells **(Figure 23D).** No mice receiving either Raji cells or Raji cells + anti-CD20 mAb survived longer than 100% above the expected lifespan of a mouse, compared to 29% of mice receiving either Raji cells + apoptotic cells or Raji cells + antiCD20 + apoptotic cells **(Figure 23E).**

*Conclusion:*

Apoptotic cell infusions increased the lifespan of leukemic mice, increased the number of mice attaining complete remission, and enhanced anti-CD20 mAb therapeutic effect **(Figures 23A-23E).**

### *REFERENCE EXAMPLE 11: Effect of ApoCell (Early Apoptotic Cells) on Leukemia/Lymphoma*

*Objective:* The work presented here had three main goals: (1) Evaluating the effect of ApoCell in a leukemia-lymphoma mouse model in terms of disease onset, progression, and ensuing death; (2) Assessing the distribution of tumor cells in a mouse model of leukemia-lymphoma after treatment with ApoCell; and (3) Assessing a possible synergistic effect of ApoCell and Rituximab (RtX) in the treatment of leukemia-lymphoma in SCID-Bg mice. As part of the work to meet these objectives, measurement of the survival of leukemic mice following ApoCell administration was measured. As well, the distribution of tumor cells was measured after treatment with ApoCell.

*Methods:*

*Mice.* Female SCID-Bg mice, 7 weeks-old (ENVIGO, Jerusalem, Israel), were injected intravenously with $0.1 \times 10^6$ Raji cells per mouse. Mice received 3 doses of $30 \times 10^6$ ApoCell intravenously on days 5, 8, and 11 of the experiment. For combinational therapy, mice received one dose (day 8) of RtX (2 or 5 mg/kg; Mabthera, Roche, Basel, Switzerland) 1.5h after ApoCell administration.

Mice were followed daily and weighed twice a week. The endpoint was defined as death, or sacrifice due to the development of either of the following symptoms: paraplegia (lower body paralysis), loss of 20% from mouse start weight, lethargy, reduced mobility, or increased respiratory effort.

Survival analysis was performed according to the Kaplan-Meier method. Mice were kept in a specific-pathogen-free (SPF) animal facility in compliance with institutional Animal Care and Use Committee (IACUC) guidelines.

*Raji cell line.* This human Burkitt's lymphoma cell line was purchased from the European Collection of Authenticated Cell Cultures (ECACC, Cat. #: 85011429), and routinely cultured in complete medium (RPMI-1640 supplemented with 10% heat inactivated FBS, 1% glutamax, 1% penicillin / streptomycin).

*ApoCell.* Essentially, as described in Example 1. Briefly, an enriched mononuclear cell fraction was collected via leukapheresis from healthy, eligible donors. Following apheresis completion, cells were washed and resuspended with freezing media composed of PlasmaLyte A pH 7.4, 5% human serum albumin, 10% dimethyl sulfoxide (DMSO), 5% anticoagulant citrate dextrose solution formula A (ACD-A) and 0.5U\ml heparin. Cells were then gradually frozen and transferred to liquid nitrogen for long-term storage.

For preparation of ApoCell, cryopreserved cells were thawed, washed and resuspended with apoptosis induction media, composed of RPMI 1640 supplemented with 2 mM L-glutamine and 10 mM hepes, 10% autologous plasma, 5% ACD-A, 0.5U\ml heparin sodium and $50\mu g$/ml methylprednisolone. Cells were then incubated for 6 hours at 37°C in 5% $CO_2$. At the end of incubation, cells were collected, washed and resuspended in Hartmann's solution using a cell processing system. ApoCell was centrifuged at 290g, for 10 min at 2-8°C, and resuspended in Hartmann's solution for injection. Apoptosis and viability of ApoCell were determined using Annexin V and propidium iodide (PI, Medical & Biological Laboratories, Nagoya, Japan) using FCS express software.

*Flow cytometry.* Mouse spleen, liver, and bone marrow were collected from sacrificed mice (following deterioration of clinical signs, as defined above) and analyzed by flow-cytometry (FACSCalibur, BD, Franklin Lakes, NJ, USA) for the presence of the Raji tumor (anti-CD20).

*Results:*

*Part A: ApoCell delays disease onset and ensuing death in leukemic mice*

**Figure 24** is a Kaplan-Meier survival plot presenting 3 individual experiments (*RPMI group,* n = 15; *Raji group,* n = 23; *Raji + ApoCell group,* n = 24). In each experiment, female SCID-Bg mice (7-8 weeks of age) were injected intravenously with $0.1 \times 10^6$ Raji cells and a control group was injected with RPMI. Subsequently, mice were administered with three doses of $30 \times 10^6$ ApoCell by intravenous administration (IV), on days 5, 8, and 11. Mice were followed daily and weighed twice a week. Endpoint was defined as death, or sacrifice due to the development of either of the following symptoms: paraplegia (lower body paralysis), loss of 20% from mouse start weight, lethargy, reduced mobility, or increased respiratory effort. Experimental details are given in Table **11.** Significant beneficial effect by ApoCell was seen (p=0.002, Log-rank (Mantel-Cox) test).

**Table 11: Experimental details of Figure A plot**

| Group | Number of mice | Survival details | | Notes |
| | | Mean day of sacrifice | Range (days) | |
|---|---|---|---|---|
| **RPMI** | 15 | | | DFS* is shown for all mice |
| **Raji** | 23 | 22 | 21 - 25 | |
| **Raji + ApoCell** | 24 | 28 | 19 - >53 | 2 mice DFS* on day 53 / 60 (termination of experiment) |
| * DFS = disease free survival | | | | |

The data for the individual studies is presented in **Figures 25A-25C.**

As depicted above, mice treated with 3 doses of ApoCell after the administration of Raji cells had a slower disease progression, and died significantly later (p=0.0020) than untreated mice.

Leukemic mice treated with ApoCell had a significant delay in the onset of symptoms, demonstrated a slower disease progression, and died later than the control untreated mice. Interestingly, about 10 percent of the mice administered with Raji cells and ApoCell did not develop any of the expected symptoms characteristic in this leukemia / lymphoma model, and remained healthy until termination of the experiments (day 53 or 60).

*ApoCell reduce tumor load in leukemic mice*

Upon sacrifice, following the deterioration of clinical signs as described above, organs of interest were collected for analysis, namely the liver, spleen, and bone-marrow. Cells of these target organs were analyzed by flow cytometry for the presence of human tumor cells (Raji cells are positive for CD20).

The data below (Table 12) describes the average percent cell population in target organs of the sacrificed mice from the 3 experiments described above; values of individual mice in each experiment can be found in Tables 13-18, which follow.

**Table 12: Average percent of tumor population in Spleen, Bone Marrow and Liver (Flow cytometry)**

| Tissue | Treatment | # of mice | % CD20$^+$ cells (average $\pm$ SD) |
|---|---|---|---|
| **Spleen** | RPMI | 5 | 0 |
| | Raji | 10 | 0 |
| | Raji + ApoCell | 8 | **1.1** $\pm$3 |
| **Bone-Marrow** | RPMI | 5 | 0 |
| | Raji | 10 | **16.5** $\pm$8.8 |
| | Raji + ApoCell | 8 | **6.8** $\pm$9.2 **(P=0.02, t-test)** |
| **Liver** | RPMI | 5 | 0 |
| | Raji | 10 | **11.2** $\pm$12 |
| | Raji + ApoCell | 8 | **4.5** $\pm$7.5 **(P=0.06, t-test)** |

*In vivo experiment 011*

**FACS analysis of CD20+ cells in bone marrow, spleen, and liver:**

One mouse receiving three doses of ApoCell was healthy when sacrificed on day 60.
Liver, spleen, and bone marrow cells were collected and analyzed by flow cytometry (FACSCalibur, BD) for the presence of human CD20-FITC (Biolegend, Cat. # 302206); mIgG1-FITC (Biolegend, Cat. # 400110).

*In vivo experiment 019*

**FACS analysis of tumor cells in bone marrow, spleen, and liver:**

Mouse spleen, liver, and bone marrow cells were collected from mice who were sacrificed following clinical deterioration, as defined in the methods) and analyzed by flow cytometry (FACSCalibur, BD) for the presence of **human CD20** (FITC).
The results of analysis of Spleen, Bone marrow, and Liver are presented in Tables 13-15 below.

**Table 13: Spleen**

| Spleen | | | |
|---|---|---|---|
| Mouse | Treatment | Day of sacrifice | CD20+ |
| A1 | | | 0 |
| A2 | RPMI | | 0 |
| A3 | | | 0 |
| B2 | | 22 | 0 |
| B3 | | 22 | 0 |
| B5 | | 22 | 0 |
| B6 | Raji | 22 | 0 |
| B2 (020) | | 25 | 0 |
| B3 (020) | | 25 | 0 |
| C1 | | 28 | 0 |
| C2 | | 53 (healthy) | 8.7 |
| C4 | Raji + ApoCell | 22 | 0 |
| C6 | | 22 | 0 |
| C7 | | 28 | 0 |

**Table 14: Bone Marrow**

| Bone marrow | | | |
|---|---|---|---|
| Mouse | Treatment | Day of sacrifice | CD20+ |
| A1 | | | 0 |
| A2 | RPMI | | 0 |
| A3 | | | 0 |

(continued)

| Bone marrow | | | |
|---|---|---|---|
| Mouse | Treatment | Day of sacrifice | CD20+ |
| B2 | Raji | 22 | 18.7 |
| B3 | | 22 | 11.3 |
| B5 | | 22 | 10.5 |
| B6 | | 22 | 14.5 |
| B2 (020) | | 25 | 21 |
| B3 (020) | | 25 | 0 |
| C1 | Raji + ApoCell | 28 | 1 |
| C2 | | 53 (healthy) | 0.5 |
| C4 | | 22 | 0.6 |
| C6 | | 22 | 17.5 |
| C7 | | 28 | 1.4 |

**Table 15: Liver**

| Liver | | | |
|---|---|---|---|
| Mouse | Treatment | Day of sacrifice | CD20+ |
| A1 | RPMI | | 0 |
| A2 | | | 0 |
| A3 | | | 0 |
| B2 | Raji | 22 | 4.4 |
| B3 | | 22 | 6.5 |
| B5 | | 22 | 1.7 |
| B6 | | 22 | 5.4 |
| B2 (020) | | 25 | 26.4 |
| B3 (020) | | 25 | 5.9 |
| C1 | Raji + Apo Cell | 28 | 9.8 |
| C2 | | 53 (healthy) | 0.5 |
| C4 | | 22 | 3 |
| C6 | | 22 | 5 |
| C7 | | 28 | 22.7 |

*In vivo experiment 023*

Expression (%) of CD20 tumor cells in the spleen, bone marrow, and liver, as determine by flow cytometry. Mouse spleen, liver, and bone marrow were collected from sacrificed mice (following clinical deterioration, as defined in methods) and analyzed by flow cytometry (FACSCalibur, BD) for the presence of **human CD20** (FITC). The results for Spleen, bone marrow, and liver for the individual mice are presented in Tables 16-18 below.

**Table 16: Spleen**

| Spleen | | | |
|---|---|---|---|
| Treatment | Mouse | Day of sacrifice | CD20+ |
| Raji | B1 | 22 | 0 |
| | B2 | 25 | 0 |
| | B3 | 22 | 0 |
| | B4 | 22 | 0.2 |
| Raji + ApoCell | C1 | 22 | 0.2 |
| | C4 | 22 | 0.3 |
| | C5 | 41 | 0.1 |
| | C6 | 47 | 0 |
| Raji + RtX 2mg/kg | E1 | 32 | 0 |
| | E2 | 32 | 0.7 |
| | E6 | 32 | 0 |
| Raji + RtX 2mg/kg + ApoCell | F1 | 40 | 0 |
| | F2 | 43 | 0.1 |
| | F4 | 35 | 0 |
| | F5 | 32 | 0 |
| | F6 | 35 | 0 |
| | F7 | 57 | 0.4 |
| Raji + RtX 5mg/kg | G1 | 34 | 0 |
| | G3 | 34 | 0.1 |
| | G4 | 43 | 0 |
| | G5 | 40 | 0 |
| | G7 | 40 | 0.1 |
| Raji + RtX 5mg/kg + ApoCell | H1 | 40 | 0.4 |
| | H3 | 36 | |
| | H4 | 36 | |
| | H5 | 40 | 0 |
| | H6 | 40 | 0 |
| | H7 | 53 | 0 |

**Table 17: Bone Marrow**

| Bone marrow | | | |
|---|---|---|---|
| Treatment | Mouse | Day of sacrifice | CD20+ |
| Raji | B1 | 22 | 18 |
| | B2 | 25 | 22.5 |
| | B3 | 22 | 33.6 |
| | B4 | 22 | 14.9 |

(continued)

| Bone marrow | | | |
|---|---|---|---|
| Treatment | Mouse | Day of sacrifice | CD20+ |
| Raji + ApoCell | C1 | 22 | 24.3 |
| | C4 | 22 | 1.8 |
| | C5 | 41 | 7.8 |
| | C6 | 47 | 0 |
| Raji + RtX 2mg/kg | E1 | 32 | 0.8 |
| | E2 | 32 | 3.1 |
| | E6 | 32 | 3 |
| Raji + RtX 2mg/kg + ApoCell | F1 | 40 | 4.1 |
| | F2 | 43 | 0.4 |
| | F4 | 35 | 0.2 |
| | F5 | 32 | 0.8 |
| | F6 | 35 | 0.2 |
| | F7 | 57 | 0.4 |
| Raji + RtX 5mg/kg | G1 | 34 | 2.1 |
| | G3 | 34 | 0.5 |
| | G4 | 43 | 4.3 |
| | G5 | 40 | 2.2 |
| | G7 | 40 | 2.6 |
| Raji + RtX 5mg/kg + ApoCell | H1 | 40 | 0.9 |
| | H3 | 36 | 0 |
| | H4 | 36 | 0 |
| | H5 | 40 | 1.6 |
| | H6 | 40 | 1.3 |
| | H7 | 53 | 1.8 |

**Table 18: Liver**

| Liver | | | |
|---|---|---|---|
| Treatment | Mouse | Day of sacrifice | CD20+ |
| Raji | B1 | 22 | 6.4 |
| | B2 | 25 | 42.6 |
| | B3 | 22 | 5 |
| | B4 | 22 | 8.1 |
| Raji + ApoCell | C1 | 22 | 2.5 |
| | C4 | 22 | 2.2 |
| | C5 | 41 | 0.4 |
| | C6 | 47 | 0 |

(continued)

| Liver | | | |
| --- | --- | --- | --- |
| Treatment | Mouse | Day of sacrifice | CD20+ |
| Raji + RtX 2mg/kg | E1 | 32 | 2.1 |
| | E2 | 32 | 1.2 |
| | E6 | 32 | 0.4 |
| Raji + RtX 2mg/kg + ApoCell | F1 | 40 | 0 |
| | F2 | 43 | |
| | F4 | 35 | 0 |
| | F5 | 32 | 7.3 |
| | F6 | 35 | 5 |
| | F7 | 57 | 0 |
| Raji + RtX 5mg/kg | G1 | 34 | 1 |
| | G3 | 34 | 7.3 |
| | G4 | 43 | 1.4 |
| | G5 | 40 | 0.9 |
| | G7 | 40 | 5.7 |
| Raji + RtX 5mg/kg + ApoCell | H1 | 40 | 0.8 |
| | H3 | 36 | 0.1 |
| | H4 | 36 | 0 |
| | H5 | 40 | 0.5 |
| | H6 | 40 | 25.1 |
| | H7 | 53 | 3.4 |

*Preliminary Conclusions:* In conclusion, tumor distribution in the mouse organs correlated the beneficial effect seen in survival plots and was significantly reduced in bone-marrow and liver in treated mice.

### Part B: Synergistic effect of ApoCell and Rituximab (RtX) in the treatment of leukemia/lymphoma

Next, it was examined whether the ApoCell treatment was synergistic with other conventional treatments of leukemia/lymphoma by evaluating the combined effect of RtX and ApoCell on leukemic mice in two experiments.

*Objectives:* Measurement of the survival of leukemic mice following RtX and ApoCell administration, and detect tumor cells in bone marrow, liver, and spleen in leukemic mice.

*Methods:* The following work is a representative description of the results obtained in the combination therapy experiments (ApoCell and rtx). Briefly, female SCID-Beige mice were injected intravenously with $0.1 \times 10^6$ Raji cells (n = 7 in all groups). Mice received three doses of $30 \times 10^6$ ApoCell intravenously on days 5, 8, and 12. On day 8, 1.5 h after ApoCell injection, the mice received a single IV dose of 2 or 5mg/kg RtX. Mice were followed daily and weighted twice a week. The endpoint was defined as death, or sacrifice due to the development of one or more of the following symptoms: paraplegia (lower body paralysis), loss of 20% from starting weight, lethargy, reduced mobility, or increased respiratory effort.

### Results:

As shown in **Figure 26,** ApoCell had a beneficial effect corroborating the results presented in **Figure 24.** Rituxan (RtX) alone had a superior effect to ApoCell both in 2 mg and 5 mg dosage but the combination of ApoCell and Rituxan had a synergistic effect at both in 2 mg (p=0.104) and in 5 mg dosage, although the synergistic effect seen in 5 mg did not reach statistical significance. Tumor distribution in the mouse organs correlated the beneficial effect (Table 19).

**Table 19:** Statistical analysis of the survival distributions (Log-rank (Mantel-Cox) test)

| Compared groups | | Statistical test (P value) |
|---|---|---|
| Group 1 | Group 2 | Log-rank (Mantel-Cox) Test |
| Raji | Raji + rtx (2mg/Kg) | 0.0002 |
| Raji | Raji + rtx (2mg/Kg) + ApoCell | 0.0002 |
| Raji | Raji + rtx (5mg/Kg) | 0.0002 |
| Raji | Raji + rtx (5mg/Kg) + ApoCell | 0.0002 |
| Raji + Rituxan (2mg/Kg) | Raji + Rituxan (2mg/Kg) + ApoCell | 0.0104 |

*End of experiment - Day 57*

One mouse (Raji + RtX 2mg/kg + ApoCell) was declared disease free upon termination of the experiment.

The synergistic effect in one 2 mg RtX dose was measured in an additional experiment. As was clearly shown in this experiment **(Figure 27),** the synergistic effect of ApoCell and RtX was again verified as significant (p=0.01).

As shown in Table 20, the spleen was not populated by tumor cells (0.1-0.3 represents background staining) and was used as a control. In contrast, bone marrow and liver were tumor targets. There was a reduced tumor population (Rajji-cells, as measured using CD20 marker) in bone marrow and liver following treatment by ApoCell and RtX separately, and the benefit increased when the two were given in combination; p= 0.0034 (**) for Raji + rtx (2mg/Kg) + ApoCell, and 0.0031 (**) for Raji + rtx (5mg/Kg) + ApoCell (T-test. As expected, RtX significantly reduces tumor burden in the target organs of the leukemic mice. Interestingly, treatment with ApoCell alone reduced tumor cells in those organs to levels comparable to treatment with conventional RtX therapy.

**Table 20:** Average tumor cell population in the spleen, bone-marrow, and liver (flow cytometry)

| Tissue | Treatment | # of mice | Statistical test (P value) | |
|---|---|---|---|---|
| | | | CD20+ | T-Test |
| **Spleen** | Raji | 7 | 0 | |
| | Raji + ApoCell | 4 | **0.3** ±0.4 | |
| | Raji + rtx (2mg/Kg) | 3 | **0.2** ±0.4 | |
| | Raji + rtx (2mg/Kg) + ApoCell | 6 | **0.1** ±0.1 | |
| | Raji + rtx (5 mg/Kg) | 7 | 0 | |
| | Raji + rtx (5 mg/Kg) + ApoCell | 4 | **0.1** ±0.2 | |
| **Bone Marrow** | Raji | 6 | **18.3** ±11 | |
| | Raji + ApoCell | 4 | **8.5** ±11 | |
| | Raji + rtx (2mg/Kg) | 3 | **2.3** ±1.3 | |
| | Raji + rtx (2mg/Kg) + ApoCell | 6 | **1** ±1.5 | 0.0034 (**) |
| | Raji + rtx (5 mg/Kg) | 7 | **1.7** ±1.5 | |
| | Raji + rtx (5 mg/Kg) + ApoCell | 6 | **0.9** ±0.8 | **0.0031 (**)** |
| **Liver** | Raji | 6 | **15.7** ±15.4 | |
| | Raji + ApoCell | 4 | **1.3** ±1.3 | |
| | Raji + rtx (2mg/Kg) | 3 | **1.2** ±0.8 | |
| | Raji + rtx (2mg/Kg) + ApoCell | 5 | **2.5** ±3.5 | |
| | Raji + rtx (5 mg/Kg) | 7 | **3** ±2.5 | |
| | Raji + rtx (5 mg/Kg) + ApoCell | 7 | **4.4** ±9.2 | |

*Conclusion:* In summary, the survival plots **(Figure 24, Figure 26, and Figure 27)** clearly demonstrate the beneficial effects of ApoCell and Rtx along as well as the synergistic effect of ApoCell and RtX in combination. Of note, when the conventional RtX treatment was combined with ApoCell, survival times increased significantly, regardless of the RtX dose, indicating a potent synergistic effect of the two therapies. A supporting clinical observation was the decrease in the of tumor cell population in the bone marrow and liver (Table 12).

Surprisingly, ApoCell preparation had a remarkably beneficial effect on disease progression and survival in a leukemic mouse model, independent of any other treatment. 10-20% of mice had prolonged survival in Kaplan-Meier analysis **(Figure 24, Figure 26, and Figure 27),** and the tumor cell burden was reduced in the liver and bone marrow. Furthermore, there was a marked synergistic effect when ApoCell and RtX were administered in combination, further delaying disease onset and progression, and improving survival.

## REFERENCE EXAMPLE 12: Use of Pooled Apoptotic Cell Preparation in GVHD Leukemia/Lymphoma Models

In the following preliminary work, the effect of the same infusion in GvHD leukemia/lymphoma models was examined. The safety and efficacy of an irradiated multiple donor single apoptotic cell infusion (a pooled mononuclear irradiated apoptotic cell preparation) for the prevention of acute GvHD in mice undergoing bone marrow transplantation (BMT) was examined. In this model, BMT rescued irradiated mice (80-100%).

The question regarding the possible loss of graft versus leukemia (GvL) effect arises in every successful treatment that potentially avoids high grade aGVHD, since this effect was found to correlate with the severity of GVHD.

*Methods*

Apoptotic cells were prepared as per Example 1 above, except that in the current experiments, preparation was done simultaneously from 4 donors. Following preparation from 4 donors, the cell preparations were combined at the last step (prior to irradiation), irradiated immediately after, and injected immediately after irradiation. Irradiation was at 25 Gy.

*Results*

The two graphs presented in **Figures 28 and 29,** show the clear effect (p<0.01) of a single injection of apoptotic cell from multiple individual donors (dotted line), both on survival and weight loss. **Figure 28 is** a Kaplan-Meier survival curve in a GvHD mouse model that was treated with a single dose irradiated apoptotic cells from multiple individual donors where survival was significantly ameliorated. **Figure 29** is percentage of weight loss of the 2 compared groups that follow and correlate with the findings of **Figure 28.**

In summary, the single infusion of multiple-donor irradiated apoptotic cells successfully and significantly improved life expectancy in a mouse model of GvHD.

## EXAMPLE 13: Stability Criteria for Apoptotic Cells from Multiple Individual Donors

The objective of this study is to develop stability criteria for apoptotic cells from multiple individual donors with comparability studies to non-irradiated HLA-matched apoptotic cells (Mevorach et al. (2014) Biology of Blood and Marrow Transplantation 20(1): 58-65; Mevorach et al. (2015) Biology of Blood and Marrow Transplantation 21(2): S339-S340).

Apoptotic cell final product preparations will be evaluated for cell number, viability, apoptotic phenotype and potency after storage at 2 to 8°C for 8, 24, 48, and 60 hours with sampling at each time point. Apoptotic cell final product lots will be prepared following standard operating procedures (SOPs) (Example 1; Reference Example 5) and batch records (BRs; i.e., specific manufacturing procedures). For potency evaluation, samples of early apoptotic cell preparation final product lots will be tested for inhibition of lipopolysaccharide (LPS) induced upregulation of MHC-II expression on immature dendritic cells (time points 0-24h) or monocytes (time points 0-6) and will be performed according to SOPs and recorded on BR. These series of test will be performed on pooled and non-pooled products that are in preparations originating from multiple individual donors and from single donors, respectively.

In addition, flow cytometric analysis of CD3 (T cells), CD19 (B cells), CD14 (monocytes), CD15$^{high}$ (granulocytes) and CD56 (NK cells) will be documented. The aims of these studies are to demonstrate consistency with a narrow range of results. Preliminary results are consistent with these goals and no deviations from the SOP are noted and no technical problems are reported. However, further studies are needed in order to conclude the range and stability of effective treatment. Preliminary results show equivalence in all these parameters. Further, single donor stability studies showed stability at least through a 48 hour period (See, Example 1).

## REFERENCE EXAMPLE 14: Safety & Efficacy Of Multiple Donor Irradiated Apoptotic Cells As Prophylaxis For Acute Graft- Versus-Host Disease

**Objective:** A phase 1/2a, multicenter, open-label study evaluating the safety, tolerability and preliminary efficacy of a single dose administration of irradiated apoptotic cells, from multi-, unmatched-donors, for the prevention of graft versus host disease in hematopoietic malignancies in human leukocyte antigen-matched, related and unrelated patients undergoing allogeneic hla-matched hematopoietic stem cell transplantation

*Primary Objective:* To determine safety and tolerability of multiple donor irradiated apoptotic cell treatment.

*Secondary Objective:* To determine efficacy of irradiated apoptotic cells from multiple individual donors as prophylaxis measure for acute GVHD (aGVHD) in patients with hematopoietic malignancies scheduled to undergo hematopoietic stem cell transplantation (HSCT). For the purposes of this study, HSCT can be either bone marrow transplant (BMT) or peripheral blood stem cell transplantation (PBSCT).

**Therapeutic Indication:** Graft vs. Host Disease (GVHD) post-transplantation in hematopoietic malignancies in human leukocyte antigen (HLA)-matched, related and unrelated patients.

**Study Design:** This is an open labeled study, multi-center, phase-1/2a study in patients diagnosed with hematopoietic malignancies scheduled to undergo HSCT (either bone marrow transplantation or peripheral blood stem cell transplantation) from an HLA-matched related or unrelated donor, following either full myeloablative or reduced intensity myeloablative conditioning regimens.

After a signing of informed consent by recipient patient, donors screening period and cell collection before initiating conditioning regimen, eligible recipient patients will be assigned (stratified by prophylactic treatment and related versus non-related transplant donors in 1:1 ratio to receive intravenous (IV) injection 12-36 hours prior to HSCT transplantation to) either:

*Investigational Arm*: single dose of $140 \times 10^6 \pm 20\%$ cell/kg from multiple individual donors of irradiated early apoptotic cells/kg body weight in phosphate buffer solution (PBS).

All patients will also be treated with the institutional standard of care (SOC) immunosuppressive regimen: cyclosporine/methotrexate or tacrolimus/methotrexate for full myeloablation and mycofenolate/cyclosporine or mycophenolate/tacrolinus for reduced intensity. Patients will be hospitalized as medically indicated.

Patients will be followed up for 180 days for the secondary efficacy endpoint and for 1 year for the primary safety and tertiary efficacy endpoints. Number of visits for patients participating in this study will be comparable to those customary for patients in their condition. For donor, study specific visit will be for apheresis procedure during the screening period.

As these patients have many underlying medical conditions and may experience symptoms compatible with aGVHD, it may be difficult to absolutely determine if toxicity is related to apoptotic cells or not although basic data exist from a former phase 1-2a study using apoptotic cells for GvHD prophylaxis (Mevorach et al. (2014) Biology of Blood and Marrow Transplantation 20(1): 58-65) Single Infusion of Donor Mononuclear Early Apoptotic Cells as Prophylaxis for Graft-versus-Host Disease in Myeloablative HLA-Matched Allogeneic Bone Marrow Transplantation: A Phase I/IIa Clinical Trial. BBMT 20(1)58-65).

Data Safety Monitoring Board (DSMB) will meet as specified in the DSMB charter, including at the time of the scheduled interim analysis (180 days) assuming no safety concerns were raised beforehand.

**Study Procedures:**

The study will comprise of screening, treatment and follow-up periods.

*1. Screening Period (Day -60 to Day -2)*

Potential recipient patients will sign informed consent prior to conduct of any study related procedures. The standard assessments before approval, will be performed by the transplantation center for the donor during the screening period and usually include: demographic data, medical history, HLA match status verification (no matching is needed), physical examination, height and weight, vital signs, pregnancy test (all women), hematology, blood chemistry, infectious disease screen, ECG and urinalysis.

The recipients (study patients) will undergo the following assessments during the screening period: demographic data, medical history, Karnofsky performance status, HLA match verification, physical exam, height and weight, vital signs, pregnancy test (all women), ECG, pulmonary function test, hematology, blood chemistry, coagulation markers, infectious disease screen, and urinalysis.

After the initial screening evaluations, if recipient is eligible to participate in the study, the recipient patient will be assigned on the first day of the conditioning regimen to receive single IV infusion of $140 \times 10^6 \pm 20\%$ cell/kg of multiple donor apoptotic cells. The conditioning regimen to be completed on the day before or day of Apoptotic Cell infusion scheduled for Study Day -1.

Apoptotic cell dosage will be calculated for each recipient patient and presumed apheresis collection number and number of donors will be decided accordingly.

*For peripheral stem cell transplant donors:* Between Days -6 to -1, the donor will receive one or more once daily injections of G-CSF to mobilize progenitor cells and on Day 0 will undergo apheresis to produce donor hematopoietic blood stem cells for transplantation. Preparation of the hematopoietic blood stem cells for bone marrow transplantation will be performed in accordance with the center's standard practice by trained hospital staff. The hematopoietic blood stem cells for HSCT will not be manipulated or T cell-depleted prior to administration.

*For bone marrow transplant donors:* Bone marrow will harvested and prepared per center standard practice and will not be otherwise manipulated.

*2. Treatment Day (Day -1)*

On Day -1 (12-36 hours prior to HSCT), eligible patients will receive single IV infusion of either 140x10$^6$ $\pm$20% cell/kg of multiple individual donors irradiated Early apoptotic Cellsl. Vital signs will be monitored every hour during infusion and every 4 hours for the first 24 hours afterwards. Treatment-related AEs will be assessed immediately following infusion.

On Day 0, patients will undergo hematopoietic stem cell transplantation according to local institution guidelines.

*3. Short-term Follow-up Period (Day 0 to Day 180)*

Patients will be followed-up to Study Day 180 for assessment of the primary endpoint safety and tolerability and secondary and tertiary endpoints: cumulative incidence of aGVHD grade II-IV ("modified Glucksberg" consensus based on Przepiorka et al cumulative incidence of any grade and high grade aGVHD, i.e., time to development of aGVHD, grades II-IV; any systemic treatment of GVHD, and the development of cGVHD.

The short term follow up visits will be daily while hospitalized for the transplantation (usually at least Days -1 to +14 or more) and weekly visits during the first 7 weeks after discharge; days +7, +14, +21. +28, +35, +42, and then on Days 60, 100, 140, and 180. The visit window will be $\pm$5 days for each weekly visit (first 7 weeks) and $\pm$5 days for biweekly or more visits during the subsequent follow up period up to 180 days.

Blood samples will be obtained on days 1, 3, 7, +7, +28, +42, 60, 100, 140 and 180 and examined for documentation of engraftment, immunological recovery, plasma and serum biomarkers ("Michigan") and cell subpopulations.

*4. Long-term Follow up Period (Day 181 to Day 365/1 Year )*

Patients will be followed for one year post-HSCT for the longer term secondary endpoints: non-relapse mortality and overall survival (OS), relapse incidence, leukemia free survival (LFS) and chronic GVHD. There will be at least two long-term follow-up visits, the last one being, 12$\pm$1 months following the HSCT.

**Study Duration:** For each participating patient, the duration in the study will be up to 14 months as follows:

| | |
|---|---|
| Screening | Up to 60 days (2 months |
| Treatment | 1 day |
| Follow-up | 365 days (12 months) consisting of |
| Short-term: | 180 days |
| Long-term | +180 days |

**Study Population:** A total of 25 patients diagnosed with hematologic malignancies scheduled to undergo HSCT (either bone marrow transplantation or peripheral blood stem cell transplantation) ,with at least 15 unrelated donors , following either myeloablative or reduced intensity conditioning regimens, per center standard practice will be included in this study and will be compared to historical controls.

**Inclusion/Exclusion Criteria:**

*Recipient Patient Exclusion Criteria*

1. Patients, Age > 18, who are eligible for allogeneic HSCT for the following malignancies:

Acute myeloid or undifferentiated or biphenotypic, leukemia, in complete remission (any remission) or beyond but with <5% blasts by morphology in bone marrow.
Acute myeloid leukemia (AML) in complete remission if it has evolved from myelodysplastic syndrome (MDS) (there should be documented diagnosis of MDS at least 3 months prior to diagnosis of acute myeloid leukemia). Or evolved from polycythemia vera or essential thrombocytosis.

Acute lymphoblastic leukemia (ALL) in complete remission (any remission) with <5% blasts by morphology in bone marrow.

Chronic myeloid leukemia (CML) in chronic or accelerated phase

Myelodysplastic syndromes - refractory cytopenia with multilineage dysplasia (RCMD), RA (refractory anemia), RA with ringed sideroblast (RARS; all < 5% blasts), RA with excess blasts (RAEB; 5 to 20% blasts).

The transplant donor and recipient patient must have at least an 8/8 HLA match at the HLA A, B, C, DQ, and DR loci and no antigen or allele mismatch. However the donor(s) of leukocytes for apoptotic cell formation is not restricted to HLA matching.

Performance status score of at least 70% at time of the screening visit (Karnofsky for adults and Lansky for recipient < 16 years old.

Cardiac left ventricular ejection fraction $\geq$ 40% in adults within 4 weeks of initiation of conditioning; MUGA scan or cardiac ECHO required if prior anthracycline exposure or history of cardiac disease.

Pulmonary function test with DLCO[1], FEV1 (forced expiratory volume) and FVC (forced vital capacity) of $\geq$60% predicted.

Oxygen saturation of at least 90% on room air.

Patients must have adequate organ function as defined below:

AST (SGOT)/ALT (SGPT) <3 x upper limit of normal (ULN).
Serum creatinine <2.0 mg/dL (adults, >16 y) or <O.8 (1-2 y), < 1(3-4 y), <1.2 (5-9 y), <1.6 (10-13 y), and 1.8 (14-15 y).
Serum bilirubin <3 mg/dL unless due to Gilbert's disease or hemolysis.

Signed written informed consent to participate in the study independently by patient, or guardian in the case of minors.

Ability to comply with the requirements of the study.

For duration of 4 weeks (from day -1), both female and male must agree to: Use an acceptable method of birth control or be surgically sterile for the first month or more if there are BMT related restrictions.

To have a negative pregnancy test regardless of child-bearing potential.

**Recipient Patient Exclusion Criteria**

All diseases eligible for HSCT not specified in the Inclusion Criteria.

Participation in an interventional investigational trial within 30 days of the screening visit.

Have progressive or poorly controlled malignancies.

If BMT plan include T-cell depleted allograft

If BMT plan include anti-thymocyte globulin (ATG) or alemtuzumab as part of immunosuppressive regimen or high dose Cyclophosphamide therapy for the prevention of GVHD after transplantation

Uncontrolled infections including sepsis, pneumonia with hypoxemia, persistent [1] Diffusing capacity of the lung for carbon monoxide bacteremia, or meningitis within two weeks of the screening visit.

Current known active acute or chronic infection with HBV or HCV.

Known human immunodeficiency virus (HIV) infection.

Patients with severe or symptomatic restrictive or obstructive lung disease or respiratory failure requiring ventilator support.

Patients with other concurrent severe and/or uncontrolled medical condition which could compromise participation in the study (i.e. active infection, uncontrolled diabetes, uncontrolled hypertension, congestive cardiac failure, unstable angina, ventricular arrhythmias, active ischemic heart disease, myocardial infarction within six months, chronic liver or renal disease, active upper gastrointestinal tract ulceration).

Any chronic or acute condition susceptible of interfering with the evaluation of investigational product effect.

Any form of substance abuse (including drug or alcohol abuse), psychiatric disorder or any chronic condition susceptible, in the opinion of the investigator, of interfering with the conduct of the study.

Organ allograft or previous history of stem cell transplantation (allogeneic only).

Breast feeding in women of childbearing potential.

Patients who are likely to be non-compliant or uncooperative during the study.

**Investigational Product Route and Dosage Form**

Apoptotic cells will be administered as an IV infusion of 140x10$^6$ $\pm$ 20% cell/kg of irradiated multiple donor apoptotic cell product 12-36 hours prior to HSCT.

Apoptotic cells are a cell-based therapeutic composed of multiple individual donors apoptotic cells. The product contains allogeneic donor mononuclear enriched cells in the form of liquid suspension with at least 40% early apoptotic cells. The suspension is prepared from multiple individual donors with PBS solution in accordance with GMP regulations and should be stored at 2-8°C until infusion. The final product will be in a total volume of 300-600 mL in an opaque transfer pack and will be irradiated with 25 Gy following preparation. Investigational product should be administered to the patient within 48 hours of completing the manufacturing process.

**Safety Outcomes/Efficacy Endpoints/Outcome Measures**

*Primary:*

Safety and tolerability endpoints include time to engraftment and a physical examination to determine adverse events, concomitant medications and safety laboratories on Day 180 and Day 360 (1 year). Further, it is expected that irradiated pooled apoptotic cell preparations will show a lack of *in vitro* and *in vivo* cell proliferation and lack of *in vivo* activation. Such a showing identifies the pooled apoptotic cell preparation as safe for use.

*Secondary:*

Cumulative incidence of aGVHD grade II-IV using "modified Glucksberg" consensus based on (Rowlings et al. 1997) IBMTR Severity Index for grading acute graft-versus-host disease: retrospective comparison with Glucksberg grade. Br J Haematol. 1997 Jun;97(4):855-64) on Day 180

1-year non-relapse mortality and overall survival (OS)
1-year relapse incidence
1-year leukaemia free survival (LFS)
Maximum grade of aGVHD within the first 180 days
Cumulative incidence of grade III-IV aGVHD
Incidence of chronic GVHD according to (Jagasia et al., 2015) on Days 180 and 360 (1 year). Any "systemic treatment" including corticosteroids (both used or not and cumulative dosage) for the treatment of aGvHD on Day 20 through Day 180
Immune reconstitution and function on Days +28, 100, 180 and 360 (1 year) in relation to T, B, NK, and Monocytes
Major infection rate (including lung infiltrates, CMV reactivation and any other infections that require hospitalization) through Day 180 and 1 year.

*Tertiary/Exploratory:*

Percent of hospitalization days to total days at risk, or total days alive and out of the hospital. Or total hospitalization days till first discharge post transplantation.

Organ specific GVHD
T regs, CD4 Tcon, CD8, NK and B cells levels on Day 180

**Statistical Analysis:**

Study outcome will be compared to historical control with individuals with comparable baseline characteristics.

Descriptive statistics will be used to summarize outcome measures and baseline characteristics. In this analysis all available data will be presented with no imputation for any missing data. Subjects will contribute the data available up to the point of withdrawal or study completion or death. The descriptive statistics such as means, median, standard deviation, minimum and maximum will be used to summarize continuous variables. All subjects who receive the apoptotic cells infusion will be included in the safety analysis. Subjects who also receive the HSCT will be included in the efficacy analysis. As this study is exploratory in nature, ad hoc analyses are planned.

*Sample size consideration*

A total of 25 patients will be included at least 15 matched unrelated patients will be enrolled. Apoptotic cells (active will be given to all, stratifying on GVHD prophylaxis regimen, and related versus unrelated transplant donor.

*Population Analysis definition*

All efficacy analyses will be conducted on the Intent-to-Treat (ITT) population and compared to adequate historical control. The safety population will be defined as all patients who receive a dose of study medication.

*Statistical methods*

Patient, disease, and transplant characteristics will be described using frequencies and percentages or median (range) as appropriate.

**Safety analysis**

Descriptive statistics will be used to summarize safety outcomes with focus on the AEs reported between study treatment infusion and HSCT procedure (24-30 hour window). No alterations in the conduct of the study will be initiated as a consequence of the DSMB review, including sample size adjustment. As such, no penalty adjustment in the overall Type I error as a consequence of the interim analysis will be required.

*Secondary Endpoint Analysis*

Grade II-IV aGVHD will be described using the cumulative incidence estimator with death prior to aGVHD as a competing event.
Neutrophil and platelet recovery, Grade III-IV aGVHD, chronic GVHD, infection, relapse, and transplant related mortality will be described using cumulative incidence with relapse as competing event for TRM and death as the competing event for all others.. Overall survival and leukemia free survival will be described using the Kaplan-Meier estimator, and. The maximum grade of aGVHD within the first 180 days and the need for steroids at 180 days will be described using frequencies and percentages using the Mann-Whitney U-test and chi-square test respectively. Immune recovery of each cell subset and TREGs will be described at each time point using median and range Mann-Whitney tests.

**REFERENCE EXAMPLE 15: Comparison of Pooled Apoptotic Cell Preparation vs. Single Donor Apoptotic Cell Preparation in GVHD Leukemia/Lymphoma Models**

*Objective:* Compare the beneficial clinical effect of humearly apoptotic cells obtained from a single donor on the severity of GvHD in a murine model of GvHD, to the clinical effect, if any, of humearly apoptotic cells obtained from multiple individual donors on the severity of GvHD in the murine model of GvHD, wherein the multiple individual donors represented HLA-unmatched heterologous donors.
Reference Example 12 above shows the beneficial effect of irradiated apoptotic cells pooled from multiple individual donors. The results shown in **Figure 28** and **Figure 29** were surprising as a skilled artisan may recognize that the multiple sources of unmatched cells may have increased the diversity of antigenicity of the cells, and thus would have expected a dramatic reduction in the clinical effect. Unexpectedly, the known, beneficial effect of early apoptotic cells on the reduction of GvHD severity, and therefore a prolongation of the number of days till mortality, was also alleviated by pooled unmatched early apoptotic cells **(Figure 28),** which would purportedly have increased antigenicity due to the pooled multiple unmatched source cells.
An additional objective was to understand if there is a difference between the use of irradiated early apoptotic cells and non-irradiated apoptotic cells.
A skilled artisan would appreciate that unmatched, irradiated cells keep their antigenic profile as recognized by the APC mechanism and so by T-Cells of the host into which they have been infused. Accordingly, concerns when pooling heterologous unmatched populations of cells included cross-reactivity between the individual populations being pooled, mixed-cell lymphatic reactions of pooled populations, or T-cell immune reactions between pooled populations that could reduce or eliminate cells, or any combination thereof.

*Methods*

*Mouse model:* Female 7-9 week-old BALB/c mice (H-2$^d$) were used as recipients and female 8-9 week-old C57BL/6 mice (H-2$^b$) were used as donors in mismatched GVHD model. Recipients were total body irradiated at 850 cGy 24 hours before bone marrow and splenocyte transplantation. Donor bone-marrow cells were used for bone-marrow reconstitution. Bone marrow cells were extracted from the femoral and tibial bones with RPMI 1640. Red blood cells were lysed, then cells were washed and resuspended with PBS. Viability was assessed using trypan blue dye exclusion (>90% viability). Donor splenocytes were used for the induction of GVHD. Spleens were removed and homogenized and single cell suspension was obtained. Red blood cells were lysed and splenocytes were resuspended with PBS. At least 90% viable cells were assessed using trypan blue dye.

*Early apoptotic cells:* Apoptotic cells were produced from mononuclear enriched cell fraction apheresis from healthy donors similar to Example 1. In brief:

Enriched fractions of mononuclear cells (MNCs) were obtained from healthy, eligible donors via leukapheresis procedure. Cells were collected via Spectra OPTIA® apheresis system from 12 liters of blood, in addition to 400-600ml of autologous plasma. The estimated yield of the enriched mononuclear cell fraction from a donor was expected to be approximately 1.2- 1.5 x 10$^{10}$ cells. Prior to leukapheresis procedure, donors are tested and confirmed negative to the below viral vectors:

1. Human Immunodeficiency virus (HIV), types 1 and 2;
2. Hepatitis B virus (HBV);
3. Hepatitis C virus (HCV);
4. Cytomegalovirus (CMV);
5. *Treponema pallidum* (syphilis);
6. Human T-lymphotropic virus (HTLV), types I and II

Following cell collection, the cells were washed with RPMI and frozen as follows. The freezing formulation was composed of PlasmaLyte A for injection pH 7.4, 10% DMSO, 5% Human Serum Albumin and 5% Anticoagulant Citrate Dextrose solution inoculated with 10U\ml heparin.

Freezing media was prepared in bags and the freezing procedure performed in a closed system under cGMP conditions.

Following leukapheresis procedure completion, enriched MNC fraction was washed with PlasmaLyte A and resuspended with ice-cold freezing media to a concentration of 50-65x10$^6$ cells\ml. Cells were then transferred to freezing bags, bags were transferred to pre-cooled aluminum cassettes and cassettes were transferred immediately to -18- (-25)$^0$C for two hours.

Following the two hours, cassettes were transferred to -80°C for an additional 2 hours and then to long-term storage in liquid nitrogen (> -135°C).

Autologous plasma was divided to 50gr aliquots. Plasma aliquots were transferred to - 80°C for 2 hours and then to a long-term storage in -18- (-25)$^0$C.

For apoptosis induction cells were thawed and washed with pre-warmed RPMI1640 containing 10mM Hepes buffer, 2mM L-Glutamine and 5% Anticoagulant Citrate Dextrose solution inoculated with 10U\ml heparin. After supernatant extraction cells were resuspended at final concentration of 5x10$^6$/ml in RPMI 1640 supplemented with 10mM Hepes, 2mM L-glutamine, with addition of 10% autologous plasma and. 50$\mu$g\ml Methylprednisolone and 5% Anticoagulant Citrate Dextrose solution inoculated with 10U\ml heparin. Cells are then transferred to cell culture bags, and incubated at humidified incubator 37°C, 5% CO$_2$ for 6 hours to stabilize apoptosis.

Following incubation cells were harvested, washed with PBS and resuspended in PBS.

Early apoptotic cell product was produced from one single donor or combined 10 different individual donors, in which case cells were combined just prior to irradiation. Since interference may occur between components in the multiple donor product, for example between living non-apoptotic cells, the early apoptotic cell product was subdivided and a sample of early apoptotic cells to be tested *in vivo* was irradiated with 2500 cGy prior to administration (sample F below), and stored at 2-8°C until administration. Table 3 of Reference Example 6 below presents details of the Annexin V positive/Propidium iodide negative ratio and cell surface markers of the early apoptotic cell product, establishing that consistency of apoptotic cells administered to mice is maintained. The final product was stable for 48 hours at 2-8°C.

On the day of transplantation, mice received 5x10$^6$ bone-marrow cells, 3x10$^6$ splenocytes and 30x10$^6$ single- or multiple-donor early apoptotic cell product, according to the following experimental design:

Irradiation control
Reconstitution control - irradiation + Bone-Marrow transplantation (BM)
GVHD control - irradiation + Bone-Marrow and splenocyte transplantation
Single donor, irradiated - irradiation + Bone-Marrow and splenocyte transplantation + irradiated early apoptotic cell product from single donor
Single donor, non-irradiated - irradiation + Bone-Marrow and splenocyte transplantation + non-irradiated early apoptotic cell product from single donor

Multiple donor, irradiated - irradiation + Bone-Marrow and splenocyte transplantation + irradiated early apoptotic cell product from multiple donor

Multiple donor, non-irradiated - irradiation + Bone-Marrow and splenocyte transplantation + non-irradiated early apoptotic cell product from multiple individual donors.

Monitoring - Transplanted mice were tagged and survival was monitored. Body weight was assessed every two days for the first two weeks of the experiment and then every day. Loss of 35% from initial body weight was determined as primary end point and mice were sacrificed and survival curve was updated accordingly. Body weight results were comparable to those observed in Reference Example 12 **Figure 29.**

The severity of GVHD was assessed using a known scoring system (Cooke KR, et al. An experimental model of idiopathic pneumonia syndrome after bone marrow transplantation. I. The roles of minor H antigens and endotoxin. Blood. 1996; 8:3230-3239) that incorporates five clinical parameters: weight loss, posture (hunching), activity, fur texture and skin integrity. Mice were evaluated and graded from 0 to 2 for each criterion. By summation of the five clinical scores a clinical index value was generated (index number increases with the severity of GVHD).

*Results*

Percent survival of the different population of mice is presented graphically in **Figure 30.** The irradiation only control mice died between day 8 and 12 (n=13), as expected from mice that did not receive bone marrow reconstitution. The majority of GVHD control mice (received bone-marrow and spleen) died between day 6 and 27. One mouse did not die (n=18). In bone-marrow reconstitution control group (BM) 3 out of 7 mice died between day 6 and 8. In the remaining mice, bone marrow was reconstituted by donor bone-marrow and mice remained alive (>50 days).

Single donor, non-irradiated mice died between day 15 and 36. Thus, as previously shown, single donor non-irradiated early apoptotic cells had a beneficial effect and survival was prolonged (p<0.01).

Single donor, irradiated mice died between day 7 and 35, one mouse remained disease free survival (>50 days). This demonstrated that single donor irradiated apoptotic cells also provided the beneficial effect with respect to GVHD. Thus, irradiation did not harm the immunomodulatory effect of early apoptotic cells. All had beneficial effect on survival in the GVHD murine model compared to GVHD control (p<0.01).

Non-irradiated multiple donor treatment did not provide a beneficial effect compared to GVHD control (n=11). Survival pattern was similar to GVHD control and mice died between day 6 and 28 (p=NS-not significant). Surprisingly and in contrast to the non-irradiated apoptotic cells, irradiated-multiple individual donor apoptotic cells (treatment F) (n=10) had a beneficial effect similar to single donor treatment, as compared with GVHD control. GVHD symptoms appeared significantly later and mice died between day 18 and 34 (p<0.01).

Irradiated-multiple individual donor (n=10), irradiated single donor (n=10) and non-irradiated single donor treatment (n=10) had similar survival patterns and no significant difference in effect on survival was observed between these three treatment groups.

The experiments indicated a clear effect of apoptotic cells infusion in GVHD induced mice. There was a significant prolonged survival effect for the treatments of irradiated multiple individual donors and irradiated- and non-irradiated single donor apoptotic cells.

Multiple donor treatment did not prolonged survival of mice when not irradiated but the irradiation of the apoptotic cell product prior to administration to mice improved results and treatment had close survival pattern as single- donor treatments.

As stated above, **Figure 30** shows, non-irradiated apoptotic cells obtained from multiple unmatched donors have significantly lower positive clinical effect on reduction in GvHD and mortality (% survival), as compared to (1) non-irradiated apoptotic cells obtained from single unmatched donors; (2) irradiated apoptotic cells obtained from single unmatched donors; and (3) irradiated apoptotic cells obtained from multiple unmatched donors. In addition, all three (non-irradiated early apoptotic cells, single donor; irradiated early apoptotic cells, single donor; and irradiated early apoptotic cells, multiple individual donors) have similar effects.

This data was surprising since the antigenicity of the non-irradiated apoptotic cells obtained from multiple individual donors was expected to be similar to that of irradiated apoptotic cells obtained from multiple individual donors, why would not both have similar hostile antigenic reaction with the implanted bone marrow, and why would both not be able to reduce GvHD and mortality rate?

If antigenicity is the main issue here, it was expected to see differences between the clinical effects of non-irradiated apoptotic cells obtained from single donor and irradiated apoptotic cells obtained from single donor. However the data does not show this difference.

One possibility is that the lack of efficacy of non-irradiated pooled apoptotic cell preparations prepared from multiple individual donors, resulted from cross-interaction between the individual mononuclear populations present in the pooled preparation. These interactions do not appear to be directly attributable to antigenicity towards the host, as irradiated cells maintain their antigenicity but the efficacy differed significantly from non-irradiated cells. Therefore, it appears that the cross-interaction in the pooled early apoptotic cell preparations receiving irradiation was unexpectedly eliminated and the host responded well to administration of the cells.

As shown, irradiated pooled donors had essentially the same effect as a single non irradiated donor.

## *EXAMPLE 16: EFFECT OF IRRADIATION ON FINAL APOPTOTIC CELL PRODUCT*

Apoptotic cells are increasingly used in novel therapeutic strategies because of their intrinsic immunomodulatory and anti-inflammatory properties. Early apoptotic cell preparations may contain as much as 20-40% viable cells (as measured by lack of PS exposure and no PI admission; Annexin V negative and Propidium iodide negative) of which some may be rendered apoptotic after use in a transfusion but some will remain viable. In the case of bone marrow transplantation from a matched donor, the viable cells do not represent a clinical issue as the recipient is already receiving many more viable cells in the actual transplant. However, in the case of a third party transfusion, (or fourth party or more as may be represented in a pooled mononuclear apoptotic cell preparation) use of an apoptotic cell population that includes viable cells may introduce a second GvHD inducer. Furthermore, the implication of irradiation on the immunomodulatory potential of early apoptotic cells has so far been not assessed. A skilled artisan may consider that additional irradiation of an early apoptotic cell population may lead cells to progress into later stages of apoptosis or necrosis. As this appears a particularly relevant question with regard to clinical applications, the experiments presented below were designed to address this issue, with at least one goal being to improve the biosafety of functional apoptotic cells.

Thus, the aim was to facilitate the clinical utilization of apoptotic cells for many indications wherein the potency of apoptotic cells may rely on a bystander effect rather than engraftment of the transplanted cells.

*Objective:* Examine the effect of irradiation on early apoptotic cells, wherein irradiation occurs following induction of apoptosis.

*Methods (in brief):* The cells were collected according and early apoptotic cells were prepared essentially as described in Reference Example 5.

Three separate early apoptotic cell batches were prepared on different dates (collections 404-1, 0044-1 and 0043-1).

Each final product was divided into three groups:

Untreated
2500rad
4000rad.

Following irradiation, early apoptotic cells were tested immediately ($t_0$) for cell count, AnnexinV positive-PI negative staining, cell surface markers (% population of different cell types) and potency (dendritic cells **(DCs))**. Following examination at $t_0$, early apoptotic cells were stored at 2-8°C for 24 hours, and examined the next day using the same test panel ($t_{24h}$) (cell count, Annexin V positive-PI negative staining, and cell surface markers and potency).

Previously, a post-release potency assay was developed, which assesses the ability of donor mononuclear early apoptotic cells (Early apoptotic Cellsl) to induce tolerance (Mevorach et al, BBMT 2014 ibid). The assay is based on using flow cytometric evaluation of MHC-class II molecules (HLA-DR) and costimulatory molecule (CD86) expression on iDC membranes after exposure to LPS. As previously and repeatedly shown, tolerogenic DCs can be generated upon interaction with apoptotic cells (Verbovetsky et al., J Exp Med 2002, Krispin et al., Blood 2006), and inhibition of maturation of LPS-treated DCs (inhibition of DR and CD86 expression), occurs in a dose dependent manner.

During phase 1/2a of the early apoptotic cell clinical study, the post-release potency assay was conducted for each early apoptotic cell batch (overall results n=13) in order to evaluate the ability of each batch to induce tolerance (Results are shown in Figure 1, Mevorach et al. (2014) Biology of Blood and Marrow Transplantation 20(1): 58-65).

DCs were generated for each early apoptotic cell batch from fresh buffy coat, collected from an unknown and unrelated healthy donor, and were combined with early apoptotic cells at different ratios (1:2, 1:4 and 1:8 DC:Early apoptotic Cells, respectively). After incubation with early apoptotic cells and exposure to LPS, potency was determined based on downregulation of DC membrane expression of either HLA DR or CD86 at one or more ratios of DC: early apoptotic cells. In all 13 assays, early apoptotic cells demonstrated a tolerogenic effect, which was seen with preparations at most DC: early apoptotic cells ratios, and for both markers, in a dose dependent manner.

Monocyte obtained immature DCs (iDCs) were generated from peripheral blood PBMCs of healthy donors and cultured in the presence of 1% autologous plasma, G-CSF and IL-4. iDCs were then pre-incubated for 2 hours at 1;2, 1;4 and 1;8 ratios with apoptotic cells either freshly prepared final product or final product stored at 2-8°C for 24 hours. The two final products were examined simultaneously in order to determine whether storage affects potency ability of apoptotic cells. Following incubation, LPS was added to designated wells were left for additional 24 hours. At the end of incubation, iDCS were collected, washed and stained with both DC-sign and HLA-DR or CD86 in order to determine changes in expression. Cells were analyzed using flow cytometer and analysis performed using FCS-express software from DC-sign positive cells gate to assure analysis on DCs only.

**Figures 31A and 31B** and **Figures 32A and 32B** show potency test of irradiated pooled apoptotic cells compared to non-irradiated single donor cell.

*Results:*

*Single Donor preparations*

Table 21 presents the comparative results of non-radiated and irradiated apoptotic cells; Average cell loss (%) at 24 hours; Annexin positive([+]) Propidium Iodide (PI) negative([-]) % at 0 hours and 24 hrs (% of early apoptotic cells; Annexin positive ([+]) Propidium Iodide (PI) positive ([+]) % at 0 hours and 24 hrs (% of late apoptotic cells); presence of cell surface antigens CD3 (T cells), CD19 (B cells), CD56 (NK cells), CD14 (monocytes), and CD15$^{high}$ (granulocyte), at 0 hours and 24 hours.

**Table 21:**

| Final product description | Apoptotic Cell | Apoptotic Cell 2500rad | Apoptotic Cell 4000rad |
|---|---|---|---|
| **An$^+$PI$^-$** $t_0$ (%)<br>Range (min-max) | 59.2<br>(52.6- 66.1) | 59.6<br>(51.6- 68.7) | 58.4<br>(50.4- 65.1) |
| **An$^+$PI$^-$** $t_{24h}$ (%)<br>Range (min-max) | 62.6<br>(53.6- 76.3) | 68.1<br>(52.0- 81.3) | 66.7<br>(52.9- 77.1) |
| **An$^+$PI$^+$** $t_0$ (%)<br>Range (min-max) | 4.9<br>(3.2- 7.0) | 6.0<br>(5.2- 7.4) | 6.1<br>(4.0-9.1) |
| **An$^+$PI$^+$** $t_{24h}$ (%)<br>Range (min-max) | 7.3<br>(5.0- 11.8) | 8.6<br>(6.4- 11.8) | 9.0<br>(6.0- 14.9) |
| **CD3+** $t_0$ (%)<br>Range (min-max) | 56.9<br>(47.4- 66.3) | 58.3<br>(48.8- 67.7) | 57.5<br>(48.6- 66.4) |
| **CD3+** $t_{24h}$ (%)<br>Range (min-max) | 56.8<br>(49.6- 64.0) | 57.1<br>(48.0- 66.1) | 56.6<br>(49.7- 63.4) |
| **CD19+** $t_0$ (%)<br>Range (min-max) | 10.6<br>(10.1- 11.0) | 9.5<br>(7.7- 11.3) | 9.6<br>(8.5- 10.7) |
| **CD19+** $t_{24h}$ (%)<br>Range (min-max) | 11.8<br>(10.2- 13.4) | 9.2<br>(6.9- 11.5) | 8.8<br>(7.5- 10.1) |
| **CD56+** $t_0$ (%)<br>Range (min-max) | 12.2<br>(7.0- 17.3) | 13.0<br>(7.6- 18.4) | 14.4<br>(21.2- 7.6) |
| **CD56+** $t_{24h}$ (%)<br>Range (min-max) | 12.9<br>(8.8- 13.4) | 14.1<br>(10.4- 17.8) | 17.1<br>(10.0- 24.1) |
| **CD14+** $t_0$ (%)<br>Range (min-max) | 23.1<br>(13.1- 33.1) | 25.2<br>(13.8- 36.5) | 24.6<br>(14.0- 35.2) |
| **CD14+** $t_{24h}$ (%)<br>Range (min-max) | 21.9<br>(13.8- 30.0) | 23.7<br>(13.8- 33.6) | 24.4<br>(15.4- 33.4) |
| **CD15$^{high}$** $t_0$ (%)<br>Range (min-max) | 0.0 | 0.0 | 0.01<br>(0.0- 0.02) |
| **CD15 $^{high}$** $t_{24h}$ (%)<br>Range (min-max) | 0.0 | 0.0 | 0.01<br>(0.0- 0.02) |

The results in Table 21 show that both non-irradiated apoptotic cells and irradiated apoptotic cells had comparable percentages of early (rows 2 and 3) and late (rows 4 and 5) apoptotic cells. Thus, 25 or 40 Gy irradiation did not accelerate the apoptotic or necrotic process induced prior to this high level of gamma-irradiation. Further, there was consistency between irradiated cell populations vs. control non-irradiated population with regard to cell type.

The results of potency assays, presented in **Figures 31A-31B** (HLA-DR expression) and **Figures 32A-32B** (CD86 expression) show that there was no change in the immune modulatory capacity of fresh **(Figure 32A, Figure 32A)** and 24 hour- stored **(Figure 31B and Figure 32B)** irradiate apoptotic cells when compared with non-irradiated apoptotic cells.

In both **Figures 31A-31B** and **Figures 32A-32B** there is a clear upregulation in both HLA-DR and CD86 expression, following exposure to maturation agent LPS. Significant (p<0.01), dose-dependent down regulation of both co-stimulatory markers was observed in the presence of freshly prepared apoptotic cells both from a single donor or irradiated pooled donors.. In addition, dose dependent down regulation was maintained in both markers in the presence of apoptotic cells stored at 2-8°C for 24 hours, indicating final product stability and potency following 24 hours of storage.

**Effect on dendritic cells,** In order to test the immunomodulatory capacity of apoptotic cells a post release potency assay was used (Mevorach et al., (2014) BBMT, *ibid*). No change in immune modulatory assay in dendritic cells was observed. (Data not shown)

**Effect on Mixed Lymphocyte Reaction (MLR).** In order to further test the immunomodulatory effect a standardized MLR assay was established. Here, co-cultivation of stimulator and responder cells, i.e. a MLR, yielded strong and reliable proliferation. Upon addition of non-irradiated apoptotic cells to the MLR, the lymphocyte proliferation was significantly reduced by >5- fold, clearly demonstrating cell inhibition of proliferation. Inhibition of lymphocyte proliferation in MLRs mediated by irradiated apoptotic cells was completely comparable. (Data not shown)

The next step was to evaluate *in vivo,* irradiated and non-irradiated apoptotic cells in a completely mismatched mouse model. As shown in **Figures 28 and 29,** irradiated and non-irradiated early apoptotic cell preparations had comparable *in vivo* beneficial effect.

*Single Donor Preparations Conclusion:*

In conclusion, irradiation of 25 Gy or 40 Gy did not significantly accelerate apoptosis or induced necrosis in populations of apoptotic cells. Significantly, these populations maintained the immunomodulatory effect of apoptotic cells both *in vitro* and *in vivo.*

*Multiple Donor preparations*

Next, experiments were performed to verify that the phenomenon observed with single donor, third party preparation was also true for multiple third party donors. Unexpectedly, when using pooled individual donor apoptotic cell preparations, the beneficial effect of a single unmatched donor was lost **(Figure 30).** This was not due to GvHD, as the beneficial effect of each donor separately was maintained (test results no shown). One possibility is that the beneficial effect of the early apoptotic cell preparation was lost due to the interaction of the individual donor cells among themselves. It was further examined whether this possible interaction of different donors could be avoided by gamma irradiation.

As shown in **Figure 30,** the beneficial effect of a single donor was completely restored following gamma irradiation, wherein the irradiated multiple donor preparation and the single donor preparation (irradiated or non-irradiated) had similar survival patterns.

*Conclusion:*

It is shown here for the first time that surprisingly irradiation (and possibly any method leading to T-cell Receptor inhibition) not only avoided unwanted proliferation and activation of T-cells but also allowed for the beneficial effects of immune modulation when using a preparation of multiple donor third party apoptotic cells.

### *EXAMPLE 17: In vivo Preclinical Analysis of Apoptotic Cell on the Treatment of Sepsis*

**Objective:** To develop an adjunctive immunomodulating cell-therapy for sepsis prevention of organ failure and mortality in patients with sepsis. Further, to study the effect of early apoptotic cells (Allocetra-OTS) and wide spectrum antibiotics on the course of severe cecal ligation puncture (CLP) natural history Shown here are the surprising and unexpected effect of early apoptotic cells (Allocetra-OTS), given 4h after the end of CLP procedure, in combination with ertapenem antibiotic on the development of CLP- induced sepsis in female C57Bl/6 mice. The effect was tested in three separated experiments.

**Methods:**

*The cecal ligation puncture (CLP) mouse model for sepsis*

The cecal ligation puncture (CLP) model has been proposed to more closely replicate the nature and course of clinical sepsis in humans, as compared to other models, and is considered by many researchers as the gold-standard animal model of sepsis. The CLP model involves the ligation of the cecum, usually below the ileocecal junction (to prevent bowel obstruction), and at least one centimeter above the distal end of the cecum, otherwise the sepsis induced is very mild. The length of ligated cecum, defined as the distance from the distal end of the cecum to the ligation point, determines the severity of the sepsis induced. Following ligation, the cecum is perforated, and this step too can be adjusted to modulate the severity of the sepsis ensuing. The perforation of the cecum is followed by the release of fecal material into the peritoneal cavity to induce a polymicrobial infection, which results in an exacerbated immune response. The benefits of the CLP model are its reproducibility and potential to alter the severity of sepsis by controlling needle size, number of cecal punctures, and antibiotics utilization.

The CLP model was used to evaluate the effect of donor apoptotic cells that were shown to have a rebalancing effect on the immune system (Mevorach, D., Zuckerman, T., Reiner, I., Shimoni, A., Samuel, S., Nagler, A., Rowe, J.M., and Or, R. (2014). Single infusion of donor mononuclear early apoptotic cells as prophylaxis for graft-versus-host disease in myeloablative HLA-matched allogeneic bone marrow transplantation: a phase I/IIa clinical trial. Biol. Blood Marrow Transplant. 20, 58-65; Trahtemberg and Mevorach, 2017 *ibid.*) in combination with fluid resuscitation and antibiotic treatment.

*Study Design*

The study reported here summarizes the effect of apoptotic cells administered 4 hours after the end of CLP on the development of CLP-induced sepsis in a female C57BL/6 mouse model.

**Table 22:** Study Design using CLP mouse model

| Experiment | Needle gauge* | Ligation | Anesthesia | Pain relief | Fluids reconstitution |
|---|---|---|---|---|---|
| CLP | 19G, 2 through and through | 75% above the distal end | Isoflurane. | Tramadol (100 mg/2ml). | 0.5 ml before suturing into the peritonitis. |
| [0879] * We used 75% cecal ligation with 19G needle and two through and through puncturing. | | | | | |

**Table 23:** Cohorts

| Group | CLP | Allocetra-OTS | Ertapenem |
|---|---|---|---|
| A | + | - | - |
| B | + | - (Hartmann) | + |
| C | + | + | + |

*Mice*

C57BL/6 female mice, 10-13wk old, were purchased from ENVIGO (Israel). Mice were kept in an SPF animal facility in compliance with institutional IACUC guidelines. Mice were weighed daily and monitored 2-3 times a day for clinical signs and determination of the murine sepsis score (MSS) clinical score (according to Shrum, B., Anantha, R. V., Xu, S.X., Donnelly, M., Haeryfar, S.M.M., McCormick, J.K., and Mele, T. (2014). A robust scoring system to evaluate sepsis severity in an animal model. BMC Res. Notes 7, 1-11). The endpoint was defined as total score of 15 or maximum score in one of the categories in the table.

*Cecal Ligation and Puncture (CLP) procedure*

The procedure was performed as briefly described below: Cecal Ligation and Puncture (CLP) - Experimental method. Briefly, the mice were operated under isoflurane anesthesia machine. Mice were anesthetized by 4% isoflurane in the chamber for about 1 minute, following anesthesia mice were transferred to the operation table and connected to the isoflurane nuzzle with 2% isoflurane. Mice were administered analgesics by sub-cutaneous (S.C.) injection of tramadol 5 mg/kg in 0.1ml of pre-warmed 0.9% saline solution. After opening the abdomen of the mouse by a midline incision, the cecum was exposed. The cecum was ligated 75% above its distal end with a 4-0 silk suture. Following ligation, the cecum was perforated twice with a 19-gauge needle, using the through-and-through technique (introducing a needle through the cecum). The perforation of the cecum was followed by the release of fecal material into the peritoneal cavity. Afterwards, the cecum was returned to the peritoneal cavity and 0.5ml of pre-warmed saline was administered to the peritoneal cavity, which was subsequently sutured with a 4-0 vicryl suture. The skin was then closed with 9mm clips and the mice were placed under a heating lamp to recuperate. Mice received tramadol every 12h for the first 36h after the procedure. In certain runs of the *in vivo* experiment, mice received the second dose of tramadol in 100$\mu$l of saline and in other runs of the *in vivo* experiments, the mice received the second dose of tramadol in 0.5ml saline in order to add fluid as supportive care. Mice that died during the first 24 hours after surgery were considered as perioperative mortality and were immediately excluded from the experiment, as their death was due to perioperative complications and not to sepsis.

*Allocetra-OTS (irradiated early apoptotic cells)*

Enriched mononuclear cell fraction was collected via Leukapheresis procedure from healthy, eligible donors. Following apheresis completion, cells were washed and resuspended with freezing media composed of PlasmaLyte A pH 7.4, 5% Human Serum Albumin, 10% dimethyl sulfoxide (DMSO), 5% Anticoagulant Citrate Dextrose Solution-Formula A (ACD-A) and 0.5U\ml heparin. Cells were then gradually frozen and transferred to liquid nitrogen for long term storage.

For preparation of Allocetra-OTS, cryopreserved cells were thawed, washed and resuspended with apoptosis induction media, composed of RPMI 1640 supplemented with 2 mM L-glutamine and 10 mM Hepes, 10% autologous plasma, 5% ACD-A, 0.5U\ml heparin sodium and 50$\mu$g/ml methylprednisolone. Cells were then incubated for 6 hours at 37°C in 5% $CO_2$. At the end of incubation, cells were collected, washed and resuspended in Hartmann's solution using the LOVO cell processing system (Fresenius Kabi, Germany). Following manufacturing completion, Allocetra was irradiated at 4000 cGy at the radiotherapy unit (Gammacell 220 excel, MDS nordion), Hadassah Ein Kerem Medical center, Jerusalem, Israel. Allocetra-OTS cells were centrifuged at 290g, for 10 min at 2-8°C, and resuspended in Hartmann's solution for injection.

Apoptosis and viability of Allocetra-OTS were determined using AnnexinV and PI staining (MBL, MA, USA) by flow-cytometry (FACSCalibur, BD). Results analyzed using FCS express software. Cells were >50% An$^+$PI$^-$ and <7% An$^+$PI$^+$.

In some cases, variable amounts of Allocetra-OTS cells were injected i.v. per mouse 4h after the end of CLP procedure. In other instances, $20\times10^6$ Allocetra-OTS cells were injected IV per mouse. The CLP procedures required about 20 minutes per mouse and the overall procedure lasted for about 5 hours. Allocetra-OTS was injected into each mouse 4h after its procedure had ended. Control mice received Hartmann vehicle solution at the same time point. For dose calibration of Allocetra-OTS cells, each mouse received 1, 3, 6, 10, or $20\times10^6$ cells. Control mice received Hartmann vehicle solution.

*Antibiotic treatment*

Mice received 75mg/kg Ertapenem *i.p* immediately after Allocetra-OTS administration and then every 24h for 3 days.

*Serum cytokines/chemokines.* At the indicated times (pre-CLP, 24h, 48h, and 72h post-CLP) ~500$\mu$l blood was collected in a pre-labeled Eppendorf tube and left for 30min to allow clotting. The samples were centrifuged at 1800 g (3000 rpm) for 10min at 4°C, 200$\mu$l. Serum was transferred to a new pre-labeled Eppendorf tube and kept at 4°C. Excess serum was stored at -80°C. Cytokine/chemokine measurement was performed using the Luminex MAGPIX system, and analysis was performed with Milliplex software.

*Organ dysfunction tests.* 22-24h after CLP, mice were weighed, assigned an MSS clinical score and sacrificed for the evaluation of organ dysfunction. Mice were bled through the retro-orbital sinus (venous blood). Naive mice were bled under isoflurane analgesia; CLP mice were bled without analgesia due to the concern of death.

*Blood pressure.* Mice were measured for blood pressure using a CODA noninvasive blood pressure system (Kent scientific corporation). Blood pressure was measured the day before the CLP procedure, in order to establish a baseline, and every 4 hours following the CLP procedure. For each mouse, 3 measurements were made each time blood pressure was assessed to provide more accurate data.

*Blood gas.* 100$\mu$l blood was collected using heparin-coated capillary tube (Paul Marienfeld, KG, Lauda-Königshofen, Germany) and immediately tested using STAT profile prime machine (Nova Biomedical, Waltham, MA, USA).

*Hematology.* 250$\mu$l blood was collected into EDTA tubes (MiniCollect, Greiner Bio-One, Kresmünster, Austria), tubes were rotated to prevent blood clotting and kept at 4°C. Hematology analysis was performed by AML laboratories (Herzliya, Israel).

*Biochemistry.* About 500$\mu$l blood was collected to a pre-labeled Eppendorf tube and left for ~30min to allow clotting. The samples were centrifuged at 1800 g (3000 rpm) for 10min at 4°C, 200$\mu$l serum was transferred to a new pre-labeled Eppendorf tube and kept at 4°C. Excess serum was stored in -80°C. Biochemistry analysis was performed by AML laboratories (Herzliya, Israel).

*Lungs.* 24 hours post-CLP, mice were weighed and then sacrificed; lungs were harvested and weighed, and lung-to-body weight ratios were calculated.

*NGAL, Cystatin C, Complement (C5a, C3a).* Blood was collected to pre-labeled Eppendorf tubes and left for ~30min to allow clotting. Tubes were centrifuged at 1800 g (3000 rpm) for 10min at 4°C. Serum was transferred to new pre-labeled Eppendorf tubes and stored at -80°C for Luminex (NGAL and Cystatin C) and ELISA (C3a and C5a) evaluation.

*Luminex® analysis.* Cytokine/chemokine measurement was performed using the Luminex MAGPIX system, and analysis was performed with Milliplex software. NGAL and Cystatin C were tested by Luminex Multiplex kit (Millipore, MKI2MAG-P4k). All reagents were provided with the kit, and all reagents were prepared according to the manufacturers' protocols. The assays were performed in 96-well plates according to the protocol provided. Plate reading was performed with the Luminex MAGPIX system (Luminex Corp.) and analyzed using Milliplex software (Millipore). The analysis software was set to acquire data using 50μl of sample per well, to collect not less than 50 beads (range 200-800 events per single bead set). The raw data was measured as mean fluorescence intensity (MFI) and the concentration of each analyte for each sample was calculated using a 4- or 5-parameter logistic fit curve generated for each analyte from the 7 standards. The lower limit of quantification (LLOQ) was determined using the lowest standard that was at least 3 times above background. The calculation of the LLOQ was performed by subtracting the MFI of the background (diluent) from the MFI of the lowest standard concentration and back-calculating the concentration from the standard curve.

*ELISA.* Complement components were tested by sandwich ELISA kits: C3a (TECO, TEI038) and C5a (EA100633, OriGene, Rockville, MD, USA). All reagents were provided with the kits and prepared according to the manufacturers' protocols. Assays were performed in 96-well plate according to the protocols provided. OD plate reading was performed with the Infinite F50 (TECAN, Männedorf, Switzerland) and analyzed using Magellan software (TECAN). The raw data was measured as 450nm optical density (OD) and the concentration was calculated using a linear standard curve generated from 6-7 standards. The lower limit of quantification (LLOQ) was determined using the lowest standard. The calculation of the LLOQ was performed by subtracting the OD of the background (diluent) from the OD of the lowest standard concentration and back-calculating the concentration from the standard curve.

*2D Echocardiography.* 24 hours after CLP, naive mice (n=5) or Ertapenem-treated CLP mice (n=10) were anesthetized with isoflurane and their left ventricle (LV) was imaged by echocardiography using a high-resolution imaging system (Vevo 770, Visual Sonics, Canada). LV internal distances, heart rate, and posterior wall thickness were measured for the calculation of various parameters of LV structure and function. LV volume and ejection fraction (EF) were calculated using the Teichholz method, and related parameters were calculated as previously described (Stypmann et al., 2009).

*Bioenergetics analysis. Cell isolation, seeding, and analysis.* 24 hours after CLP, mice were euthanized, the spleen was extracted and splenocytes were dissociated. cells were seeded at a density of $0.5 \times 10^6$ cells/well into XF96 well plates pre-coated with poly-D-lysine (100 μg/mL) to maximize adherence and allowed to adhere overnight. After recording of basal measurements, the Mito Stress Test (Agilent, Santa Clara, CA, USA) injection strategy consisted of oligomycin (1 μM), FCCP (1 μM), and rotenone/antimycin A in combination (1 μM). The Glycolytic Stress Test (Agilent) injection strategy consisted of glucose (10 mM) and oligomycin (1 μM), followed by 50 mM 2-deoxyglucose (2DG). Oxygen consumption rate (OCR) and extracellular acidification rate (ECAR) were measured with the XF96 Extracellular Flux Analyzer (Seahorse Bioscience, North Billerica, MA, USA) using three 3 min cycles of mix and measurement following each injection. *Normalization:* Upon completion of the extracellular flux assay, plated cells were lysed, and their protein concentrations were quantified using the BCA assay. Briefly, cells were lysed with 50 μl RIPA lysis medium supplemented with protease inhibitors for each well and agitated for 5 min, cells were incubated at RT for 30 min, and after incubation lysate samples were added to BCA working reagent medium and measured for absorbance at 562 μm.

*Data analysis:* Assay data were analyzed with MS Excel, using the XF Report Generator, macro-enabled spreadsheet (Agilent).

*Statistical method*

Unless differently indicated, data are presented as the median and the error bars represent the 5-95 percentile range. Differences between groups were examined for statistical significance using the Mann-Whitney nonparametric test. Differences between multiple groups were examined for statistical significance using Kruskal-Wallis one-way analysis of variance (non-parametric ANOVA) with multiple-comparisons adjusted by using the Dunn's test. Lung/body weight ratio was examined using one-way analysis of variance (ANOVA). Correlation of any parameter to clinical score was evaluated by a Spearman's rank correlation coefficient, with a coefficient higher than 0.7 or lower than -0.7 being a strong correlation. All statistical analyses were done using GraphPad Prism. Survival analysis was performed according to the Kaplan-Meier method. Log rank statistical test was performed using GraphPad (CA, USA).

**Results:**

The effect of Allocetra-OTS, given 4 hours after the end of CLP procedure, in combination with the ertapenem antibiotic, a highly effective antibiotic commonly used for the treatment of severe or high-risk bacterial infections, including urinary or abdominal infections, was evaluated in several studies. Mice were weighed daily and monitored two to three times per day for clinical signs and determination of the murine sepsis score. The endpoint was defined as survival (either death or sacrifice when a total clinical score of 15 or maximum score in one of the categories was reached).

*Evaluation of the MSS clinical scoring system as a surrogate indicator for organ dysfunction in CLP mice.*

Sepsis elicits dysregulated immune responses, which in turn dramatically disrupt the physiological homeostasis of vital organs including the kidney, liver, lungs, and heart. This imbalance often rapidly escalates into Multiple organ dysfunction syndrome (MODS), which is usually associated with poor outcomes. A MODS-like disease has been previously reported in murine CLP models ( Coletta, C., Módis, K., Oláh, G., Brunyánszki, A., Herzig, D.S., Sherwood, E.R., Ungvári, Z., and Szabo, C. (2014). Endothelial dysfunction is a potential contributor to multiple organ failure and mortality in aged mice subjected to septic shock: preclinical studies in a murine model of cecal ligation and puncture. Crit. Care 18, 511; Drechsler, S., Weixelbaumer, K.M., Weidinger, A., Raeven, P., Khadem, A., Redl, H., van Griensven, M., Bahrami, S., Remick, D., Kozlov, A., et al. (2015). Why do they die? Comparison of selected aspects of organ injury and dysfunction in mice surviving and dying in acute abdominal sepsis. Intensive Care Med. Exp. 3, 48; Osterbur, K., Mann, F.A., Kuroki, K., and DeClue, A. (2014). Multiple organ dysfunction syndrome in humans and animals. J. Vet. Intern. Med. 28, 1141-1151; Ruiz, S., Vardon-Bounes, F., Merlet-Dupuy, V., Conil, J.-M., Buléon, M., Fourcade, O., Tack, I., and Minville, V. (2016). Sepsis modeling in mice: ligation length is a major severity factor in cecal ligation and puncture. Intensive Care Med. Exp. 4, 22; Seemann, S., Zohles, F., and Lupp, A. (2017). Comprehensive comparison of three different animal models for systemic inflammation. J. Biomed. Sci. 24); however, histopathological analysis of organ dysfunction may not be an effective research tool for the development of therapeutic approaches in this model because it is a terminal procedure, requiring a large number of mice. In addition, histopathological results often show no differences between experimental groups and fail to correlate with disease severity and outcomes. Thus, finding diagnostic tests for organ dysfunction in septic

mice that strongly correlate with the MSS clinical score may be a clinically relevant research tool for sepsis.

Therefore, 24 hours post-CLP, each mouse (Fluids and Ertapenem-treated; N=40) was assigned with a clinical score, weighed, and blood samples were drawn from the retro-orbital sinus for further analyses. Mice were sacrificed and their lungs were harvested and weighed. To elucidate the effects of CLP on organ dysfunction and its correlation with the MSS clinical score, blood was tested for multiple parameters of organ dysfunction relating to five major systems: cardiovascular, respiratory, renal, hepatic, and hematological, as well as complement and several metabolites (Tables 24A and 24B).

**Table 24A: Organ dysfunction analysis 24 hours post CLP.**

| System | Parameter | Median of Naïve [IQR] | | Median of CLP + Ertapenem [IQR] | | [1]P-Value | [2]Correlation to Clinical Score | [3]AUC of ROC Curve |
|---|---|---|---|---|---|---|---|---|
| **Respiratory** | venous pCO2 (mmHg) | 44.75 [42.0, 59.28]; | N=8 | 53.2 [43.4, 79.8]; | N=11 | n.s | No | N/A |
| | venous pO2 (mmHg) | 46.45 [39.15, 59.98]; | N=8 | 57.4 [51.1, 63.3]; | N=13 | n.s | 0.5934 | N/A |
| | ‡pH | 7.3245 [7.31, 7.341]; | N=9 | 7.1995 [7.02, 7.274]; | N=12 | 0.0089 | -0.7792 | 0.8438 |
| | ‡Lung/body weight (w/w) | 0.0069 [0.0065, 0.007]; | N=21 | 0.0079 [0.0071, 0.0089]; | N=40 | <0.0001 | 0.743 | 0.8494 |

(continued)

| System | Parameter | Median of Naïve [IQR] | | Median of CLP + Ertapenem [IQR] | | [1]P-Value | [2]Correlation to Clinical Score | [3]AUC of ROC Curve |
|---|---|---|---|---|---|---|---|---|
| Renal | Creatinine (mg/dL) | 0.17 [0.145, 0.205]; | N=9 | 0.16 [0.11, 0.31]; | N=15 | n.s | No | N/A |
| | ‡urea (mg/dL) | 37.1 [33.9, 42.5]; | N=9 | 116.6 [67.8, 789.1]; | N=15 | <0.0001 | 0.8852 | 1 |
| | Cystatin C (ng/ml) | 650 [600, 700]; | N=4 | 750 [525, 1575]; | N=16 | n.s | 0.6196 | N/A |
| | ‡NGAL (ng/ml) | 150 [100, 275]; | N=4 | 35850 [23350, 50975]; | N=16 | 0.0004 | 0.7572 | 1 |
| Hepatic | ‡total protein (g/dL) | 5.54 [5.405, 5.61]; | N=9 | 4.14 [3.77, 4.32]; | N=15 | <0.0001 | -0.865 | 1 |
| | ‡Albumin (g/dL) | 4.2 [4.05, 4.3]; | N=9 | 2.9 [2.6, 3.1]; | N=15 | <0.0001 | -0.8333 | 1 |
| | Globulin (g/dL) | 1.33 [1.235, 1.41]; | N=9 | 1.26 [1.12, 1.36]; | N=15 | n.s | -0.5312 | N/A |
| | ‡AST (U/L) | 345 [290, 519]; | N=9 | 1003 [873, 1328]; | N=15 | <0.0001 | 0.7268 | 0.9852 |
| | ‡ALT (U/L) | 133 [94.5, 197.5]; | N=9 | 374 [327, 502]; | N=15 | <0.0001 | 0.8216 | 0.9926 |
| | ‡Alkaline Phosphatase (U/L) | 192 [169, 202]; | N=9 | 101 [93, 110]; | N=15 | <0.0001 | -0.8432 | 1 |
| | total Bilirubin (mg/dL) | 0.09 [0.065, 0.1]; | N=9 | 0.09 [0.07, 0.12]; | N=15 | n.s | No | N/A |

\* Significant differences between CLP mice and naive mice with a strong correlation to MSS Clinical score (-0.7 > ρ-Spearman > 0.7)
[1] Mann-Whitney 2-tailed nonparametric t-test; [2] ρ- Spearman; [3] Naive versus CLP mice

**Table 24B: Organ dysfunction analysis 24 hours post CLP.**

| System | Parameter | Median of Naïve [IQR] | | Median of CLP + Ertapenem [IQR] | | [1]P-Value | [2]Correlation to Clinical Score | [3]AUC of ROC Curve |
|---|---|---|---|---|---|---|---|---|
| Hematopoietic | ‡WBC ($10^3/\mu L$) | 2.585 [2.19, 3.605]; | N=8 | 1.94 [1.06, 2.18]; | N=15 | 0.0017 | No | 0.8833 |
| | RBC ($10^6/\mu L$) | 9.935 [8.28, 10.17]; | N=8 | 8.36 [8.12, 9]; | N=15 | n.s | No | N/A |
| | Hemoglobin (g/dL) | 14.85 [12.48, 15]; | N=8 | 12.4 [12, 14]; | N=15 | n.s | No | N/A |
| | HCT (%) | 46.4 [41.55, 47.7]; | N=8 | 40.9 [39.3, 43.1]; | N=15 | n.s | No | N/A |
| | MCV (fL) | 47.7 [46.25, 50.45]; | N=8 | 47.9 [46.3, 49.4]; | N=15 | n.s | No | N/A |
| | MCH (pg) | 14.95 [14.75, 15.1]; | N=8 | 14.9 [14.7, 15.3]; | N=15 | n.s | No | N/A |

(continued)

| System | Parameter | Median of Naïve [IQR] | | Median of CLP + Ertapenem [IQR] | | [1]P-Value | [2]Correlation to Clinical Score | [3]AUC of ROC Curve |
|---|---|---|---|---|---|---|---|---|
| | MCHC (g/dL) | 31.7 [30.23, 32.28]; | N=8 | 31.2 [30.5, 33.3]; | N=15 | n.s | No | N/A |
| | ‡Platelets ($10^3/\mu$L) | 642.5 [548.5, 812.8]; | N=8 | 99 [87.25, 228.5]; | N=14 | <0.0001 | -0.7099 | 1 |
| | Neutrophils ($10^3/\mu$l) | 0.51 [0.3175, 0.8125]; | N=8 | 0.31 [0.17, 0.39]; | N=15 | 0.0382 | -0.4531 | N/A |
| | Lymphocytes ($10^3/\mu$l) | 1.965 [1.723, 3.175]; | N=8 | 1.55 [0.68, 1.9]; | N=15 | 0.0275 | No | N/A |
| | Monocytes ($10^3/\mu$l) | 0 [0, 0]; | N=8 | 0 [0, 0.04]; | N=15 | N/A | No | N/A |
| | Eosinophils ($10^3/\mu$l) | 0 [0, 0]; | N=8 | 0 [0, 0]; | N=15 | N/A | N/A | N/A |
| | Basophils ($10^3/\mu$l) | 0 [0, 0]; | N=8 | 0 [0, 0]; | N=15 | N/A | N/A | N/A |
| Complement | ‡C3a (ng/ml) | 8903 [7769, 11426]; | N=4 | 4835 [4216, 5652]; | N=16 | 0.0029 | -0.7183 | 0.9531 |
| | C5a (ng/ml) | 1102 [992, 1664]; | N=4 | 1809 [1631, 2492]; | N=16 | 0.0219 | No | 0.875 |
| Metabolites | Cholesterol (mg/dL) | 97 [92.5, 100]; | N=9 | 108 [88, 140]; | N=15 | n.s | No | N/A |
| | Glucose (mg/dL) | 151 [118.5, 178]; | N=17 | 59.5 [42.75, 122.3]; | N=28 | 0.0001 | -0.3227 | 0.8288 |
| | Lactate (mg/dL) | 19.5 [17.25, 24.75]; | N=8 | 13 [9.5, 20]; | N=13 | 0.0255 | No | N/A |
| Electrolytes | Phosphorus (mg/dL) | 8.8 [8.3, 9.1]; | N=9 | 10.2 [8.5, 13.7]; | N=15 | n.s | 0.6705 | N/A |
| | Sodium (mmol/L) | 151 [147.8, 153]; | N=17 | 154.1 [150.1, 158]; | N=26 | 0.0125 | No | N/A |
| | Potassium (mmol/L) | 5.7 [5.225, 5.9]; | N=17 | 6.6 [6.1, 7.3]; | N=27 | 0.0001 | 0.4993 | 0.8279 |
| | Chloride (mmol/L) | 115.6 [113.5, 117]; | N=17 | 121.1 [119, 125.3]; | N=27 | <0.0001 | 0.5063 | 0.939 |

‡ Significant differences between CLP mice and naïve mice with a strong correlation to MSS Clinical score (-0.7 > ρ-Spearman > 0.7)
[1] Mann-Whitney 2-tailed nonparametric t-test; [2] ρ- Spearman; [3] Naïve versus CLP mice

CLP mice were compared to naïve mice (MSS score of 0; N=21). The CLP mice were divided into three sub-groups, based on their clinical scores: 1-4 (mild sepsis), 7-12 (moderate sepsis), and 13+ (severe sepsis). 24 hours post-CLP, most mice exhibited severe clinical signs, with a median MSS clinical score of 13 (95% CI of 9-14), indicating moderate to severe sepsis (Figure 34A).

To study cardiac function of CLP-mice, 24 hours post-CLP, the left ventricle (LV) of naive mice (n=5) or Ertapenem-treated CLP mice (n=10) was imaged by echocardiography and various structural and functional cardiac parameters were tested for their correlation with the clinical score (Table 25).

**Table 25: 2D Echocardiography parameter analysis**

| Parameter | Median of Naïve [IQR] | | Median of CLP [IQR] | | [1]P-Value | [2]Correlation to Clinical Score |
|---|---|---|---|---|---|---|
| *Heart rate (BPM); HR | 500 [458, 561]; | N=5 | 358.5 [270, 409]; | N=10 | 0.003 | - 0.878 |
| [3]Fractional shortening (%); FS | 30 [28.45, 40.8]; | N=5 | 40.4 [30.6, 51.6] ; | N=9 | n.s | No |
| [4]Ejection fraction (%); EF | 57.7 [55.3, 71.45]; | N=5 | 71.9 [59.1, 84.15]; | N=9 | n.s | No |
| Posterior wall thickness (mm); PWT | 25 [19.55, 37.65]; | N=5 | 15 [5.3, 50.25]; | N=9 | n.s | No |
| [5]LV Volumediastole ($\mu$l); LVEDV | 68.3 [56.8, 82.7]; | N=5 | 39.1 [26.9, 50.05]; | N=9 | 0.0035 | - 0.701 |
| [5]LV Volume- systole ($\mu$l); LVESV | 29 [17.35, 37.05]; | N=5 | 11.8 [4.2, 17.25]; | N=9 | 0.018 | - 0.597 |
| LV Area- diastole (mm$^2$); LVEDA | 10.3 [9.4, 11.58]; | N=5 | 8.44 [5.95, 8.72]; | N=9 | 0.002 | No |
| LV Area- systole (mm$^2$); LVESA | 5.15 [4.62, 5.94]; | N=5 | 3.57 [2.09, 4.76]; | N=9 | 0.042 | No |
| [6]Fractional area shortening (%); FAS | 54.42 [26.32, 58.36]; | N=5 | 54.54 [40.37, 70.93]; | N=9 | n.s | No |
| [7]LV Stroke volume ($\mu$l); SV | 41.3 [38.5, 45.65]; | N=5 | 23.4 [21.25, 37.5]; | N=9 | 0.007 | - 0.691 |
| [8,‡]Cardiac output (ml/min); CO | 20.62 [18.41, 24.6]; | N=5 | 9.34 [7.33, 11.92]; | N=9 | 0.002 | - 0.799 |

- LV internal distances (diastole/systole, LVIDD, and LVIDS, respectively), HR and PWT were measured in duplicates or triplicates using the M-Mode view of the echocardiograms; LVEDA and LVESA were measured using the B-Mode view of the echocardiograms.
- *Significant differences between CLP mice and naive mice with a strong correlation to MSS Clinical Score (-0.7 > $\rho$-Spearman > 0.7)
- [1] Mann-Whitney 2-tailed nonparametric t-test; [2] $\rho$- Spearman; [3]

$$FS\ (\%) = \frac{LVIDD - LVIDS}{LVIDd} \times 100;\ ^4 EF\ (\%) = \frac{LVEDV - LVESV}{LVEDV} \times 100$$

- [5] Teichholz method for LV volume calculation: *LVEDV*

$$LVEDV\ (\mu l) = \frac{7 \times LVIDD^3}{[2.4 + LVIDD]};\ LVESV\ (\mu l) = \frac{7 \times LVIDS^3}{[2.4 + LVIDS]};\ ^6 FAS\ (\%) = \frac{LVEDA - LVESA}{LVEDA} \times 100\ ;$$

$$^7 SV\ (\mu l) = LVEDV - LVESV;\ ^8 CO\ \left(\frac{ml}{min}\right) = SV \times HR\ /\ 1000$$

*Severe cardiovascular and respiratory dysfunction in CLP mice.* The cardiovascular system is among the first to be affected in mice with CLP-induced sepsis. Accordingly, the attempts at non-invasive measurement of the murine blood pressure were not successful because the systolic blood pressure was below the instrument's detection limit of <50 mmHg, further emphasizing the severity of sepsis. Lung dysfunction was evident by the increased lung weight (normalized to body weight), due to fluid retention; this significant increase of lung weight strongly correlated with the MSS clinical score (Table 24; $\rho$ Spearman=0.743), and accordingly was even more significant in mice with moderate and severe sepsis **(Figure 34B;** $p \le 0.01$ and $p \le 0.0001$ for MSS clinical scores of 7-12 and 13+, respectively). Though CLP mice had no apparent structural myocardial damage **(Figure 34C,** top view; B-Mode) they had a significantly lower heart rate **(Figure 34C,** M-Mode) than naïve mice, with a strong inverse correlation to the MSS clinical score (Table 25; $\rho$ Spearman= -0.878); this reduced heart-rate was most significant in mice with severe sepsis **(Figure 34D;** $p \le 0.0194$). Although the fractional shortening (FS) and ejection fraction (EF) were not significantly different between the groups (Table 25; p= n.s), CLP-mice had significantly lower diastolic LV volume, with a strong inverse correlation to clinical score (Table 25; ρ Spearman= -0.701); severely septic mice had the lowest LV volume **(Figure 34E;** p≤0.0343). The systolic LV volume and the measured LV area were also significantly lower in septic mice (Table 25). Accordingly, cardiac output of CLP mice was severely impaired and, again, strongly and inversely correlated with disease severity (Table 25, ρ Spearman= -0.799 and **Figure 34F).**

*Acute kidney injury (AKI).* An exaggerated inflammatory response combined with cardiovascular dysfunction in sepsis can seriously damage renal function. Therefore, renal dysfunction was evaluated by measuring creatinine and urea, as well as newer markers, i.e. cystatin C and NGAL. Though slightly elevated, CLP mice had no significant increase in serum creatinine and cystatin C (Tables 24A and 24B), indicating probably a relatively late effect on creatinine levels. However, urea levels were significantly elevated in CLP mice with low (1-4) and moderate (7-12) clinical score **(Figure 35A;** p≤0.01 for both groups), and strongly correlated with MSS clinical score (Tables 24A and 24B; ρ Spearman=0.8852). In contrast to the late effect on serum creatinine, NGAL was suggested to correlate well to AKI in sepsis model mice ( Otto, G.P., Busch, M., Sossdorf, M., and Claus, R.A. (2013). *Impact of sepsis-associated cytokine storm on plasma NGAL during acute kidney injury in a model of polymicrobial sepsis.* Crit. Care 17, 419). Indeed, NGAL serum concentration was dramatically increased, especially in CLP mice with severe sepsis **(Figure 35B;** p≤0.01; MSS clinical score of 13+), and strongly correlated with the clinical score (Tables 24A and 24B; ρ Spearman=0.7572). Together with a moderate but significant increase in serum potassium in CLP mice **(Figures 35C;** p≤0.01; MSS clinical score of 13+), these results are indicative of AKI.

*Markers for acute liver injury strongly correlate with MSS clinical score in CLP mice.* Liver dysfunction occurs in almost 40% of sepsis patients; it can be diagnosed by an increase of serum bilirubin and liver transaminases, and a decrease in protein production, including albumin. CLP mice were shown to follow the same trend. In this study, CLP mice with severe sepsis had a mild but insignificant increase of serum bilirubin **(Figure 36A,** p>0.93). Nevertheless, both AST and ALT transaminase levels were significantly elevated in CLP mice, compared to naive mice (Tables 24A and 24B; p≤0.001), especially in mice with severe sepsis **(Figures 36B and 36C;** p≤0.01). The dramatic increase in AST and ALT were clearly reflected in murine MSS clinical scores (Tables 24A and 24B; ρ Spearman=0.7268 and 0.8216, respectively). A substantial release of liver transaminases that is not accompanied by significant increase of bilirubin is typical of hypoxic hepatitis and may suggest this mechanism of ALI. Alkaline phosphatase (ALP) is also elevated human sepsis patients, possibly as an anti-inflammatory and anti-microbial agent with a protective function against acute kidney injury. Indeed, in severe sepsis as in this model and with severe AKI, ALP serum concentration in CLP mice was substantially reduced in comparison to naive mice, and with a strong inverse correlation to MSS clinical score (Tables 24A and 24B; p<0.0001, ρ Spearman=-0.8432). This reduction of ALP was most prominent in mice with moderate and severe sepsis (**Figure 36D**; p≤0.017 and p≤0.001 for MSS clinical scores of 7-12 and 13+, respectively).

AP are endogenous metalloenzymes found in serum and in multiple organs throughout the body including bone, liver, intestine, and kidney. These enzymes are well established as biomarkers of liver and bone disease, but their physiologic roles remain incompletely understood. Recent evidence points towards a potential protective effect of AP in the mitigation of AKI through dephosphorylation of nephrotoxic molecules including extracellular adenine nucleotides and endotoxin. Less is known about ALP serum concentration in CLP mice, although a few studies demonstrated an increase of ALP following CLP in mice, the opposite observation seen here seems to reflect the severity of CLP. 24 hours post-CLP, both total protein serum levels, and serum albumin levels had significantly dropped (Tables 24A and 24B; p<0.0001); these decreased protein levels are probably attributed to liver dysfunction, as albumin (which is produced primarily in the liver), but not globulin, was decreased (**Figures 36E** and **36F**). Interestingly, glucose levels were significantly decreased, mainly in mildly septic mice (**Figure 36G**; p≤0.01 for MSS clinical scores of 1-4), but also in general (Tables 24A and 24B; p≤0.0001). This phenomenon may be related to liver dysfunction of gluconeogenesis.

*Marked thrombocytopenia and lymphopenia in septic mice.* The hematological system is the first and one of the most affected in sepsis. Hematological aberrations in sepsis patients include thrombocytopenia, lymphopenia) and neutropenia or neutrophilia; all of which are associated with poor outcomes. CLP mice are no different. Hematological dysfunction in septic mice was thus evaluated by full blood count, including red blood cells (RBC), platelets, white blood cells (WBC, both general and sub-populations), and other parameters (hemoglobin, hematocrit, and cell volume). As seen in Tables 24A and 24B, the most dramatic effect on the hematological system was a sharp decrease (-6.49 × fold change) of CLP-mice platelet count, in comparison to naive mice (p<0.0001). This thrombocytopenia was in strong correlation to MSS clinical score (ρ Spearman=-0.7099), and more prominent in mice with moderate and severe sepsis, with median platelet counts below $100 \times 10^3/\mu$l (**Figure 37A**; 95% CI range of 37-260 $10^3/\mu$l). There were no differences between CLP-mice and healthy mice in RBC, hemoglobin, hematocrit, and cell volume, (Tables 24A and 24B; p=NS). A slight neutrophilia was also observed in septic mice, with a moderate inverse correlation to clinical score (Tables 24A and 24B; p≤0.0382; ρ Spearman=-0.4531). However, total WBC count in septic mice was significantly lower than that in healthy mice (Tables 24A and 24B; p≤0.0017) and with possibly inverse correlation to MSS clinical score. Interestingly, the lowest WBC count was in mice with mild sepsis rather than mice with severe sepsis (**Figure 37B;** p≤0.01 for MSS clinical scores of 1-4). This was mainly due to lymphocytes that were with lower counts in septic mice (Tables 24A and 24B; $p \leq 0.0275$), which was mainly attributed to severe lymphopenia in mildly septic mice (**Figure 37C**; $p \leq 0.01$ for MSS clinical scores of 1-4).

*Aberrant complement activation pattern following CLP.* The complement immune system is a major responder to infection, and as such is highly activated in sepsis. The effect of sepsis on the complementary immune system was evaluated by measuring the serum concentration of C3a and C5a, 24 hours post-CLP. As expected, C5a serum concentrations were elevated in CLP-mice; however, they did not correlate with MSS clinical score (Tables 24A and 24B; $p \leq 0.0219$). As seen in **Figure 37D,** C5 was active in all CLP-mice, regardless of their clinical score. Interestingly, C3a levels were significantly decreased in CLP-mice, and strongly correlated with MSS clinical score (Tables 24A and 24B; $p \leq 0.0029$, $\rho$ Spearman= -0.7183); This decrease was the most significant in mice with a severe clinical score (**Figure 37E**; $p \leq 0.012$ for MSS clinical scores of 13+).

*CLP mice presented adverse metabolic changes.* Because the pathogenesis of sepsis involves dramatic metabolic changes, it is of interest to explore some of the major metabolic and bioenergetic markers of sepsis and to find their correlation to disease severity. CLP mice had a significantly lower blood pH than naive mice, with a strong inverse correlation to clinical score (Tables 24A and 24B; $p \leq 0.0089$, $\rho$ Spearman=-0.7792); the median blood pH of mice with severe sepsis, compared to naive mice, was even lower (**Figure 38A**; 7.044 and 7.325, respectively), suggesting respiratory or metabolic acidosis. As noted above **(Figure 36G),** glucose levels were significantly decreased in septic mice, which may also be related to a state of hypoxia (whereas glucose is rapidly catabolized in the glycolysis pathway). In order to further explore these phenomena, bioenergetics analysis was performed using the XF96 Extracellular Flux Analyzer (Seahorse Bioscience, North Billerica, MA, USA) to measure the oxygen consumption rate (OCR) and extracellular acidification rate (ECAR) of freshly isolated PBMCs from naive and CLP-mice. These levels directly reflect mitochondrial function and glycolysis. The general mitochondrial respiration of PBMCs from CLP-mice was compromised, especially in mice with severe clinical scores **(Figure 38B),** as manifested by significantly decreased maximal respiration ($P<0.05$, Table 3), a mildly increased proton leak, and reduced spare respiratory capacity (p=n.s, Table 3).

**Table 26: Mitochondrial function and glycolysis assay analysis of mice derived splenocytes, 24h post-CLP (N= number mice)**

| *Assay | Parameter | Median of Naïve [IQR] | | Median of CLP [IQR] | | [1]P-Value | [2]Correlation to Clinical Score |
|---|---|---|---|---|---|---|---|
| **Mitochondrial Respiration (OCR)** | Non-mitochondrial oxygen consumption | 5.77 [5.16, 5.86]; | N=3 | 4.57 [4.09, 5.26]; | N=7 | 0.0667 | -0.8247 |
| | Basal respiration | 14.78 [12.72, 16.08]; | N=3 | 13.88 [11.89, 14.59] ; | N=7 | 0.3833 | No |
| | Maximal respiration | 39.94 [25.74, 45.41]; | N=3 | 26.55 [24.5, 33.16]; | N=7 | 0.1833 | No |
| | Proton leak | 3.33 [2.06, 3.47]; | N=3 | 4.1 [3.25, 4.22]; | N=7 | 0.1833 | No |
| | ATP production | 11.31 [10.67, 12.75]; | N=3 | 9.68 [8.9, 10.15]; | N=7 | 0.0667 | -0.7547 |
| | Spare respiratory capacity | 23.87 [13.01, 30.63]; | N=3 | 12.61 [10.7, 19.23]; | N=7 | 0.1167 | -0.8528 |
| | Spare respiratory capacity as a % | 249 [200, 308]; | N=3 | 204 [184, 234]; | N=7 | 0.2667 | -0.8528 |
| | Coupling efficiency (%) | 79.2 [76.3, 83.6]; | N=3 | 69.7 [69, 72.9]; | N=7 | **0.0167** | -0.6688 |

(continued)

| *Assay | Parameter | Median of Naïve [IQR] | | Median of CLP [IQR] | | [1]P-Value | [2]Correlation to Clinical Score |
|---|---|---|---|---|---|---|---|
| **Glycolytic function (ECAR)** | Non-glycolytic acidification | 0.89 [0.82, 1]; | N=3 | 0.92 [0.79, 0.98]; | N=7 | >0.9999 | No |
| | Glycolytic capacity | 0.54 [0.41, 0.81]; | N=3 | 0.81 [0.6, 0.83]; | N=7 | 0.25 | No |
| | Glycolysis | 1.24 [1.09, 1.25]; | N=3 | 1.05 [0.87, 1.31]; | N=7 | 0.5167 | No |
| | Glycolytic reserve | 0.68 [0.43, 0.71]; | N=3 | 0.35 [0.18, 0.50]; | N=7 | 0.2667 | -0.7301 |
| | Glycolytic reserve as a % | 232 [157, 268]; | N=3 | 142 [124, 175]; | N=7 | 0.1167 | -0.8528 |

Significantly reduced ATP production and coupling efficiency were also strongly and inversely correlated with the MSS clinical score (Table 26; p ≤ 0.001, ρ Spearman < -0.7 and **Figure 38C**). ECAR analysis of the same cells revealed very mild changes in glycolytic function, changes that were seen only in moderately septic mice, which had slightly increased glycolysis (**Figure 38D,** MSS clinical scores of 7-8.5). The only glycolytic parameter that was in correlation with the clinical score was the glycolytic reserve (Table 26; p = NS, ρ Spearman= -0.499; Figure 38E). In agreement with the lack of increasing glycolysis. the expected increase in lactate levels (typical of sepsis) was not found but rather a slight decrease (**Figure 38F** and Tables 24A and 24B; p≤0.0255). The apparent inability of PBMCs from septic mice to shift from damaged mitochondrial respiration to the glycolysis pathway reflects their disease severity and their failure to meet the energy demands of the immune system.

Taken together, these results show that in this CLP model of severe sepsis, the majority of significantly altered parameters of organ dysfunction strongly correlated with the MSS clinical score. These markers cover five of the main systems and organs that are damaged in sepsis. Furthermore, based on the large area under the curves (AUC) of their ROCs, all markers that had a strong correlation with the clinical score can be used for prognosis in severe sepsis (Tables 24A and 24B; AUC > 0.840). Therefore, the MSS clinical scoring system strongly reflects the pathophysiological status of the mice, and as such can be used to evaluate the efficacy of our Allocetra-OTS treatment.

*Allocetra-OTS treatments*

*Adding Allocetra-OTS to ertapenem, dramatically increased the survival of CLP mice.* Since apoptotic cells were shown to bring an exaggerated cytokine/chemokine response back to homeostasis, treating CLP-induced septic mice with Allocetra-OTS was envisioned to try rebalancing the immune response as a potential therapy for sepsis. Mice underwent CLP procedures to induce sepsis, as detailed in *Methods* above. Perioperative survival of mice from the CLP procedure using the isoflurane anesthesia machine was considered high; only 3 out of 54 mice (5.5%) died during the first 24 h after the procedure (interval of 6.5-20h) and were excluded from the study. 15 of 16 mice (94%) in the control group (CLP mice with vehicle injection-only) died of sepsis 24-72 hours after CLP. Compared to the CLP control group, ertapenem treatment with vehicle control (n=15) had no significant effect on mouse survival, with only a slightly higher median survival (P>0.99; 31h and 48h, respectively), and similar mortality of 93%.

*Initial results*

Shown in **Figure 33A,** antibiotic treatment showed a non-significant tendency to ameliorate mortality of the mice (CLP+ Ertapenem + vehicle, n=15) compared to the control group (CLP only, n=16). Treating CLP mice with the combination of antibiotics and Allocetra-OTS significantly delayed mortality and even prevented mortality in 60% of the animals (CLP+ Ertapenem +Allocetra-OTS, n=20, p<0.001). In this model 90-100% of mice die of sepsis within 50 hours. In comparison to the control group, the treated group reflected an approximately 10-fold improvement in the survival rate (p<0.001 in a log-rank analysis). As shown in Figure 33B, Allocetra-OTS-treated mice had significantly lower clinical scores indicating superior clinical condition.

Finally, the clinical score to serum cytokines/chemokines was correlated with *in vivo* measurements early on, and as shown in **Figure 33C,** Allocetra-OTS downregulated proinflammatory cytokines/chemokines related to monocyte/-macrophage and dendritic cells activation. In the preclinical study, Allocetra-OTS delayed and prevented mortality in animal models with sepsis by reducing pro-inflammatory cytokines/chemokines and resetting the sepsis-related excessive immune response following the initial immune response.

In order to determine the exact dosage that should be utilized, the Allocetra-OTS dose dependency in the CLP model was examined. As shown in **Figure 33D,** the Allocetra-OTS effect was clear using $1 \times 10^6$ cells and $3 \times 10^6$ cells. However, at $6 \times 10^6$ cells it became indistinguishable from 10 and $20 \times 10^6$ cells, indicating that the minimum dose that should be used is between $1-6 \times 10^6$ cells per 25 grams mouse. **Figure 33E** supports these findings presenting additional dosages.

A single dose of $1-6 \times 10^6$ Allocetra-OTS per 25-gram mouse, is equivalent to a dose of $40-240 \times 10^6$. cells/kg cells in humans. In a previous human study that focused on preventing complications post bone-marrow transplantations, it was determined that $70 \times 10^6$ apoptotic cells/kg were sufficient to cause a partial effect in humans and $140-210 \times 10^6$ apoptotic cells/kg caused a significant effect (Mevorach et al., 2014, *ibid).* Therefore, it was concluded that one and two dosages of $140 \times 10^6$ cells/kg should be examined, where the highest dosage would be $280 \times 10^6$/kg (in two equal doses of $140 \times 10^6$/kg).

**Table 27:** Spreadsheet for monitoring weight, survival, and clinical score of mice analyzed

| | CLP (cecal ligation and puncture) only | CLP + Ertapenem + Allocetra-OTS-$1 \times 10^6$ | CLP + Ertapenem + Allocetra-OTS-$3 \times 10^6$ | CLP + Ertapenem + Allocetra-OTS $6 \times 10^6$ | CLP + Ertapenem + Allocetra-OTS $10 \times 10^6$ | CLP + Ertapenem + Allocetra-OTS-$20 \times 10^6$ |
|---|---|---|---|---|---|---|
| | A | B | C | B | C | D |
| | n=6 | n=8 | n=7 | n=8 | n=8 | n=8 |
| **Hours** | | | | | | |
| 0 | 100 | 100 | 100 | 100 | 100 | 101 |
| 0.999 | 100 | 100 | 100 | 100 | 100 | 101 |
| 7 | 100 | 100 | 100 | 100 | 100 | 101 |
| 7.999 | 100 | 100 | 100 | 100 | 100 | 101 |
| 10 | 100 | 100 | 100 | 100 | 100 | 101 |
| 23 | 100 | 100 | 100 | 100 | 100 | 101 |
| 23.999 | 100 | 100 | 100 | 100 | 100 | 101 |
| 24 | 100 | 100 | 100 | 100 | 100 | 101 |
| 24.999 | 100 | 100 | 100 | 100 | 100 | 101 |
| 25 | 100 | 100 | 100 | 100 | 100 | 101 |
| 26.999 | 100 | 100 | 100 | 100 | 100 | 101 |
| 27 | 100 | 100 | 100 | 100 | 87.5 | 101 |
| 27.999 | 100 | 100 | 100 | 100 | 87.5 | 76 |
| 28 | 100 | 100 | 100 | 100 | 75 | 76 |
| 28.999 | 100 | 100 | 100 | 100 | 75 | 76 |
| 29 | 66.6 | 87.5 | 100 | 87.5 | 75 | 76 |
| 29.999 | 66.6 | 87.5 | 100 | 87.5 | 75 | 76 |
| 30 | 50 | 87.5 | 100 | 87.5 | 75 | 76 |
| 47.499 | 50 | 87.5 | 100 | 87.5 | 75 | 76 |
| 47.5 | 33.3 | 87.5 | 71.4 | 87.5 | 75 | 76 |
| 48.999 | 33.3 | 87.5 | 71.4 | 87.5 | 75 | 76 |
| 49 | 16.6 | 87.5 | 71.4 | 87.5 | 75 | 76 |
| 50 | 16.6 | 87.5 | 71.4 | 87.5 | 75 | 76 |
| 53.499 | 16.6 | 87.5 | 71.4 | 87.5 | 75 | 76 |
| 53.5 | 16.6 | 62.5 | 57.1 | 87.5 | 75 | 76 |
| 55.999 | 16.6 | 62.5 | 57.1 | 87.5 | 75 | 76 |

(continued)

| | CLP (cecal ligation and puncture) only | CLP + Ertapenem + Allocetra-OTS-1X10$^6$ | CLP + Ertapenem + Allocetra-OTS-3X10$^6$ | CLP + Ertapenem + Allocetra-OTS 6x10$^6$ | CLP + Ertapenem + Allocetra-OTS 10x10$^6$ | CLP + Ertapenem + Allocetra-OTS-20X10$^6$ |
|---|---|---|---|---|---|---|
| 56 | 16.6 | 62.5 | 42.8 | 87.5 | 75 | 76 |
| 72.4999 | 16.6 | 62.5 | 42.8 | 87.5 | 75 | 76 |
| 72.5 | 0 | 62.5 | 42.8 | 87.5 | 75 | 76 |
| 122 | | 62.5 | 42.8 | 87.5 | 75 | 76 |
| 177 | | 62.5 | 42.8 | 87.5 | 75 | 76 |

The initial results showed that the recuperation rate of mice from the CLP procedure using the isoflurane anesthesia machine was very high. Only 3 out of 42 mice had died during the first night after the procedure (interval of 6.5-20h after procedure and were excluded from the study without referring to different groups due to perioperative mortality).

Fifteen out of 16 mice of the Control group (CLP mice with vehicle injection-only), died from sepsis 24-51 hours after CLP (94%). Ertapenem treatment with vehicle control (n=15) slightly prolonged mice survival to 30-97h (p=NS), with the same survival rate: 14 out of 15 died (6.6%). Allocetra-OTS treatment combined with Ertapenem significantly prolonged CLP- induced sepsis survival of mice (P<0.001, log-rank, 10-fold increase in survival). Mice treated with Allocetra-OTS combined with Ertapenem died 29-146h after CLP (n=20). Furthermore, 60% of mice were still alive in good condition, at day 7 (end of experiment, log-rank p value <0.001).

The cytokine/ chemokines levels of typical mice from each group is shown in **Figure 33C.**

In attempt to see dose-dependence, it was demonstrated that even 1 and 3 million of Allocetra-OTS have an effect in severe sepsis.

*Follow-up Analysis*

As described above, Allocetra-OTS treatment, combined with ertapenem, significantly prolonged the survival of the mice following CLP-induced sepsis (**Figure 39A** (As shown in **Figure 33A** now with significance added); ***P≤0.0005, log-rank test). Among the mice treated with Allocetra-OTS and ertapenem, eight of 20 (40%) died within 29-146 hours after CLP; however, the majority of the mice remained alive at the end of the experiments 6-8 days post-CLP, and with significantly increased median survival time of 160h (**Figure 39B**; P≤0.0074, Kruskal-Wallis nonparametric ANOVA, multiple-comparisons adjusted with Dunn's test; 95% CI: 48h-172h).

***Allocetra-OTS attenuates sepsis severity.*** Murine survival had a strong reversecorrelation with clinical score (r-Pearson of -0.924; ***p < 0.0001). Accordingly, treatment with Allocetra-OTS and ertapenem, substantially attenuated the appearance of clinical symptoms. The final clinical score of Allocetra-OTS-treated mice was significantly lower than that of the CLP control group and that mice treated with Ertapenem alone (**Figure 39C**; MSS plateau values of 7.78, 14.81, and 14.37, respectively; ***p < 0.0001, ordinary one-way ANOVA).

A dose-dependent, beneficial effect of Allocetra-OTS, was also observed; CLP mice that were treated with Allocetra-OTS doses between $1\times10^6$ and 20x10$^6$ cells per mouse survived 42.85%-87.5% longer than vehicle-treated CLP mice (**Figure 39D** (as shown above in **Figure 33E** now with significance_; *P≤0.0115, log-rank test). Although even low doses of 1 and 3 million Allocetra-OTS cells per mouse had a clear effect in severe sepsis, robust effects were seen only when using 6 million Allocetra-OTS cells or more. Doses of 3-6 million Allocetra-OTS cells were not examined.

***Allocetra-OTS effects on sepsis severity are achieved by rebalancing the immuneresponse.*** Previously, it was suggested that the dramatic effect of a single apoptotic cell infusion on sepsis progression in the CLP model was attributed rebalancing of the immune systems via interaction with monocytes, macrophages, and dendritic cells (Trahtemberg and Mevorach, 2017, *ibid)*. To examine this concept, a wide panel of serum cytokines/chemokines was tested following CLP, using the Luminex Multiplex kit (Millipore, Waltham, MA, USA). As summarized in Table 28, 33 different cytokine and chemokine levels were elevated 24h post-CLP in CLP mice compared to naïve C57BL/6 mice.

**Table 28: 24-hour serum cytokine/chemokine change in mice after CLP-induced sepsis (N=6) compared to naïve mice (N=2)**

| Analyte | CLP compared to naïve mice |
|---|---|
| CRP | ↑↑ |

(continued)

| Analyte | CLP compared to naïve mice |
|---------|---------------------------|
| ENA-78 | ↑↑ |
| Eotaxin | ↑↑ |
| G-CSF | ↑↑↑ |
| GM-CSF | ↑↑ |
| Gro-α | ↑↑ |
| IL-1α | ↑↑ |
| IL-1β | ↑↑↑ |
| IL-2 | ↑↑ |
| IL-2R | ↑↑ |
| IL-5 | ↑↑ |
| IL-6 | ↑↑↑ |
| IL-9 | ↑↑ |
| IL-10 | ↑↑↑ |
| IL-12p70 | ↑↑ |
| IL-17A | ↑↑↑ |
| IL-18 | ↑↑ |
| IL-22 | ↑↑↑ |
| IL-23 | ↑↑↑ |
| IL-27 | ↑↑↑ |
| IL-28 | ↑↑ |
| IL-31 | ↑↑ |
| IFNγ | ↑↑ |
| IP-10 | ↑↑↑ |
| LIF | ↑↑↑ |
| MCP-1 | ↑↑ |
| MCP-3 | ↑↑ |
| MIP-1α | ↑↑ |
| MIP-1β | ↑↑ |
| MIP-2 | ↑↑↑ |
| RANTES | ↑↑↑ |
| TNFα | ↑↑ |
| VEGF-A | ↑↑ |

Interestingly and unexpectedly, while treatment with ertapenem antibiotics alone had no beneficial effects on cytokine/chemokine levels, combined treatment with ertapenem and Allocetra-OTS attenuated and even abolished cytokine/chemokine release at 24h and even 48h after sepsis induction **(Figures 40A-40L).** Reduced cytokine/chemokine release was observed for both proinflammatory and anti-inflammatory cytokines/chemokines. The cytokine storm rebalancing effect of Allocetra-OTS corresponded well to the effect treatment with of Allocetra-OTS plus ertapenem on murine survival and sepsis severity. These findings strongly suggest that Allocetra-OTS confers its effects through breaking the exaggerated cytokine storm that occurs in sepsis and rebalancing the immune response.

**Discussion and Conclusions:**

Triggering the innate immune system assures a common response pattern, regulated by the level of and variation in the repertoire of PAMPs and DAMPs, and the resulting signaling pathways that are activated. The complementary nature of the pathways explains the overlapping yet unique early inflammatory response to common Gram-negative bacteria, Gram-positive bacteria, fungal, and viral infections, as well as tissue injury.

Sepsis is generally initiated by simultaneous recognition of either PAMPs or DAMPs by complement, toll-like receptors, NOD-like receptors, RIG-like receptors, mannose-binding lectin, and scavenger receptors. Recognition induces a complex intracellular signaling system with redundant and complementary activities, and activation of these multiple signaling pathways ultimately leads to the expression of several common classes of genes that are involved in inflammation, adaptive immunity, and cellular metabolism. Apoptotic cells were shown to have a beneficial effect on cytokine storms with downregulation of both anti- and pro-inflammatory cytokines derived from PAMPs and DAMPs, both in animal models and in *in vitro* models (*See for example,* Examples 2-8 above). Clearance of apoptotic cells allows immune homeostasis, generally leads to a non-inflammatory state for both macrophages and dendritic cells (DCs) and contributes to the maintenance of peripheral homeostasis of almost all immune-triggered mechanisms in sepsis.

In this study of sepsis, a CLP-induced murine model for sepsis was used that successfully emulated human sepsis. This model simulated severe sepsis with acute multiple organ dysfunction. Importantly, the MSS Clinical Score method, adopted from Shrum et al (Shrum et al., 2014, *ibid),* was used to assess disease severity in tandem with multiple organ analysis, and the correlation between parameters of organ dysfunction and disease severity was assessed. Indeed, CLP mice had low blood pressure, poor cardiac output and lung dysfunction, as well as AKI, AKL, and thrombocytopenia that correlated with their clinical score. These cardiovascular and pulmonary failures are well documented in patients with severe sepsis and septic shock, and in murine sepsis models.

The complement system mediates the activation of the innate immune response against bacterial infection. However, this system is excessively activated in sepsis, which may lead to deleterious effects. Accordingly, high levels of the complement proteins C3a and C5a were detected in sepsis patients. While excessive generation of C5a causes harmful effects such as impaired neutrophil function and hyper-inflammation, some cohort studies of sepsis patients showed links between higher C3a levels and survival or C3 depletion and high mortality. The opposing protective effects of C3 and the harmful effects of C5 have been well-demonstrated in murine sepsis models of C3$^{-/-}$ and C5$^{-/-}$ or C3aR$^{-/-}$ and C5aR$^{-/-}$ after CLP; in these studies, C3-deficient mice had the poorest survival in comparison to WT and C5-deficient animals. Furthermore, C5aR$^{-/-}$ mice were resistant to Gram-negative bacteremia while C3aR$^{-/-}$ were much more sensitive to this infection.

Interestingly and surprisingly, the results of this study support those opposing effects, whereas it seems that the increased levels of C5a, together with decreased C3a levels corresponded to the high severity of sepsis in our model. An explanation for this phenomenon may be in the fact that neutrophils have only C5a receptor whereas macrophages have both C5aR and C3aR.

The pathogenesis of sepsis is strongly related to vast changes in metabolic homeostasis. Respiratory and cardiovascular dysfunction as well as malnutrition lead to energetic crisis, while the hyperactivation of the immune system greatly raises energy demands. Recent studies have provided evidence for metabolic switching from oxidative phosphorylation to aerobic glycolysis to meet the increasing energy demands of activated leukocytes in inflammation and sepsis. Accordingly, lactate, which is the main by-product of aerobic glycolysis, was found to be greatly increased in both sepsis patients and *in vivo* animal models of sepsis. In contrary to most sepsis patients, who are diagnosed with hyperglycemia, mice in the CLP-based sepsis models present hypoglycemia, as also observed by here. It was speculated that this hypoglycemia was the consequence of rapid glucose catabolism for aerobic glycolysis by immune cells. This was supported by the pro-inflammatory cytokine profile following CLP; however, although a significant reduction of pH was seen, it was surprisingly not accompanied by the expected hyperlactatemia. Closer examination of mitochondrial respiratory function and glycolytic capacity of PBMCs revealed heavily damaged mitochondria in CLP mice. The defects in energy production by oxidative phosphorylation were very severe and could not be rescued by the alternative glycolysis pathway. The inability to accelerate glycolysis as well as the apparent hypoglycemia, together with marked liver failure in CLP mice could be explained by mitochondrial dysfunction and severely impaired gluconeogenesis.

Overall, the majority of the significantly altered markers of organ dysfunction, covering five of the main systems and organs that are damaged in sepsis, also strongly correlated with the MSS Clinical Score. This correlation enabled the use of the MSS Clinical Score system as a surrogate method that reflects organ function (or dysfunction) in our CLP mice model.

Using this model, it has been shown here that, surprisingly, a single dose of Allocetra-OTS not only significantly increased murine survival, but did so in a dose-dependent manner. Furthermore, the combined treatment with ertapenem antibiotic and Allocetra-OTS significantly attenuated disease severity by almost fifty percent. Furthermore, in patients undergoing bone marrow transplantation who had an elevated cytokine profile, Allocetra was shown to be a safe and efficient with a clear dose-dependent effect starting at $140 \times 10^6$ cells/kg. This was the basis for selecting this dose for sepsis patients in the current trial.

The properties of apoptotic cells enable the mechanisms that lead to their successful use as a therapeutic modality in sepsis, where most of these mechanisms are activated, as well as in various autoimmune diseases, organ transplantation, and graft-versus-host disease (GvHD). All of these conditions are characterized by cytokine storm. Indeed, in the present study, it has been clearly shown that the beneficial effects of Allocetra-OTS are achieved via its ability to interact with and rebalance the immune system.

Use of early apoptotic cells in the treatment of sepsis provided a dramatic, unexpected effect on survival in severe sepsis. Furthermore, sepsis death was not only delayed but prevented. This surprising effect was observed even with a dose of 1 million cells. Moreover, the effect on proand anti-inflammatory cytokines chemokines was unexpected as an effect was expected only for proinflammatory cytokines. Such an effect on very high spectrum of cytokines chemokines was unknow before this study.

The resemblance between the murine model and human sepsis, and the ability to monitor sepsis severity as a derivative of organ dysfunction, as well as the initial promising positive effects of Allocetra-OTS, provide a valuable research tool for sepsis therapy. These results may lay the path for future use of Allocetra-OTS as the first effective therapy for sepsis.

It should be emphasized that treatment aiming to modulate the immune response is not administered instead of antibiotic treatment, fluid resuscitation, and vasopressors. Rather apoptotic cells are an adjuvant and complementary treatment that rebalances the immune response.

### EXAMPLE 18: In vivo Phase II Study of Sepsis

**Objective:** After completing all pre-clinical safety and efficacy testing in animals noted above in Example 17, a randomized, multi-center, vehicle-controlled, comparative, open-label, study evaluating safety and efficacy of Allocetra-OTS for the prevention of organ dysfunction in patients with sepsis will be performed.

The primary objective will be to evaluate the safety of Allocetra-OTS in patients with sepsis. Secondary Objectives will be to assess preliminary clinical efficacy and to support the proposed mechanism of action and biological effect. Exploratory objectives will be to further explore the potential mechanisms of action and biological effect.

Each patient will be followed for a period of 28 days, which is the accepted standard for sepsis studies.

### Methods:

This study is planned to be conducted in three clinical sites. The study includes three study groups (n=42 for all groups) that will be enrolled into the study, two groups will be treated with one or two Allocetra-OTS doses, and one group will be treated with vehicle (control group). Patients in each arm will be treated using the institutional standard of care (SOC) for sepsis.

### Results:

It is expected that successful treatment of sepsis in humans will follow the trajectory provided in the pre-clinical trials in mice, wherein there is an increase in survival, an anti-inflammatory effect on cytokines, and a reduction in organ damage or failure.

### EXAMPLE 19: Phase I Trial: PREVENTION OF SEPSIS-RELATED ORGAN DYSFUNCTION WITH ALLOCETRA-OTS (P-SOFA-1)

### Objectives:

*Primary Objective*: To evaluate the safety of one dose and two doses of Allocetra-OTS in patients with sepsis.

*Secondary Objectives:* To assess preliminary clinical efficacy and to support the proposed mechanism of action and biological effect.

*Exploratory Objective:* To explore possible additional mechanisms of action.

### Methods:

*Study Design:* Open label of one and two Allocetra-OTS doses in patients with sepsis. (See below for Allocetra-OTS preparation of dosage) Six (six) eligible patients were identified and recruited in the Emergency Room (ER) as they were scheduled to be admitted to Intensive Care Unit (ICU) or Intermediate Unit (IMU). Patients were followed for safety and efficacy assessments for 28 days following Investigational Product (IP) administration.

The first three (3) patients were administered one dose of Allocetra-OTS by Intravenous (IV) infusion 140x10$^6$ ±20% Allocetra-OTS cells/kg within 24±6 hours following the time of meeting sepsis criteria - *See below* (time 0).

Following 14 days (inclusive), an additional three patients were recruited and received one IV dose of 140x10$^6$ ±20% cell/kg of Allocetra-OTS, as described above.

Following 14 days (inclusive), follow-up data of the additional three patients (patients 4-6) will be submitted to the DSMB. DSMB evaluations and recommendations will be submitted to the MoH. Following MoH approval and notification to the EC, additional four (4) patients will be recruited. These four (4) patients (patients 7-10) will be administered with two doses of 140x10$^6$ ±20% Allocetra-OTS cells/kg; the first within 24±6 hours following diagnosis of sepsis, and the second 48±6 hours following the first treatment.

All study patients were treated using the institutional Standard of Care (SOC) for sepsis based on the Surviving Sepsis Campaign (Levy et al. (2018) The Surviving Sepsis Campaign Bundle: 2018 Update. Crit Care Med., 46(6):997-1000; Rhodes et al. (2017) Surviving Sepsis Campaign: International Guidelines for Management of Sepsis and Septic Shock: 2016. Intensive Care Med. 2017 Mar;43(3):304-377).

*Study Procedures*

The study included pre-screening, screening, treatment and follow-up periods.

Pre-Screening period

Based on standard local ER procedures, patients were identified as meeting clinical criteria for sepsis and start sepsis SOC. The time of sepsis diagnosis was recorded as time 0.

Screening and Eligibility Confirmation Period (Time 0 to 24±6 hours)

Only patients with verbal Glasgow Coma Score (GCS) of 5 and total GCS >13 were asked to participate in the study. Following patient signing of the Inform Consent Form (ICF), screening procedures were performed. Screening procedures included collection of demographic data, complete and disease-related medical history and concomitant medications, blood tests (biochemistry; hematology and coagulation), urinalysis, vital signs (Systolic and Diastolic Blood Pressures (BP), Respiratory Rate (RR), Temperature (T), Heart Rate (HR) and Oxygen Saturation), physical examination, 12-lead ECG, infectious disease screening, echocardiogram (if indicated), pregnancy tests (for woman of childbearing potential), as well as qSOFA and SOFA scores calculations. Procedures that were conducted in the ER prior to the patient signing the consent form as part of standard care were accepted. Following screening procedures and verification of inclusion and exclusion criteria, eligibility was confirmed.

In addition to screening procedures, baseline tests were performed and included urine albumin/creatinine ratio, APACHE II score calculation, donor-specific antibodies, autoimmune serology and blood samples for pro- and anti-inflammatory cytokines, cell phenotype, transcriptional data in leukocytes, immune functions and apoptosis, metabolomics in serum and leukocytes, cell free DNA and endocrine parameters.

Treatment Period (Day 1 for patients that were treated with one dose and Day 1 to 3 for patients that will be treated with two doses):

Prior to investigational product (IP; Allocetra-OTS) treatment, patients must have been admitted to either the ICU or IMU and treated with SOC for sepsis.

Premedication: patients received premedication prior to any investigational product (IP; Allocetra-OTS) infusion, as follows:

- 40 mg methylprednisolone as IV injection- 5±4 hours prior to Allocetra-OTS transfusion.
- 12.5 mg promethazine as IV injection- 30±30 minutes prior to investigational product (IP; Allocetra-OTS) administration.

Patients treated with one dose (Patients 1-6): Within 24±6 hours following meeting sepsis criteria (time 0) patients received premedication as indicated above, followed by one dose of Allocetra-OTS by IV infusion, using a volumetric pump, at a maximal rate of 102 mL/hour, of 140x10$^6$ ±20% cell/kg of Allocetra-OTS, in 375 ml Ringer Lactate solution.

Treatment day was designated as day 1. During investigational product (IP; Allocetra-OTS) infusion and the following 24 hours, procedures will be conducted as follows:

- Vital signs (Systolic and Diastolic Blood Pressures (BP), Respiratory Rate (RR), Temperature (T), Heart Rate (HR) and Oxygen Saturation) measurements were made - every 15±10 min during the first hour of infusion, every 30±15 min from the second hour till completion of investigational product (IP; Allocetra-OTS) infusion, and every $6\pm3$ hours following infusion completion.

Biochemistry and hematology blood tests at $60\pm60$ minutes prior to investigational product (IP; Allocetra-OTS) administration.

Blood samples for pro- and anti-inflammatory cytokines, cell phenotype, transcriptional data in leukocytes, immune functions and apoptosis, metabolomics in serum and leukocytes, cell free DNA, endocrinology panel and ELISpot will be collected at $60\pm60$ minutes prior to investigational product (IP; Allocetra-OTS) administration.

SOFA and GCS calculation- were made $60\pm60$ minutes Prior to investigational product (IP; Allocetra-OTS) administration.

Patients treated with two doses (Patients 7-10): Within $24\pm6$ hours following sepsis diagnosis (time 0) patients will receive premedication as indicated above, followed by the first dose of IV infusion, and after $48\pm6$ hours from the first treatment will receive premedication as indicated above, followed by the second dose of IV infusion. All infusion will be administered using a volumetric pump at a maximal rate of 102 mL/hour, of 140x10$^6$ $\pm20\%$ cell/kg of Allocetra-OTS, in 375 ml Ringer Lactate solution.

Treatment day of the first dose will be designated as day 1, and treatment day of the second dose will be designated as day 3. During the treatment period (days 1 to 3) procedures will be conducted as follows:

- Vital signs (Systolic and Diastolic Blood Pressures (BP), Respiratory Rate (RR), Temperature (T), Heart Rate (HR) and Oxygen Saturation) measurements as follows: on days 1 and 3: every $15\pm10$ min during the first hour of infusion, every $30\pm15$ min until completion of investigational product (IP; Allocetra-OTS) infusion, and every $6\pm3$ hours following infusion completion; on day 2 every $6\pm3$ hours.

Blood tests (biochemistry and hematology):

- Day 1: $60\pm60$ minutes prior to investigational product (IP; Allocetra-OTS) administration.
- Day 2: $24\pm6$ hours following completion of first investigational product (IP; Allocetra-OTS) infusion.
- Day 3: $48\pm6$ hours following completion of first investigational product (IP; Allocetra-OTS) infusion and prior to second investigational product (IP; Allocetra-OTS) infusion.

Blood samples for pro- and anti-inflammatory cytokines, cell phenotype, transcriptional data in leukocytes, immune functions and apoptosis, metabolomics in serum and leukocytes and cell free DNA, as follows:

- Day 1: $60\pm60$ minutes prior to first investigational product (IP; Allocetra-OTS) administration.

- Day 2: $24\pm6$ hours following completion of first investigational product (IP; Allocetra-OTS) infusion.

- Day 3: $60\pm60$ minutes prior to second investigational product (IP; Allocetra-OTS) administration.

ELISpot on day 1- $60\pm60$ minutes prior to first investigational product (IP; Allocetra-OTS) administration.
12-lead ECG: on day 2, $24\pm2$ hours following completion of the first investigational product (IP; Allocetra-OTS) infusion.
SOFA and Glasgow coma scale (GCS) scores calculation: on days 1, 2 and 3.
Follow Up Period (Day 2 to 28 for the single-dose patients and Day 4 to 28 for the two-dose patients):
Patients were followed-up to study day 28 following first investigational product (IP; Allocetra-OTS) treatment for safety and efficacy evaluations as follows:

- Adverse events and concomitant medication: continuously.
- Vital signs (Systolic and Diastolic Blood Pressures (BP), Respiratory Rate (RR), Temperature (T), Heart Rate (HR) and Oxygen Saturation), as follows: for single dose - twice daily on days 2 and 3 and once daily as long as the patient was hospitalized. For two doses - twice daily on days 4 and 5 and once daily as long as the patient is in hospitalized. If patient is discharged, vital signs will be measured on day 7, 14 and 28 visits.
- Blood tests (biochemistry and hematology) were taken once daily until day 7 and on days $11\pm1$, $14\pm1$, $18\pm1$, $21\pm1$, $24\pm1$ and $28\pm2$ if the patient was hospitalized. If patient was discharged, these tests were measured on day $7\pm1$, $14\pm2$ and $28\pm2$ visits.
- Blood samples for pro- and anti-inflammatory cytokines, immune functions and apoptosis, metabolomics in serum and leukocytes and cell free DNA on days 2, 3, 4, $7\pm1$, $14\pm2$ and $28\pm2$.
- Blood samples for cell phenotype and transcriptional data in leukocytes on days 2, 3, 4 and $28\pm2$.

- Blood samples for endocrinology panel on days 4±1 and 28±2.
- 12-lead ECG: on day 2 for patients treated with one dose and on day 4 for patients treated with two doses (24±2 hours following investigational product (IP; Allocetra-OTS) administration), as well as on days 14±2 and 28±2 for all patients.
- SOFA scores were documented on days 2, 3, 7, 14±2, 28±2.
- Donor-specific antibodies, ELISpot and autoimmune serology will be evaluated on day 28±2.

*Study Duration*:

For each participating patient, the duration in the study was 28 days from investigational product (IP; Allocetra-OTS) treatment.

*Study Population:*

As of this time, the Study included 6 patients, with a goal of having a total of 10 patients.

*Inclusion / Exclusion Criteria:*

*Inclusion Criteria*:

- Suspected, presumed or documented infection from any source.
- Initiation of antibiotics.
- Meets Sepsis 3 criteria: The presence of organ dysfunction as identified by a total SOFA score $\geq$ 2 points above baseline.
- Adult male or female, age between 18 and 85.
- GCS of >13 with verbal score of 5.
- Signed written informed consent by the patient.

*Exclusion Criteria*:

- Participation in an interventional investigational trial within 30 days prior to diagnosis of sepsis.
- Significant trauma requiring hospitalization within 30 days prior to diagnosis of sepsis.
- Surgical intervention, or plan for surgical intervention, or hospitalization within 30 days prior or after the scheduled investigational product (IP; Allocetra-OTS) infusion.
- Pregnancy or breast-feeding female.
- Progressive or poorly controlled malignancies or < 6 month after active treatment for cancer (chemotherapy or irradiation).
- Terminally ill patients defined as patients that prior to the current hospitalization are expected to live < 6 months (as assessed by the physician responsible for the patient).
- Known active acute or chronic viral infections, e.g. Hepatitis B Virus (HBV) or Hepatitis C Virus (HCV), Human Immunodeficiency Virus (HIV) or other chronic infection.
- Known severe chronic respiratory health problems with severe pulmonary hypertension ($\geq$40 mmHg) or respirator dependency.
- Known active upper gastrointestinal (GI) tract ulceration or hepatic dysfunction including: biopsy-proven cirrhosis; portal hypertension; episodes of past upper GI bleeding attributed to portal hypertension; or prior episodes of hepatic failure, encephalopathy, or coma.
- Known New York Heart Association (NYHA) class IV heart failure or unstable angina, ventricular arrhythmias, active ischemic heart disease, or myocardial infarction within six months prior to diagnosis of sepsis.
- Known immunocompromised state or medications known to be immunosuppressive.
- Organ allograft or previous history of stem cell transplantation.

*Allocetra-OTS Product, Route of Administration, and Dosage Form*

Allocetra-OTS is a cell-based therapeutic composed of donor early apoptotic cells. The Allocetra-OTS product contained allogeneic donor mononuclear enriched cells in the form of a liquid suspension with at least 40% early apoptotic cells. Early apoptotic cells were prepared as per Example 1 above. The suspension was prepared with Ringer's lactate solution and stored at 2-8°C until 45±25 minutes before infusion and at room temperature thereafter. Allocetra-OTS contains $140 \times 10^6 \pm 20\%$ cells per kg of recipient body weight in a total volume of 375 mL in a transfer pack that underwent irradiation and was administrated using a volumetric pump, at a starting rate of 48 mL/hour (16 drops per minute) with a gradual increase every 15-25 minutes of 15 mL/hour (additional 5 drops per minute) to a maximal rate of 102 mL/hour, as follows: 63 (21 drops) mL/hour; 78 (26 drops) mL/hour; 93 (31 drops) mL/hour ; 102 (34 drops) mL/hour.

During investigational product (IP; Allocetra-OTS) administration no other I.V. fluids such as Ringer's lactate or Normal Saline were given in parallel, unless medically indicated due to volume depletion.

The Allocetra-OTS was administered to the patient within 72 hours of completing the manufacturing process.

*Standard of Care (SOC)*

The SOC was according to the Surviving Sepsis Campaign guidelines (Levy et al., 2018, *ibid*; Rhodes et al., 2017, *ibid)* with allowance for variance based on institutional standards. Institutional SOC may include intravenous (IV) fluids including crystalloids with or without albumin, other blood products according to accepted indications, vasopressors and inotropes, antibiotics, anti-viral or anti-fungal agents and corticosteroids if indicated.

*Premedication*

Patients received 40 mg methylprednisolone and 12.5 mg of I.V. promethazine, as IV injections, prior to Allocetra-OTS transfusion.

*Concomitant Medications*

Prohibited medications: Significant immune suppressing agents including chronic corticosteroids > 10 mg/day, azathioprine, cyclosporine, cyclophosphamide, and any biological treatment.

*Safety Endpoint (primary)*

Assessment of safety was accomplished by determining the clinical outcome and the number of participants with any Adverse Events (AE), Serious Adverse Events (SAE) and fatal SAE.

*Efficacy Endpoints/Outcome Measures (secondary)*

The following efficacy endpoints were measured throughout the study period:

- Any one of the following organ function or support measurements recorded throughout the 28-days study period:

  o Ventilator-free days, and/or
  o Vasopressor-free days, and/or
  o Days without renal replacement therapy (dialysis) and/or days with creatinine $\leq$ baseline +20%, and/or
  o

  Days with $\geq 100 \times 10^9$/L platelets count, and/or

  o

  Days with $\leq$ three times normal ALT and AST levels and/or $\leq$ two times normal bilirubin levels, and/or

  o Days with return to GCS 15.

- Mortality from any cause.
- Cumulative days in ICU or IMU and/or in hospital.

## Time to CRP < 20 mg/L.

- Time to normal + 20% lactate levels.

*Exploratory Endpoints*

The following exploratory biological tests were or will be measured at baseline and at days 1, 2, 3, 4, 7, 14 and 28 post first investigational product (IP; Allocetra-OTS) treatment:

- Pro- and anti-inflammatory cytokines : MIP-1 beta, TNF alpha, MCP-1, IL-1R, IL-1 beta, IL-2, IL-2R, IL-4, IL-5, IL-6, IL-7, IL-8, IL-9, IL-15, MIP-1 alpha, IL-22 and GMCSF.
- Immune functions and apoptosis: HMGB1, histone levels, and complement.
- Metabolomics in serum and leukocytes: Pyruvate, FFAs, glycolysis, mitochondrial function, leptin, ghrelin and glucagon.
- Cell free DNA

The following exploratory blood test were or will be measured at baseline and at days 1,2, 3 (prior investigational product (IP; Allocetra-OTS) treatment for patients treated with two doses), 4, and 28 post first investigational product (IP; Allocetra-OTS) treatment:

- Cell Phenotype: T regs, CD4, CD8, NK and B, monocytes, dendritic cells and function and expressions of PD-1, PDL-1 and BTLA.
- Transcriptional data in leukocytes.

ELISpot was or will be measured on baseline, day 1 and day 28.

- Endocrinology panel at baseline and on days 1, 4 and 28: Cortisol, ACTH, FT3, FT4, TSH, growth hormone and insulin.

The following exploratory blood tests was or will be measured at baseline and on day 28:

- Donor specific antibodies: Panel Reactive Antibodies (PRA).
- Autoimmune serology panel: ANA, Anti-DNA, Anti-RNP, Anti-SSA, Anti-SSB, Cardiolipin IgG and Cardiolipin IgM

*Statistical Analysis*

Data from all clinical assessments, whether explicitly referred to in the statistics section or not, was and will be listed and, where appropriate, summarized by dose group and by other categorical information of interest using descriptive statistics.

Summary statistics (arithmetic mean, standard deviation, minimum value, lower quartile, median, upper quartile, maximum value, number of non-missing values) was and will be presented for continuous variables (absolute values at each time point and changes from baseline, if appropriate) and counts and percentages will be presented for categorical variables. Where appropriate, the presentation of results includes shift tables, plots, statistical tests or confidence intervals.

**Results:**

The results presented in **Figures 41A-41C** through **Figures 50A-50B** provide positive interim efficacy data from an ongoing Phase Ib clinical trial in patients with severe sepsis. The interim analysis is based on a dataset with a total of 43patients with severe sepsis (6 treated patients and 37 historical controls), all hospitalized at Hadassah Medical Center (**Figures 41A-41C and 42**). Six patients admitted to intensive care unit with sepsis were administered with off-the-shelf Allocetra ("OTS Allocetra"; early apoptotic cells as described above) upon their admission into the intensive care unit, while 37 patients are matched controls that have received standard of care during 2016-2019 but did not received Allocetra treatment.

The primary safety parameter was ± mortality at 28 days. Surprisingly, no Allocetra-treated patients died (0/6 (0%)) compared with 11/37 (29%) in the matched control group (**Figures 43, 46A, and 46B**). Efficacy parameters that were analyzed include organ failure clinical SOFA score (the higher the score, the negative the clinical condition of various organs) (**Figures 48A-48D**, **49A-49B,** and **50A-50B**), as well as mortality (**Figures 46A-46B**), recovery from sepsis (**Figures 44A-44B** and **Figure 45**), number of days of hospitalization in the intensive care unit (**Figure 45** and **Figures 47A-47B**).

Table 29 below provides a summary of the interim results showing the robust safety and efficacy profile demonstrated by Allocetra-OTS administration to patients with severe sepsis. The matching of the 37 patients to the OTS Allocetra-treated group was based on similar organ failure clinical SOFA score at admission, overall clinical state, age group, sex, and source of severe sepsis (pneumonia, endovacular, or urinal tract infections). All matched patients were treated at the same hospital as the Allocetra-treated group. A summary of the matching characteristics is presented in Table 30.

**Table 29: Summary Interim Results**

| Sepsis Outcomes | Matched Untreated Group | Treated with OTS Allocetra |
|---|---|---|
| % of patients that recovered from Sepsis within 28 days | 48% | 100% |
| % Mortality of patients | 29% | 0% |
| Organ failure clinical SOFA score at admission vs maximal reached during hospitalization | Avg. at admission: 3.98 | Avg. at admission: 4.5 |
| Organ failure clinical score at admission vs maximal reached during hospitalization | Avg. Maximal: 8.11 | Avg. Maximal: 4.5 |
| % of patients with organ failure clinical score that increased by 28 days | Median at admission: 4 | Median at admission: 4.5 |
| % of patients with organ failure SOFA clinical score that increased by 4 or more during 28 days | Median Maximal: 8 | Median Maximal: 4.5 |
| % of patient mortality among those with organ failure clinical SOFA score that increased by 4 or more during 28 days | 78% | 0% |
| % of patients still in intensive care unit after 6 days | 57% | 0% |

**Table 30: Summary Allocetra-OTS Patient and Matching Control Characteristics**

| Matching Characteristics | Matched Untreated Group | Treated with OTS Allocetra |
|---|---|---|
| **Source of sepsis** | | |
| Pneumonia | 67% | 68% |
| Endovascular ( (MRSA) | 16% | 17% |
| Urinal tract infection | 17% | 15% |
| **Age group distribution** | Avg. age: 69.2 Median age: 69 | Avg. age: 69.8 Median age: 70.5 |
| **SOFA at admission** | Avg. at admission: 3.98 Median at admission: 4 | Avg. at admission: 4.50 Median at admission: 4.50 |
| **Sex** | All male | All male |

**Summary:** The interim results of the Phase Ib clinical trial demonstrate positive interim efficacy for safety and efficacy, in treating patients with sepsis using Allocetra-OTS ("Off the Shelf") early apoptotic cells. No mortalities were observed and time to recover from sepsis (time to SPFA score<2) and hospital length of stay, were significantly reduced compared to historical controls.

## Claims

1. A composition comprising an early apoptotic cell population for use in treating, preventing, inhibiting, or reducing the incidence of organ failure in a subject having sepsis, wherein said population comprises a mononuclear enriched early apoptotic cell population, optionally wherein said population comprises a mononuclear apoptotic cell population comprising a decreased percentage of non-quiescent non-apoptotic cells, a suppressed cellular activation of any living non-apoptotic cells, or a reduced proliferation of any living non-apoptotic cells, or any combination thereof.

2. The composition for use according to claim 1, wherein the sepsis comprises mild, severe, acute, or highly aggressive sepsis.

3. The composition for use according to claim 1 or claim 2, wherein the composition increases the survival of the subject, or wherein the composition reduces the incidence of organ failure or organ dysfunction, or organ damage, or a combination thereof.

4. The composition for use according to claim 3, wherein the organ failure comprises acute multiple organ failure.

5. The composition for use according to any of claims 1-4, wherein the early apoptotic cell population comprises a pooled population of early apoptotic cells; or comprises an irradiated population of early apoptotic cells, wherein said irradiation was post induction of apoptosis; or comprises a pooled population of irradiated early apoptotic cells, wherein said irradiation was post induction of apoptosis.

6. The composition for use according to any of claims 1-5, wherein the subject is a human subject.

7. The composition for use according to any of claims 1-6, wherein a single infusion of the early apoptotic cell population or multiple infusions of the apoptotic cell population are administered to the subject.

8. The composition for use according to claim 7, wherein said administering comprises intravenous administration.

9. The composition for use according to any of claims 1-8, wherein an additional therapy for treating, preventing, inhibiting, reducing the incidence of, ameliorating, or alleviating sepsis, or any combination thereof, is administered to the subject.

10. The composition for use according to claim 9, wherein the additional therapy is administered prior to, concurrent with, or following administration of the early apoptotic cells.

11. The composition for use according to any of claims 1-10, wherein the composition comprises a first-line therapy or comprises an adjuvant therapy.

12. The composition for use according to any of claims 1-11, wherein the composition reduces the secretion of one or more proinflammatory cytokine/chemokine.

13. The composition for use according to any of claims 1-11, wherein the composition reduces the secretion of one or more anti-inflammatory cytokine/chemokine.

14. The composition for use according to any of claims 1-11, wherein the composition reduces secretion of one or more pro-inflammatory cytokines/chemokines and one or more anti-inflammatory cytokines/chemokines.

15. The composition for use according to any of claims 1-14, wherein the composition prevents, inhibits, reduces the incidence of, or reduces the severity of a cytokine and chemokine storm in the subject.

**Patentansprüche**

1. Zusammensetzung, umfassend eine Population von frühen apoptotischen Zellen zur Verwendung beim Behandeln, Verhindern, Hemmen oder Verringern der Inzidenz eines Organversagens bei einem Probanden mit Sepsis, wobei die Population eine Population von mononukleären angereicherten frühen apoptotischen Zellen umfasst, optional wobei die Population eine Population von mononukleären apoptotischen Zellen umfasst, die einen reduzierten Prozentanteil von nichtruhenden nichtapoptotischen Zellen, eine supprimierte Zellaktivierung von beliebigen lebenden nichtapoptotischen Zellen oder eine verringerte Proliferation von beliebigen lebenden nichtapoptotischen Zellen oder eine beliebige Kombination davon umfasst.

2. Zusammensetzung zur Verwendung nach Anspruch 1, wobei die Sepsis milde, schwere, akute oder hochaggressive Sepsis umfasst.

3. Zusammensetzung zur Verwendung nach Anspruch 1 oder Anspruch 2, wobei die Zusammensetzung das Überleben des Probanden verbessert, oder wobei die Zusammensetzung die Inzidenz eines Organversagens oder einer Organdysfunktion oder einer Organschädigung oder einer Kombination davon verringert.

4. Zusammensetzung zur Verwendung nach Anspruch 3, wobei das Organversagen akutes Multiorganversagen umfasst.

5. Zusammensetzung zur Verwendung nach einem der Ansprüche 1-4, wobei die Population von frühen apoptotischen Zellen eine gepoolte Population von frühen apoptotischen Zellen umfasst oder eine bestrahlte Population von frühen apoptotischen Zellen umfasst, wobei die Bestrahlung nach einer Induktion einer Apoptose war; oder eine gepoolte Population von bestrahlten frühen apoptotischen Zellen umfasst, wobei die Bestrahlung nach einer Induktion einer Apoptose war.

6. Zusammensetzung zur Verwendung nach einem der Ansprüche 1-5, wobei der Proband ein menschlicher Proband ist.

7. Zusammensetzung zur Verwendung nach einem der Ansprüche 1-6, wobei eine einzige Infusion der Population von frühen apoptotischen Zellen oder mehrere Infusionen der Population von apoptotischen Zellen an den Probanden verabreicht werden.

8. Zusammensetzung zur Verwendung nach Anspruch 7, wobei das Verabreichen eine intravenöse Verabreichung umfasst.

9. Zusammensetzung zur Verwendung nach einem der Ansprüche 1-8, wobei eine Zusatztherapie zum Behandeln, Verhindern, Hemmen, Verringern der Inzidenz, Lindern oder Mildern von Sepsis oder einer beliebigen Kombination davon an den Probanden verabreicht wird.

10. Zusammensetzung zur Verwendung nach Anspruch 9, wobei die Zusatztherapie vor, gleichzeitig mit oder nach einer Verabreichung der frühen apoptotischen Zellen verabreicht wird.

11. Zusammensetzung zur Verwendung nach einem der Ansprüche 1-10, wobei die Zusammensetzung eine Primärtherapie umfasst oder eine unterstützende Therapie umfasst.

12. Zusammensetzung zur Verwendung nach einem der Ansprüche 1-11, wobei die Zusammensetzung die Sekretion eines oder mehrerer proinflammatorischer Zytokine/Chemokine verringert.

13. Zusammensetzung zur Verwendung nach einem der Ansprüche 1-11, wobei die Zusammensetzung die Sekretion eines oder mehrerer antiinflammatorischer Zytokine/Chemokine verringert.

14. Zusammensetzung zur Verwendung nach einem der Ansprüche 1-11, wobei die Zusammensetzung die Sekretion eines oder mehrerer proinflammatorischer Zytokine/Chemokine und eines oder mehrerer antiinflammatorischer Zytokine/Chemokine verringert.

15. Zusammensetzung zur Verwendung nach einem der Ansprüche 1-14, wobei die Zusammensetzung die Inzidenz eines Zytokin- und Chemokinsturms bei dem Probanden verhindert, hemmt, verringert oder die Schwere dieses verringert.

**Revendications**

1. Composition, comprenant une population de cellules apoptotiques précoces destinée à être utilisée dans le traitement, la prévention, l'inhibition, ou la réduction de l'incidence d'une défaillance d'organe chez un sujet ayant une sepsie, dans laquelle ladite population comprend une population de cellules apoptotiques précoces enrichies mononucléées, facultativement dans laquelle ladite population comprend une population de cellules apoptotiques mononucléées comprenant un pourcentage réduit de cellules non apoptotiques non quiescentes, une activation cellulaire supprimée de toute cellule non apoptotique vivante, ou une prolifération réduite de toute cellule non apoptotique vivante, ou une quelconque combinaison de ceux-ci.

2. Composition destinée à être utilisée selon la revendication 1, dans laquelle la sepsie comprend une sepsie légère, sévère, aiguë ou hautement agressive.

3. Composition destinée à être utilisée selon la revendication 1 ou la revendication 2, dans laquelle la composition augmente la survie du sujet, ou dans laquelle la composition réduit l'incidence de défaillance d'organe ou de dysfonctionnement d'organe, ou de dommage d'organe, ou une combinaison de ceux-ci.

4. Composition destinée à être utilisée selon la revendication 3, dans laquelle la défaillance d'organe comprend une défaillance aiguë de plusieurs organes.

5. Composition destinée à être utilisée selon l'une quelconque des revendications 1 à 4, dans laquelle la population de cellules apoptotiques précoces comprend une population groupée de cellules apoptotiques précoces ; ou comprend une population irradiée de cellules apoptotiques précoces, dans laquelle ladite irradiation a eu lieu après l'induction de l'apoptose ; ou comprend une population groupée de cellules apoptotiques précoces irradiées, dans laquelle ladite irradiation a eu lieu après l'induction de l'apoptose.

6. Composition destinée à être utilisée selon l'une quelconque des revendications 1 à 5, dans laquelle le sujet est un sujet humain.

7. Composition destinée à être utilisée selon l'une quelconque des revendications 1 à 6, dans laquelle une seule infusion de la population de cellules apoptotiques précoces ou plusieurs infusions de la population de cellules apoptotiques sont administrées au sujet.

8. Composition destinée à être utilisée selon la revendication 7, dans laquelle ladite administration comprend une administration par voie intraveineuse.

9. Composition destinée à être utilisée selon l'une quelconque des revendications 1 à 8, dans laquelle un traitement supplémentaire pour le traitement, la prévention, l'inhibition, la réduction de l'incidence de, l'amélioration, ou l'atténuation de la sepsie, ou une quelconque combinaison de ceux-ci, est administrée au sujet.

10. Composition destinée à être utilisée selon la revendication 9, dans laquelle le traitement supplémentaire est administré avant, simultanément à, ou après l'administration des cellules apoptotiques précoces.

11. Composition destinée à être utilisée selon l'une quelconque des revendications 1 à 10, dans laquelle la composition comprend un traitement de première intention ou comprend un traitement adjuvant.

12. Composition destinée à être utilisée selon l'une quelconque des revendications 1 à 11, dans laquelle la composition réduit la sécrétion d'une ou plusieurs cytokines/chimiokines proinflammatoires.

13. Composition destinée à être utilisée selon l'une quelconque des revendications 1 à 11, dans laquelle la composition réduit la sécrétion d'une ou plusieurs cytokines/chimiokines anti-inflammatoires.

14. Composition destinée à être utilisée selon l'une quelconque des revendications 1 à 11, dans laquelle la composition réduit la sécrétion d'une ou plusieurs cytokines/chimiokines proinflammatoires et d'une ou plusieurs cytokines/chimiokines anti-inflammatoires.

15. Composition destinée à être utilisée selon l'une quelconque des revendications 1 à 14, dans laquelle la composition empêche, inhibe, réduit l'incidence de, ou réduit la sévérité d'un choc cytokinique et chimiokinique chez le sujet.

FIGURE 1A

# CAR + APOPTOTIC CELLS/SUPERNATANT(using patient's own cells)

**Gene**

T-Cell

LEUKAPHERESIS

ACTIVATION &
SELECTION

GENE TRANSFER

CELL EXPANSION

INFUSION

SCRS

MONONUCLEAR
CELL SELECTION

PROCESSING
INTO APOPTOTIC CELLS
OR SUPERNATANT

INFUSION 24 HR PRIOR
OR CONCURRENT
WITH CAR-T CELLS

FIGURE 1B

CAR + APOPTOTIC CELLS/SUPERNATANT(using donor cells)

LEUKAPHERESIS • ACTIVATION & SELECTION • GENE TRANSFER • CELL EXPANSION • INFUSION

DONOR BLOOD COLLECTION • MONONUCLEAR CELL SELECTION • PROCESSING INTO APOPTOTIC CELLS OR SUPERNATANT

INFUSION 24 HR PRIOR OR CONCURRENT WITH CAR-T CELLS

sCRS

FIGURE 2

**FIGURE 3**

FIGURE 4A

FIGURE 4B

FIGURE 4C

**FIGURE 4D**

FIGURE 4E

FIGURE 4F

**FIGURE 4G**

FIGURE 4H

EP 3 883 584 B1

FIGURE 4I

IL-2, t24

FIGURE 4J

FIGURE 5

EP 3 883 584 B1

FIGURE 6

## T4+ CAR-T Cells Cytotoxicity Assay

**FIGURE 7**

T4+ CAR-T Cells Cytoxicity Assay in the Presence of Apoptotic Cells

**FIGURE 8**

FIGURE 9

FIGURE 10

FIGURE 11A

FIGURE 11B

FIGURE 12

Tumor growth 0.5x10^6 SKOV3-luc

FIGURE 13A

Tumor growth 0.5x10^6 SKOV3-luc

FIGURE 13B

Average SKOV3-luc Tumor Growth

Avg. Radiance (p/s/cm²/sr)

- - -x- - - PBS-Control
——x—— 0.5x10⁶ SKOV3-luc
— * — 1x10⁶ SKOV3-luc
- ·-x-· - 4.5x10⁶ SKOV3-luc

Time (Days post SKOV3-luc injection)

**FIGURE 13D**

Tumor growth 4.5x10⁶ SKOV3-luc

- T=11d
- T=18d
- T=25d
- T=32d
- T=39d

Avg. Radiance (p/s/cm²/sr)

Mouse number

**FIGURE 13C**

FIGURE 14

FIGURE 15

FIGURE 16

FIGURE 17

EP 3 883 584 B1

# Experiment Scheme

Survival

Days:              1        6        12        18        24        30

Raji        CAR T-CD19           Expected              End points
Injection   +Apo Injection       Death (18-21 d)

FIGURE 18A

Survival Curve: SCID injected with CD19+Raji

FIGURE 18B

FIGURE 19A

FIGURE 19B

FIGURE 19C

FIGURE 20A

FIGURE 20B

Survival Curve: SCID injected with CD19+Raji

FIGURE 21

FIGURE 22A

FIGURE 22B

FIGURE 22C

FIGURE 22D

100%   100% 100%

**86%**

**FIGURE 23A**

100% 100%

**29%**

0%

**FIGURE 23B**

100%

**57%**

**29%**

0%

**FIGURE 23C**

85%

**29%**

**14%**

0%

**FIGURE 23D**

**29%**   29%

0%   0%

**FIGURE 23E**

Figure 24

| Compared groups | | Statistical test (P value) |
|---|---|---|
| Group 1 | Group 2 | Log-rank (Mantel-Cox) Test |
| Raji | Raji + ApoCell | 0.0334 |

FIGURE 25A

| Compared groups | | Statistical test (P value) |
|---|---|---|
| Group 1 | Group 2 | Log-rank (Mantel-Cox) Test |
| Raji | Raji + ApoCell | 0.1031 |

**FIGURE 25B**

| Compared groups | | Statistical test (P value) |
|---|---|---|
| Group 1 | Group 2 | Log-rank (Mantel-Cox) Test |
| Raji | Raji + ApoCell | 0.3231 |

**FIGURE 25C**

**FIGURE 26**

**FIGURE 27**

FIGURE 28

FIGURE 29

**FIGURE 30**

FIGURE 31A

FIGURE 31B

**FIGURE 32A**

**FIGURE 32B**

FIGURE 33A

FIGURE 33B

148

FIGURE 33C

FIGURE 33D

FIGURE 33E

FIGURE 34A

FIGURE 34B

FIGURE 34C

EP 3 883 584 B1

Heart rate

LV volume (Diastola)

Cardiac Output

P≤0.0194

P≤0.0343

P≤0.0168

FIGURE 34D

FIGURE 34E

FIGURE 34F

FIGURE 35A

FIGURE 35B

FIGURE 35C

FIGURE 36A

FIGURE 36B

FIGURE 36C

Alkaline phosphatase

P≤0.017

Clinical Score

0   1-4   7-12   13+
N=9  N=4   N=4   N=7

**FIGURE 36D**

Albumin

P≤0.040

Clinical Score

0   1-4   7-12   13+
N=9  N=4   N=4   N=7

**FIGURE 36E**

Globulin

Clinical Score

0   1-4   7-12   13+
N=9  N=4   N=4   N=7

**FIGURE 36F**

FIGURE 36G

FIGURE 37A

FIGURE 37B

FIGURE 37C

EP 3 883 584 B1

FIGURE 37D

FIGURE 37E

FIGURE 38A

FIGURE 38B

FIGURE 38C

**FIGURE 38F**

Lactate

Clinical Score

□0 □1-4 □7-12 ■13+
N=8 N=3 N=4 N=6

**FIGURE 38E**

Glycolytic Reserve

Clinical Score

□0 □7-8.5 □10-11.5 □14-15 □16-17
N=6 N=2 N=3 N=3 N=9

**FIGURE 38D**

Glycolytic Function

Time (min)

◆0 ▨7-8.5 ◆10-11.5 ✦14-15 ✦16-17
N=6 N=2 N=3 N=3 N=9

FIGURE 39B

FIGURE 39A

**FIGURE 39C**

**FIGURE 39D**

EP 3 883 584 B1

FIGURE 40A    FIGURE 40B    FIGURE 40C    FIGURE 40D

EP 3 883 584 B1

**FIGURE 40E**

**FIGURE 40F**

**FIGURE 40G**

**FIGURE 40H**

IL-17a

IP-10

VEGF-α

IL-12p70

FIGURE 40I

FIGURE 40J

FIGURE 40K

FIGURE 40L

EP 3 883 584 B1

**ALL Patients, SOFA at Admission**

FIGURE 41A

**ALL Patients, Age Distribution**

FIGURE 41B

**All Patients, Source of Sepsis**

- Pneumonia
- Endovascular (MRSA)
- UTI

FIGURE 41C

| Patient # | Patient Treated With Allocetra | Patient Admission Date | Gender (M/F) | Age (y) | Source of Sepsis | Sofa Score at Admission | Sofa Score Maximal | Baseline (Non-Sepsis) SOFA Score | Sepsis Resolved Within 28 Days | APACHII 2 Score | # of Hospitalization Days in MICU/ICU | # of Hospitalization Days in Medicine | Mortality |
|---|---|---|---|---|---|---|---|---|---|---|---|---|---|
| 1 | Yes | 3.3.2019 | M | 67 | Pneumonia | 6 | 6 | 0 | Yes | 12 | 1 | 5 | No |
| 2 | Yes | 2.4.2019 | M | 52 | Pneumonia | 3 | 3 | 0 | Yes | 9 | 2 | 9 | No |
| 3 | Yes | 6.4.2019 | M | 74 | Pneumonia | 6 | 6 | 2 | Yes | 15 | 2 | 8 | No |
| 4 | Yes | 10.6.2019 | M | 79 | Pneumonia | 3 | 3 | 1 | Yes | 11 | 2 | 5 | No |
| 5 | Yes | 25.6.2019 | M | 65 | MRSA | 6 | 6 | 4 | Yes | 18 | 6 | 22 | No |
| 6 | Yes | 3.7.2019 | M | 82 | UTI | 3 | 3 | 0 | Yes | 9 | 5 | 2 | No |
| 7 | No | 19.6.2016 | M | 72 | Pneumonia | 4 | 16 | 0 | No | 17 | 6 | 3 | Yes |
| 8 | No | 23.4.2016 | M | 67 | Pneumonia | 6 | 14 | 0 | No | 14 | 30 | 3 | Yes |
| 9 | No | 28.1.2016 | M | 63 | Pneumonia | 3 | 10 | 0 | Yes | 8 | 28 | 19 | No |
| 10 | No | 6.2.2016 | M | 64 | Pneumonia | 8 | 14 | 0 | Yes | 27 | 16 | 52 | No |
| 11 | No | 23.9.2016 | M | 65 | Pneumonia | 9 | 13 | 0 | Yes | 30 | 16 | 41 | No |
| 12 | No | 4.5.2017 | M | 67 | Pneumonia | 4 | 9 | 0 | No | 15 | 7 | 29 | Yes |
| 13 | No | 17.1.2017 | M | 64 | Pneumonia | 9 | 10 | 0 | Yes | 17 | 13 | 132 | No |
| 14 | No | 8.2.2016 | M | 54 | Pneumonia | 4 | 11 | 0 | No | 8 | 23 | 13 | Yes |
| 15 | No | 16.1.2016 | M | 57 | Pneumonia | 1 | 8 | 0 | Yes | 11 | 17 | 48 | No |
| 16 | No | 7.5.2016 | M | 54 | Pneumonia | 3 | 3 | 0 | Yes | 7 | 5 | 12 | No |
| 17 | No | 21.7.2017 | M | 53 | Pneumonia | 3 | 7 | 0 | Yes | 12 | 11 | 11 | No |
| 18 | No | 9.6.2017 | M | 53 | Pneumonia | 3 | 7 | 2 | Yes | 12 | 8 | 4 | No |
| 19 | No | 31.8.2017 | M | 55 | Pneumonia | 3 | 5 | 0 | Yes | 12 | 2 | 30 | No |
| 20 | No | 13.10.2017 | M | 55 | Pneumonia | 5 | 11 | 0 | No | 22 | 6 | 0 | No |
| 21 | No | 9.3.2016 | M | 69 | Pneumonia | 5 | 13 | 0 | No | 14 | 12 | 85 | No |
| 22 | No | 1.1.2017 | M | 70 | Pneumonia | 7 | 8 | 0 | No | 18 | 2 | 5 | No |
| 23 | No | 3.9.2016 | M | 76 | Pneumonia | 1 | 13 | 0 | No | 11 | 15 | 16 | Yes |
| 24 | No | 17.11.2016 | M | 75 | Pneumonia | 2 | 4 | 0 | Yes | 6 | 7 | 12 | No |
| 25 | No | 2.3.2016 | M | 78 | Pneumonia | 3 | 9 | 0 | No | 13 | 15 | 3 | Yes |
| 26 | No | 19.8.2016 | M | 80 | Pneumonia | 2 | 6 | 0 | Yes | 11 | 5 | 9 | No |
| 27 | No | 18.11.2016 | M | 79 | Pneumonia | 5 | 9 | 0 | Yes | 10 | 9 | 8 | No |
| 28 | No | 9.12.2016 | M | 79 | Pneumonia | 5 | 5 | 0 | Yes | 16 | 2 | 3 | No |
| 29 | No | 3.3.2016 | M | 78 | Pneumonia | 4 | 17 | 1 | No | 12 | 7 | 0 | Yes |
| 30 | No | 5.5.2016 | M | 79 | Pneumonia | 3 | 9 | 0 | No | 12 | 6 | 16 | Yes |
| 31 | No | 7.1.2017 | M | 74 | Pneumonia | 3 | 9 | 0 | No | 12 | 10 | 0 | Yes |
| 32 | No | 19.06.2019 | M | 67 | MRSA | 3 | 3 | 1 | Yes | 19 | 0 | 20 | No |
| 33 | No | 05.05.2019 | M | 65 | MRSA | 4 | 6 | 4 | No | 15 | 8 | 40 | No |
| 34 | No | 14.1.2018 | M | 89 | MRSA | 4 | 6 | 1 | No | 19 | 0 | 8 | No |
| 35 | No | 25.9.2016 | M | 66 | MRSA | 6 | 10 | 4 | No | 21 | 22 | 80 | Yes |
| 36 | No | 11.6.2017 | M | 62 | MRSA | 4 | 10 | 4 | No | 25 | 14 | 6 | Yes |
| 37 | No | 13.10.2016 | M | 60 | MRSA | 2 | 2 | 0 | No | 14 | 0 | 4 | No |
| 38 | No | 21.06.2016 | M | 81 | UTI | 2 | 6 | 0 | Yes | 11 | 14 | 0 | No |
| 39 | No | 8.10.2016 | M | 78 | UTI | 3 | 3 | 2 | Yes | 9 | 0 | 3 | No |
| 40 | No | 5.2.2017 | M | 84 | UTI | 3 | 4 | 0 | Yes | 12 | 0 | 3 | No |
| 41 | No | 8.3.2017 | M | 78 | UTI | 5 | 5 | 2 | Yes | 19 | 0 | 6 | No |
| 42 | No | 13.7.2017 | M | 84 | UTI | 4 | 4 | 0 | No | 6 | 0 | 2 | No |
| 43 | No | 13.8.2016 | M | 86 | UTI | 2 | 2 | 0 | No | 6 | 0 | 2 | No |

Figure 42

All Patient Mortalities Within Matched Controls Group As Function Of
SOFA at Admission

Figure 43

ALL Subjects, % Patients Complete Recovery
From Sepsis Within 28 Days

Figure 44A

Excluding UTI Subjects, % Patients Complete
Recovery From Sepsis Within 28 Days

Figure 44B

Figure 45

Figure 46A

Figure 46B

ALL Patients, % Still In ICU Post Admission

Figure 47A

ALL Excluding UTI Patients, % Still In ICU Post Admission

Figure 47B

ALL Subjects, Avg Baseline SOFA & Maximum SOFA

Figure 48A

Figure 48B

Figure 48C

Figure 48D

**ALL Subjects, % Patients With SOFA Increase From Time Of Admission**

Figure 49A

**% Patients With SOFA Increase From Time Of Admission, Excluding UTI Patients**

Figure 49B

**% Mortality of Patients With SOFA Increase >= 4**

Figure 50A

Figure 50B

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087408 A
- US 20130156794 A
- US 4036945 A, Goldenberg
- US 4331647 A
- US 4946778 A, Ladner
- US 4816567 A
- US 5545807 A
- US 5545806 A
- US 5569825 A
- US 5625126 A
- US 5633425 A
- US 5661016 A
- US 20150275175 A1
- US 6489311 B

**Non-patent literature cited in the description**

- **ST. CLAIR EW**. The calm after the cytokine storm: Lessons from the TGN1412 trial. *J Clin Invest*, 2008, vol. 118, 1344-1347
- **VENKATESH, B.** ; **FINFER, S.** ; **COHEN, J.** ; **RAJBHANDARI, D.** ; **ARABI, Y.** ; **BELLOMO, R.** ; **BILLOT, L.** ; **CORREA, M.** ; **GLASS, P.** ; **HARWARD, M. et al.** Adjunctive Glucocorticoid Therapy in Patients with Septic Shock.. *N. Engl. J. Med.*, 2018, vol. 378, 797-808
- **FINFER, S.** ; **BELLOMO, R.** ; **LIPMAN, J.** ; **FRENCH, C.** ; **DOBB, G.** ; **MYBURGH, J.** Adult-population incidence of severe sepsis in Australian and New Zealand intensive care units. *Intensive Care Med.*, 2004, vol. 30, 589-596
- **FLEISCHMANN, C.** ; **SCHERAG, A.** ; **ADHIKARI, N.K.J.** ; **HARTOG, C.S.** ; **TSAGANOS, T.** ; **SCHLATTMANN, P.** ; **ANGUS, D.C.** ; **REINHART, K.** Assessment of Global Incidence and Mortality of Hospital-treated Sepsis. Current Estimates and Limitations.. *Am. J. Respir. Crit. Care Med.*, 2016, vol. 193, 259-272
- **LIU, V.** ; **ESCOBAR, G.J.** ; **GREENE, J.D.** ; **SOULE, J.** ; **WHIPPY, A.** ; **ANGUS, D.C.** ; **IWASHYNA, T.J.** Hospital Deaths in Patients With Sepsis From 2 Independent Cohorts. *JAMA*, 2014, vol. 312, 90
- **MACHADO, F.R.** ; **CAVALCANTI, A.B.** ; **BOZZA, F.A.** ; **FERREIRA, E.M.** ; **ANGOTTI CARRARA, F.S.** ; **SOUSA, J.L.** ; **CAIXETA, N.** ; **SALOMAO, R.,** **ANGUS, D.C.** ; **PONTES AZEVEDO, L.C. et al.** The epidemiology of sepsis in Brazilian intensive care units (the Sepsis PREvalence Assessment Database, SPREAD): an observational study.. *Lancet Infect. Dis.*, 2017, vol. 17, 1180-1189
- **REINHART, K.** ; **DANIELS, R.** ; **KISSOON, N.** ; **MACHADO, F.R.** ; **SCHACHTER, R.D.** ; **FINFER, S.** Recognizing Sepsis as a Global Health Priority - A WHO Resolution.. *N. Engl. J. Med.*, 2017, vol. 377, 414-417
- **RHEE, C.** ; **DANTES, R.** ; **EPSTEIN, L.** ; **MURPHY, D.J.** ; **SEYMOUR, C.W.** ; **IWASHYNA, T.J.** ; **KADRI, S.S.** ; **ANGUS, D.C.** ; **DANNER, R.L.** ; **FIORE, A.E. et al.** Incidence and Trends of Sepsis in US Hospitals Using Clinical vs Claims Data, 2009-2014.. *JAMA*, 2017, vol. 318, 1241
- **HOTSHKISS, R.S.** ; **MOLDAWER, L.L.** ; **OPAL, S.M.** ; **REINHART, K.** ; **TURNBULL, I.R.** ; **VINCENT, J.-L.** Sepsis and septic shock.. *Nat. Rev. Dis. Prim.*, 2016, vol. 2, 16045
- **TANG, D.** ; **KANG, R.** ; **COYNE, C.B.** ; **ZEH, H.J.** ; **LOTZE, M.T.** PAMPs and DAMPs: signal 0s that spur autophagy and immunity.. *Immunol. Rev.*, 2012, vol. 249, 158-175
- **CHAUDHRY, H.** ; **ZHOU, J.** ; **ZHONG, Y.** ; **ALI, M.M.** ; **MCGUIRE, F.** ; **NAGARKATTI, P.S.** ; **NAGARKATTI, M.** Role of cytokines as a double-edged sword in sepsis.. *In Vivo*, 2015, vol. 27, 669-684
- **MATSUMOTO, H.** ; **OGURA, H.** ; **SHIMIZU, K.** ; **IKEDA, M.** ; **HIROSE, T.** ; **MATSUURA, H.** ; **KANG, S.** ; **TAKAHASHI, K.** ; **TANAKA, T.** ; **SHIMAZU, T.** The clinical importance of a cytokine network in the acute phase of sepsis. *Sci. Rep.*, 2018, vol. 8, 1-4
- **MARSHALL, J.C.** ; **COOK, D.J.** ; **CHRISTOU, N. V** ; **BERNARD, G.R.** ; **SPRUNG, C.L.** ; **SIBBALD, W.J.** Multiple organ dysfunction score: a reliable descriptor of a complex clinical outcome. *Crit. Care Med.*, 1995, vol. 23, 1638-16525
- **VINCENT, J.-L.** Organ Dysfunction in Patients with Severe Sepsis.. *Surg. Infect. (Larchmt).*, 2006, vol. 7

- **TRAHTEMBERG, U.** ; **MEVORACH, D.** Apoptotic cells induced signaling for immune homeostasis in macrophages and dendritic cells. *Front. Immunol*, 2017, vol. 8
- Remington's Pharmaceutical Sciences. Mack Publishing Co.
- REMINGTON'S PHARMACEUTICAL SCIENCE. May 1985
- **HARLOW** ; **LANE**. Antibodies: A Laboratory Manual. Cold Spring Harbor Laboratory, 1988
- **PORTER, R. R.** *Biochem. J.*, 1959, vol. 73, 119-126
- **INBAR et al.** *Proc. Nat'l Acad. Sci. USA*, 1972, vol. 69, 2659-62
- **WHITLOW** ; **FILPULA**. *Methods*, 1991, vol. 2, 97-105
- **BIRD et al.** *Science*, 1988, vol. 242, 423-426
- **PACK et al.** *Bio/Technology*, 1993, vol. 11, 1271-77
- **LARRICK** ; **FRY**. *Methods*, 1991, vol. 2, 106-10
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **RIECHMANN et al.** *Nature*, 1988, vol. 332, 323-327
- **VERHOEYEN et al.** *Science*, 1988, vol. 239, 1534-1536
- **HOOGENBOOM** ; **WINTER**. *J. Mol. Biol.*, 1991, vol. 227, 381
- **MARKS et al.** *Mol. Biol.*, 1991, vol. 222, 581
- **COLE et al.** Monoclonal Antibodies and Cancer Therapy, Alan R.. *Liss*, 1985, 77
- **BOERNER et al.** *J. Immunol.*, 1991, vol. 147 (1), 86-95
- **MARKS et al.** *Bio/Technology*, 1992, vol. 10, 779-783
- **LONBERG et al.** *Nature*, 1994, vol. 368, 856-859
- **MORRISON**. *Nature*, 1994, vol. 368, 812-13
- **FISHWILD et al.** *Nature Biotechnology*, 1996, vol. 14, 845-51
- **NEUBERGER**. *Nature Biotechnology*, 1996, vol. 14, 826
- **LONBERG** ; **HUSZAR**. *Intern. Rev. Immunol.*, 1995, vol. 13, 65-93
- **LEE DW et al.** *Blood*, 2014, vol. 124 (2), 188-195
- **MEVORACH et al.** *Biology of Blood and Marrow Transplantation*, 2014, vol. 20 (1), 58-65
- **MEVORACH et al.** *Biology of Blood and Marrow Transplantation*, 2015, vol. 21 (2), S339-S340
- Single Infusion of Donor Mononuclear Early Apoptotic Cells as Prophylaxis for Graft-versus-Host Disease in Myeloablative HLA-Matched Allogeneic Bone Marrow Transplantation: A Phase I/IIa Clinical Trial.. *BBMT*, vol. 20 (1), 58-65
- **ROWLINGS et al.** IBMTR Severity Index for grading acute graft-versus-host disease: retrospective comparison with Glucksberg grade. *Br J Haematol*, June 1997, vol. 97 (4), 855-64
- *Blood*, 1996, vol. 8, 3230-3239
- **VERBOVETSKY et al.** *J Exp Med*, 2002
- **KRISPIN et al.** *Blood*, 2006
- **MEVORACH et al.** *BBMT*, 2014
- **MEVORACH, D.** ; **ZUCKERMAN, T.** ; **REINER, I.** ; **SHIMONI, A.** ; **SAMUEL, S.** ; **NAGLER, A.** ; **ROWE, J.M.** ; **OR, R**. Single infusion of donor mononuclear early apoptotic cells as prophylaxis for graft-versus-host disease in myeloablative HLA-matched allogeneic bone marrow transplantation: a phase I/IIa clinical trial. *Biol. Blood Marrow Transplant.*, 2014, vol. 20, 58-65
- **SHRUM, B.** ; **ANANTHA, R. V.** ; **XU, S.X.** ; **DONNELLY, M.** ; **HAERYFAR, S.M.M.** ; **MCCORMICK, J.K.** ; **MELE, T.** A robust scoring system to evaluate sepsis severity in an animal model.. *BMC Res. Notes*, 2014, vol. 7, 1-11
- **COLETTA, C.** ; **MÓDIS, K.** ; **OLÁH, G.** ; **BRUNYÁNSZKI, A.** ; **HERZIG, D.S.** ; **SHERWOOD, E.R.** ; **UNGVÁRI, Z.** ; **SZABO, C.** Endothelial dysfunction is a potential contributor to multiple organ failure and mortality in aged mice subjected to septic shock: preclinical studies in a murine model of cecal ligation and puncture.. *Crit. Care*, 2014, vol. 18, 511
- **DRECHSLER, S.** ; **WEIXELBAUMER, K.M.** ; **WEIDINGER, A.** ; **RAEVEN, P.** ; **KHADEM, A.** ; **REDL, H.** ; **VAN GRIENSVEN, M.** ; **BAHRAMI, S.** ; **REMICK, D., KOZLOV, A. et al.** Why do they die? Comparison of selected aspects of organ injury and dysfunction in mice surviving and dying in acute abdominal sepsis. *Intensive Care Med*, 2015, vol. 3, 48
- **OSTERBUR, K.** ; **MANN, F.A.** ; **KUROKI, K.** ; **DECLUE, A.** Multiple organ dysfunction syndrome in humans and animals.. *J. Vet. Intern. Med.*, 2014, vol. 28, 1141-1151
- **RUIZ, S.** ; **VARDON-BOUNES, F.** ; **MERLET-DUPUY, V.** ; **CONIL, J.-M.** ; **BULÉON, M.** ; **FOURCADE, O.** ; **TACK, I.** ; **MINVILLE, V.** Sepsis modeling in mice: ligation length is a major severity factor in cecal ligation and puncture.. *Intensive Care Med. Exp.*, 2016, vol. 4, 22
- **SEEMANN, S.** ; **ZOHLES, F.** ; **LUPP, A**. Comprehensive comparison of three different animal models for systemic inflammation.. *J. Biomed. Sci.*, 2017, vol. 24
- **LEVY et al.** The Surviving Sepsis Campaign Bundle: 2018 Update.. *Crit Care Med.*, 2018, vol. 46 (6), 997-1000

- **RHODES et al.** Surviving Sepsis Campaign: International Guidelines for Management of Sepsis and Septic Shock: 2016. *Intensive Care Med.,* March 2017, vol. 43 (3), 304-377